(19) Europäisches Patentamt
European Patent Office
Office européen des brevets

(11) EP 2 535 416 A1

(12) **EUROPEAN PATENT APPLICATION**

(43) Date of publication:
**19.12.2012 Bulletin 2012/51**

(51) Int Cl.:
***C12N 15/82*** *(2006.01)*

(21) Application number: **12168070.6**

(22) Date of filing: **15.05.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.05.2011 EP 11167250**

(83) **Declaration under Rule 32(1) EPC (expert solution)**

(71) Applicant: **BASF Plant Science Company GmbH**
**67056 Ludwigshafen (DE)**

(72) Inventors:
- **Schultheiß, Holger**
  **67459 Böhl-Iggelheim (DE)**
- **Böhme, Timo**
  **67063 Ludwigshafen (DE)**

(74) Representative: **Patzelt, Andrea**
**BASF SE**
**GVX/B - C 6**
**Carl-Bosch-Straße 38**
**67056 Ludwigshafen (DE)**

(54) **Development of phytophthora resistant potato with increased yield**

(57) The present invention relates to transgenic potato plants having an increased resistance against *Phytophthora infestans* and a comparable yield of potato tubers compared with the wildtype potato plants, wherein the blb1-gen and blb2-gen are integrated within a specific genetic background into the potato plant.

EP 2 535 416 A1

## Description

The present invention relates to transgenic potato plants having an increased resistance against Phytophthora infestans and a comparable yield of potato tubers compared with the wildtype potato plants, wherein the blb1-gene and blb2-gene are integrated within a specific genetic background into the potato plant.

Late blight caused by the oomycete Phytophthora infestans is one of the most severe threats to potato production worldwide. Despite many years of resistance breeding, the only effective way to prevent crop failures or reduced yields is the application of fungicides that prevent or cure an infection by P. infestans. As the disease development of late blight is extremely fast, it is necessary to run a tight fungicide regime, which has to start before first symptoms occur. Furthermore, P. infestans seems to have a high potential to adapt to specific fungicides and to develop resistance, as already seen in the case of metalaxyl-fungicides (Gisi U, Cohen Y (1996) Resistance to phenylamide fungicides: A case study with Phytophthora infestans involving mating type and race structure Annual Rev Phytopathol. 34: 549-572).

In several Western European countries, legislation on the use of plant protection products is becoming more restrictive regarding the application of specific fungicides, making chemical control of the disease and the prevention of resistance development more difficult.

An alternative and/or complementary approach to the use of fungicides is the development of potato cultivars that harbour improved resistance to P. infestans.

In recent years, two potato varieties containing S. bulbocastanum derived resistance were developed via conventional breeding. Both varieties, Toluca and Bionica, contain a single S. bulbocastanum resistance gene that confers full resistance against P. infestans. But from an agronomical point of view the two potato varieties do not match modern potato varieties in terms of yield potential.

As the introgression of the S. bulbocastanum derived resistance into modern potato varieties turned out to be difficult and time consuming, a much more efficient approach is the isolation of the genes that code for Phytophthora resistance in S. bulbocastanum and their transfer into current potato cultivars by biotechnological methods.

To generate the durably resistant potato plants, the Rpi-blb1 and the Rpi-blb2 genes were combined under control of their native regulation elements. The resultant vector construct contained the genomic sequence of the Rpi-blb1 gene under control of the native Rpi-blb1 promoter and Rpi-blb1 terminator, all derived from S. bulbocastanum, in combination with the genomic sequence of the Rpi-blb2 gene under control of the native Rpi-blb2 promoter and Rpi-blb2 terminator all from S. bulbocastanum (WO 2008/034876). The obtained transgenic potatoes expressing blb1-protein and blb2-protein showed increased resistance to Phytophthora infestans. However, it was found that the yield of the developed potato plants was decreased.

It is an object of the present invention to provide potato plants having an improved resistance to Phytophthora infestans and a comparable yield with the wildtype potato.

The present invention may be understood more readily by reference to the following detailed description of the preferred embodiments of the invention and the examples included herein. Unless otherwise noted, the terms used herein are to be understood according to conventional usage by those of ordinary skill in the relevant art. In addition to the definitions of terms provided herein, definitions of common terms in molecular biology may also be found in Rieger et al., 1991 Glossary of genetics: classical and molecular, 5th Ed., Berlin: Springer-Verlag; and in Current Protocols in Molecular Biology, F.M. Ausubel et al., Eds., Current Protocols, a joint venture between Greene Publishing Associates, Inc. and John Wiley & Sons, Inc., (1998 Supplement). It is to be understood that as used in the specification and in the claims, "a" or "an" can mean one or more, depending upon the context in which it is used. Thus, for example, reference to "a cell" can mean that at least one cell can be utilized. It is to be understood that the terminology used herein is for the purpose of describing specific embodiments only and is not intended to be limiting.

Throughout this application, various publications are referenced. The disclosures of all of these publications and those references cited within those publications in their entireties are hereby incorporated by reference into this application in order to more fully describe the state of the art to which this invention pertains. Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al., 1989 Molecular Cloning, Second Edition, Cold Spring Harbor Laboratory, Plainview, N.Y.; Maniatis et al., 1982 Molecular Cloning, Cold Spring Harbor Laboratory, Plainview, N.Y.; Wu (Ed.) 1993 Meth. Enzymol. 218, Part I; Wu (Ed.) 1979 Meth Enzymol. 68; Wu et al., (Eds.) 1983 Meth. Enzymol. 100 and 101; Grossman and Moldave (Eds.) 1980 Meth. Enzymol. 65; Miller (Ed.) 1972 Experiments in Molecular Genetics, Cold Spring Harbor Laboratory, Cold Spring Harbor, N.Y.; Old and Primrose, 1981 Principles of Gene Manipulation, University of California Press, Berkeley; Schleif and Wensink, 1982 Practical Methods in Molecular Biology; Glover (Ed.) 1985 DNA Cloning Vol. I and II, IRL Press, Oxford, UK; Hames and Higgins (Eds.) 1985 Nucleic Acid Hybridization, IRL Press, Oxford, UK; and Setlow and Hollaender 1979 Genetic Engineering: Principles and Methods, Vols. 1-4, Plenum Press, New York. Abbreviations and nomenclature, where employed, are deemed standard in the field and commonly used in professional journals such as those cited herein.

The object of the present invention is solved by the provision of Phytophthora-resistant-transgenic potato plant, seed, tuber, plant cell or tissue thereof having a specific integration site for the blb1-gene and blb2-gene.

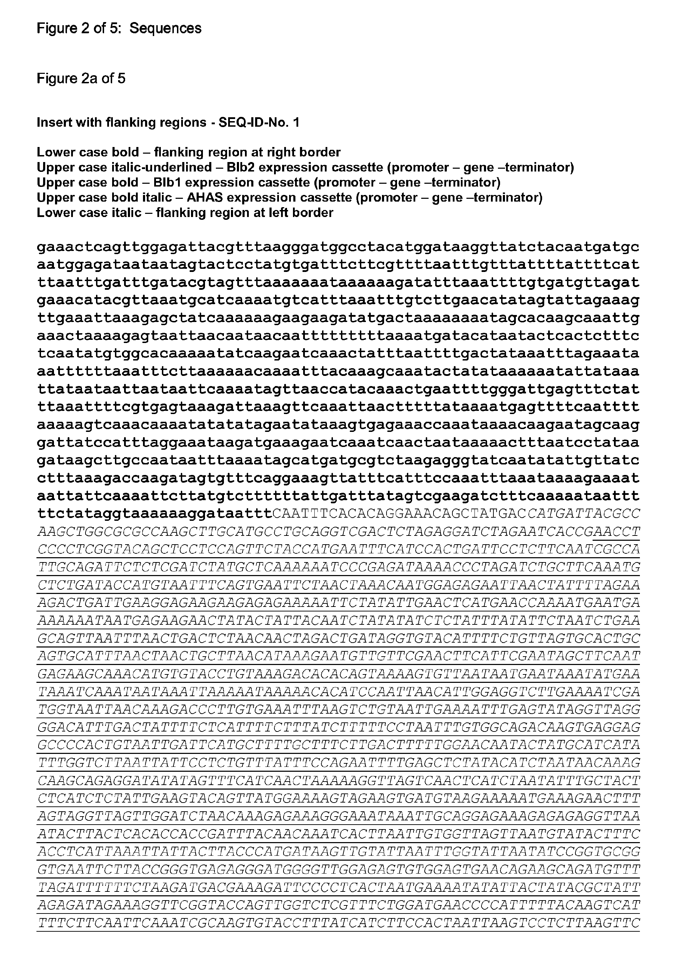

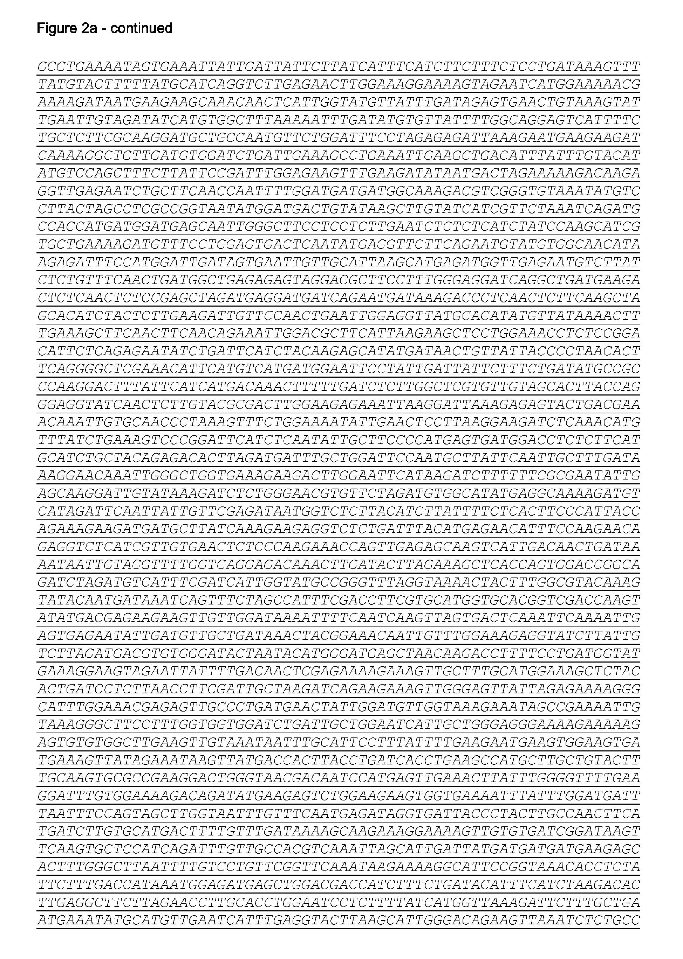

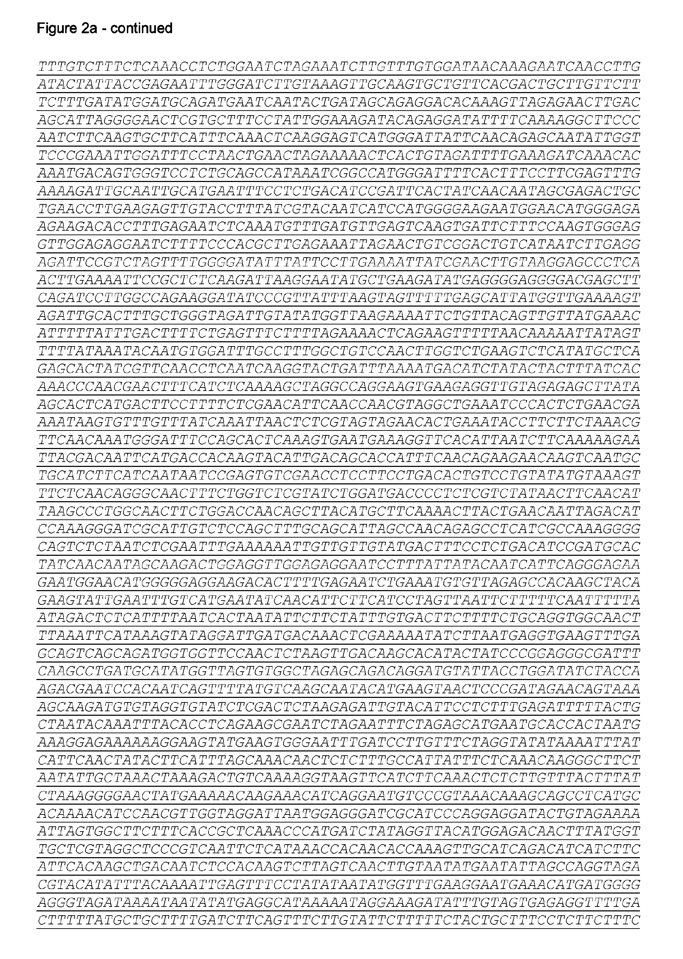

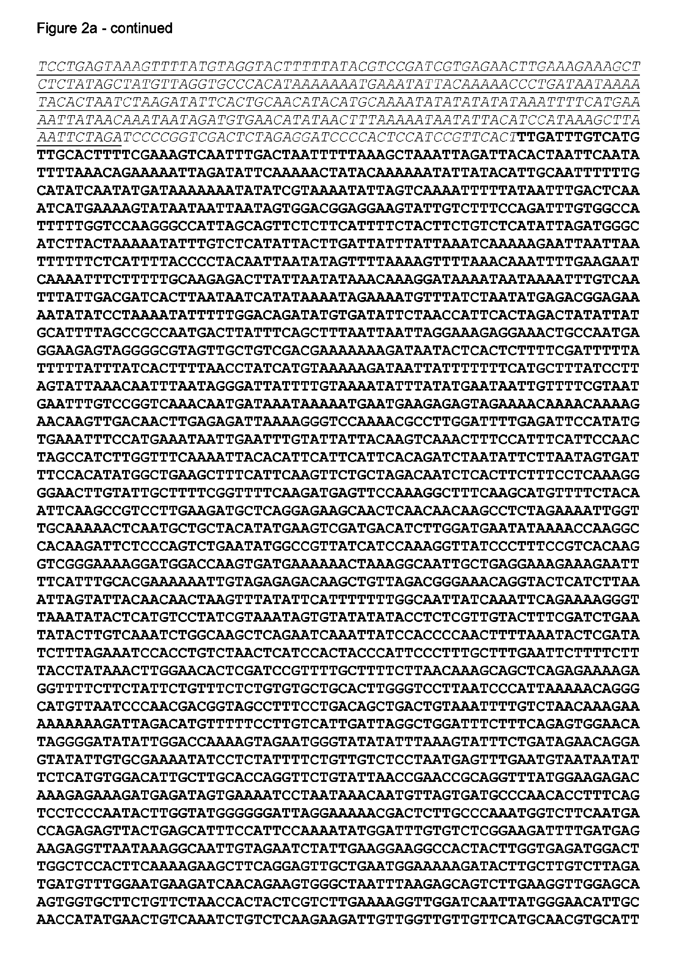

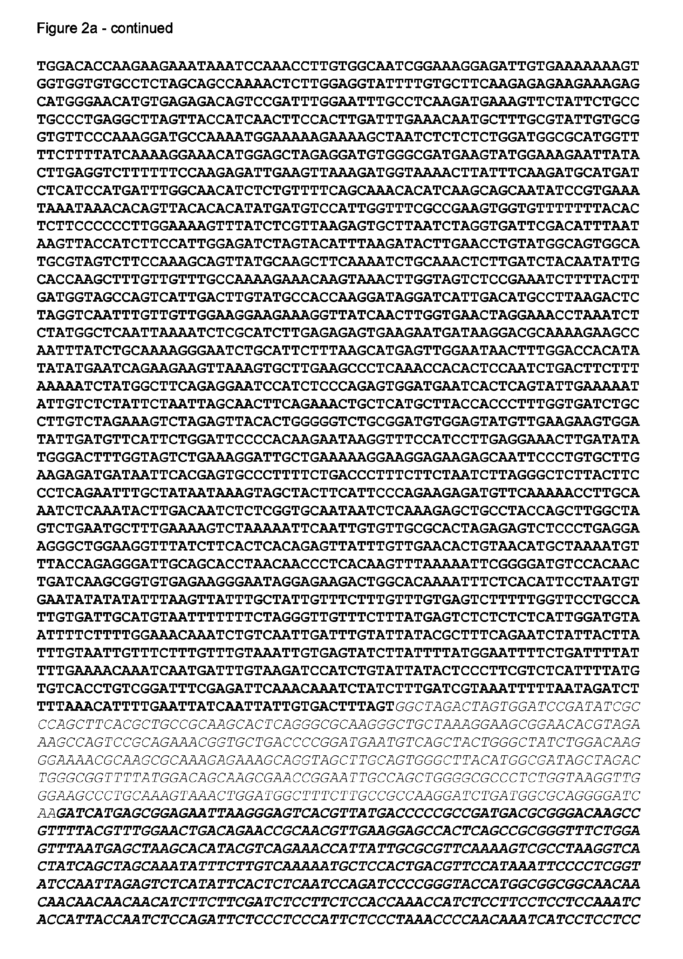

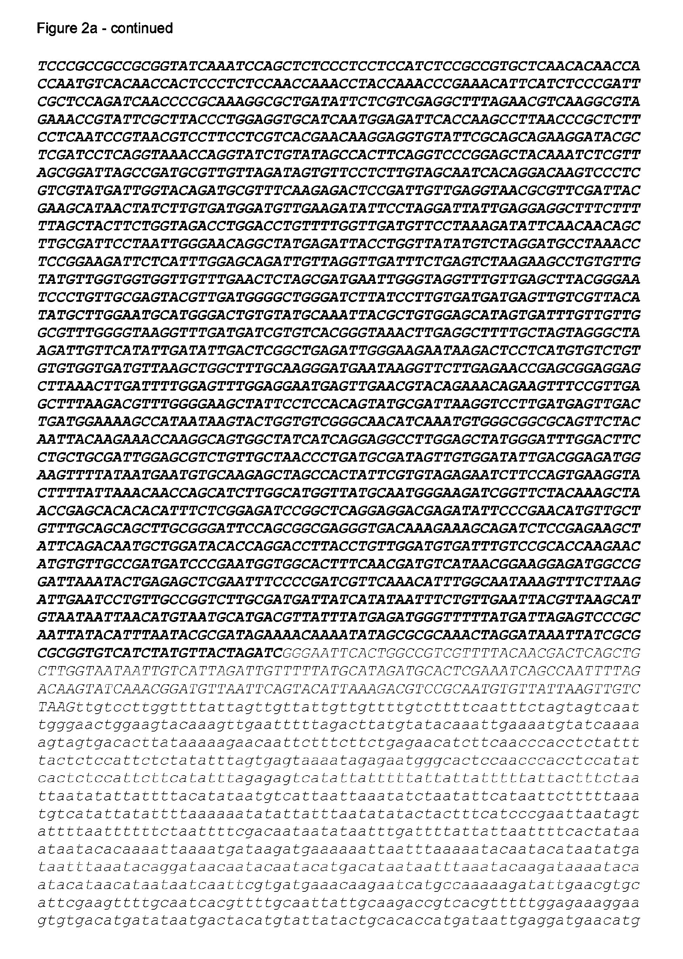

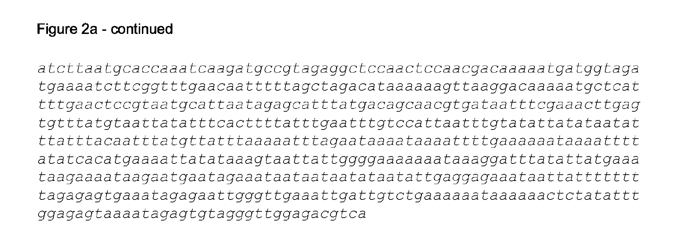

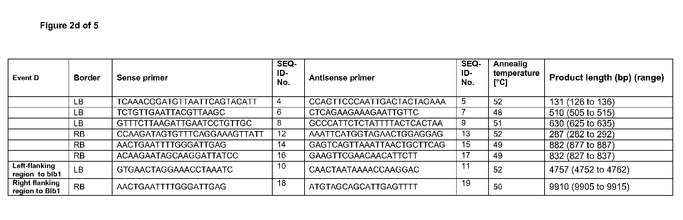

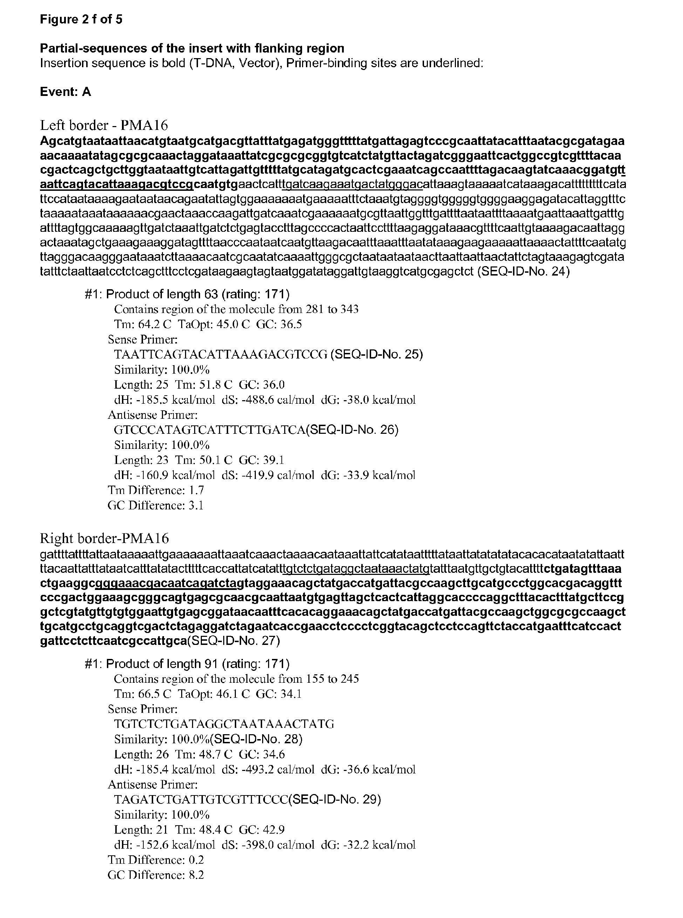

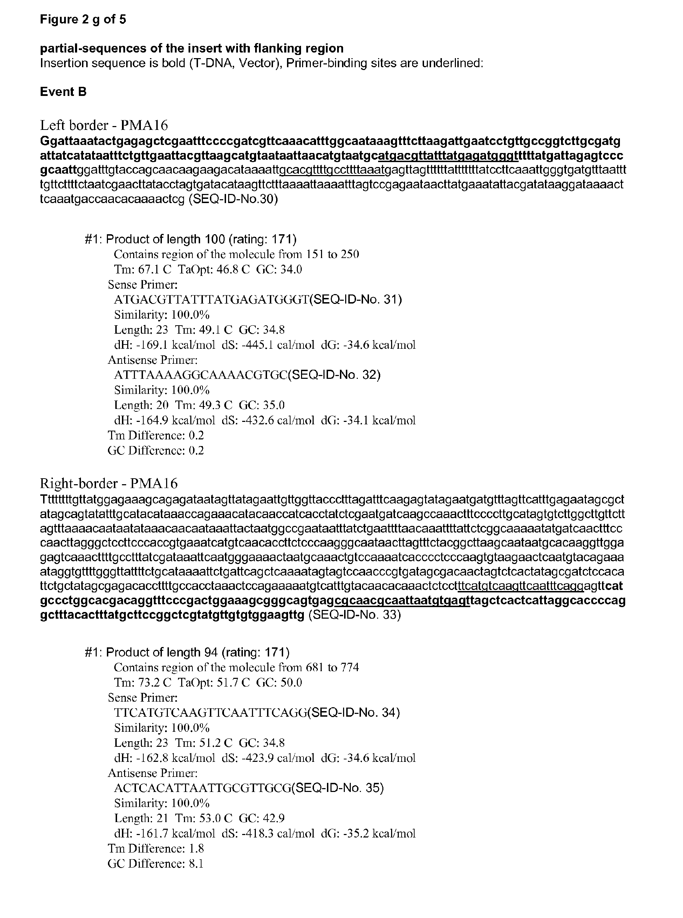

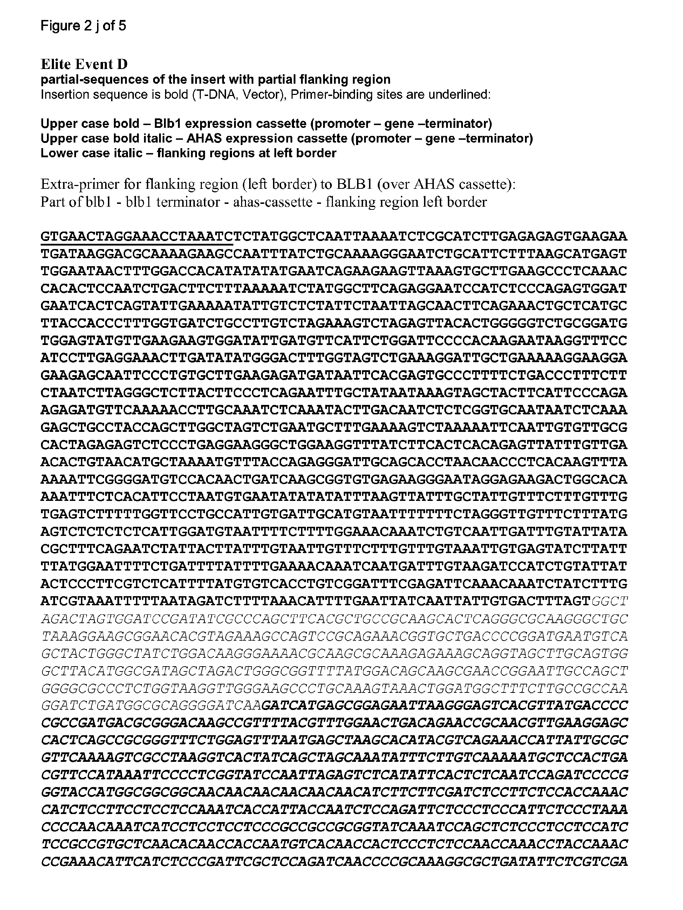

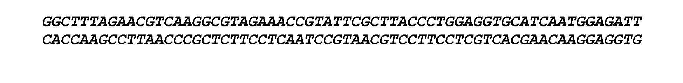

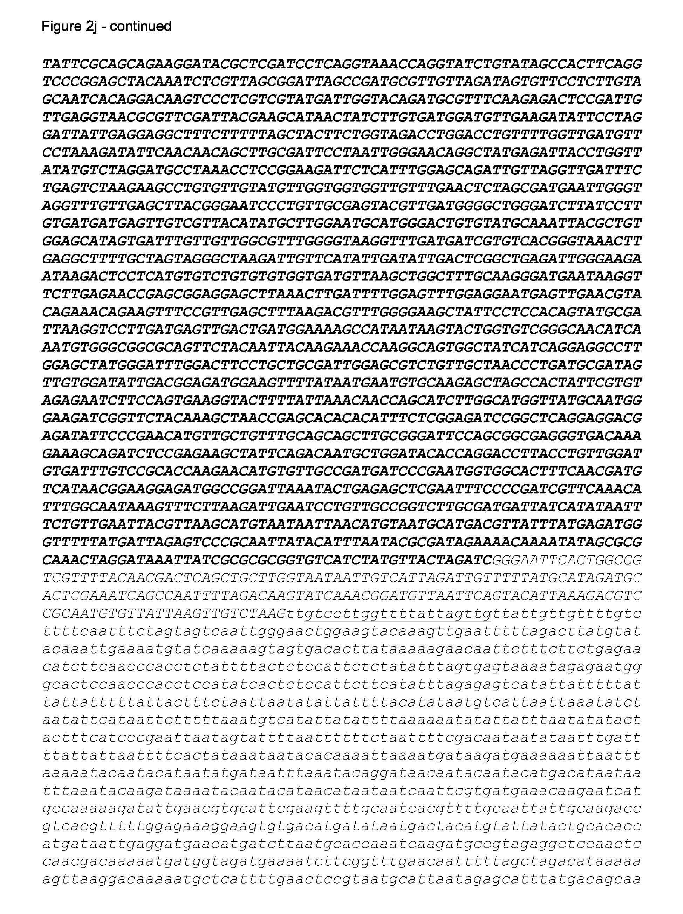

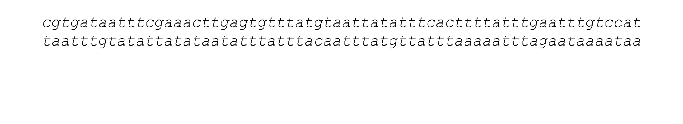

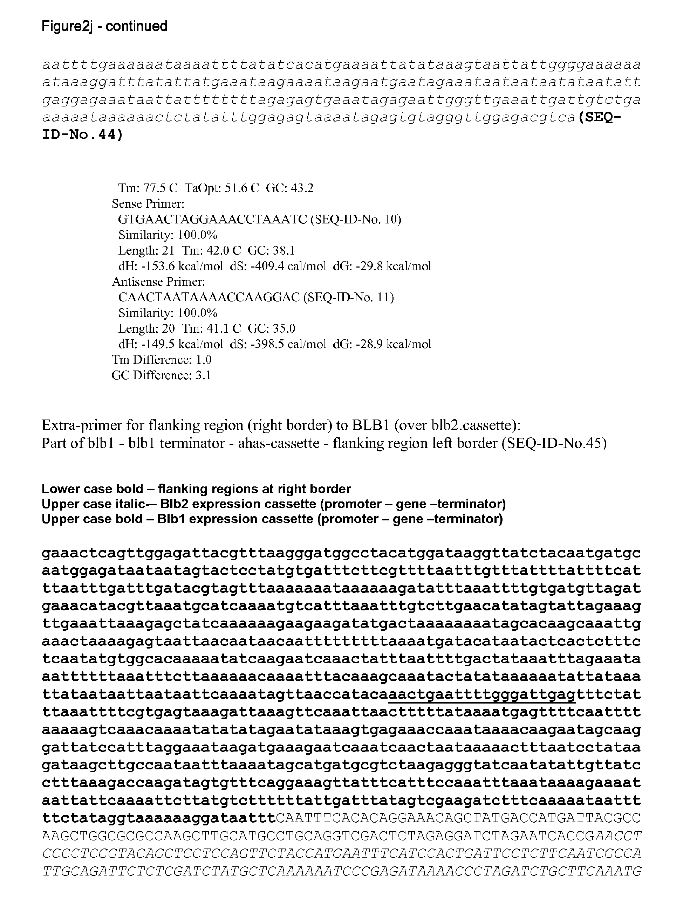

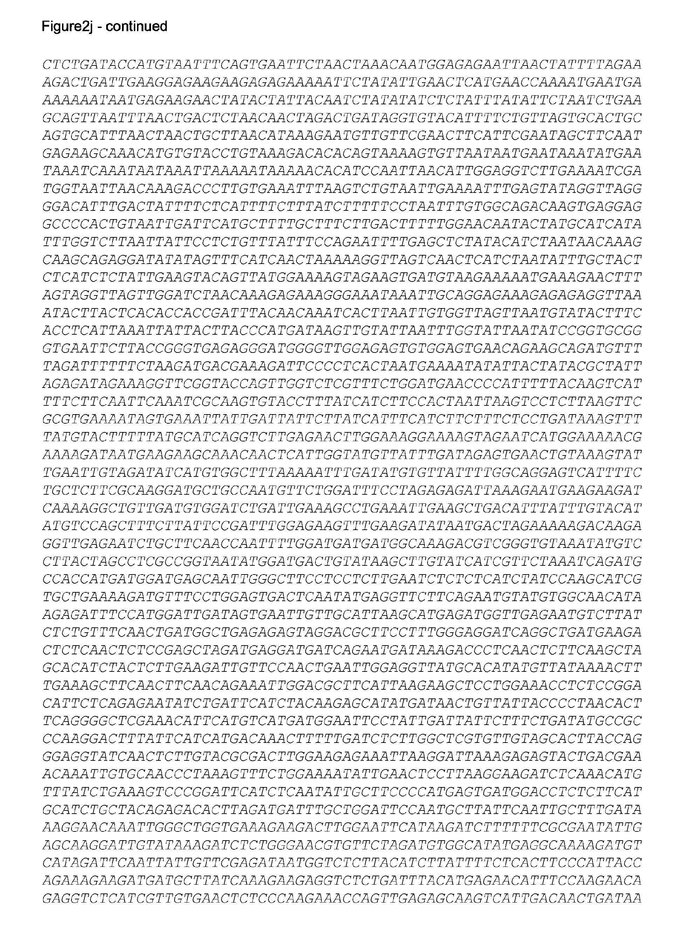

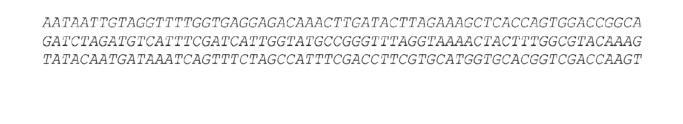

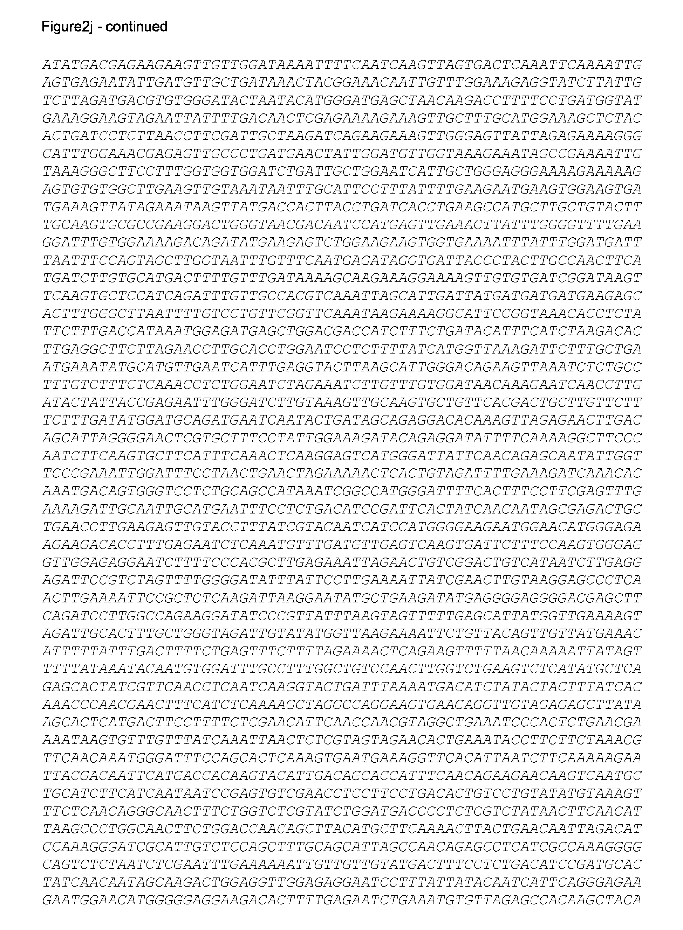

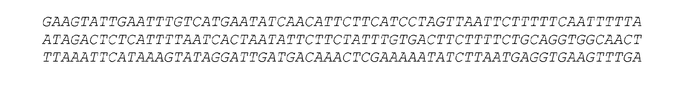

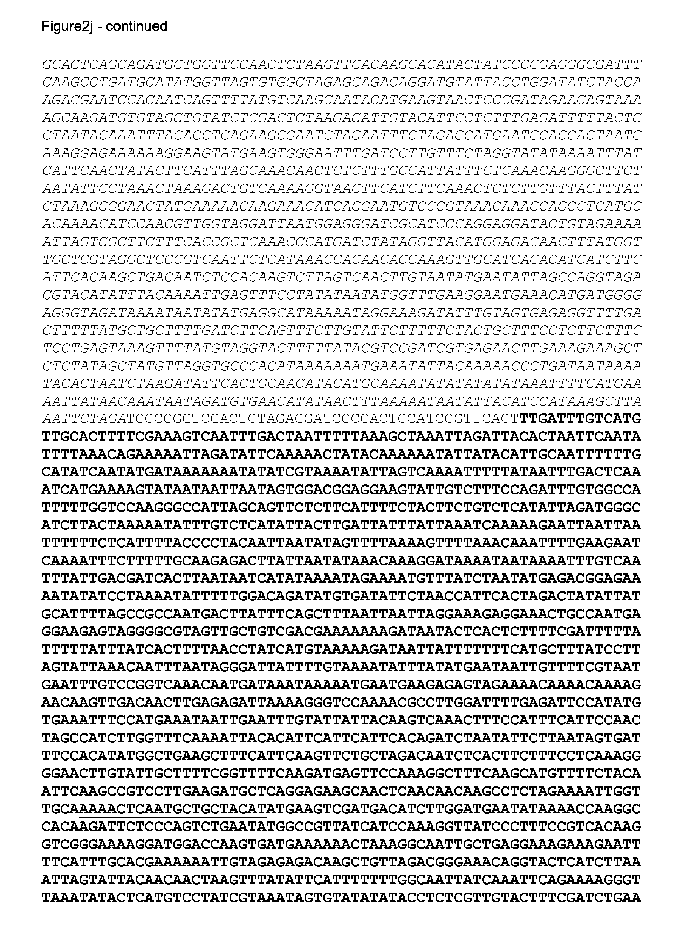

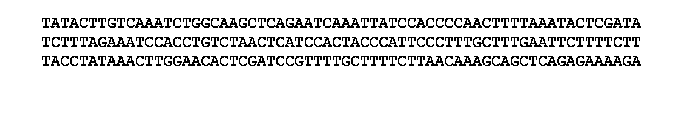

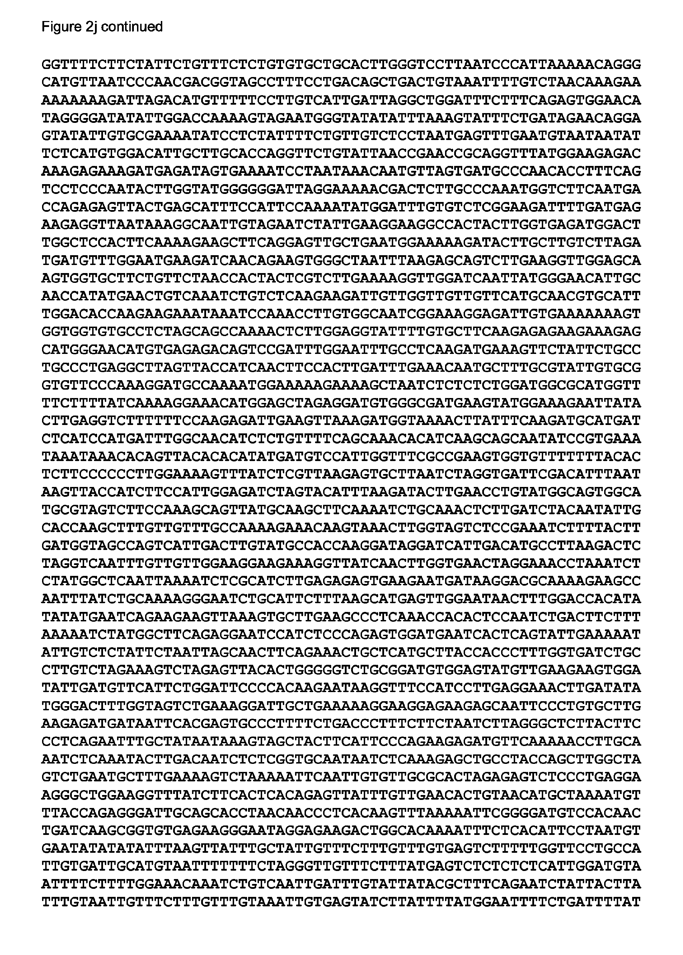

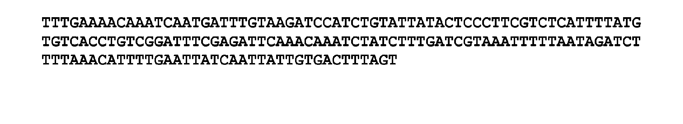

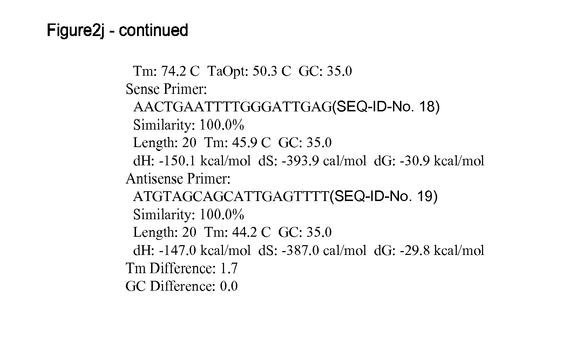

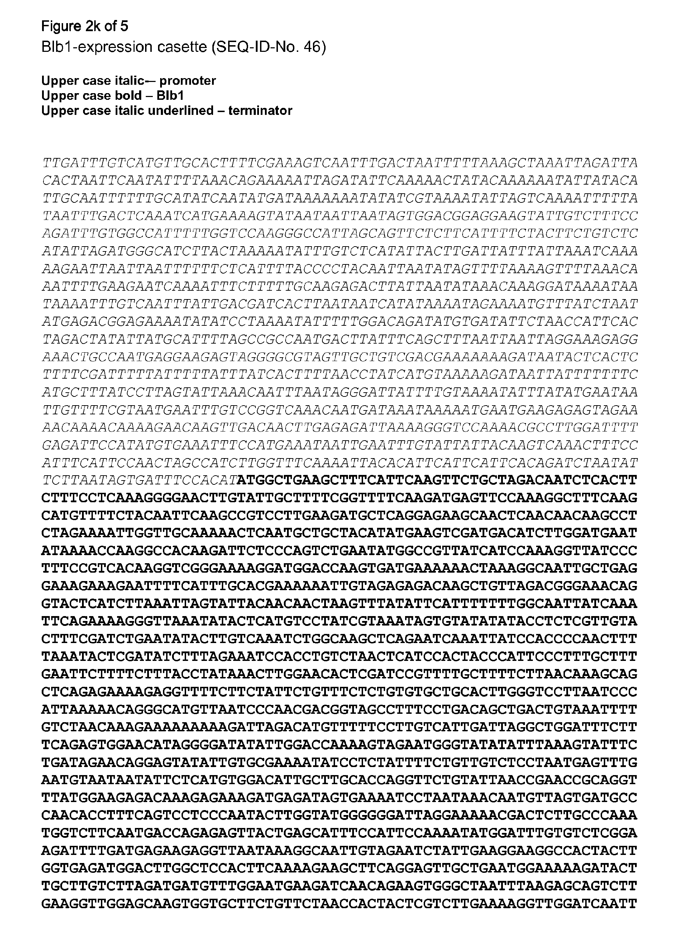

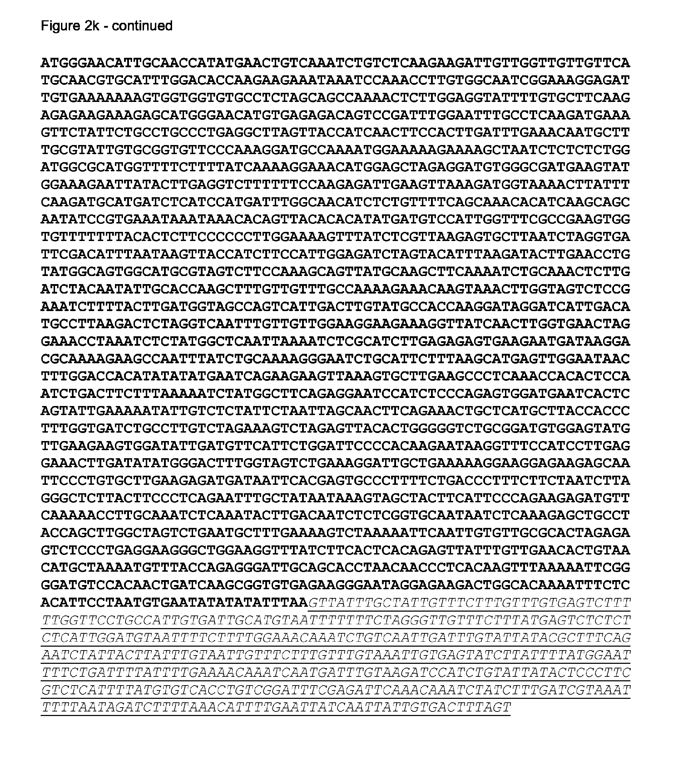

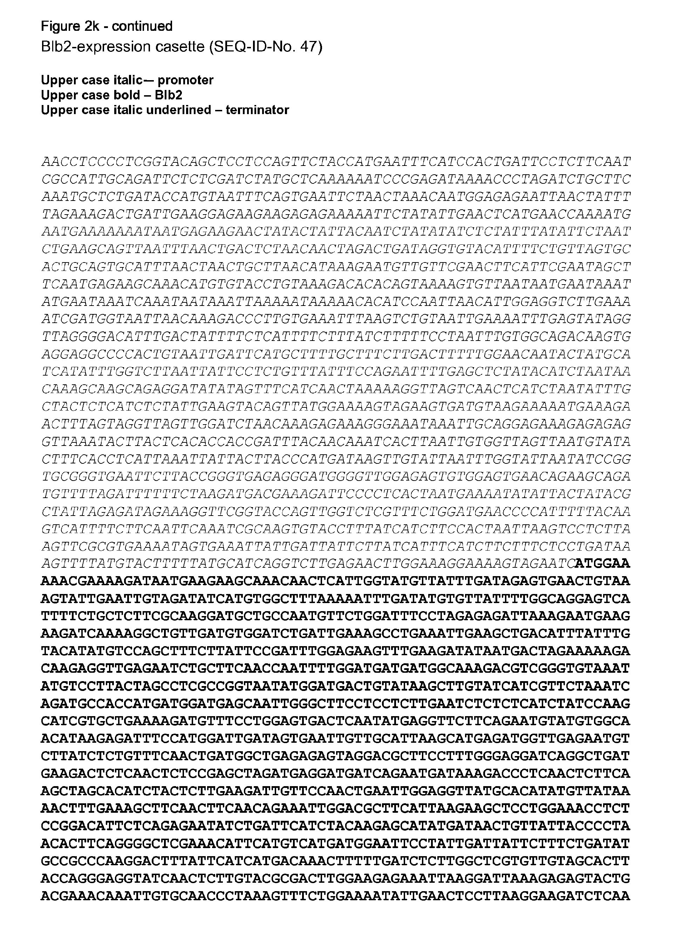

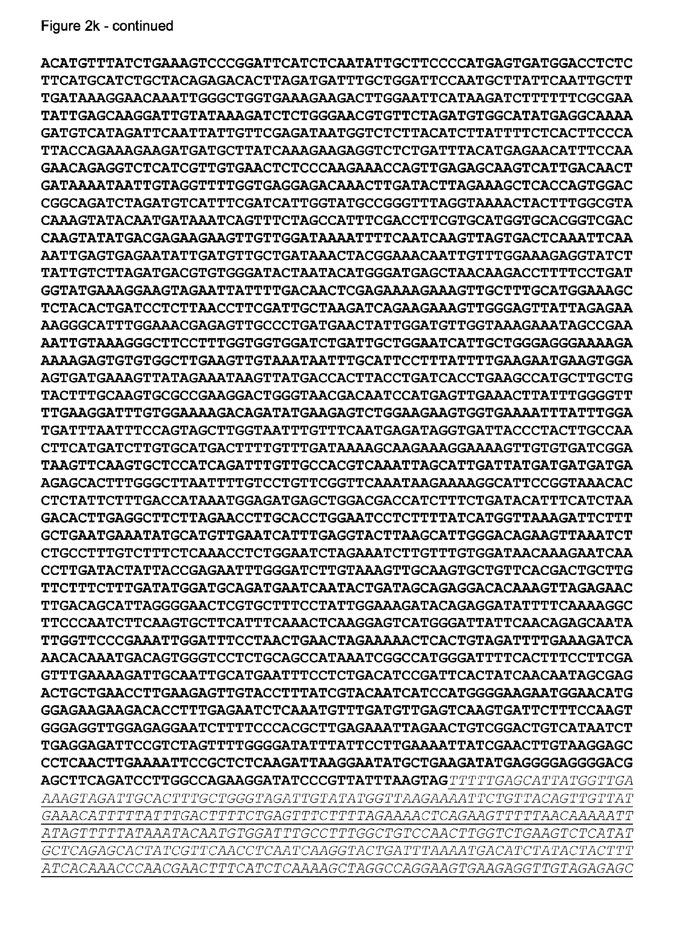

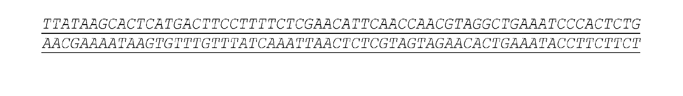

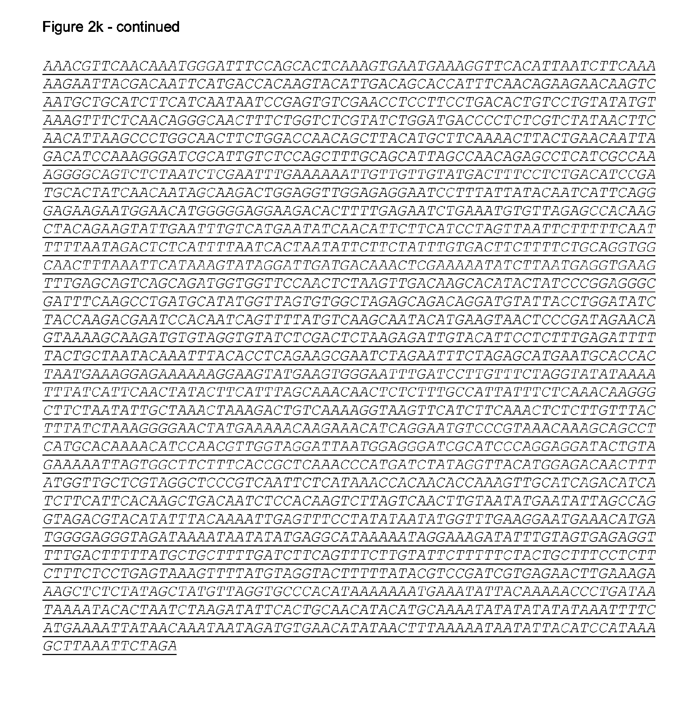

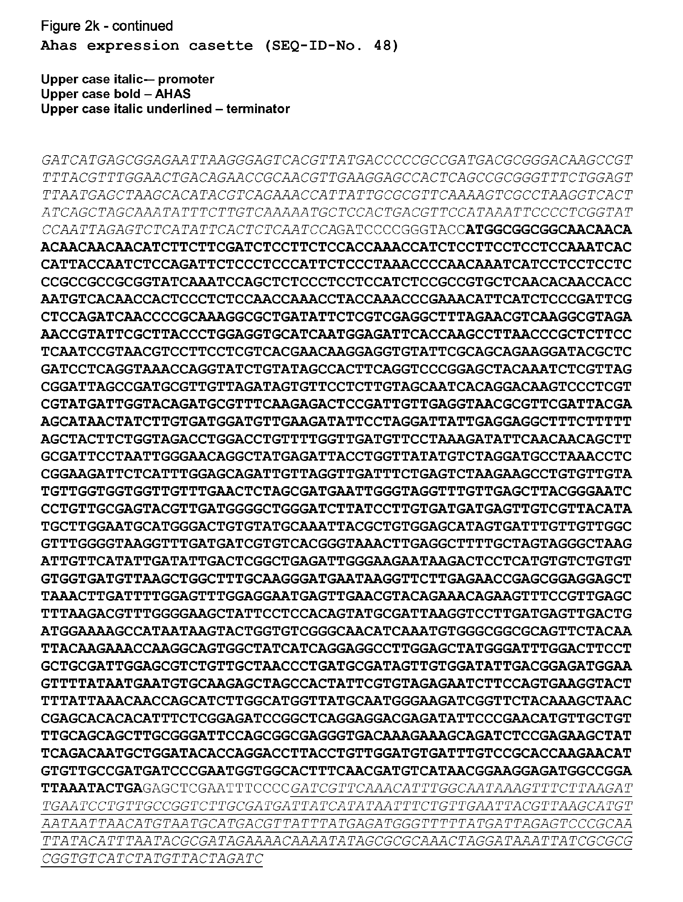

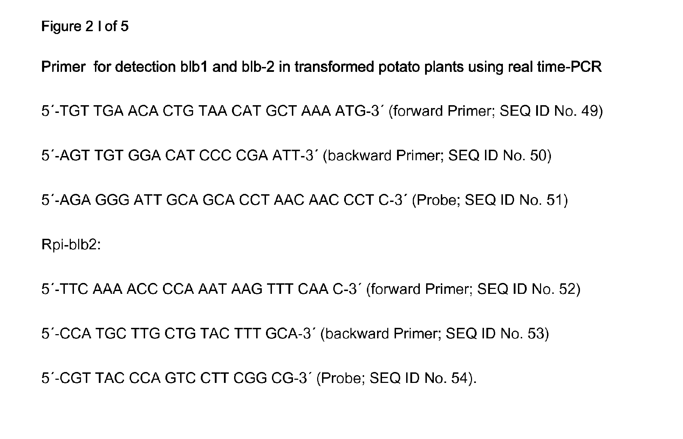

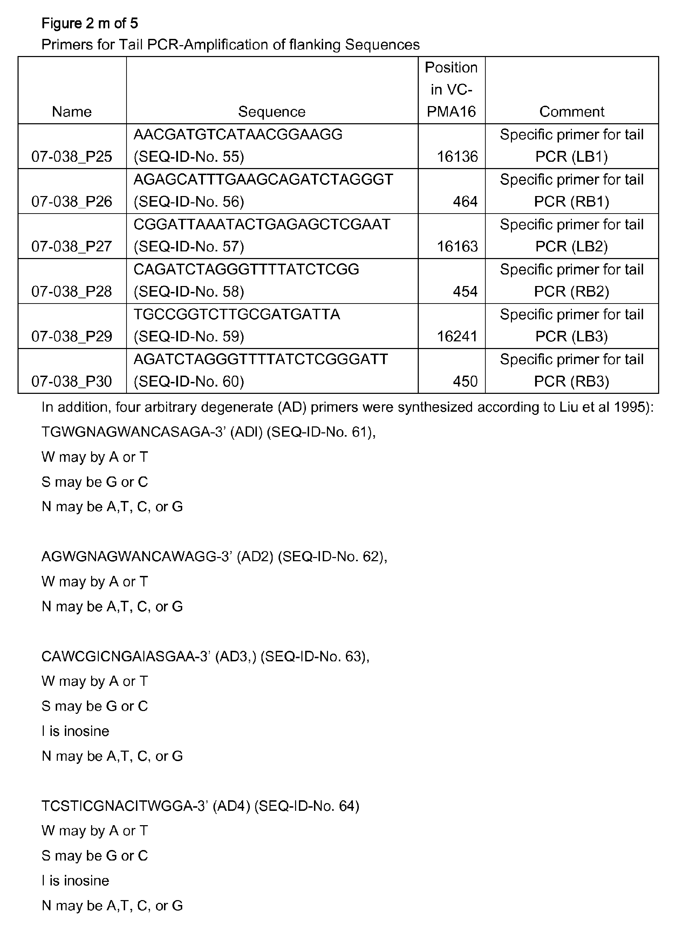

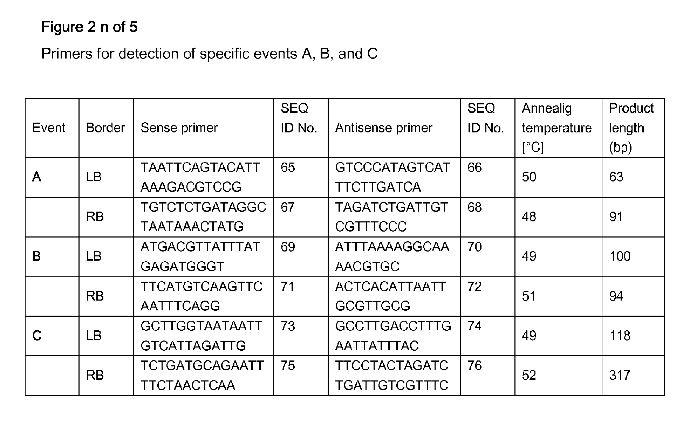

One embodiment according to the present invention provides a Phytophthora-resistant transgenic potato plant, seed, tuber, plant cell or tissue, preferably comprising a nucleotide sequence having at least 80 % identity with SEQ-ID-No. 1 (cf. Figure 2a).

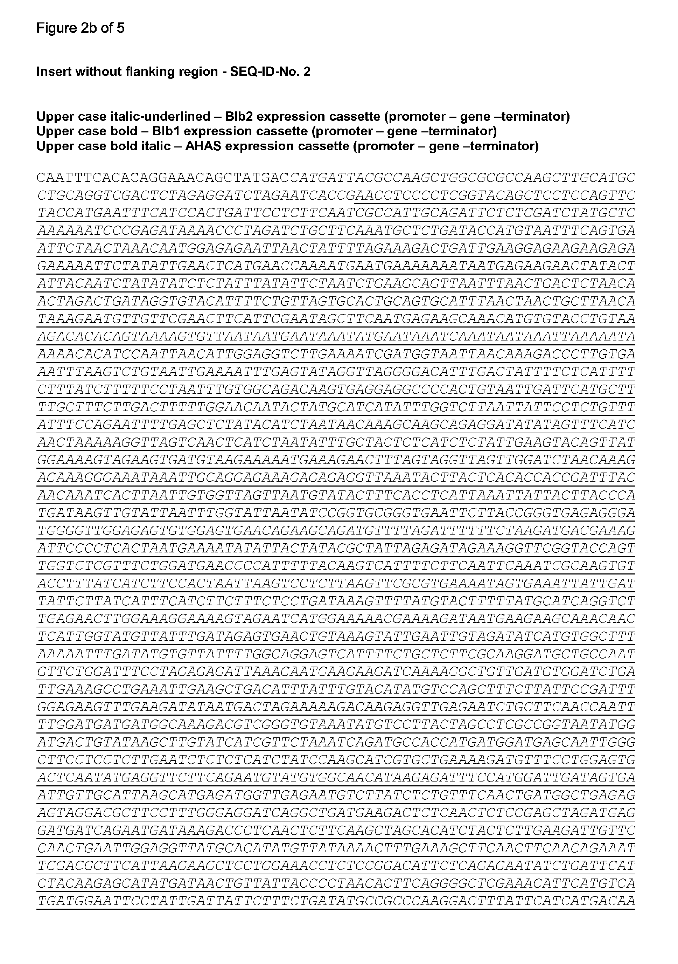

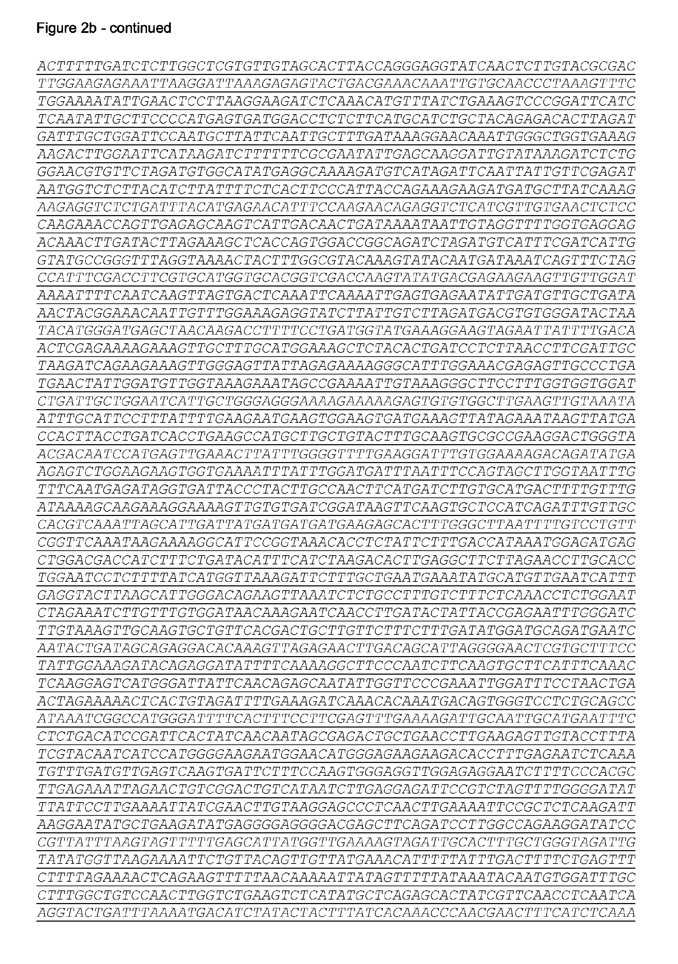

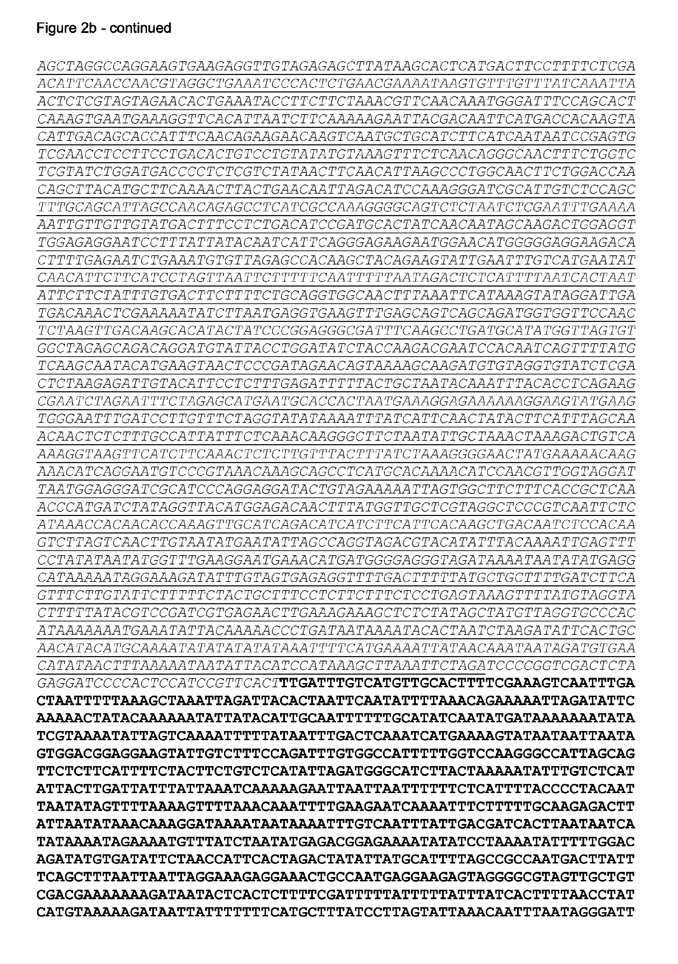

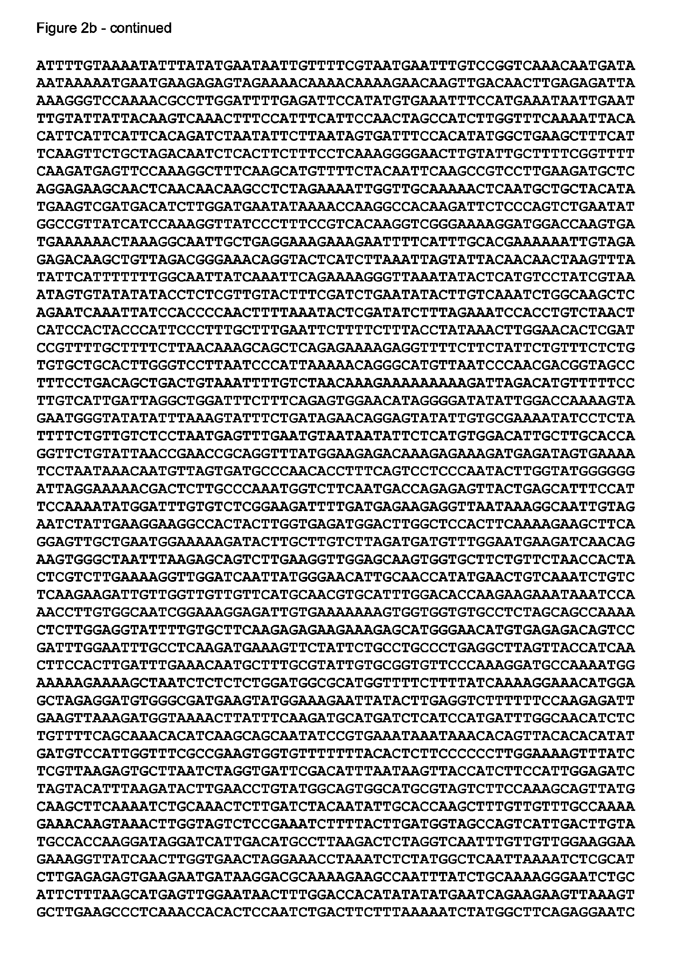

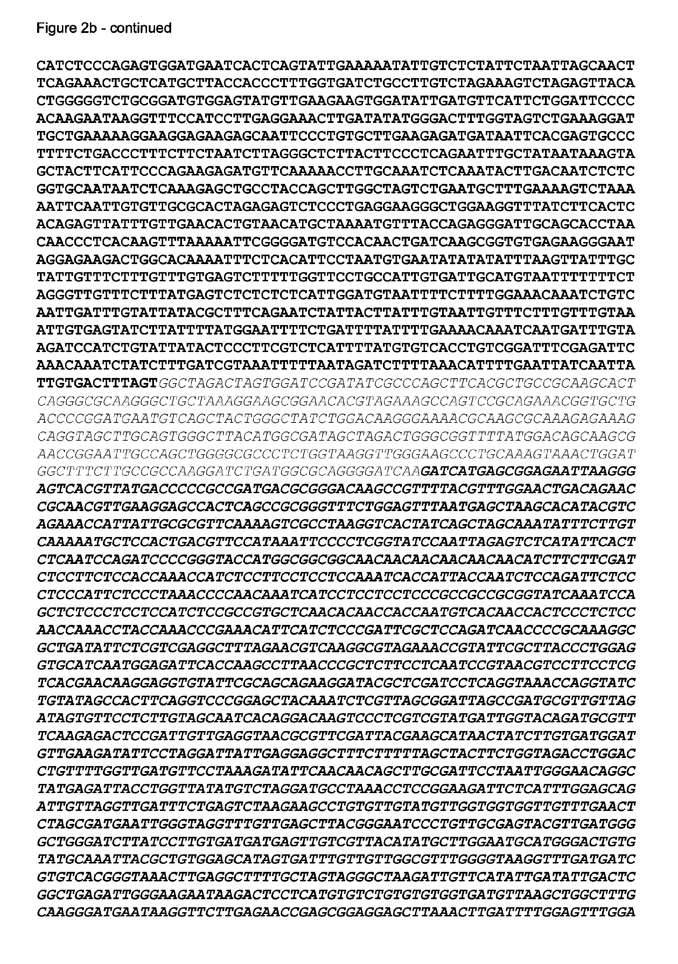

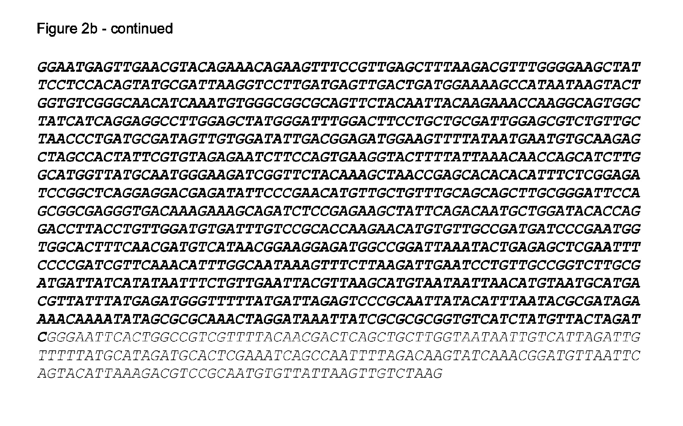

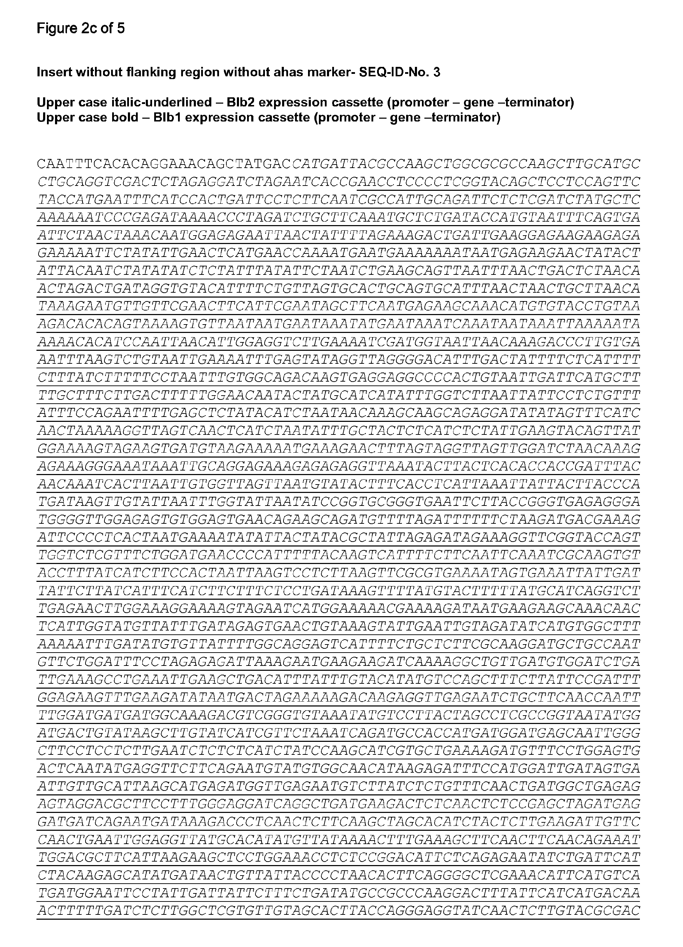

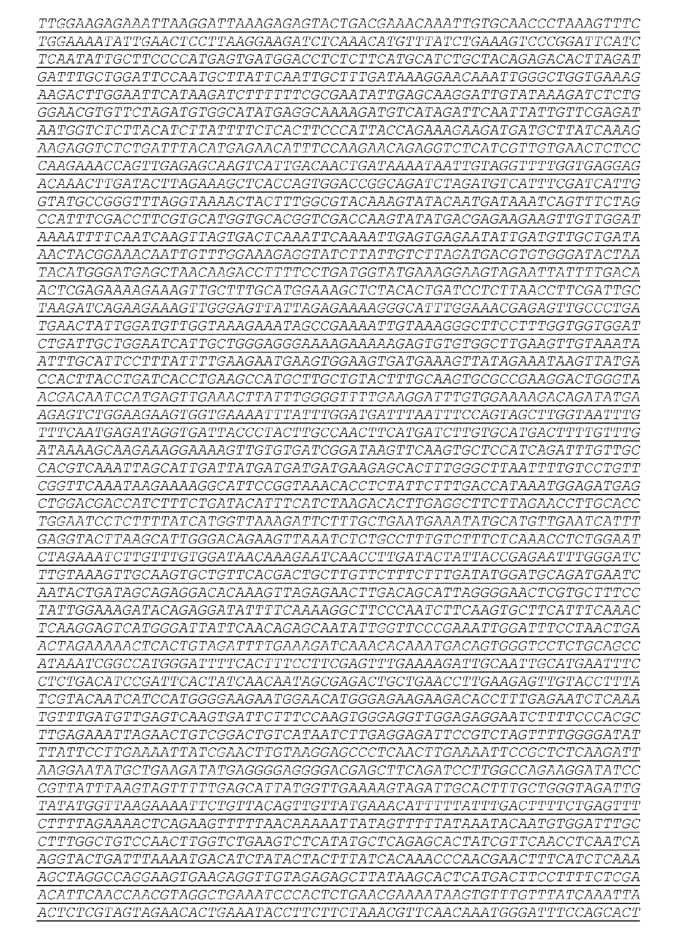

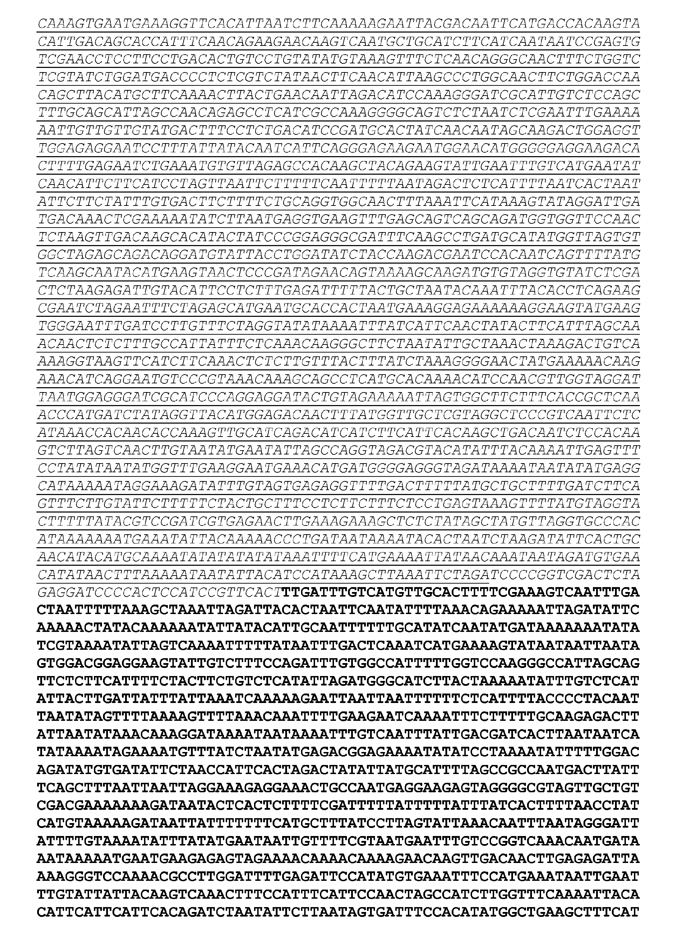

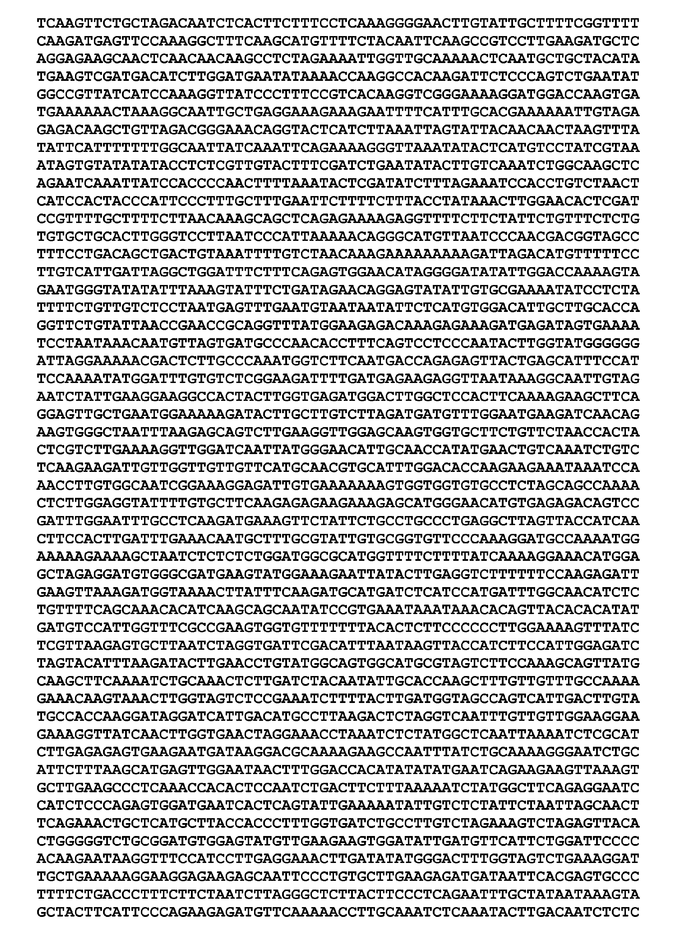

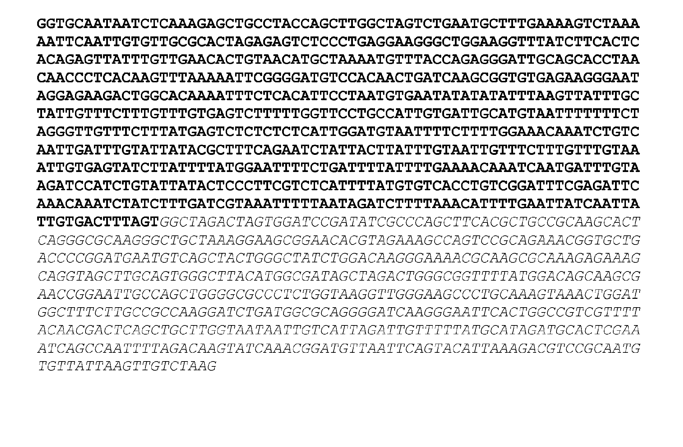

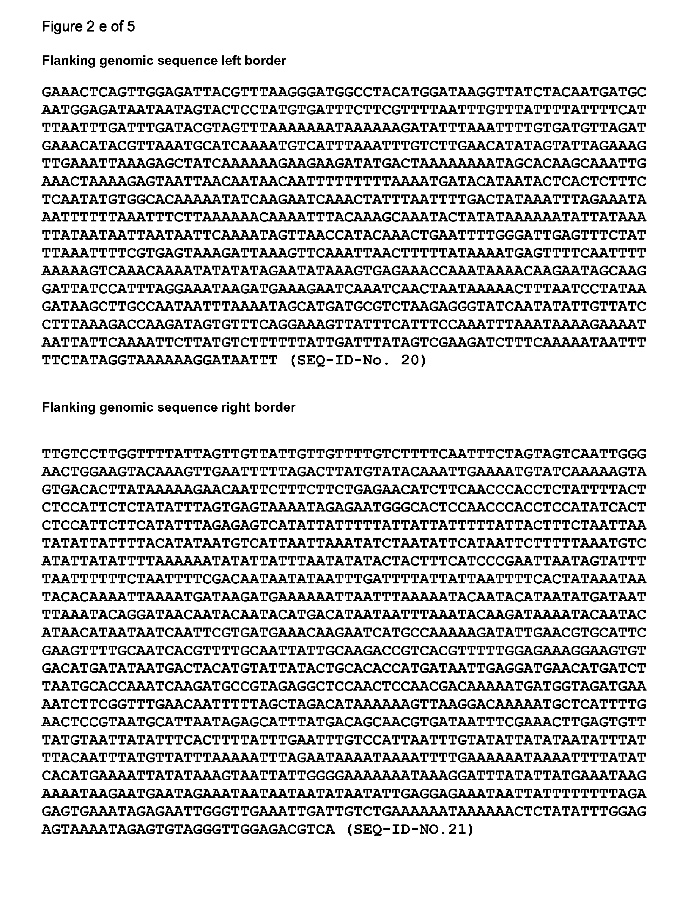

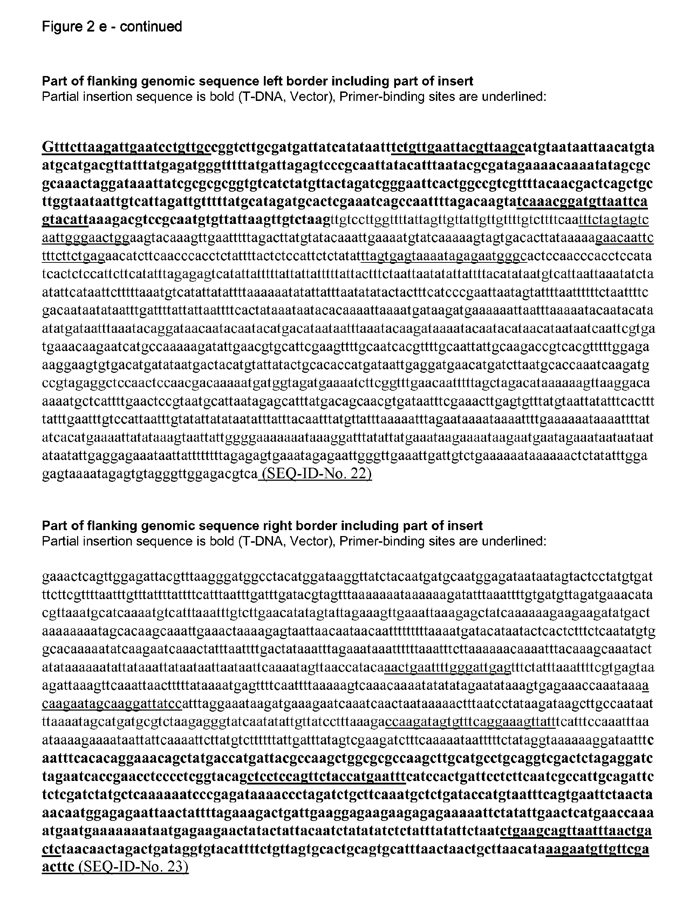

One embodiment according to the present invention provides a Phytophthora-resistant transgenic potato plant, seed, tuber, plant cell or tissue comprising a nucleotide sequence having at least 80 % identity with SEQ-ID-No. 2 or SEQ-ID-No. 3 flanked by flanking regions having at least 80% identity with SEQ-ID-No. 20 and/or SEQ-ID-No. 21 (cf. Figs 2b,c ,e).

SEQ-ID-No. 1 refers to the part of the recombinant construct inserted into the plant genome including blb1-gene, blb2-gene and ahas-gene, wherein SEQ-ID-No. 1 further includes the flanking genomic sequences of the plant (cf. Fig.2a).

SEQ-ID-No.2 refers to the part of the recombinant construct inserted into the plant genome including blb1-gene, blb2-gene and the ahas-gene. Preferably, blb1-gene, blb2-gene and optionally the ahas-gene are expressed, if a sequence having at least 80 % identity to SEQ-ID-No. 2 is inserted into the plant genome. Preferably, blb1-gene, blb2-gene and or ahas-gene are expressed, if a sequence having at least 80 % identity to SEQ-ID-No. 2 is inserted into the plant genome (cf. Fig. 2b).

SEQ-ID-No. 3 refers to the part of the recombinant construct inserted into the plant genome including blb1-gene, blb2-gene but without the ahas-marker-gene. Preferably, blb1-gene, blb2-gene are expressed, if a sequence having at least 80 % identity to SEQ-ID-No. 3 is inserted into the plant genome (cf. Fig. 2c).

SEQ-ID-Nos. 2 and/or 3 may be referred to herein later as insert.

SEQ-ID-No. 20 refers to the left flanking region of the insert having SEQ-ID-Nos. 2 or 3.

SEQ-ID-No. 21 refers to the right flanking region of the insert having SEQ-ID-Nos. 2 or 3.

The term "flanking region" refers the region of the plant genome flanking either the right or left site of the insert which is integrated into the plant genome.

One embodiment according to the present invention provides a Phytophthora-resistant transgenic potato plant, seed, tuber, plant cell or tissue thereof comprising a) a recombinant construct having at least 80 % identity with SEQ-ID-No. 2 or SEQ-ID-No.3 and

further comprising a junction sequence selected from the group consisting of b)

i) a nucleic acid sequence that can be used to amplify a nucleotide fragment of between 126 and 136 basepairs using a polymerase chain reaction with two primers having the nucleotide sequences of SEQ-ID-No. 4 and SEQ-ID-No. 5,
ii) a nucleic acid sequence that can be used to amplify a nucleotide fragment of between 505 and 515 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of between 625 and 635 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of between 4752 and 4762 basepairs using a polymerase chain reaction with two primers having the nucleotide sequences of SEQ-ID-No. 10 and SEQ-ID-No. 11,

and/or
further comprising a junction sequence selected from the group consisting of

v) a nucleic acid sequence that can be used to amplify a nucleotide fragment of between 282 and 292 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of between 877 and 887 basepairs using a polymerase chain reaction with two primers having the nucleotide sequences of SEQ-ID-No. 14 and SEQ-ID-No. 15,
vii) a nucleic acid sequence that can be used to amplify a nucleotide fragment of between 827 and 837 basepairs using a polymerase chain reaction with two primers having the nucleotide sequences of SEQ-ID-No. 16 and SEQ-ID-No. 17, and/or
viii) a nucleic acid sequence that can be used to amplify a nucleotide fragment of between 9905 and 9915 basepairs using a polymerase chain reaction with two primers having the nucleotide sequences of SEQ-ID-No. 18 and SEQ-ID-No. 19.

A preferred embodiment of the present invention provides a Phytophthora-resistant transgenic potato, seed, tuber, plant cell or tissue thereof

a) a recombinant construct having at least 80 % identity with SEQ-ID-No. 2 or SEQ-ID-No.3 and
further comprising a junction sequence selected from the group consisting of
b)

i) a nucleic acid sequence that can be used to amplify a nucleotide fragment of 131 basepairs using a polymerase chain reaction with two primers having the nucleotide sequences of SEQ-ID-No. 4 and SEQ-ID-No. 5,
ii) a nucleic acid sequence that can be used to amplify a nucleotide fragment 510 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of 630 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of 4757 basepairs using a polymerase chain reaction with two primers having the nucleotide sequences of SEQ-ID-No. 10 and SEQ-ID-No. 11

and/or
further comprising a junction sequence selected from the group consisting of

v) a nucleic acid sequence that can be used to amplify a nucleotide fragment of 287 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of 882 basepairs using a polymerase chain reaction with two primers having the nucleotide sequence of SEQ-ID-No. 14 and SEQ-ID-No. 15,
vii) a nucleic acid sequence that can be used to amplify a nucleotide fragment of 832 basepairs using a polymerase chain reaction with two primers having the nucleotide sequences of SEQ-ID-No. 16 and SEQ-ID-No. 17, and/or
viii) nucleic acid sequence that can be used to amplify a nucleotide fragment of 9910 basepairs using a polymerase chain reaction with two primers having the nucleotide sequences of SEQ-ID-No. 18 and SEQ-ID-No. 19.

A nucleic acid sequence that can be used to amplify a nucleotide fragment of a certain length using a polymerase chain reaction with two primers means the product of said polymerase chain reaction with two primers. In particular, this means a nucleic acid sequence that is amplified to a nucleotide fragment of a certain length using a polymerase chain reaction with two primers.

A junction sequence includes either a right or left part of the recombinant construct inserted into the plant genome and partially includes plant genomic sequences of the flanking region of said right or left part of the recombinant construct. In particular, the junction sequence comprises either a left part of the recombinant construct having at least 80 % identity with SEQ-ID-No. 2 or 3 and a part of the flanking region having at least 80% identity to SEQ-ID-No. 20 or a right part of the recombinant construct having at least 80 % identity to SEQ-ID-No. 2 or 3 and a part of the flanking region having at least 80 % identity with SEQ-ID-No. 21. Identity with respect to partial sequences of the recombinant construct means in this case identity over the entire length of said left or right part of the recombinant construct (Fig. 2e).

In particular, there is at least a partial overlap of either a part of the left part of the recombinant construct having at least 80 % identity with SEQ-ID-No. 2 or 3 and the junction sequence having at least 80 % identity with SEQ-ID-No. 22 or a part of the right part of the recombinant construct having at least 80 % identity with SEQ-ID-No. 2 or 3 and the junction sequence having at least 80 % identity with SEQ-ID-No. 23. Identity with respect to partial sequences of the recombinant construct means in this case identity over the entire length of said left or right part of the recombinant construct (Fig. 2e).

PCR means polymerase chain reaction, i.e. the selective enrichment of nucleic acids of defined length within a mixture of nucleic acids with primers specific for said nucleic acid by using Taq-polymerase or the like (US 5,656,493; Sambrook et al. 1989, Molecular Cloning, Second Edition, Cold Spring Harbor Laboratory, Plainview, N.Y.).

An alternative embodiment provides a Phytophthora-resistant transgenic potato, seed, tuber, plant cell or tissue comprising

a) a recombinant construct having at least 80 % identity with SEQ-ID-No. 2 or SEQ-ID-No.3 and
further comprising a junction sequence selected from the group consisting of
a sequence having at least 80 % identity to SEQ-ID-No. 22 and/or SEQ-ID-No. 23.

One embodiment according to the present invention provides a Phytophthora-resistant transgenic potato plant, seed, tuber, plant cell or tissue obtainable from the seeds as deposited under Accession-No. NCIMB 41841 (Solanum tuberosum) at NCIMB (NCIMB Ltd , Ferguson Building, Craibstone Estate Bucksburn, Aberdeen AB21 9YA, Scotland, Great Britain) on May 12, 2011. A transgenic potato plant, seed, tuber, plant cell or tissue according to the present invention comprising the recombinant construct which is amplifiable as defined above may be obtained by propagation or crossing a potato plant with a potato plant obtained from the seeds deposited under Accession-No. NCIMB 41841 (of elite-event D) and subsequent selection of the plants carrying the recombinant construct by detection with PCR using the above defined primer pairs. The berries containing the seeds have been hand-harvested, extracted and dried recently. The seeds may be stored at room temperature. Seed may be treated with 0,04% GA (giberellic acid) in order to break dormacy and enhance germination.

In one embodiment for crossing the pollen of the father plant is transferred from its stamen to the isolated carpel of the mother plant. The true seed bearing berries are harvested and the seeds are separated from the peel and flesh of the berries. The seeds are replanted, grown to plants and subsequently the plants carrying the recombinant construct are selected by detection with PCR using the above defined primer pairs.

The mother plant and/or the father plants may be the phythophthora-resistant transgenic potato plant according to the present invention. In one embodiment the mother plant is phythophthora-resistant transgenic potato plant according to the present invention and the father plant may be a non-transgenic plant, e.g. selected from the group consisting of Agria, Sarpo Mira, Cara, Valor, Innovator, Diamant and Bintje. In an alternative embodiment the father plant is the phythophthora-resistant transgenic potato plant according to the present invention and the mother plant may be a non-transgenic plant, e.g. selected from the group consisting of Agria, Sarpo Mira, Cara, Valor, Innovator, Diamant and Bintje.

One embodiment of the present invention provides a method for providing a Phytophthora-resistant transgenic potato plant or part thereof comprising the following steps:

a) introducing a recombinant nucleic acid having at least 80 % identity with SEQ-ID- No. 2 or SEQ-ID-No. 3 into the genome of potato plant cells,
b) integrating said recombinant nucleic acid into the genome,
c) regenerating plant from said plant cells,
d) selecting plant comprising a nucleic acid having at least 80 % identity with SEQ-ID-No. 2 or SEQ-ID-No. 3 and a junction sequence selected from the group consisting of a sequence having at least 80 % identity to SEQ-ID-No. 22 and/or SEQ-ID-No. 23.

One embodiment of the present invention provides a method for providing a Phytophthora-resistant transgenic potato plant or part thereof comprising the following steps:

a) introducing a recombinant nucleic acid having at least 80 % identity with SEQ-ID- No. 2 or SEQ-ID-No. 3 into the genome of potato plant cells,
b) integrating said recombinant nucleic acid into the genome,
c) regenerating plant from said plant cells,
d) selecting plant comprising a nucleic acid having at least 80 % identity with SEQ-ID-No. 2 or SEQ-ID-No. 3 and a junction sequence selected from the group consisting of

i) a nucleic acid sequence that can be used to amplify a nucleotide fragment of between 126 and 136 basepairs using a polymerase chain reaction with two primers having the nucleotide sequences of SEQ-ID-No. 4 and SEQ-ID-No. 5,
ii) a nucleic acid sequence that can be used to amplify a nucleotide fragment of between 505 and 515 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of between 625 and 635 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of between 4752 and 4762 basepairs using a polymerase chain reaction with two primers having the nucleotide sequences of SEQ-ID-No. 10 and SEQ-ID-No. 11,

and/or
further comprising a junction sequence selected from the group consisting of

v) a nucleic acid sequence that can be used to amplify a nucleotide fragment of between 282 and 292 basepairs, using a polymerase chain reaction with two primers having the nucleotide sequences of SEQ-ID-No. 12 and SEQ-ID-No. 13,

vi) a nucleic acid sequence that can be used to amplify a nucleotide fragment of between 877 and 887 basepairs, using a polymerase chain reaction with two primers having the nucleotide sequences of SEQ-ID-No. 14 and SEQ-ID-No. 15,
vii) a nucleic acid sequence that can be used to amplify a nucleotide fragment of between 827 and 837 basepairs, using a polymerase chain reaction with two primers having the nucleotide sequences of SEQ-ID-No. 16 and SEQ-ID-No. 17

and/or

viii) a nucleic acid sequence that can be used to amplify a nucleotide fragment of between 9905 and 9915 basepairs, using a polymerase chain reaction with two primers having the nucleotide sequence of SEQ-ID-No. 18 and SEQ-ID-No. 19.

One method of the present invention provides a method for providing a Phytophthora-resistant transgenic potato plant or part thereof,
wherein in step d) plants are selected comprising a nucleic acid having at least 80 % identity with SEQ-ID-No. 2 or SEQ-ID-No. 3 and a junction sequence selected from the group consisting of

i) a nucleic acid sequence that can be used to amplify a nucleotide fragment of 131 basepairs using a polymerase chain reaction with two primers having the nucleotide sequences of SEQ-ID-No. 4 and SEQ-ID-No. 5,
ii) a nucleic acid sequence that can be used to amplify a nucleotide fragment 510 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of 630 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of 4757 basepairs using a polymerase chain reaction with two primers having the nucleotide sequences of SEQ-ID-No. 10 and SEQ-ID-No. 11,

and/or

v) a nucleic acid sequence that can be used to amplify a nucleotide fragment of 287 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of 882 basepairs using a polymerase chain reaction with two primers having the nucleotide sequences of SEQ-ID-No. 14 and SEQ-ID-No. 15,
vii) a nucleic acid sequence that can be used to amplify a nucleotide fragment of 832 basepairs using a polymerase chain reaction with two primers having the nucleotide sequences of SEQ-ID-No. 16 and SEQ-ID-No. 17, and/or
viii) a nucleic acid sequence that can be used to amplify a nucleotide fragment of 9910 basepairs using a polymerase chain reaction with two primers having the nucleotide sequences of SEQ-ID-No. 18 and SEQ-ID-No. 19.

One embodiment of the present invention provides a kit for the detection of the specific integration place, in particular for the detection of elite-event D as deposited under Accession-No. NCIMB 41841, comprising the primer pairs

SEQ-ID-No. 4 and 5,
SEQ-ID-No. 6 and 7,
SEQ-ID-No. 8 and 9,
SEQ-1 D-No. 10 and 11,
SEQ-ID-No. 12 and 13,
SEQ-ID-No. 14 and 15,
SEQ-ID-No. 16 and 17, and/or
SEQ-ID-No. 18 and 19.

The kit disclosed can be used for purposes of quality control (e.g., purity of seed lots), detection of the specific integration place, in particular elite-event D as deposited at NCIMB having deposition-No. NCIMB 41841 on May 12, 2011, in plant material or material comprising or derived from plant material, such as french fries, potato meal, mash potatoes etc. but not limited to food or feed products.
Briefly, genomic DNA is amplified by PCR using a primer which specifically recognizes the 5' or 3' flanking sequence of the insertion site in the elite event D, in particular the above mentioned primer pairs, e.g. primers SEQ-ID-No. 4 and SEQ-ID-No. 5, SEQ-ID-No. 6 and SEQ-ID-No. 7, SEQ-ID-No. 8 and SEQ-ID-No. 9, SEQ-ID-No. 10 and SEQ-ID-No. 11, SEQ-ID-No. 12 and SEQ ID NO: 13, SEQ ID NO: 14 and SEQ ID NO: 15, SEQ ID NO: 16 and SEQ ID NO:

17, SEQ-ID-No. 18 and SEQ-ID-No. 19, respectively. If PCR using above mentioned primer combinations on the plant material yields a fragment of

126 to 136 bp (131 bp),
505 to 515 bp (510 bp),
625 to 536 bp (630 bp),
282 to 292 bp (287 bp),
877 to 887 bp (882 bp),
827 to 837 bp (832 bp),
4752 to 4762 bp (4757 bp)
9905 to 9915 bp (9910), respectively,

the transgenic plant is determined to have the herein defined specific integration place, e.g. the selected elite-event D. The fragment length can be determined by gel electrophoresis using markers Sambrook et al., 1989 Molecular Cloning, Second Edition, Cold Spring Harbor Laboratory, plainview, N.Y.).

One embodiment of the present invention provides a detection method for a specific integration place, preferably for the identification of elite event D, comprising the steps of

a) isolating DNA from a potato plant as a test sample
b) exposing the test sample, a positive and a negative sample a primer pair as defined above under PCR-conditions, and
c) evaluating the amplification of a DNA-fragment

i) of between 126 and 136 basepairs when using a polymerase chain reaction with two primers having the nucleotide sequences of SEQ-ID-No. 4 and SEQ-ID-No. 5,
ii) of between 505 and 515 basepairs when using a polymerase chain reaction with two primers having the nucleotide sequences of SEQ-ID-No. 6 and SEQ-ID-No. 7,
iii) of 625 and 635 basepairs when using a polymerase chain reaction with two primers having the nucleotide sequences of SEQ-ID-No. 8 and SEQ-ID-No. 9, and/or
iv) of 4752 and 4762 basepairs when using a polymerase chain reaction with two primers having the nucleotide sequences of SEQ-ID-No. 10 and SEQ-ID-No. 11,

and/or

v) of between 282 and 292 basepairs when using a polymerase chain reaction with two primers having the nucleotide sequence of SEQ-ID-No. 12 and SEQ-ID-No. 13,
vi) of 877 and 292 basepairs when using a polymerase chain reaction with two primers having the nucleotide sequences of SEQ-ID-No. 14 and SEQ-ID-No. 15,
vii) of 827 and 837 basepairs when using a polymerase chain reaction with two primers having the nucleotide sequences of SEQ-ID-No. 16 and SEQ-ID-No. 17, and/or
viii) a nucleic acid sequence that can be used to amplify a nucleotide fragment of 9905 and 9915 basepairs, using a polymerase chain reaction with two primers having the nucleotide sequences of SEQ-ID-No. 18 and SEQ-ID-No. 19

compared with said positive and negative control.

In a preferred embodiment in step e)
evaluating means the amplification of a nucleotide fragment selected from the group consisting of a nucleotide fragment

i) of 131 basepairs when using a polymerase chain reaction with two primers having the nucleotide sequences of SEQ-ID-No. 4 and SEQ-ID-No. 5,
ii) of 510 basepairs when using a polymerase chain reaction with two primers having the nucleotide sequences of SEQ-ID-No. 6 and SEQ-ID-No. 7,
iii) of 630 basepairs when using a polymerase chain reaction with two primers having the nucleotide sequences of SEQ-ID-No. 8 and SEQ-ID-No. 9, and/or
iv) of 4757 basepairs when using a polymerase chain reaction with two primers having the nucleotide sequences of SEQ-ID-No. 10 and SEQ-ID-No. 11

and/or
of a nucleotide fragment selected from the group consisting of a nucleotide fragment

i) of 287 basepairs when using a polymerase chain reaction with two primers having the nucleotide sequences of SEQ-ID-No. 12 and SEQ-ID-No. 13,
ii) of 882 basepairs when using a polymerase chain reaction with two primers having the nucleotide sequences of SEQ-ID-No. 14 and SEQ-ID-No. 15,
iii) of 832 basepairs when using a polymerase chain reaction with two primers having the nucleotide sequences of SEQ-ID-No. 16 and SEQ-ID-No. 17, and/or
iv) a nucleic acid sequence that can be used to amplify a nucleotide fragment of 9910 basepairs, using a polymerase chain reaction with two primers having the nucleotide sequences of SEQ-ID-No. 18 and SEQ-ID-No. 19

compared with said positive and negative control.
The test sample comprises genomic DNA isolated from transformed plant material, e.g. from plants, seed, tuber, plant cells or an tissue thereof. The positive sample is a sample comprising genomic DNA isolated from plants including SEQ-ID-No. 1, e.g. genomic DNA isolated from plants grown from seeds deposited under Accession-No. NCIMB 41841. The negative sample is a sample comprising genomic DNA isolated from the non-transgenic original variety used for transformation, e.g. the Fontane variety.
The PCR reaction can be run with various DNA polymerases, such as the Pfu Ultra, Pfu Turbo or Herculase DNA Polymerase (Agilent Technologies, Santa Clara, CA, US). The composition for the protocol of the Pfu Ultra, Pfu Turbo or Herculase DNA polymerase may be as follows: 1x PCR buffer, 0.2 mM of each dNTP, 1$\mu$g genomic DNA of Sample, 50 pmol forward primer, 50 pmol reverse primer, 1 u Pfu Ultra, Pfu Turbo or Herculase DNA polymerase.
The amplification cycles may be as follows:
1 cycle of 60 seconds at 98°C, followed by 35 cycles of in each case 10 seconds at 98°C, 30 seconds at the annealing temperature given in the table below and 60 seconds per 1000bp product length (see table below) at 72°C, followed by 1 cycle of 10 minutes at 72°C, then 4°C.

Table1

| Event D | Border | Sense primer | SEQ-ID No. | Antisense primer | SEQ ID No | Annealig temperature [°C] |
|---|---|---|---|---|---|---|
| | LB | TCAAACGGATGTTAATTCAGTACATT | 4 | CCAGTTCCCAATTGACTACTAGAAA | 5 | 52 |
| | LB | TCTGTTGAATTACGTTAAGC | 6 | CTCAGAAGAAAGAATTGTTC | 7 | 48 |
| | LB | GTTTCTTAAGATTGAATCCTGTTGC | 8 | GCCCATTCTCTATTTTACTCACTAA | 9 | 51 |
| | R B | CCAAGATAGTGTTTCAGGAAAGTTATT | 12 | AAATTCATGGTAGAACTGGAGGAG | 1 3 | 52 |
| | R B | AACTGAATTTTGGGATTGAG | 14 | GAGTCAGTTAAATTAACTGCTTCAG | 1 5 | 49 |
| | R B | ACAAGAATAGCAAGGATTATCC | 16 | GAAGTTCGAACAACATTCTT | 1 7 | 49 |
| Left flanking region to blb1 | LB | GTGAACTAGGAAACCTAAATC | 10 | CAACTAATAAAACCAAGGAC | 1 1 | 52 |
| Right flanking region to blb1 | R B | AACTGAATTTTGGGATTGAG | 18 | ATGTAGCAGCATTGAGTTTT | 1 9 | 50 |

One embodiment according to the present invention provides a plant, tuber, seed, detectable by the above defined kit or by the above defined detection method.

One embodiment of the present invention provides a polynucleotide comprising

a) a recombinant nucleic acid having at least 80 % identity with SEQ-ID-No. 2 or SEQ-ID-No. 3 and
b) further comprising a junction sequence selected from the group consisting of

i) a nucleic acid sequence that can be used to amplify a nucleotide fragment of between 126 and 136 basepairs using a polymerase chain reaction with two primers having the nucleotide sequences of SEQ-ID-No. 4 and SEQ-ID-No. 5,
ii) a nucleic acid sequence that can be used to amplify a nucleotide fragment of between 505 and 515 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of between 625 and 635 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of between 4752 and 4762 basepairs using a polymerase chain reaction with two primers having the nucleotide sequences of SEQ-ID-No. 10 and SEQ-ID-No. 11,

and/or
further comprising a junction sequence selected from the group consisting of

v) a nucleic acid sequence that can be used to amplify a nucleotide fragment of between 282 and 292 basepairs, using a polymerase chain reaction with two primers having the nucleotide sequences of SEQ-ID-No. 12 and SEQ-ID-No. 13
vi) a nucleic acid sequence that can be used to amplify a nucleotide fragment of between 877 and 887 basepairs, using a polymerase chain reaction with two primers having the nucleotide sequences of SEQ-ID-No. 14 and SEQ-ID-No. 15
vii) a nucleic acid sequence that can be used to amplify a nucleotide fragment of between 827 and 837 basepairs, using a polymerase chain reaction with two primers having the nucleotide sequences of SEQ-ID-No. 16 and SEQ-ID-No. 17 and/or
viii) a nucleic acid sequence that can be used to amplify a nucleotide fragment of between 9905 and 9915 basepairs, using a polymerase chain reaction with two primers having the nucleotide sequences of SEQ-ID-No. 18 and SEQ-ID-No. 19.

One preferred embodiment of the present invention provides a polynucleotide comprising

a) a recombinant nucleic acid having at least 80 % identity with SEQ-ID-No. 2 or SEQ-ID-No. 3 and
b) further comprising a junction sequence selected from the group consisting of

i) a nucleic acid sequence that can be used to amplify a nucleotide fragment of 131 basepairs, using a polymerase chain reaction with two primers having the nucleotide sequences of SEQ-ID-No. 4 and SEQ-ID-No. 5,
ii) a nucleic acid sequence that can be used to amplify a nucleotide fragment 510 basepairs, using a polymerase chain reaction with two primers having the nucleotide sequences of SEQ-ID-No. 6 and SEQ-ID-No. 7,
iii) a nucleic acid sequence that can be used to amplify a nucleotide fragment of 630 basepairs, using a polymerase chain reaction with two primers having the nucleotide sequences of SEQ-ID-No. 8 and SEQ-ID-No. 9, and/or
iv) a nucleic acid sequence that can be used to amplify a nucleotide fragment of 4757 basepairs, using a polymerase chain reaction with two primers having the nucleotide sequences of SEQ-ID-No. 10 and SEQ-ID-No. 11

and/or

v) a nucleic acid sequence that can be used to amplify a nucleotide fragment of 287 basepairs, using a polymerase chain reaction with two primers having the nucleotide sequences of SEQ-ID-No. 12 and SEQ-ID-No. 13,
vi) a nucleic acid sequence that can be used to amplify a nucleotide fragment of 882 basepairs, using a polymerase chain reaction with two primers having the nucleotide sequences of SEQ-ID-No. 14 and SEQ-ID-No. 15,
vii) a nucleic acid sequence that can be used to amplify a nucleotide fragment of 832 basepairs, using a polymerase chain reaction with two primers having the nucleotide sequences of SEQ-ID-No. 16 and SEQ-ID-No. 17, and/or

viii) a nucleic acid sequence that can be used to amplify a nucleotide fragment of 9910 basepairs, using a polymerase chain reaction with two primers having the nucleotide sequences of SEQ-iD-No. 18 and SEQ-ID-No. 19

stably integrated into a potato plant cell nucleus.

One embodiment of the present invention provides a polynucleotide having at least 80% identity with SEQ-ID-Nos. 22 or 23.

A preferred embodiment according to the present invention is a polynucleotide comprising a nucleotide sequence having at least 80 % identity with SEQ-No. 1 preferably stably integrated into a potato plant cell nucleus.

In a further embodiment, the polynucleotide comprises a nucleotide sequence having at least 80%, at least 85%, at least 90%, at least 91 %, at least 92%, at least 93%, at least 94%, at least 95%, at least 96%, at least 97%, at least 98%, at least 99%, or 100% sequence identity to SEQ ID NO: 1, 2, 3, 44 and/or 45, preferably stably integrated into the genome of a potato plant cell.

In yet another embodiment, the polynucleotide comprises a nucleotide sequence having at least 80%, at least 85%, at least 90%, at least 91 %, at least 92%, at least 93%, at least 94%, at least 95%, at least 96%, at least 97%, at least 98%, at least 99%, or 100% sequence identity to SEQ ID NO: 1, 2, 3, 44 and/or 45 and comprising the blb1 and the blb2 genes, stably integrated into the genome of a potato plant cell. The polynucleotide can also further comprise one or more of SEQ ID NO: 4, 5, 6, 7, 8, 9, 10, 11, 12, 13, 14, 15, 16, 17, 18, and/or 19 in the regions flanking the inserts.

"Polynucleotides" according to the present invention may be isolated polynucleotides and/or recombinant polynucleotides. Recombinant polynucleotides or recombinant construct mean any polynucleotide produced by gene technology modification e.g. by man. The gene technology modification may be transforming a plant cell with a nucleotide sequence preferably using agrobacteria.

"Identity" between two nucleic acids and/or refers in each case over the entire length of the nucleic acids.

For example the identity may be calculated by means of the Vector NTI Suite 7.1 program of the company Informax (USA) employing the Clustal Method (Higgins DG, Sharp PM. Fast and sensitive multiple sequence alignments on a microcomputer. Comput Appl. Biosci. 1989 Apr; 5(2):151-1) with the following settings:

Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | on |
| K-tuple size | 1 |
| Gap penalty | 3 |
| Window size | 5 |
| Number of best diagonals | 5 |

Alternatively the identity may be determined according to Chenna, Ramu, Sugawara, Hideaki, Koike, Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, the web page: http://www.ebi.ac.uk/Tools/clustalw/index.html and the following settings

| | |
|---|---|
| DNA Gap Open Penalty | 15.0 |
| DNA Gap Extension Penalty | 6.66 |

(continued)

| | |
|---|---|
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al., 1989 Molecular Cloning, Second Edition, Cold Spring Harbor Laboratory.

Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). At least 80% sequence identity, preferably at least 85% sequence identity, especially preferred at least 90%, at least 95 %, at least 98%, at least 99% sequence identity, or even 100% sequence identity, with the nucleic acid having SEQ-ID-Nos. 1, 2, 3, 20, 21, 22 and/or 23 is preferred.

The recombinant construct may encompass nucleotides having nucleic acid substitutions, deletions and/or insertions relative to the unmodified nucleic acid in question, wherein the protein coded by such nucleic acids has similar or higher functional activity as the unmodified protein coded by the unmodified nucleic acid from which they are derived. In the substitutions may be based on the degenerative amino acid code.

A "deletion" refers to removal of one or more amino acids from a protein or to the removal of one or more nucleic acids from DNA.

An "insertion" refers to one or more nucleic acid residues being introduced into a predetermined site in the nucleic acid.

Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gene in vitro mutagenesis (USB, Cleveland, OH), QuickChange Site Directed mutagenesis (Stratagene, San Diego, CA), PCR-mediated site-directed mutagenesis or other site-directed mutagenesis protocols.

As used herein, the term "recombinant construct" preferably refers to an expression cassette having at least 80 % identity with SEQ-ID-No. 2 and/or 3. In one embodiment homologues of the expression cassette have at the DNA level at least 80%, preferably of at least 90%, especially preferably of at least 95%, quite especially preferably of at least 98%, at least 99% or 100% identity over the entire DNA region of SEQ-No. 2 and/or 3. Preferably, the recombinant construct comprises the blb1-gene including the blb1-promotor and the blbl-1-terminator as well as the blb2-gene including the blb2-promotor and the blbl-2-terminator as defined below as well as a mutated ahas-gene including the p-nos-promotor and the t-nos-promotor, which are preferably capable to express the blb1 and blb2 gene and optionally the ahas gene. Said recombinant construct may be introduced in a plant cell by gene technological methods e.g. agrobacteria transformation.

In one embodiment, the recombinant construct or expression cassette or transgenic plant comprises the nucleotide sequence of SEQ ID NO: 1, 2, 3, 44 and/or 45.

As used herein, the term "blb1-gene" refers to a gene having at least 80 % identity with SEQ-ID-No. 46. In one embodiment homologues of the blb1-gene have at the DNA level at least 90%, preferably of at least 95%, especially preferably of at least 98%, at least 99% or 100% identity over the entire DNA region of SEQ-ID-No. 46.

As used herein, the term "blb2-gene" refers to a gene having at least 80 % identity with SEQ-ID-No. 47. In one embodiment homologues of the blb2-gene have at the DNA level at least 90%, preferably of at least 95%, especially preferably of at least 98%, at least 99% or 100% identity over the entire DNA region of SEQ-ID.No. 47.

As used herein, the term "mutated ahas-gene" refers to a gene having at least 80 % identity with SEQ-ID-No. 48. In one embodiment homologues of the mutated ahas-gene have at the DNA level at least 90%, preferably of at least 95%, especially preferably of at least 98%, at least 99% or 100% identity over the entire DNA region of SEQ-ID-No. 48.

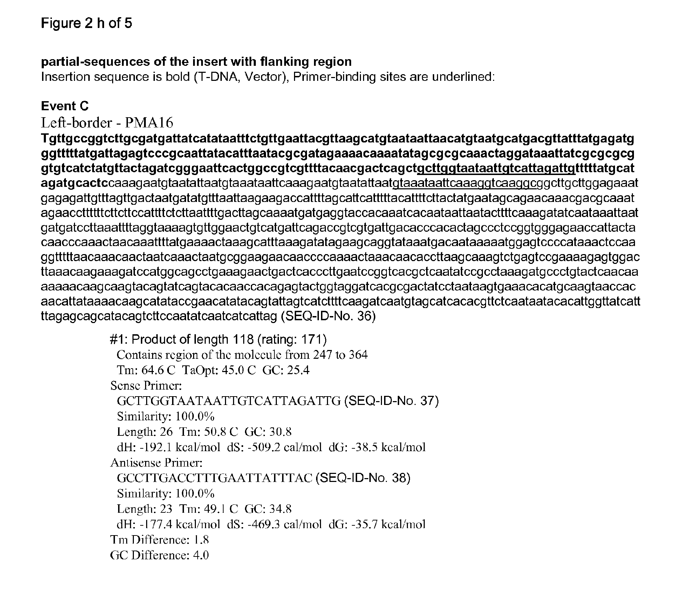

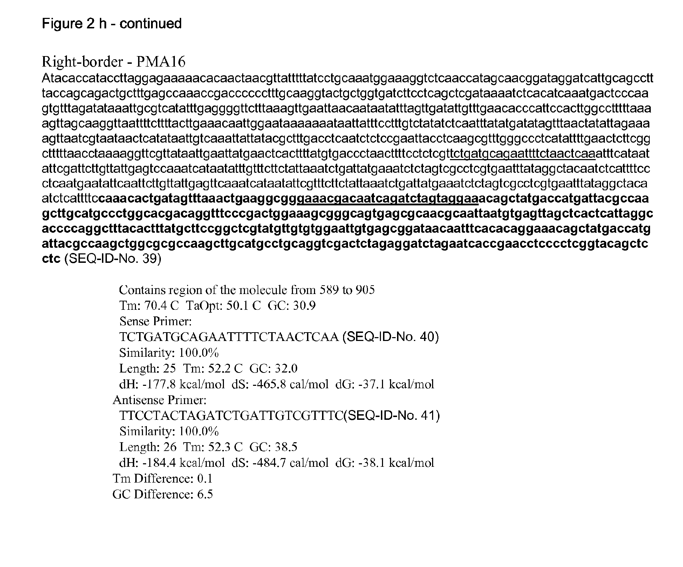

Preferably, the Phythophthora-resistant transgenic potato plant, seed, tuber, plant cell or tissue thereof expresses a functional protein corresponding to the blb1- and blb2-genes and optionally the ahas gene. Preferably, the Phythophthora-resistant transgenic potato plant, seed, tuber, plant cell or tissue thereof expresses SEQ-No. 46 and 47 and optionally SEQ-ID-No. 48 and/or the corresponding protein (cf. Fig. 2h).

The transgenic plant, seed, tuber, plants cell or tissue according to the present invention have a Phytophthora-resistance compared to the wildtype plant.

The wild type plant is a plant of a similar, more preferably identical, genotype as the transgenic plant having increased resistance to the Phytophthora-resistance, but does not comprise a recombinant nucleic acid comprising the blb1-gene and blb2-gene preferably regulated by their respective natural promotors and terminators.

As used herein the term "Phytophthora-resistance" or "Phytophthora-resistant", means reducing or preventing an infection with Phytophthora infestans. Phytophthora-resistance does not imply that the plant necessarily has 100% resistance to said infection. In preferred embodiments, the resistance to infection Phytophthora infestans in a resistant plant is greater than 10%, 15%, 20%, 25 %, 30%, 35 %, 40%, 45 %, 50%, 55 %, 60%, 65 %, 70%, 75 %, 80%, 85 %, 90%, or 95% in comparison to a wild type plant that is not resistant to Phytophthora infestans.

The term "Phytophthora-resistance" as used herein refers to the ability of a plant, as compared to a wild type plant, to avoid infection by Phytophthora infestans, to be killed by Phytophthora infestans, to hamper, to reduce, to delay, to stop the development, growth and/or multiplication of Phytophthora infestans. The level of Phytophthora infestans resistance of a plant can be determined in various ways, e.g. by scoring/measuring the infected leaf area in relation to the overall leaf area. Another possibility to determine the level of resistance is to count the number of Phytophthora infestans colonies on the plant or to measure the amount of spores produced by these colonies. Another way to resolve the degree of fungal infestation is to specifically measure the amount of Phytophthora infestans by quantitative (q) PCR. (e.g. Llorente et al (2010) A quantitative real-time PCR method for in planta monitoring of Phytophthora infestans growth. Lett Appl Microbiol. 51 (6):603-1 0.)

Furthermore, the transgenic plant, seed, tuber, plants cell or tissue according to the present invention provides a "comparable yield" compared to the wildtype plant, seed, tuber, plants cell or tissue.

The term "comparable yield" as used herein refers to the ability of the transgenic potato plant compared to wildtype plant to provide a similar amount of tubers. A similar amount of tubers means that the relative yield of transgenic tubers based on the yield/ha of wildtype tubers (kg/ha) is at least 95 % , preferably at least 96%, at least 97%, at least 98%, at least 99% of the yield/ha of the wildtype tubers or more preferably the same or more than the yield/ha of the wildtype tubers.

The %-relative yield is calculated as follows:

$$\% = \text{transgenic tubers (kg/ha)} \times 100\% / \text{wildtype tubers (kg/ha)}.$$

The term "plant" is intended to encompass plants at any stage of maturity or development, as well as any tissues or organs (plant parts) taken or derived from any such plant unless otherwise clearly indicated by context. Plant parts include, but are not limited to, plant cells, stems, roots, flowers, ovules, stamens, seeds, leaves, embryos, meristematic regions, callus tissue, anther cultures, gametophytes, sporophytes, pollen, microspores, protoplasts, hairy root cultures, and/or the like. As used herein, a "plant cell" includes, but is not limited to, a protoplast, gamete producing cell, and a cell that regenerates into a whole plant. Tissue culture of various tissues of plants and regeneration of plants there from is well known in the art and is widely.

The present invention also includes seeds produced by the plants of the present invention. Preferably, the seeds comprise a nucleic acid sequence having at lest 80 % identity with SEQ-ID-No.1. The generated transformed plants may be propagated by clonal propagation or classical breeding techniques.

For the purposes of the invention, "recombinant construct" or "recombinant nucleic acid" means an expression cassette or a vector construct comprising the blb1-gene and the blb2-gene in combination with their natural promoters and terminators. Said expression cassette comprising the blb1-gene, blb2-gene, blb1-promotor, blb2-promotor, blb1-terminator and blb2-terminator are defined above.

As used herein, the term "transgenic" preferably refers to any plant, plant cell, tuber, callus, plant tissue, or plant part that contains the recombinant construct or a part thereof which is preferably introduced by non-essentially biological processes, preferably agrobacteria transformation. The recombinant construct or a part thereof is stably integrated into a chromosome, so that it is passed on to successive generations by clonal propagation, vegetative propagation or sexual propagation. Said successive generations are also transgenic. Essentially biological processes may be crossing of plants and/or natural recombination.

A transgenic potato plant, seed tuber, plants cell or tissue for the purposes of the invention is thus understood as meaning that the recombinant cassette integrated into the genome. Preferably, the blb1-gene and/or the blb2-gene and/or the ahas-gene are not present in the genome of the original plant and preferably are present in the genome of the transgenic plant not at their natural locus of the genome of the original plant.

Natural locus means the location on a specific chromosome, preferably the location between certain genes, more preferably the same sequence background as in the original plant which is transformed.

Preferably, the transgenic potato plant, seed tuber, plants cell or tissue thereof expresses the blb1-gene and the blb2-gene. The term "expression" or "express" means the transcription of a specific gene or specific genes or specific genetic vector construct. The term "expression" or "express" in particular means the transcription of a gene or genes or genetic vector construct into structural RNA (rRNA, tRNA) or mRNA with preferably a subsequent translation of the latter into a protein.

The term "increased expression" or "overexpression" or "increase of content" as used herein means any form of expression that is additional to the original wild-type expression level. For the purposes of this invention, the original wild-type expression level might also be zero (absence of expression or absence of respective gene(s)).

The wildtype plant cells may be transformed with one of the above described recombinant construct. Suitable methods for transforming host cells including plant cells are well known in the art of plant biotechnology. Any method may be used to transform the recombinant expression vector into plant cells to yield the transgenic plants of the invention. The wildtype plants cells may be e.g. from Fontane, Agria, Bientje, Sarpo Mira, Cara, Valor, Innovator, Diamant.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). Agrobacterium based transformation techniques are well known in the art. The Agrobacterium strain (e.g., Agrobacterium tumefaciens or Agrobacterium rhizogenes) comprises a plasmid (Ti or Ri plasmid) and a T-DNA element which is transferred to the plant following infection with Agrobacterium. The T-DNA (transferred DNA) is integrated into the genome of the plant cell. The T-DNA may be localized on the Ri- or Ti-plasmid or is separately comprised in a so-called binary vector. Methods for the Agrobacterium-mediated transformation are described, for example, in Horsch RB et al. (1985) Science 225:1229. The transformation of potatoe by Agrobacteria is described in, for example WO 2008/ß34876). Transformation may result in transient or stable transformation and expression. Although a nucleotide sequence of the present invention can be inserted into any plant and plant cell falling within these broad classes, it is particularly useful in potato plant cells.

The genetically modified plant cells can be regenerated via all methods with which the skilled worker is familiar. Suitable methods can be found in the abovementioned publications by S.D. Kung and R. Wu (White FF, Vectors for Gene Transfer in Higher Plants, Transgenic Plants, Vol. 1, Engineering and Utilization, edited by S.D. Kung and R. Wu, Academic Press, 1993, pp. 15 - 38; Jenes B et al. Techniques for Gene Transfer, Transgenic Plants, Vol. 1, Engineering and Utilization, edited by S.D. Kung and R. Wu, Academic Press, 1993, pp. 128-143) Potrykus (Potrykus (1991) Annu Rev Plant Physiol Plant Molec Biol 42:205- 225.) or or Höfgen and Willmitzer (Höfgen R, Willmitzer L (1988) Storage of competent cells for Agrobacterium transformation. Nucleic Acids Res 16:9877).

The recombinant construct may comprise a mutated ahas-gene as a selection marker. Plants carrying the construct are resistant to immidazolines. For selection of transgenic potato plants chemical compounds inhibiting the AHAS enzyme can be used. Useful compounds are the imidazoline type herbicides. Especially useful compounds are selected from the group consisting of imazethapyr (Pursuit®), imazamox (Raptor®), imazamethabenz (Assert®), imazapyr (Arsenal®), imazapic (Cadre®) and imazaquinon (Scepter®). For selection of transgenic plants chemical compounds as described in the review article by Duggleby, R.G. and Pang, S.S. in Journal of Biochemistry and Molecular Biology 33(1), 1-36 (2000) can be used.

The transformed plant tissue may be exposed to 0,5 μM imazamox such selecting the plant material carrying the construct.

Following DNA transfer and regeneration, putatively transformed plants may also be evaluated, for instance using Southern analysis, for the presence of the whole recombinant construct, copy number and/or genomic organisation.

Gene targeting in plants is possible, but it is a quite rare event (Hanin & Paszkowski 2003 Current Opinion Plant Biol. 6(2):157-62). However, the person skilled in the art will know how to improve gene targeting frequency. For example, one could increase gene targeting frequency by expressing proteins, which facilitate the process of homologous recombination such as yeast RAD54 (Shaked et al. 2005 Proc Natl Acad Sci USA 102(34):12265-9). Another approach is to facilitate detection of gene targeting lines by a strong positive-negative selection system (Iida & Terada 2005 Plant Mol. Biol 59: 205-219). In such approach a negative selectable marker is located outside of the homologous sequences on the transformation construct. In consequence, only those transgenic plants with random insertion of the transgenic sequences contain the negative selectable marker, while transgenic lines obtained through gene targeting do not comprise the negative selectable marker.

Furthermore, gene targeting frequency can be drastically increased by introducing a DNA double strand break at or near the desired insertion site. The person skilled in the art will know how to achieve this. For example, natural occurring homing endonucleases (also referred to as meganucleases, e.g. I-CreI) can be modified such that they recognize and cut a novel DNA sequence, i.e. the sequence at or near the desired insertion site in the genome (WO 07/047859, WO 07/049156). Alternatively, one could design so called zink finger nucleases, which are comprised of a unspecific nuclease domain (usually obtained from FokI nuclease) linked to a zink finger, which specifically recognizes the desired DNA sequence (compare for example Trends Biotechnol. 2005 23(12):567-9; Cell Mol Life Sci. 2007 64(22): 2933-44; WO 08/021207). Another method is the usage of TAL (transcription activator like) effectors linked to a DNA specific nuclease (e.g. Fok1) as described in Mahfouz et al. 2011. De novo-engineered transcription activator-like effector (TALE) hybrid nuclease with novel DNA binding specificity creates double-strand breaks; PNAS 108(6)2623-2628. By using this method, a TAL effector variant binding to the desired target sequence can be easily generated by changing the well defined aminoacids binding to the DNA (the code can be found in WO/2010/079430).

Gene targeting may be used to obtain a line similar to the elite-event D by inserting a transgenic construct comprising the recombinant construct at essentially the same insertion site as found in elite-event D. The person skilled in the art will know that the insertion site may differ in a few base pairs or up to a few kilo base pairs, but still obtaining a similar line with similar beneficial characteristics as compared to deposited elite-event. Gene targeting may in particular be used to establish a line similar to deposited elite-event D in a potato variety other than Fontane. It may be of interest to establish such a corresponding line based on other varieties more particularly suited for environmental conditions found in different potato growing regions.

## Figures

Figure 1: Vector card VCPMA 16
Figure 2: Sequences of the present application

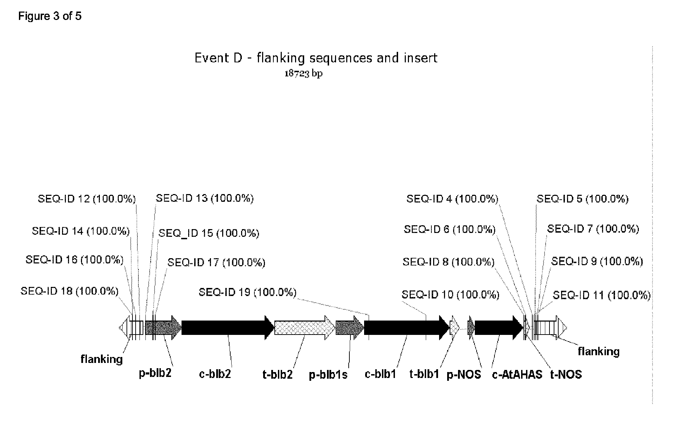

Figure 3: Overview of primers
Figure 4: Chart comparing the relative yield of events A to D, Bintje (standard variety) with Fontane (mother variety of events A to D).The average yield/ha of Fontane, events A - D and Bintje was measured over 3 years at 15 locations in the field. The average yield/ha of the Fontane variety was set to 100% and relative yields of events A - D and Bintje were calculated accordingly.
Figure 5: Chart shows the result of Phytophthora screening of Fontane compared with events A to D and Bientje. Diseased leaf area was scored in the field after natural infection. The mother variety Fontane was set to 100%. All events show full resistance against Phytophthora infestans.

## Examples

The following examples are not intended to limit the scope of the claims to the invention, but are rather intended to be exemplary of certain embodiments. Any variations in the exemplified methods that occur to the skilled artisan are intended to fall within the scope of the present invention.

### Example 1: General methods

The cloning steps carried out for the purposes of the present invention such as, for example, restriction cleavages, agarose gel electrophoresis, purification of DNA fragments, transfer of nucleic acids to nitrocellulose and nylon membranes, linking DNA fragments, transformation of E. coli cells, bacterial cultures, phage multiplication and sequence analysis of recombinant DNA, are carried out as described by Sambrook et al. Cold Spring Harbor Laboratory Press (1989), ISBN 0-87969-309-6.

The chemical synthesis of oligonucleotides can be affected, for example, in the known fashion using the phosphoamidite method (Voet, Voet, 2nd Edition, Wiley Press New York, pages 896-897). The sequencing of recombinant DNA molecules is carried out with an MWG-Licor laser fluorescence DNA sequencer following the manual of the manufacturer based on the method by Sanger (Sanger et al., Proc. Natl. Acad. Sci. USA 74, 5463 (1977).

### Example 2 Cloning of transformation vector VC-PMA16

pSUNAHASmod was used as backbone for the construction of VCPMA16. pSUNAHASmod is based on the plasmid pSUN1 (WO 02/00900). The T-DNA of pSUNAHASmod contains a mutated AHAS (Acetohydroxyacid-Synthase)-gene (S653N), which enhances the resistance of the transformed plant against imidazolinone herbicides (e.g. Imazamox: (R/S)-2-(4-isopropyl-4-methyl-5-oxo-2-imidazolin-2-yl)-5-methoxymethylnicotinic acid). The use of the mutated AHAS gene as selection marker is described in Andersson et al. (2003) Plant Cell Rep 22:261-267 and WO 2004/005516. The AHAS selection cassette was constructed by fusing the nos promoter fragment from pGPTVKan (Becker et al., Molecular plant Biology 20, 1195 - 1197), the mutated AHAS gene from Arabidopsis and the nos-terminator from pGPTVKan (Becker et al., 1992 Molecular plant Biology 20, 1195 - 1197).

The blb1-gene fragment, including the 1173 bp blb1-promotor-region and the 406 bp bib1 terminator region was ligated into pSUNAHASmod by using the XbaI restriction site.

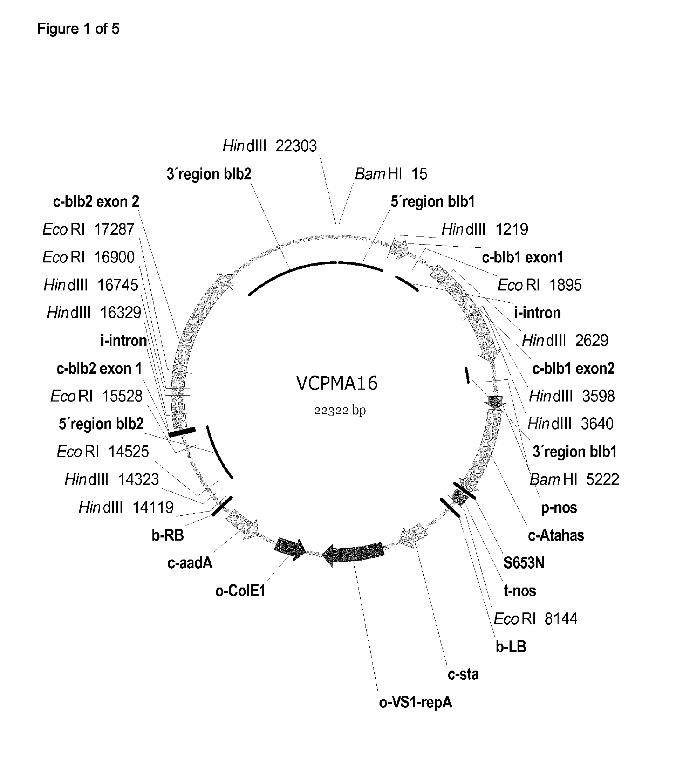

The blb2-gene expression cassette comprises the Rpi-blb2 gene (3890 bp), the blb2-promoter sequence (1530 bp) and 2530 bp blb2 termination sequence. To insert the blb2-expression cassette into the bib1 containing pSUNAHASmod, the vector was cut with PstI. The resulting sticky restriction sites were blunted and the blb2 expression cassette was inserted in a blunt-blunt ligation. The resulting vector was named VCPMA16 (Figure 1).

**Example 3 Transformation of Agrobacterium tumefaciens (A. tumefaciens) with VCPMA16**

The construct VCPMA16 was transformed into the A. tumefaciens strains LBA4404, AGL0 or AGL1 by using direct transformation as described by Walkerpeach & Velten (Agrobacterium-mediated gene transfer to plant cells: co-integrate and binary vector systems, Gelvin SB, Schilperoot RA (Hrsg.), Plant Molecular Biology Manual, 2nd edn, Kluwer Academic Publishers, Dordrecht, Netherlands, pp. B1/1-B1/19, 1994). Transformed bacteria were grown on YEB agar plates containing 1 μg/ml spectinomycin.

**Example 4 Cultivation and transformation of potato cultivar Fontane using A. tumefaciens**

The potato variety Fontane was transformed with VCPMA16 by using Agrobacterium mediated transformation as described by Visser (Visser RGF, 1991, "Regeneration and transformation of potato by Agrobacterium tumefaciens." In Lindsey K (ed), "Plant Culture Manual", Kluwer Academic Publishers, Dordrecht, Netherlands. Seiten B5/1 - B5/9) but using Imazamox as selection marker (WO 2004/005516; Andersson et al., 2003, plant Cell Rep 22: 2261-267).

Potato leaf or shoot segments were incubated for 1-3 days on MC-plates (M300-Plates (4,4 g/l MS-Medium, 2 mg/l NAA, 1 mg/l BAP, 30 g/l Sucrose, pH 5,2) covered with 1,5 - 2 ml liquid M100-Medium (4,4g/l MS-Medium, 30 g/l Sucrose, 0,5 mg/ml Thiamin-Hydrochloride, 0,5 mg/ml Pyridoxin-Hydrochloride, 1 mg/l Nikotinic acid, 0,5 mg/l Kinetin, 29,8 mg/l $FeSO_4$*7$H_2O$, 1 mg/l 2,4-D, 2 g/l Casein-Hydrolysate, pH 6,5) and covered with a sterile filter paper.

After 1-3 days the tissue segments were incubated with A. tumefaciens (containing VCPMA16) in MS10-Medium (4,4 g/l MS-Medium, 10 g/l Sucrose, pH 5,8). After 8 - 10 min. the tissue segments were transferred to M300 plates (see above). After 1 - 3 days the tissue segments were transferred to MS400-plates (4,4 g/l MS-Medium, 2 mg/l zeatine, 0,01 mg/l NAA, 0,1 mg/l GA3, 10 g/l Sucrose, 400 mg/l Claforane or carbenicilline, pH 5,8) and incubated for another 3 - 5 days.

**Example 5 Selection of the transformed potato plantlets**

After 3 - 5 days the tissue segments were transferred to MS400 plates (see above) containing 0,5 μmol Imazamox as selection agent. Every 2 weeks the tissue segments were conveyed to new MS400 plates containing 0,5 μmol Imazamox. Growing (regenerated) shoots were harvested and transferred to MS30 plates (4,4 g/l MS-Medium, 30 g/l Sucrose, 200 mg/l claforane, pH 5,8) for further cultivation.

**Example 6 DNA extraction from transformed potato shoots**

DNA was extracted from putative transgenic shoots by using the Wizard Magnetic 96 DNA Plant System (Promega, Mannheim) Kit according to the instructions of the manufacturer.

Example 7 Detection of Rpi-blb1 und Rpi-blb2 in transformed potato plants using real-time PCR To detect the presence of blb1 and blb2 a real time PCR was performed using the DNA from putative transgenic potato shoots (see above) as template. Following primers were used:

blb1:

5'-TGT TGA ACA CTG TAA CAT GCT AAA ATG-3' (forward Primer; SEQ ID No. 49)

5'-AGT TGT GGA CAT CCC CGAATT-3' (backward Primer; SEQ ID No. 50)

5'-AGA GGG ATT GCA GCA CCT AAC AAC CCT C-3' (Probe; SEQ ID No. 51)

blb2:

5'-TTC AAA ACC CCA AAT AAG TTT CAA C-3' (forward Primer; SEQ ID No. 52)

5'-CCA TGC TTG CTG TAC TTT GCA-3' (backward Primer; SEQ ID No. 53)

5'-CGT TAC CCA GTC CTT CGG CG-3' (Probe; SEQ ID No. 54)

The samples were analyzed using a Roche Lightcycler480 by using 20-50 ng genomic DNA, 900 mM PCR primer (see above), 200 nM probe (see above) in 1 x LightCycler 480 Probe Master (for detailed protocol see manual of the manufacturer).

The amplification cycles of the PCR were:

1 cycle of 15 min at 95°C for denaturation, followed by 40 cycles of in each case 10 seconds at 95°C (Ramp Rate 4,8°C/sec) and 30 sec at 60°C (Ramp Rate 2,5°C /sec).

If the PCR of both fragments resulted in a positive signal, the shoots were transferred into the greenhouse to conduct Phytophthora resistance tests.

**Example 8 Determination of the resistance level of blb1 and blb2 containing transgenic potato shoots against Phytophthora infestans**

The blb1 and blb2 containing transgenic potato shoots (as determined above) were transferred into soil and adapted to soil for 2 days at 22°C with 12 h day-length and 100% humidity in a growth cabinet (Binder KBW 400). Afterwards plants were grown in the greenhouse or phytochambers under similar conditions but 70% humidity.

After 4 weeks plants were inoculated with Phytophthora infestans spores. To prove the broad spectrum resistance mediated by blb1 and blb2, a multitude of different Phytophthora isolates, e.g. Blue13, Us-22 and many locally collected strains were tested collected from all over the world, either in mixtures or as single isolates.

All Phytophthora isolates were cultivated on pea-agar

Table 2

| | |
|---|---|
| Pea-agar: | - 150 g peas |
| | - 1000 ml Millipore-ater |
| | - cook for 75 min. in a steamer |
| | - cool down for - 1 hour, incubate for 24h at RT |
| | - strain media and refill with 1l Millipore water |
| | - add 5g Glucose and 20g agar-agar |
| | - adjust pH to 6.5 |
| | - autoclave for 15 min |
| | - pour plates under sterile conditions |

Plant were inoculated with a spore density of 2,5xE05 spores/ml. The spore density was evaluated by using a Thoma counting chamber. For inoculation the complete plant was sprayed with spore suspension and transferred into a dark mist chamber. After 12-18 hours the plants were moved to the greenhouse (21°C, 12h light, >90% humidity) for one week.

First disease symptoms occurred after approx. 1 week. The rating of disease symptoms was done by trained personal evaluating the diseased leaf area, necrotic lesions, clorotic lesions and potential sporulation of P. infestans.

These values were integrated into a disease rating ranging from 0 to 100%. In the scoring system 0% disease means no macroscopically visible symptoms, whereas 100% means that all inoculated leaves are completely brownish and covered with mycelia, so the plant is essentially dead. Inoculation of the susceptible mother variety Fontane potato variety generally leads to a strong infection of all leaves. All leaves are heavily infected and green tissue is rare. In contrast the inoculation of the transformed Fontane Event A to D always leads completely healthy plants with a disease rating of 0% for all used Phytophthora isolates. As susceptible control the standard variety Bintje was used, which is known to be fully susceptible to Phytophthora infection

**Example 9 DNA isolation and quantitation methods for FST identification**

Young leaf tissue of the fungal resistant potato events were collected for DNA isolation and characterization. Upon collection, the leaf tissues were frozen with liquid nitrogen and lyophilized.

DNA was isolated from potato leaf tissue using a modified cetyl trimethyl ammonium bromide (CTAB) method (Carlson et al., 1991). Dry leaf tissue was ground with a pestle and a mortar. The ground tissue was incubated with preheated extraction buffer consisting of 2% (w/v) CTAB, 100 mM Tris-HCl, 1.4 M NaCl, 1% (w/v) polyvinylpyrrolidone (PVP), 20 mM ethylenediamine tetraacetic acid (EDTA), pH 9.5 (5 ml/1 g fresh leaf tissue) and beta-mercaptoethanol (2.5 μl/ml buffer) at 74°C for 20 min. After centrifugation at 2440 x g for 10 min, the supernatant was extracted twice with an equal volume of chloroform/isoamyl alcohol (24:1). DNA was precipitated with 0.7 volume of isopropanol and dissolved in TE buffer (10 mM Tris-HCl, 1 mM EDTA, pH 8.0) with 0.5 mg/ml RNase A (Invitrogen; Carlsbad, CA 92008 USA) added to a final concentration of about 500 ng/μl. The isolated DNA was quantified with Hoechst 33258 dye (Invitrogen) using calf thymus DNA (Invitrogen) as the DNA standard on an FLx800™ Microplate Reader (BioTek Instruments, Winooski, VT 05404, USA) according to the fluorometer user manual.

**Example 10: Tail PCR amplification of flanking sequences**

Oligonucleotide primers. T-DNA specific primers which are complementary to the AHAS coding sequence and the Blb2 promoter region in VC-PMA16, respectively, were synthesized (Table 3).

Table 3 T-DNA specific primers for cloning of flanking sequences using tail PCR

| Name | Sequence | Position in VC-PMA16 | Comment |
|---|---|---|---|
| 07-038_P25 | AACGATGTCATAACGGAAGG (SEQ-ID-NO. 55) | 16136 | Specific primer for tail PCR (LB1) |
| 07-038_P26 | AGAGCATTTGAAGCAGATCTAGGGT (SEQ-ID-NO.56) | 464 | Specific primer for tail PCR (RB1) |
| 07-038_P27 | CGGATTAAATACTGAGAGCTCGAAT (SEQ-ID-NO.57) | 16163 | Specific primer for tail PCR (LB2) |
| 07-038_P28 | CAGATCTAGGGTTTTATCTCGG (SEQ-ID-NO.58) | 454 | Specific primer for tail PCR (RB2) |
| 07-038_P29 | TGCCGGTCTTGCGATGATTA (SEQ-ID-NO.59) | 16241 | Specific primer for tail PCR (LB3) |
| 07-038_P30 | AGATCTAGGGTTTTATCTCGGGATT (SEQ-ID-NO. 60) | 450 | Specific primer for tail PCR (RB3) |
| LB = left flanking primer, RB = right flanking primer | | | |

In addition, four arbitrary degenerate (AD) primers were synthesized according to Liu et al 1995):

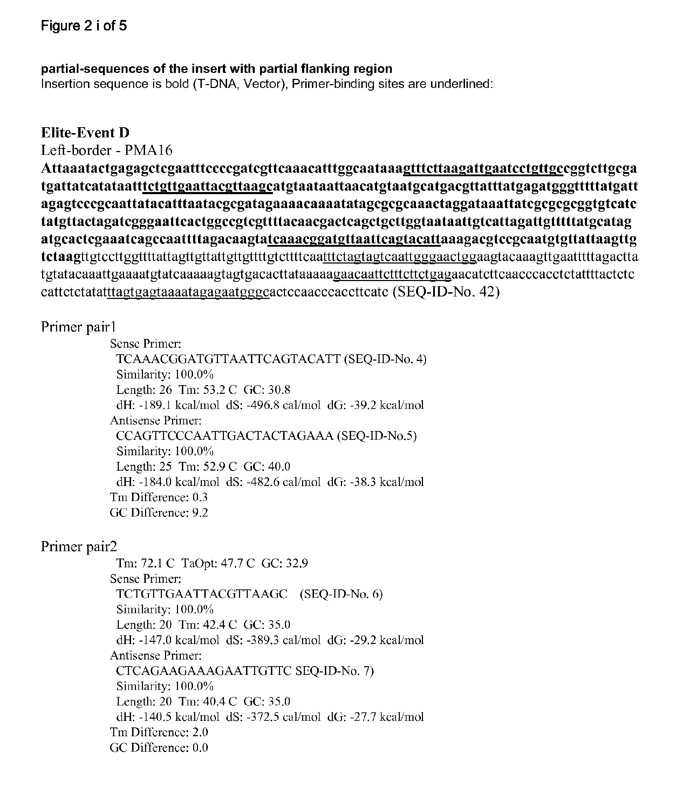

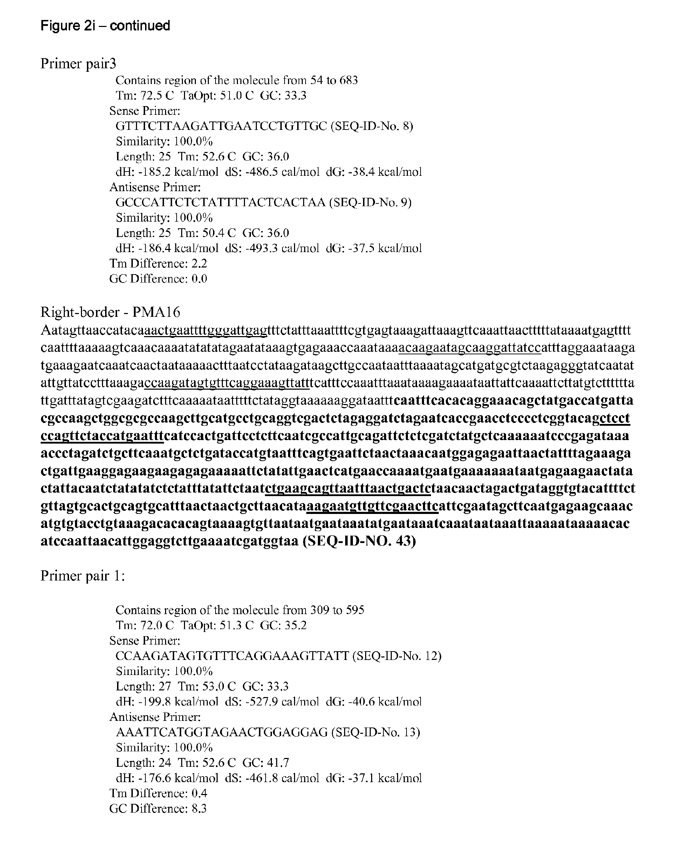

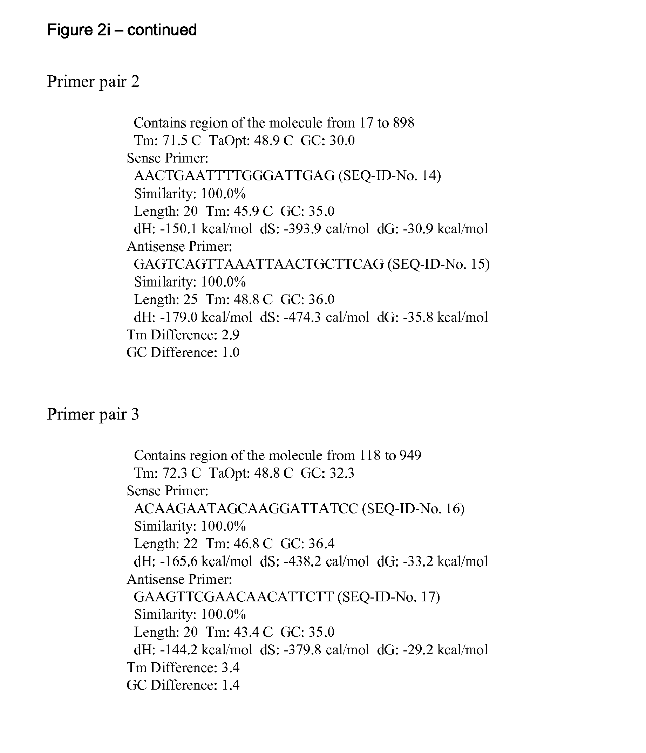

TG(A/T)GNAG(A/T)ANCA(G/C)AGA-3' (ADI) (SEQ-ID-NO. 61), AG(A/T)GNAG(A/T)ANCA (A/T)AGG-3' (AD2) (SEQ-ID-NO. 62), CA(A/T)CGICNGAIA(G/C)GAA-3' (AD3, I indicates inosine) (SEQ-ID-NO. 63), and TC(G/C)TICGNACIT(A/T)GGA-3' (AD4) (SEQ-ID-NO. 64). These AD primers have average Tm's of 47-48°C as calculated with the formula 69.3 + 0.41 (%GC) -650/L, where L is primer length (cf. Fig. 2I)

Tail PCR was performed basically following Liu et al procedure (Liu et al 1995). Primary TAIL-PCR reactions (20 μl) contained 1x PCR buffer (10 mM Tris-HCl pH 8.3, 50 mM KCl, 1.5 or 2.0 mM $MgCl_2$, 0.001 % gelatin), 200 μM each of dNTPs, 25 ng of genomic DNA, 1 unit of Taq polymerase (Invitrogen), 0.2 μM T-DNA specific primers (07-038_P25 and 07-038_P26) and a given AD primer (2 μM for AD1, 3 μM for AD2 or 4 μM for AD3 and AD4). Primary TAIL-PCR was executed according to the PCR program in Liu et al (1995) in Perkin-Elmer thermal cyclers 9700. Aliquots (1 μl) from 50-fold dilutions of the primary PCR products were applied directly to secondary TAIL-PCR reactions (20 μl) containing 1x PCR buffer, 1 unit of Taq DNA polymerase, 200 μM each of dNTPs, 0.2 μM T-DNA specific primers (07-038_P27 and 07-038_P28) and the same AD primer used in the primary reaction (1.5 μM for AD1, 2.0 μM for AD2 and AD3and AD4). After amplification with 12 super cycles, the secondary TAIL-PCR products (1 μl aliquots of 10 fold dilutions) were re-amplified in 50 μl tertiary reactions with 20 reduced-stringency cycles. Components and their concentrations were the same as in the secondary reaction except that another nested PCR primer was used (07-038_P29 for LB and 07-038_P30 for RB). Amplified products from the reactions were analyzed by agarose gel electrophoresis. Strongly amplified products were recovered and purified with Zymoclean Gel DNA recovery kit (Zymo Research, CA 92614, USA). The purified DNA was quantified with Hoechst 33258 dye (Invitrogen) using calf thymus DNA (Invitrogen) as the DNA standard on an FLx800™ Microplate Reader (BioTek Instruments, Winooski, VT 05404, USA) according to the fluorometer user manual.

Example 11 DNA sequencing

The purified tertiary PCR products were sequenced with Sanger sequencing using BigDye terminator v3.0 kit according to the manufactuer's protocol (Applied Biosystems, California 92008, USA). The same specific primer used in the tertiary PCR (unlabeled) was used for sequencing.

**Example 12: Identification of specific events by PCR**

Unless otherwise specified, standard methods as described in Sambrook et al., Molecular Cloning: A laboratory manual, Cold Spring Harbor 1989, Cold Spring Harbor Laboratory Press are used.

Genomic DNA was prepared from the particular potato events by using the DNeasy Plant Mini Kit (Quiagen) for processing of single samples and the DNeasy 96 Plant Kit (Quiagen) for processing of samples in 96 well format. Both kits were used according to the intructions described in the manual. The junction between flanking region and T-DNA insert was amplified from the cDNA by PCR as described in the protocol of the Phusion hot-start, Pfu Ultra, Pfu Turbo or Herculase DNA polymerase (Stratagene, Santa Clara, CA, US).

The composition for the protocol of the Pfu Ultra, Pfu Turbo or Herculase DNA polymerase was as follows: 1x PCR buffer, 0.2 mM of each dNTP, 100 ng cDNA of Arabidopsis thaliana (var Columbia-0) , 50 pmol forward primer, 50 pmol reverse primer, 1 u Pfu Ultra, Pfu Turbo or Herculase DNA polymerase.

The amplification cycles were as follows:

1 cycle of 60 seconds at 98°C, followed by 35 cycles of in each case 10 seconds at 98°C, 30 seconds at specific annealing temperature (see table) and 60 seconds at 72°C, followed by 1 cycle of 10 minutes at 72°C, then 4°C.

The following primer sequences and annealing temperatures were used to specifically amplify event-specific FST-T-DNA junctions:

Table 4

| Event | Flanking site | Sense primer | SEQ ID No. | Antisense primer | SEQ ID No. | Annealig temperature [°C] | Product length (bp) |
|---|---|---|---|---|---|---|---|
| A | LB | TAATTCAGTACATTAAAGACGTCCG | 65 | GTCCCATAGTCATTTCTTGATCA | 66 | 50 | 63 |
| | RB | TGTCTCTGATAGGCTAATAAACTATG | 67 | TAGATCTGATTGTCGTTTCCC | 68 | 48 | 91 |
| B | LB | ATGACGTTATTTATGAGATGGGT | 69 | ATTTAAAAGGCAAAACGTGC | 70 | 49 | 100 |
| | RB | TTCATGTCAAGTTCAATTTCAGG | 71 | ACTCACATTAATTGCGTTGCG | 72 | 51 | 94 |
| C | LB | GCTTGGTAATAATTGTCATTAGATTG | 73 | GCCTTGACCTTTGAATTATTTAC | 74 | 49 | 118 |
| | RB | TCTGATGCAGAATTTTCTAACTCAA | 75 | TTCCTACTAGATCTGATTGTCGTTTC | 76 | 52 | 317 |
| D | LB | TCAAACGGATGTTAATTCAGTACATT | 4 | CCAGTTCCCAATTGACTACTAGAAA | 5 | 52 | 131 |

(continued)

| Event | Flanking site | Sense primer | SEQ ID No. | Antisense primer | SEQ ID No. | Annealig temperature [°C] | Product length (bp) |
|---|---|---|---|---|---|---|---|
| | RB | CCAAGATAGTGTTTCAGGAAAGTTATT | 12 | AAATTCATGGTAGAACTGGAGGAG | 13 | 52 | 287 |

The resulting PCR products were analyzed on a 1.5% Agarose-gel. PCR products occur specifically to identify the event. Detailed conditions are given in Table 4.

**Example 13 Determination of yield by field trials**

The various potato events were tested in the field to determine their yield potential. The yield of the events A, B, C and the elite event D, all showing full Phytophthora resistance, was compared to the non transgenic mother line Fontane and other standard potato varieties, like e.g. Bintje under disease free conditions (full plant protection scheme of transgenic events and control varieties according to good agricultural practice).

Yield trials were performed on more than 15 locations across 3 years (2008-2010). For every experiment a randomized block design with 3-5 block-repetitions was used. Each block was about 10-15 $m^2$ in size and planted with 4-6 potato plants per $m^2$.

Potatoes were planted in April or May according to local conditions. All plant cultivation management, including plant protection, was performed based on good agricultural practice (GAP). Potato tubers were harvested two weeks after haulm killing in September/October. Harvest was performed either by hand or mechanically. The tuber yield/ha was determined by weighing of the freshly harvested potato tubers at the place of harvest. As control the potato variety Bintje was used. The yield/ha of the standard potato variety Fontane was set to 100% and the relative yield of the non-transgenic line Bintje and the transformed Fontane events A to D was calculated.

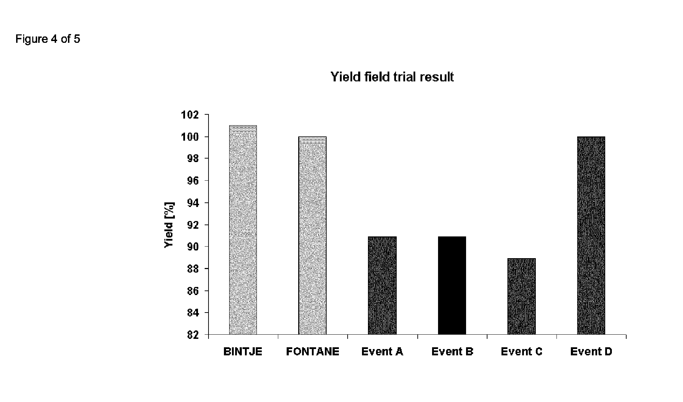

Only the elite event D showed the same yield/ha compared to the non-transgenic mother line, whereas the other events (e.g. A, B, C) showed a ~10% yield decrease (cf. Figure 4).

**Example 14 Determination of Phytophthora resistance by field trials**

The various potato events were tested in the field to determine their resistance phenotype against Phytophthora infestans. All events and the nontransgenic controls (Fontane, as on-transgenic mother line and Bintje as susceptible standard variety) were grown in the field without any fungicide treatments targeting Phytophthora infestans. Resistance trials on more than 20 locations across 5 years (2006-2010) were performed. For every experiment a randomized block design with 3-5 block-repetitions was used. Each block was about 1 -15 $m^2$ in size and planted with 4-6 potato plants per $m^2$.

Potatoes were planted in April or May according to local conditions. All plant cultivation management, excluding plant protection, was performed based on good agricultural practice (GAP). Phytophthora infection occurs naturally.

The rating of disease symptoms was done by trained personal evaluating the diseased leaf area, necrotic lesions, clorotic lesions and potential sporulation of P. infestans.

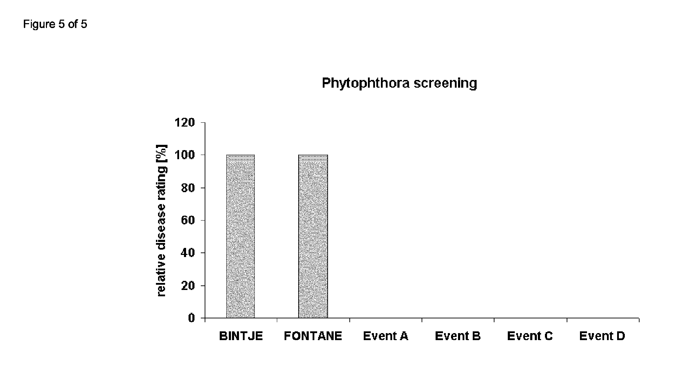

These values were integrated into a disease rating ranging from 0 to 100%. In the scoring system 0% disease means no macroscopically visible symptoms, whereas 100% means that all inoculated leaves are completely brownish and covered with mycelia, so the plant is essentially dead. The mother variety Fontane generally showed a strong infection of all leaves. All leaves are heavily infected and green tissue is rare. In contrast the inoculation of all events A to D led to completely healthy plants with a disease rating of 0%. As susceptible control the standard variety Bintje was used, which is known to be fully susceptible to Phytophthora infection and which showed strong infection (Figure 5).

## Claims

1. Phythophthora-resistant transgenic potato plant, seed, tuber, plant cell or tissue thereof comprising a nucleotide sequence having at least 80 % identity with SEQ-ID-No. 1.

2. Phythophthora-resistant transgenic potato plant, seed, tuber, plant cell or tissue thereof comprising

   a) a recombinant construct having at least 80 % identity with SEQ-ID-No. 2 or SEQ-ID-No. 3 and
   b) further comprising a junction sequence selected from the group consisting of

      i) a nucleic acid sequence that can be used to amplify a nucleotide fragment of between 126 and 136 basepairs using a polymerase chain reaction with two primers having the nucleotide sequences of SEQ-ID-No. 4 and SEQ-ID-No. 5,
      ii) a nucleic acid sequence that can be used to amplify a nucleotide fragment of between 505 and 515 basepairs using a polymerase chain reaction with two primers having the nucleotide sequences of SEQ-ID-No. 6 and SEQ-ID-No. 7,
      iii) a nucleic acid sequence that can be used to amplify a nucleotide fragment of between 625 and 635 basepairs using a polymerase chain reaction with two primers having the nucleotide sequences of SEQ-ID-No. 8 and SEQ-ID-No. 9,
      and/or
      iv) a nucleic acid sequence that can be used to amplify a nucleotide fragment of between 4752 and 4762 basepairs using a polymerase chain reaction with two primers having the nucleotide sequences of SEQ-ID-No. 10 and SEQ-ID-No. 11,
      and/or
      further comprising a junction sequence selected from the group consisting of
      v) a nucleic acid sequence that can be used to amplify a nucleotide fragment of between 282 and 292 basepairs using a polymerase chain reaction with two primers having the nucleotide sequences of SEQ-ID-No. 12 and SEQ-ID-No. 13,
      vi) a nucleic acid sequence that can be used to amplify a nucleotide fragment of between 877 and 887 basepairs using a polymerase chain reaction with two primers having the nucleotide sequences of SEQ-ID-No. 14 and SEQ-ID-No. 15,

vii) a nucleic acid sequence that can be used to amplify a nucleotide fragment of between 827 and 837 basepairs using a polymerase chain reaction with two primers having the nucleotide sequences of SEQ-ID-No. 16 and SEQ-ID-No. 17,
and/or
viii) a nucleic acid sequence that can be used to amplify a nucleotide fragment of between 9905 and 9915 basepairs using a polymerase chain reaction with two primers having the nucleotide sequences of SEQ-ID-No. 18 and SEQ-ID-No. 19.

**3.** Phythophthora-resistant transgenic potato, seed, tuber, plant cell or tissue according to claim 1 comprising

a) a recombinant construct having at least 80 % identity with SEQ-ID-No. 2 or SEQ-ID-No. 3 and further comprising a junction sequence selected from the group consisting of
b)

i) a nucleic acid sequence that can be used to amplify a nucleotide fragment of 131 basepairs using a polymerase chain reaction with two primers having the nucleotide sequences of SEQ-ID-No. 4 and SEQ-ID-No. 5,
ii) a nucleic acid sequence that can be used to amplify a nucleotide fragment 510 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of 630 basepairs using a polymerase chain reaction with two primers having the nucleotide sequences of SEQ-ID-No. 8 and SEQ-ID-No. 9, and/or
iv) a nucleic acid sequence that can be used to amplify a nucleotide fragment of 4757 basepairs using a polymerase chain reaction with two primers having the nucleotide sequences of SEQ-ID-No. 10 and SEQ-ID-No. 11

and/or
further comprising a junction sequence selected from the group consisting of

v) a nucleic acid sequence that can be used to amplify a nucleotide fragment of 287 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of 882 basepairs using a polymerase chain reaction with two primers having the nucleotide sequences of SEQ-ID-No. 14 and SEQ-ID-No. 15,
vii) a nucleic acid sequence that can be used to amplify a nucleotide fragment of 832 basepairs using a polymerase chain reaction with two primers having the nucleotide sequences of SEQ-ID-No. 16 and SEQ-ID-No. 17, and/or
viii) a nucleic acid sequence that can be used to amplify a nucleotide fragment of 9910 basepairs using a polymerase chain reaction with two primers having the nucleotide sequences of SEQ-ID-No. 18 and SEQ-ID-No. 19.

**4.** Method for providing a Phythophthora-resistant transgenic potato plant comprising the following steps:

a) introducing a recombinant nucleic acid having at least 80 % identity with SEQ-ID-No. 2 or SEQ-ID-No. 3 into the genome of potato plant cells,
b) integrating said recombinant nucleic acid into the genome,
c) regenerating plant from said plant cells,
d) selecting plant comprising a nucleic acid having at least 80 % identity with SEQ-ID-No. 2 or SEQ-ID-No. 3 and a junction sequence selected from the group consisting of

i) a nucleic acid sequence that can be used to amplify a nucleotide fragment of between 126 and 136 basepairs using a polymerase chain reaction with two primers having the nucleotide sequences of SEQ-ID-No. 4 and SEQ-ID-No. 5,
ii) a nucleic acid sequence that can be used to amplify a nucleotide fragment of between 505 and 515 basepairs using a polymerase chain reaction with two primers having the nucleotide sequences of SEQ-ID-No. 6 and SEQ-ID-No. 7,
iii) a nucleic acid sequence that can be used to amplify a nucleotide fragment of between 625 and 635 basepairs using a polymerase chain reaction with two primers having the nucleotide sequences of SEQ-ID-No. 8 and SEQ-ID-No. 9,

and/or

iv) a nucleic acid sequence that can be used to amplify a nucleotide fragment of between 4752 and 4762 basepairs, using a polymerase chain reaction with two primers having the nucleotide sequences of SEQ-ID-No. 10 and SEQ-ID-No. 11,

and/or
further comprising a junction sequence selected from the group consisting of

v) a nucleic acid sequence that can be used to amplify a nucleotide fragment of between 282 and 292 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of between 877 and 887 basepairs using a polymerase chain reaction with two primers having the nucleotide sequences of SEQ-ID-No. 14 and SEQ-ID-No. 15,
vii) a nucleic acid sequence that can be used to amplify a nucleotide fragment of between 827 and 837 basepairs using a polymerase chain reaction with two primers having the nucleotide sequences of SEQ-ID-No. 16 and SEQ-ID-No. 17,

and/or

viii) a nucleic acid sequence that can be used to amplify a nucleotide fragment of between 9905 and 9915basepairs using a polymerase chain reaction with two primers having the nucleotide sequences of SEQ-ID-No. 18 and SEQ-ID-No. 19.

**5.** Method for providing a Phythophthora-resistant transgenic potato plant according to claim 4, wherein in step d) a plant is selected comprising a nucleic acid having at least 80 % identity with SEQ-ID-No. 2 or SEQ-ID-No. 3 and a junction sequence selected from the group consisting of

i) a nucleic acid sequence that can be used to amplify a nucleotide fragment of 131 basepairs using a polymerase chain reaction with two primers having the nucleotide sequences of SEQ-ID-No. 4 and SEQ-ID-No. 5,
ii) a nucleic acid sequence that can be used to amplify a nucleotide fragment 510 basepairs,using a polymerase chain reaction with two primers having the nucleotide sequences of SEQ-ID-No. 6 and SEQ-ID-No. 7,
iii) a nucleic acid sequence that can be used to amplify a nucleotide fragment of 630 basepairs using a polymerase chain reaction with two primers having the nucleotide sequencesof SEQ-ID-No. 8 and SEQ-ID-No. 9, and/or
iv) a nucleic acid sequence that can be used to amplify a nucleotide fragment of 4757 basepairs using a polymerase chain reaction with two primers having the nucleotide sequences of SEQ-ID-No. 10 and SEQ-ID-No. 11,
and/or further comprising a junction sequence selected from the group consisting of
v) a nucleic acid sequence that can be used to amplify a nucleotide fragment of 287 basepairs using a polymerase chain reaction with two primers having the nucleotide sequences of SEQ-ID-No. 12 and SEQ-ID-No. 13,
vi) a nucleic acid sequence that can be used to amplify a nucleotide fragment of 882 basepairs using a polymerase chain reaction with two primers having the nucleotide sequences of SEQ-ID-No. 14 and SEQ-ID-No. 15,
vii) a nucleic acid sequence that can be used to amplify a nucleotide fragment of 832 basepairs using a polymerase chain reaction with two primers having the nucleotide sequences of SEQ-ID-No. 16 and SEQ-ID-No. 17, and/or
viii) a nucleic acid sequence that can be used to amplify a nucleotide fragment of 9910 basepairs using a polymerase chain reaction with two primers having the nucleotide sequences of SEQ-ID-No. 18 and SEQ-ID-No. 19.

**6.** Kit comprising the primer pairs for the detection of the specific integration place, selected from the group consisting of

SEQ-ID-No. 4 and 5,
SEQ-ID-No. 6 and 7,
SEQ-ID-No. 8 and 9,

SEQ-1 D-No. 10 and 11,
SEQ-ID-No. 12 and 13,
SEQ-ID-No. 14 and 15,
SEQ-ID-No. 16 and 17, and/or
SEQ-ID-No. 18 and 19.

**7.** Detection method for the detection of the specific integration place comprising

a) isolating a nucleic acid sequence from a potato plant, seed, tuber, plant cell or tissue thereof as a test sample,
b) exposing said test sample, a positive and a negative sample with nucleotide sequence selected from at least one set of primer pairs defined in claim 6 under PCR-conditions, and

i) evaluating the amplification of a nucleotide fragment selected from the group consisting of a nucleotide fragment of between 126 and 136 basepairs when using a polymerase chain reaction with two primers having the nucleotide sequences of SEQ-ID-No. 4 and SEQ-ID-No. 5,
ii) of between 505 and 515 basepairs when using a polymerase chain reaction with two primers having the nucleotide sequences of SEQ-ID-No. 6 and SEQ-ID-No. 7,
iii) of 625 and 635 basepairs when using a polymerase chain reaction with two primers having the nucleotide sequences of SEQ-ID-No. 8 and SEQ-ID-No. 9,

and/or

iv) of 4752 and 4762 basepairs when using a polymerase chain reaction with two primers having the nucleotide sequences of SEQ-ID-No. 10 and SEQ-ID-No. 11

and/or
selected from the group consisting of a nucleotide fragment

v) of between 282 and 292 basepairs when using a polymerase chain reaction with two primers having the nucleotide sequences of SEQ-ID-No. 12 and SEQ-ID-No. 13,
vi) of 877 and 292 basepairs when using a polymerase chain reaction with two primers having the nucleotide sequences of SEQ-ID-No. 14 and SEQ-ID-No. 15,
vii) of 827 and 837 basepairs when using a polymerase chain reaction with two primers having the nucleotide sequence of SEQ-ID-No. 16 and SEQ-ID-No. 17, and/or
vii) a nucleic acid sequence that can be used to amplify a nucleotide fragment of 9905 and 9915 basepairs, using a polymerase chain reaction with two primers having the nucleotide sequences of SEQ-ID-No. 18 and SEQ-ID-No. 19

compared with said positive and negative control.

**8.** Detection method according to claim 7 evaluating the amplification of a nucleotide fragment selected from the group consisting of a nucleotide fragment

i) of 131 basepairs when using a polymerase chain reaction with two primers having the nucleotide sequences of SEQ-ID-No. 4 and SEQ-ID-No. 5,
ii) of 510 basepairs when using a polymerase chain reaction with two primers having he nucleotide sequences of SEQ-ID-No. 6 and SEQ-ID-No. 7,
iii) of 630 basepairs when using a polymerase chain reaction with two primers having the nucleotide sequences of SEQ-ID-No. 8 and SEQ-ID-No. 9, and/or
iv) of 4757 basepairs when using a polymerase chain reaction with two primers having the nucleotide sequences of SEQ-ID-No. 10 and SEQ-ID-No. 11 and/or evaluating the amplification of a nucleotide fragment selected from the group consisting of a nucleotide fragment
selected from the group consisting of a nucleotide fragment
iv) of 287 basepairs when using a polymerase chain reaction with two primers having the nucleotide sequences of SEQ-ID-No. 12 and SEQ-ID-No. 13,
v) of 882 basepairs when using a polymerase chain reaction with two primers having the nucleotide sequences of SEQ-ID-No. 14 and SEQ-ID-No. 15,
vi) of 832 basepairs when using a polymerase chain reaction with two primers having the nucleotide sequences of SEQ-ID-No. 16 and SEQ-ID-No. 17, and/or

vii) a nucleic acid sequences that can be used to amplify a nucleotide fragment of 9910 basepairs, using a polymerase chain reaction with two primers having the nucleotide sequence of SEQ-ID-No. 18 and SEQ-ID-No. 19

compared with said positive and negative control.

**9.** Plant, seed, tuber, plant cell or tissue thereof detectable by the kit of claim 6 or by the detection method of claims 7 or 8.

**10.** Polynucleotide comprising

a) a recombinant nucleic acid having at least 80 % identity with SEQ-ID-No. 2 or SEQ-ID-No. 3 and

b) further comprising a junction sequence selected from the group consisting of

i) a nucleic acid sequence that can be used to amplify a nucleotide fragment of 131 basepairs using a polymerase chain reaction with two primers having the nucleotide sequences of SEQ-ID-No. 4 and SEQ-ID-No. 5,

ii) a nucleic acid sequence that can be used to amplify a nucleotide fragment 510 basepairs using a polymerase chain reaction with two primers having the nucleotide sequences of SEQ-ID-No. 6 and SEQ-ID-No. 7,

iii) a nucleic acid sequence that can be used to amplify a nucleotide fragment of 630 basepairs using a polymerase chain reaction with two primers having the nucleotide sequences of SEQ-ID-No. 8 and SEQ-ID-No. 9, and/or

iv) a nucleic acid sequence that can be used to amplify a nucleotide fragment of 4757 basepairs using a polymerase chain reaction with two primers having the nucleotide sequences of SEQ-ID-No. 10 and SEQ-ID-No. 11

and/or

v) a nucleic acid sequence that can be used to amplify a nucleotide fragment of 287 basepairs using a polymerase chain reaction with two primers having the nucleotide sequences of SEQ-ID-No. 12 and SEQ-ID-No. 13,

vi) a nucleic acid sequence that can be used to amplify a nucleotide fragment of 882 basepairs using a polymerase chain reaction with two primers having the nucleotide sequences of SEQ-ID-No. 14 and SEQ-ID-No. 15,

vii) a nucleic acid sequence that can be used to amplify a nucleotide fragment of 832 basepairs using a polymerase chain reaction with two primers having the nucleotide sequences of SEQ-ID-No. 16 and SEQ-ID-No. 17, and/or

viii) a nucleic acid sequence that can be used to amplify a nucleotide fragment of 9910 basepairs using a polymerase chain reaction with two primers having the nucleotide sequences of SEQ-ID-No. 18 and SEQ-ID-No. 19 stably integrated into a potato plant cell nucleus.

**11.** Polynucleotide comprising a nucleotide sequence having at least 80 % identity SEQ-No. 1 stably integrated into a potato plant cell nucleus.

Figure 1 of 5

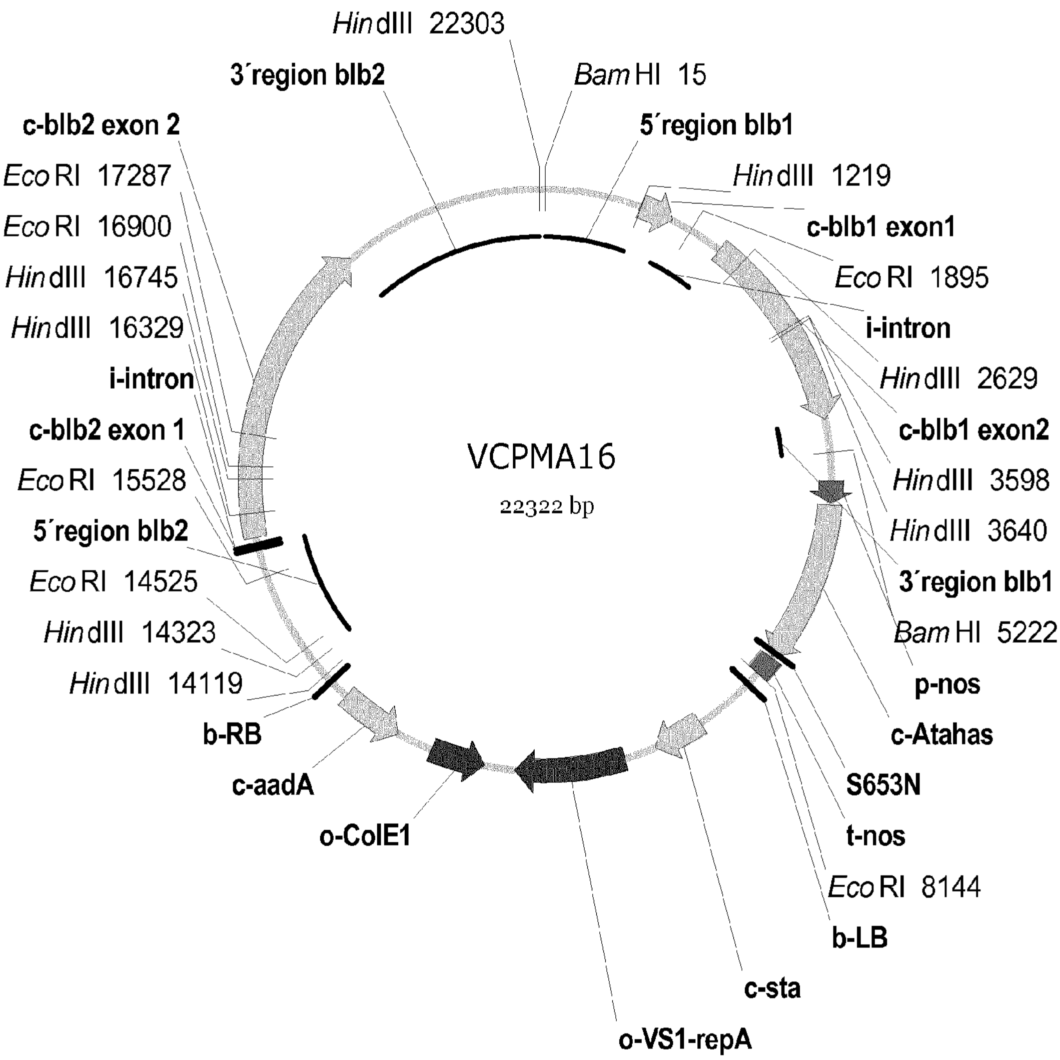
HindIII 22303
3´region blb2
BamHI 15
c-blb2 exon 2
5´region blb1
EcoRI 17287
HindIII 1219
c-blb1 exon1
EcoRI 16900
EcoRI 1895
HindIII 16745
i-intron
HindIII 16329
HindIII 2629
i-intron
c-blb1 exon2
c-blb2 exon 1
VCPMA16
HindIII 3598
EcoRI 15528
22322 bp
HindIII 3640
5´region blb2
3´region blb1
EcoRI 14525
BamHI 5222
HindIII 14323
p-nos
HindIII 14119
c-Atahas
b-RB
S653N
c-aadA
t-nos
o-ColE1
EcoRI 8144
b-LB
c-sta
o-VS1-repA

Figure 2 of 5: Sequences

Figure 2a of 5

**Insert with flanking regions - SEQ-ID-No. 1**

**Lower case bold – flanking region at right border**
**Upper case italic-underlined – Blb2 expression cassette (promoter – gene –terminator)**
**Upper case bold – Blb1 expression cassette (promoter – gene –terminator)**
**Upper case bold italic – AHAS expression cassette (promoter – gene –terminator)**
**Lower case italic – flanking region at left border**

**gaaactcagttggagattacgtttaagggatggcctacatggataaggttatctacaatgatgc**
**aatggagataataatagtactcctatgtgatttcttcgttttaatttgtttattttattttcat**
**ttaatttgatttgatacgtagtttaaaaaaataaaaaagatatttaaattttgtgatgttagat**
**gaaacatacgttaaatgcatcaaaatgtcatttaaatttgtcttgaacatatagtattagaaag**
**ttgaaattaaagagctatcaaaaaagaagaagatatgactaaaaaaaatagcacaagcaaattg**
**aaactaaaagagtaattaacaataacaatttttttttaaaatgatacataatactcactctttc**
**tcaatatgtggcacaaaaatatcaagaatcaaactatttaattttgactataaatttagaaata**
**aatttttttaaatttcttaaaaaacaaaatttacaaagcaaatactatataaaaaatattataaa**
**ttataataattaataattcaaaatagttaaccatacaaactgaattttgggattgagtttctat**
**ttaaattttcgtgagtaaagattaaagttcaaattaacttttttataaaatgagttttcaatttt**
**aaaaagtcaaacaaaatatatatagaatataaagtgagaaaccaaataaaacaagaatagcaag**
**gattatccatttaggaaataagatgaaagaatcaaatcaactaataaaaactttaatcctataa**
**gataagcttgccaataatttaaaatagcatgatgcgtctaagagggtatcaatatattgttatc**
**ctttaaagaccaagatagtgtttcaggaaagttatttcatttccaaatttaaataaaagaaaat**
**aattattcaaaattcttatgtctttttttattgatttatagtcgaagatctttcaaaaataattt**
**ttctataggtaaaaaaggataattt**CAATTTCACACAGGAAACAGCTATGAC*CATGATTACGCC*
*AAGCTGGCGCGCCAAGCTTGCATGCCTGCAGGTCGACTCTAGAGGATCTAGAATCACCGAACCT*
*CCCCTCGGTACAGCTCCTCCAGTTCTACCATGAATTTCATCCACTGATTCCTCTTCAATCGCCA*
*TTGCAGATTCTCTCGATCTATGCTCAAAAAATCCCGAGATAAAACCCTAGATCTGCTTCAAATG*
*CTCTGATACCATGTAATTTCAGTGAATTCTAACTAAACAATGGAGAGAATTAACTATTTTAGAA*
*AGACTGATTGAAGGAGAAGAAGAGAGAAAAATTCTATATTGAACTCATGAACCAAAATGAATGA*
*AAAAAATAATGAGAAGAACTATACTATTACAATCTATATATCTCTATTTATATTCTAATCTGAA*
*GCAGTTAATTTAACTGACTCTAACAACTAGACTGATAGGTGTACATTTTCTGTTAGTGCACTGC*
*AGTGCATTTAACTAACTGCTTAACATAAAGAATGTTGTTCGAACTTCATTCGAATAGCTTCAAT*
*GAGAAGCAAACATGTGTACCTGTAAAGACACACAGTAAAAGTGTTAATAATGAATAAATATGAA*
*TAAATCAAATAATAAATTAAAAATAAAAACACATCCAATTAACATTGGAGGTCTTGAAAATCGA*
*TGGTAATTAACAAAGACCCTTGTGAAATTTAAGTCTGTAATTGAAAATTTGAGTATAGGTTAGG*
*GGACATTTGACTATTTTCTCATTTTCTTTATCTTTTTCCTAATTTGTGGCAGACAAGTGAGGAG*
*GCCCCACTGTAATTGATTCATGCTTTTGCTTTCTTGACTTTTTGGAACAATACTATGCATCATA*
*TTTGGTCTTAATTATTCCTCTGTTTATTTCCAGAATTTTGAGCTCTATACATCTAATAACAAAG*
*CAAGCAGAGGATATATAGTTTCATCAACTAAAAAGGTTAGTCAACTCATCTAATATTTGCTACT*
*CTCATCTCTATTGAAGTACAGTTATGGAAAAGTAGAAGTGATGTAAGAAAAATGAAAGAACTTT*
*AGTAGGTTAGTTGGATCTAACAAAGAGAAAGGGAAATAAATTGCAGGAGAAAGAGAGAGGTTAA*
*ATACTTACTCACACCACCGATTTACAACAAATCACTTAATTGTGGTTAGTTAATGTATACTTTC*
*ACCTCATTAAATTATTACTTACCCATGATAAGTTGTATTAATTTGGTATTAATATCCGGTGCGG*
*GTGAATTCTTACCGGGTGAGAGGGATGGGGTTGGAGAGTGTGGAGTGAACAGAAGCAGATGTTT*
*TAGATTTTTTCTAAGATGACGAAAGATTCCCCTCACTAATGAAAATATATTACTATACGCTATT*
*AGAGATAGAAAGGTTCGGTACCAGTTGGTCTCGTTTCTGGATGAACCCCATTTTTACAAGTCAT*
*TTTCTTCAATTCAAATCGCAAGTGTACCTTTATCATCTTCCACTAATTAAGTCCTCTTAAGTTC*

Figure 2a - continued

GCGTGAAAATAGTGAAATTATTGATTATTCTTATCATTTCATCTTCTTTCTCCTGATAAAGTTT
TATGTACTTTTTATGCATCAGGTCTTGAGAACTTGGAAAGGAAAAGTAGAATCATGGAAAAACG
AAAAGATAATGAAGAAGCAAACAACTCATTGGTATGTTATTTGATAGAGTGAACTGTAAAGTAT
TGAATTGTAGATATCATGTGGCTTTAAAAATTTGATATGTGTTATTTTGGCAGGAGTCATTTTC
TGCTCTTCGCAAGGATGCTGCCAATGTTCTGGATTTCCTAGAGAGATTAAAGAATGAAGAAGAT
CAAAAGGCTGTTGATGTGGATCTGATTGAAAGCCTGAAATTGAAGCTGACATTTATTTGTACAT
ATGTCCAGCTTTCTTATTCCGATTTGGAGAAGTTTGAAGATATAATGACTAGAAAAAGACAAGA
GGTTGAGAATCTGCTTCAACCAATTTTGGATGATGATGGCAAAGACGTCGGGTGTAAATATGTC
CTTACTAGCCTCGCCGGTAATATGGATGACTGTATAAGCTTGTATCATCGTTCTAAATCAGATG
CCACCATGATGGATGAGCAATTGGGCTTCCTCCTCTTGAATCTCTCTCATCTATCCAAGCATCG
TGCTGAAAAGATGTTTCCTGGAGTGACTCAATATGAGGTTCTTCAGAATGTATGTGGCAACATA
AGAGATTTCCATGGATTGATAGTGAATTGTTGCATTAAGCATGAGATGGTTGAGAATGTCTTAT
CTCTGTTTCAACTGATGGCTGAGAGAGTAGGACGCTTCCTTTGGGAGGATCAGGCTGATGAAGA
CTCTCAACTCTCCGAGCTAGATGAGGATGATCAGAATGATAAAGACCCTCAACTCTTCAAGCTA
GCACATCTACTCTTGAAGATTGTTCCAACTGAATTGGAGGTTATGCACATATGTTATAAAACTT
TGAAAGCTTCAACTTCAACAGAAATTGGACGCTTCATTAAGAAGCTCCTGGAAACCTCTCCGGA
CATTCTCAGAGAATATCTGATTCATCTACAAGAGCATATGATAACTGTTATTACCCCTAACACT
TCAGGGGCTCGAAACATTCATGTCATGATGGAATTCCTATTGATTATTCTTTCTGATATGCCGC
CCAAGGACTTTATTCATCATGACAAACTTTTTGATCTCTTGGCTCGTGTTGTAGCACTTACCAG
GGAGGTATCAACTCTTGTACGCGACTTGGAAGAGAAATTAAGGATTAAAGAGAGTACTGACGAA
ACAAATTGTGCAACCCTAAAGTTTCTGGAAAATATTGAACTCCTTAAGGAAGATCTCAAACATG
TTTATCTGAAAGTCCCGGATTCATCTCAATATTGCTTCCCCATGAGTGATGGACCTCTCTTCAT
GCATCTGCTACAGAGACACTTAGATGATTTGCTGGATTCCAATGCTTATTCAATTGCTTTGATA
AAGGAACAAATTGGGCTGGTGAAAGAAGACTTGGAATTCATAAGATCTTTTTTCGCGAATATTG
AGCAAGGATTGTATAAAGATCTCTGGGAACGTGTTCTAGATGTGGCATATGAGGCAAAAGATGT
CATAGATTCAATTATTGTTCGAGATAATGGTCTCTTACATCTTATTTTCTCACTTCCCATTACC
AGAAAGAAGATGATGCTTATCAAAGAAGAGGTCTCTGATTTACATGAGAACATTTCCAAGAACA
GAGGTCTCATCGTTGTGAACTCTCCCAAGAAACCAGTTGAGAGCAAGTCATTGACAACTGATAA
AATAATTGTAGGTTTTGGTGAGGAGACAAACTTGATACTTAGAAAGCTCACCAGTGGACCGGCA
GATCTAGATGTCATTTCGATCATTGGTATGCCGGGTTTAGGTAAAACTACTTTGGCGTACAAAG
TATACAATGATAAATCAGTTTCTAGCCATTTCGACCTTCGTGCATGGTGCACGGTCGACCAAGT
ATATGACGAGAAGAAGTTGTTGGATAAAATTTTCAATCAAGTTAGTGACTCAAATTCAAAATTG
AGTGAGAATATTGATGTTGCTGATAAACTACGGAAACAATTGTTTGGAAAGAGGTATCTTATTG
TCTTAGATGACGTGTGGGATACTAATACATGGGATGAGCTAACAAGACCTTTTCCTGATGGTAT
GAAAGGAAGTAGAATTATTTTGACAACTCGAGAAAAGAAAGTTGCTTTGCATGGAAAGCTCTAC
ACTGATCCTCTTAACCTTCGATTGCTAAGATCAGAAGAAAGTTGGGAGTTATTAGAGAAAAGGG
CATTTGGAAACGAGAGTTGCCCTGATGAACTATTGGATGTTGGTAAAGAAATAGCCGAAAATTG
TAAAGGGCTTCCTTTGGTGGTGGATCTGATTGCTGGAATCATTGCTGGGAGGGAAAAGAAAAAG
AGTGTGTGGCTTGAAGTTGTAAATAATTTGCATTCCTTTATTTTGAAGAATGAAGTGGAAGTGA
TGAAAGTTATAGAAATAAGTTATGACCACTTACCTGATCACCTGAAGCCATGCTTGCTGTACTT
TGCAAGTGCGCCGAAGGACTGGGTAACGACAATCCATGAGTTGAAACTTATTTGGGGTTTTGAA
GGATTTGTGGAAAAGACAGATATGAAGAGTCTGGAAGAAGTGGTGAAAATTTATTTGGATGATT
TAATTTCCAGTAGCTTGGTAATTTGTTTCAATGAGATAGGTGATTACCCTACTTGCCAACTTCA
TGATCTTGTGCATGACTTTTGTTTGATAAAAGCAAGAAAGGAAAAGTTGTGTGATCGGATAAGT
TCAAGTGCTCCATCAGATTTGTTGCCACGTCAAATTAGCATTGATTATGATGATGATGAAGAGC
ACTTTGGGCTTAATTTTGTCCTGTTCGGTTCAAATAAGAAAAGGCATTCCGGTAAACACCTCTA
TTCTTTGACCATAAATGGAGATGAGCTGGACGACCATCTTTCTGATACATTTCATCTAAGACAC
TTGAGGCTTCTTAGAACCTTGCACCTGGAATCCTCTTTTATCATGGTTAAAGATTCTTTGCTGA
ATGAAATATGCATGTTGAATCATTTGAGGTACTTAAGCATTGGGACAGAAGTTAAATCTCTGCC

Figure 2a - continued

*TTTGTCTTTCTCAAACCTCTGGAATCTAGAAATCTTGTTTGTGGATAACAAAGAATCAACCTTG*
*ATACTATTACCGAGAATTTGGGATCTTGTAAAGTTGCAAGTGCTGTTCACGACTGCTTGTTCTT*
*TCTTTGATATGGATGCAGATGAATCAATACTGATAGCAGAGGACACAAAGTTAGAGAACTTGAC*
*AGCATTAGGGGAACTCGTGCTTTCCTATTGGAAAGATACAGAGGATATTTTCAAAAGGCTTCCC*
*AATCTTCAAGTGCTTCATTTCAAACTCAAGGAGTCATGGGATTATTCAACAGAGCAATATTGGT*
*TCCCGAAATTGGATTTCCTAACTGAACTAGAAAAACTCACTGTAGATTTTGAAAGATCAAACAC*
*AAATGACAGTGGGTCCTCTGCAGCCATAAATCGGCCATGGGATTTTCACTTTCCTTCGAGTTTG*
*AAAAGATTGCAATTGCATGAATTTCCTCTGACATCCGATTCACTATCAACAATAGCGAGACTGC*
*TGAACCTTGAAGAGTTGTACCTTTATCGTACAATCATCCATGGGGAAGAATGGAACATGGGAGA*
*AGAAGACACCTTTGAGAATCTCAAATGTTTGATGTTGAGTCAAGTGATTCTTTCCAAGTGGGAG*
*GTTGGAGAGGAATCTTTTCCCACGCTTGAGAAATTAGAACTGTCGGACTGTCATAATCTTGAGG*
*AGATTCCGTCTAGTTTTGGGGATATTTATTCCTTGAAAATTATCGAACTTGTAAGGAGCCCTCA*
*ACTTGAAAATTCCGCTCTCAAGATTAAGGAATATGCTGAAGATATGAGGGGAGGGGACGAGCTT*
*CAGATCCTTGGCCAGAAGGATATCCCGTTATTTAAGTAGTTTTTGAGCATTATGGTTGAAAAGT*
*AGATTGCACTTTGCTGGGTAGATTGTATATGGTTAAGAAAATTCTGTTACAGTTGTTATGAAAC*
*ATTTTTATTTGACTTTTCTGAGTTTCTTTTAGAAAACTCAGAAGTTTTTAACAAAAAATTATAGT*
*TTTTATAAATACAATGTGGATTTGCCTTTGGCTGTCCAACTTGGTCTGAAGTCTCATATGCTCA*
*GAGCACTATCGTTCAACCTCAATCAAGGTACTGATTTAAAATGACATCTATACTACTTTATCAC*
*AAACCCAACGAACTTTCATCTCAAAAGCTAGGCCAGGAAGTGAAGAGGTTGTAGAGAGCTTATA*
*AGCACTCATGACTTCCTTTTCTCGAACATTCAACCAACGTAGGCTGAAATCCCACTCTGAACGA*
*AAATAAGTGTTTGTTTATCAAATTAACTCTCGTAGTAGAACACTGAAATACCTTCTTCTAAACG*
*TTCAACAAATGGGATTTCCAGCACTCAAAGTGAATGAAAGGTTCACATTAATCTTCAAAAAGAA*
*TTACGACAATTCATGACCACAAGTACATTGACAGCACCATTTCAACAGAAGAACAAGTCAATGC*
*TGCATCTTCATCAATAATCCGAGTGTCGAACCTCCTTCCTGACACTGTCCTGTATATGTAAAGT*
*TTCTCAACAGGGCAACTTTCTGGTCTCGTATCTGGATGACCCCTCTCGTCTATAACTTCAACAT*
*TAAGCCCTGGCAACTTCTGGACCAACAGCTTACATGCTTCAAAACTTACTGAACAATTAGACAT*
*CCAAAGGGATCGCATTGTCTCCAGCTTTGCAGCATTAGCCAACAGAGCCTCATCGCCAAAGGGG*
*CAGTCTCTAATCTCGAATTTGAAAAAATTGTTGTTGTATGACTTTCCTCTGACATCCGATGCAC*
*TATCAACAATAGCAAGACTGGAGGTTGGAGAGGAATCCTTTATTATACAATCATTCAGGGAGAA*
*GAATGGAACATGGGGGAGGAAGACACTTTTGAGAATCTGAAATGTGTTAGAGCCACAAGCTACA*
*GAAGTATTGAATTTGTCATGAATATCAACATTCTTCATCCTAGTTAATTCTTTTTCAATTTTTA*
*ATAGACTCTCATTTTAATCACTAATATTCTTCTATTTGTGACTTCTTTTCTGCAGGTGGCAACT*
*TTAAATTCATAAAGTATAGGATTGATGACAAACTCGAAAAATATCTTAATGAGGTGAAGTTTGA*
*GCAGTCAGCAGATGGTGGTTCCAACTCTAAGTTGACAAGCACATACTATCCCGGAGGGCGATTT*
*CAAGCCTGATGCATATGGTTAGTGTGGCTAGAGCAGACAGGATGTATTACCTGGATATCTACCA*
*AGACGAATCCACAATCAGTTTTATGTCAAGCAATACATGAAGTAACTCCCGATAGAACAGTAAA*
*AGCAAGATGTGTAGGTGTATCTCGACTCTAAGAGATTGTACATTCCTCTTTGAGATTTTTACTG*
*CTAATACAAATTTACACCTCAGAAGCGAATCTAGAATTTCTAGAGCATGAATGCACCACTAATG*
*AAAGGAGAAAAAAGGAAGTATGAAGTGGGAATTTGATCCTTGTTTCTAGGTATATAAAATTTAT*
*CATTCAACTATACTTCATTTAGCAAACAACTCTCTTTGCCATTATTTCTCAAACAAGGGCTTCT*
*AATATTGCTAAACTAAAGACTGTCAAAAGGTAAGTTCATCTTCAAACTCTCTTGTTTACTTTAT*
*CTAAAGGGGAACTATGAAAAACAAGAAACATCAGGAATGTCCCGTAAACAAAGCAGCCTCATGC*
*ACAAAACATCCAACGTTGGTAGGATTAATGGAGGGATCGCATCCCAGGAGGATACTGTAGAAAA*
*ATTAGTGGCTTCTTTCACCGCTCAAACCCATGATCTATAGGTTACATGGAGACAACTTTATGGT*
*TGCTCGTAGGCTCCCGTCAATTCTCATAAACCACAACACCAAAGTTGCATCAGACATCATCTTC*
*ATTCACAAGCTGACAATCTCCACAAGTCTTAGTCAACTTGTAATATGAATATTAGCCAGGTAGA*
*CGTACATATTTACAAAATTGAGTTTCCTATATAATATGGTTTGAAGGAATGAAACATGATGGGG*
*AGGGTAGATAAAATAATATATGAGGCATAAAAATAGGAAAGATATTTGTAGTGAGAGGTTTTGA*
*CTTTTTATGCTGCTTTTGATCTTCAGTTTCTTGTATTCTTTTTCTACTGCTTTCCTCTTCTTTC*

Figure 2a - continued

*TCCTGAGTAAAGTTTTATGTAGGTACTTTTTATACGTCCGATCGTGAGAACTTGAAAGAAAGCT*
*CTCTATAGCTATGTTAGGTGCCCACATAAAAAAATGAAATATTACAAAAACCCTGATAATAAAA*
*TACACTAATCTAAGATATTCACTGCAACATACATGCAAAATATATATATATAAATTTTCATGAA*
*AATTATAACAAATAATAGATGTGAACATATAACTTTAAAAATAATATTACATCCATAAAGCTTA*
*AATTCTAGATCCCCGGTCGACTCTAGAGGATCCCCACTCCATCCGTTCACT***TTGATTTGTCATG**
**TTGCACTTTTCGAAAGTCAATTTGACTAATTTTTAAAGCTAAATTAGATTACACTAATTCAATA**
**TTTTAAACAGAAAAATTAGATATTCAAAAACTATACAAAAAATATTATACATTGCAATTTTTTG**
**CATATCAATATGATAAAAAAATATATCGTAAAATATTAGTCAAAATTTTTATAATTTGACTCAA**
**ATCATGAAAAGTATAATAATTAATAGTGGACGGAGGAAGTATTGTCTTTCCAGATTTGTGGCCA**
**TTTTTGGTCCAAGGGCCATTAGCAGTTCTCTTCATTTTCTACTTCTGTCTCATATTAGATGGGC**
**ATCTTACTAAAAATATTTGTCTCATATTACTTGATTATTTATTAAATCAAAAAGAATTAATTAA**
**TTTTTTCTCATTTTACCCCTACAATTAATATAGTTTTAAAAGTTTTAAACAAATTTTGAAGAAT**
**CAAAATTTCTTTTTGCAAGAGACTTATTAATATAAACAAAGGATAAAATAATAAAATTTGTCAA**
**TTTATTGACGATCACTTAATAATCATATAAAATAGAAAATGTTTATCTAATATGAGACGGAGAA**
**AATATATCCTAAAATATTTTTGGACAGATATGTGATATTCTAACCATTCACTAGACTATATTAT**
**GCATTTTAGCCGCCAATGACTTATTTCAGCTTTAATTAATTAGGAAAGAGGAAACTGCCAATGA**
**GGAAGAGTAGGGGCGTAGTTGCTGTCGACGAAAAAAAGATAATACTCACTCTTTTCGATTTTTA**
**TTTTTATTTATCACTTTTAACCTATCATGTAAAAAGATAATTATTTTTTTCATGCTTTATCCTT**
**AGTATTAAACAATTTAATAGGGATTATTTTGTAAAATATTTATATGAATAATTGTTTTCGTAAT**
**GAATTTGTCCGGTCAAACAATGATAAATAAAAATGAATGAAGAGAGTAGAAAACAAAACAAAAG**
**AACAAGTTGACAACTTGAGAGATTAAAAGGGTCCAAAACGCCTTGGATTTTGAGATTCCATATG**
**TGAAATTTCCATGAAATAATTGAATTTGTATTATTACAAGTCAAACTTTCCATTTCATTCCAAC**
**TAGCCATCTTGGTTTCAAAATTACACATTCATTCATTCACAGATCTAATATTCTTAATAGTGAT**
**TTCCACATATGGCTGAAGCTTTCATTCAAGTTCTGCTAGACAATCTCACTTCTTTCCTCAAAGG**
**GGAACTTGTATTGCTTTTCGGTTTTCAAGATGAGTTCCAAAGGCTTTCAAGCATGTTTTCTACA**
**ATTCAAGCCGTCCTTGAAGATGCTCAGGAGAAGCAACTCAACAACAAGCCTCTAGAAAATTGGT**
**TGCAAAAACTCAATGCTGCTACATATGAAGTCGATGACATCTTGGATGAATATAAAACCAAGGC**
**CACAAGATTCTCCCAGTCTGAATATGGCCGTTATCATCCAAAGGTTATCCCTTTCCGTCACAAG**
**GTCGGGAAAAGGATGGACCAAGTGATGAAAAAACTAAAGGCAATTGCTGAGGAAAGAAAGAATT**
**TTCATTTGCACGAAAAAATTGTAGAGAGACAAGCTGTTAGACGGGAAACAGGTACTCATCTTAA**
**ATTAGTATTACAACAACTAAGTTTATATTCATTTTTTTGGCAATTATCAAATTCAGAAAAGGGT**
**TAAATATACTCATGTCCTATCGTAAATAGTGTATATATACCTCTCGTTGTACTTTCGATCTGAA**
**TATACTTGTCAAATCTGGCAAGCTCAGAATCAAATTATCCACCCCAACTTTTAAATACTCGATA**
**TCTTTAGAAATCCACCTGTCTAACTCATCCACTACCCATTCCCTTTGCTTTGAATTCTTTTCTT**
**TACCTATAAACTTGGAACACTCGATCCGTTTTGCTTTTCTTAACAAAGCAGCTCAGAGAAAAGA**
**GGTTTTCTTCTATTCTGTTTCTCTGTGTGCTGCACTTGGGTCCTTAATCCCATTAAAAACAGGG**
**CATGTTAATCCCAACGACGGTAGCCTTTCCTGACAGCTGACTGTAAATTTTGTCTAACAAAGAA**
**AAAAAAAGATTAGACATGTTTTTCCTTGTCATTGATTAGGCTGGATTTCTTTCAGAGTGGAACA**
**TAGGGGATATATTGGACCAAAAGTAGAATGGGTATATATTTAAAGTATTTCTGATAGAACAGGA**
**GTATATTGTGCGAAAATATCCTCTATTTTCTGTTGTCTCCTAATGAGTTTGAATGTAATAATAT**
**TCTCATGTGGACATTGCTTGCACCAGGTTCTGTATTAACCGAACCGCAGGTTTATGGAAGAGAC**
**AAAGAGAAAGATGAGATAGTGAAAATCCTAATAAACAATGTTAGTGATGCCCAACACCTTTCAG**
**TCCTCCCAATACTTGGTATGGGGGATTAGGAAAAACGACTCTTGCCCAAATGGTCTTCAATGA**
**CCAGAGAGTTACTGAGCATTTCCATTCCAAAATATGGATTTGTGTCTCGGAAGATTTTGATGAG**
**AAGAGGTTAATAAAGGCAATTGTAGAATCTATTGAAGGAAGGCCACTACTTGGTGAGATGGACT**
**TGGCTCCACTTCAAAAGAAGCTTCAGGAGTTGCTGAATGGAAAAAGATACTTGCTTGTCTTAGA**
**TGATGTTTGGAATGAAGATCAACAGAAGTGGGCTAATTTAAGAGCAGTCTTGAAGGTTGGAGCA**
**AGTGGTGCTTCTGTTCTAACCACTACTCGTCTTGAAAAGGTTGGATCAATTATGGGAACATTGC**
**AACCATATGAACTGTCAAATCTGTCTCAAGAAGATTGTTGGTTGTTGTTCATGCAACGTGCATT**

Figure 2a - continued

**TGGACACCAAGAAGAAATAAATCCAAACCTTGTGGCAATCGGAAAGGAGATTGTGAAAAAAAGT**
**GGTGGTGTGCCTCTAGCAGCCAAAACTCTTGGAGGTATTTTGTGCTTCAAGAGAGAAGAAAGAG**
**CATGGGAACATGTGAGAGACAGTCCGATTTGGAATTTGCCTCAAGATGAAAGTTCTATTCTGCC**
**TGCCCTGAGGCTTAGTTACCATCAACTTCCACTTGATTTGAAACAATGCTTTGCGTATTGTGCG**
**GTGTTCCCAAAGGATGCCAAAATGGAAAAAGAAAAGCTAATCTCTCTCTGGATGGCGCATGGTT**
**TTCTTTTATCAAAAGGAAACATGGAGCTAGAGGATGTGGGCGATGAAGTATGGAAAGAATTATA**
**CTTGAGGTCTTTTTTCCAAGAGATTGAAGTTAAAGATGGTAAAACTTATTTCAAGATGCATGAT**
**CTCATCCATGATTTGGCAACATCTCTGTTTTCAGCAAACACATCAAGCAGCAATATCCGTGAAA**
**TAAATAAACACAGTTACACACATATGATGTCCATTGGTTTCGCCGAAGTGGTGTTTTTTTACAC**
**TCTTCCCCCCTTGGAAAAGTTTATCTCGTTAAGAGTGCTTAATCTAGGTGATTCGACATTTAAT**
**AAGTTACCATCTTCCATTGGAGATCTAGTACATTTAAGATACTTGAACCTGTATGGCAGTGGCA**
**TGCGTAGTCTTCCAAAGCAGTTATGCAAGCTTCAAAATCTGCAAACTCTTGATCTACAATATTG**
**CACCAAGCTTTGTTGTTTGCCAAAAGAAACAAGTAAACTTGGTAGTCTCCGAAATCTTTTACTT**
**GATGGTAGCCAGTCATTGACTTGTATGCCACCAAGGATAGGATCATTGACATGCCTTAAGACTC**
**TAGGTCAATTTGTTGTTGGAAGGAAGAAAGGTTATCAACTTGGTGAACTAGGAAACCTAAATCT**
**CTATGGCTCAATTAAAATCTCGCATCTTGAGAGAGTGAAGAATGATAAGGACGCAAAAGAAGCC**
**AATTTATCTGCAAAAGGGAATCTGCATTCTTTAAGCATGAGTTGGAATAACTTTGGACCACATA**
**TATATGAATCAGAAGAAGTTAAAGTGCTTGAAGCCCTCAAACCACACTCCAATCTGACTTCTTT**
**AAAAATCTATGGCTTCAGAGGAATCCATCTCCCAGAGTGGATGAATCACTCAGTATTGAAAAAT**
**ATTGTCTCTATTCTAATTAGCAACTTCAGAAACTGCTCATGCTTACCACCCTTTGGTGATCTGC**
**CTTGTCTAGAAAGTCTAGAGTTACACTGGGGGTCTGCGGATGTGGAGTATGTTGAAGAAGTGGA**
**TATTGATGTTCATTCTGGATTCCCCACAAGAATAAGGTTTCCATCCTTGAGGAAACTTGATATA**
**TGGGACTTTGGTAGTCTGAAAGGATTGCTGAAAAAGGAAGGAGAAGAGCAATTCCCTGTGCTTG**
**AAGAGATGATAATTCACGAGTGCCCTTTTCTGACCCTTTCTTCTAATCTTAGGGCTCTTACTTC**
**CCTCAGAATTTGCTATAATAAAGTAGCTACTTCATTCCCAGAAGAGATGTTCAAAAACCTTGCA**
**AATCTCAAATACTTGACAATCTCTCGGTGCAATAATCTCAAAGAGCTGCCTACCAGCTTGGCTA**
**GTCTGAATGCTTTGAAAAGTCTAAAAATTCAATTGTGTTGCGCACTAGAGAGTCTCCCTGAGGA**
**AGGGCTGGAAGGTTTATCTTCACTCACAGAGTTATTTGTTGAACACTGTAACATGCTAAAATGT**
**TTACCAGAGGGATTGCAGCACCTAACAACCCTCACAAGTTTAAAAATTCGGGGATGTCCACAAC**
**TGATCAAGCGGTGTGAGAAGGGAATAGGAGAAGACTGGCACAAAATTTCTCACATTCCTAATGT**
**GAATATATATATTTAAGTTATTTGCTATTGTTTCTTTGTTTGTGAGTCTTTTTGGTTCCTGCCA**
**TTGTGATTGCATGTAATTTTTTTCTAGGGTTGTTTCTTTATGAGTCTCTCTCTCATTGGATGTA**
**ATTTTCTTTTGGAAACAAATCTGTCAATTGATTTGTATTATACGCTTTCAGAATCTATTACTTA**
**TTTGTAATTGTTTCTTTGTTTGTAAATTGTGAGTATCTTATTTTATGGAATTTTCTGATTTTAT**
**TTTGAAAACAAATCAATGATTTGTAAGATCCATCTGTATTATACTCCCTTCGTCTCATTTTATG**
**TGTCACCTGTCGGATTTCGAGATTCAAACAAATCTATCTTTGATCGTAAATTTTTAATAGATCT**
**TTTAAACATTTTGAATTATCAATTATTGTGACTTTAGT***GGCTAGACTAGTGGATCCGATATCGC*
*CCAGCTTCACGCTGCCGCAAGCACTCAGGGCGCAAGGGCTGCTAAAGGAAGCGGAACACGTAGA*
*AAGCCAGTCCGCAGAAACGGTGCTGACCCCGGATGAATGTCAGCTACTGGGCTATCTGGACAAG*
*GGAAAACGCAAGCGCAAAGAGAAAGCAGGTAGCTTGCAGTGGGCTTACATGGCGATAGCTAGAC*
*TGGGCGGTTTTATGGACAGCAAGCGAACCGGAATTGCCAGCTGGGGCGCCCTCTGGTAAGGTTG*
*GGAAGCCCTGCAAAGTAAACTGGATGGCTTTCTTGCCGCCAAGGATCTGATGGCGCAGGGGATC*
*AA****GATCATGAGCGGAGAATTAAGGGAGTCACGTTATGACCCCCGCCGATGACGCGGACAAGCC***
***GTTTTACGTTTGGAACTGACAGAACCGCAACGTTGAAGGAGCCACTCAGCCGCGGGTTTCTGGA***
***GTTTAATGAGCTAAGCACATACGTCAGAAACCATTATTGCGCGTTCAAAAGTCGCCTAAGGTCA***
***CTATCAGCTAGCAAATATTTCTTGTCAAAAATGCTCCACTGACGTTCCATAAATTCCCCTCGGT***
***ATCCAATTAGAGTCTCATATTCACTCTCAATCCAGATCCCCGGGTACCATGGCGGCGGCAACAA***
***CAACAACAACAACATCTTCTTCGATCTCCTTCTCCACCAAACCATCTCCTTCCTCCTCCAAATC***
***ACCATTACCAATCTCCAGATTCTCCCTCCCATTCTCCCTAAACCCCAACAAATCATCCTCCTCC***

Figure 2a - continued

TCCCGCCGCCGCGGTATCAAATCCAGCTCTCCCTCCTCCATCTCCGCCGTGCTCAACACAACCA
CCAATGTCACAACCACTCCCTCTCCAACCAAACCTACCAAACCCGAAACATTCATCTCCCGATT
CGCTCCAGATCAACCCCGCAAAGGCGCTGATATTCTCGTCGAGGCTTTAGAACGTCAAGGCGTA
GAAACCGTATTCGCTTACCCTGGAGGTGCATCAATGGAGATTCACCAAGCCTTAACCCGCTCTT
CCTCAATCCGTAACGTCCTTCCTCGTCACGAACAAGGAGGTGTATTCGCAGCAGAAGGATACGC
TCGATCCTCAGGTAAACCAGGTATCTGTATAGCCACTTCAGGTCCCGGAGCTACAAATCTCGTT
AGCGGATTAGCCGATGCGTTGTTAGATAGTGTTCCTCTTGTAGCAATCACAGGACAAGTCCCTC
GTCGTATGATTGGTACAGATGCGTTTCAAGAGACTCCGATTGTTGAGGTAACGCGTTCGATTAC
GAAGCATAACTATCTTGTGATGGATGTTGAAGATATTCCTAGGATTATTGAGGAGGCTTTCTTT
TTAGCTACTTCTGGTAGACCTGGACCTGTTTTGGTTGATGTTCCTAAAGATATTCAACAACAGC
TTGCGATTCCTAATTGGGAACAGGCTATGAGATTACCTGGTTATATGTCTAGGATGCCTAAACC
TCCGGAAGATTCTCATTTGGAGCAGATTGTTAGGTTGATTTCTGAGTCTAAGAAGCCTGTGTTG
TATGTTGGTGGTGGTTGTTTGAACTCTAGCGATGAATTGGGTAGGTTTGTTGAGCTTACGGGAA
TCCCTGTTGCGAGTACGTTGATGGGGCTGGGATCTTATCCTTGTGATGATGAGTTGTCGTTACA
TATGCTTGGAATGCATGGGACTGTGTATGCAAATTACGCTGTGGAGCATAGTGATTTGTTGTTG
GCGTTTGGGGTAAGGTTTGATGATCGTGTCACGGGTAAACTTGAGGCTTTTGCTAGTAGGGCTA
AGATTGTTCATATTGATATTGACTCGGCTGAGATTGGGAAGAATAAGACTCCTCATGTGTCTGT
GTGTGGTGATGTTAAGCTGGCTTTGCAAGGGATGAATAAGGTTCTTGAGAACCGAGCGGAGGAG
CTTAAACTTGATTTTGGAGTTTGGAGGAATGAGTTGAACGTACAGAAACAGAAGTTTCCGTTGA
GCTTTAAGACGTTTGGGGAAGCTATTCCTCCACAGTATGCGATTAAGGTCCTTGATGAGTTGAC
TGATGGAAAAGCCATAATAAGTACTGGTGTCGGGCAACATCAAATGTGGGCGGCGCAGTTCTAC
AATTACAAGAAACCAAGGCAGTGGCTATCATCAGGAGGCCTTGGAGCTATGGGATTTGGACTTC
CTGCTGCGATTGGAGCGTCTGTTGCTAACCCTGATGCGATAGTTGTGGATATTGACGGAGATGG
AAGTTTTATAATGAATGTGCAAGAGCTAGCCACTATTCGTGTAGAGAATCTTCCAGTGAAGGTA
CTTTTATTAAACAACCAGCATCTTGGCATGGTTATGCAATGGGAAGATCGGTTCTACAAAGCTA
ACCGAGCACACACATTTCTCGGAGATCCGGCTCAGGAGGACGAGATATTCCCGAACATGTTGCT
GTTTGCAGCAGCTTGCGGGATTCCAGCGGCGAGGGTGACAAAGAAAGCAGATCTCCGAGAAGCT
ATTCAGACAATGCTGGATACACCAGGACCTTACCTGTTGGATGTGATTTGTCCGCACCAAGAAC
ATGTGTTGCCGATGATCCCGAATGGTGGCACTTTCAACGATGTCATAACGGAAGGAGATGGCCG
GATTAAATACTGAGAGCTCGAATTTCCCCGATCGTTCAAACATTTGGCAATAAAGTTTCTTAAG
ATTGAATCCTGTTGCCGGTCTTGCGATGATTATCATATAATTTCTGTTGAATTACGTTAAGCAT
GTAATAATTAACATGTAATGCATGACGTTATTTATGAGATGGGTTTTTATGATTAGAGTCCCGC
AATTATACATTTAATACGCGATAGAAAACAAAATATAGCGCGCAAACTAGGATAAATTATCGCG
CGCGGTGTCATCTATGTTACTAGATCGGGAATTCACTGGCCGTCGTTTTACAACGACTCAGCTG
CTTGGTAATAATTGTCATTAGATTGTTTTTATGCATAGATGCACTCGAAATCAGCCAATTTTAG
ACAAGTATCAAACGGATGTTAATTCAGTACATTAAAGACGTCCGCAATGTGTTATTAAGTTGTC
TAAGttgtccttggttttattagttgttattgttgttttgtcttttcaatttctagtagtcaat
tgggaactggaagtacaaagttgaattttagacttatgtatacaaattgaaaatgtatcaaaa
agtagtgacacttataaaagaacaattctttcttctgagaacatcttcaacccacctctattt
tactctccattctctatatttagtgagtaaaatagagaatgggcactccaacccacctccatat
cactctccattcttcatatttagagagtcatattatttttattattatttttattactttctaa
ttaatatattattttacatataatgtcattaattaaatatctaatattcataattcttttaaa
tgtcatattatattttaaaaatatattatttaatatatactactttcatcccgaattaatagt
attttaatttttctaattttcgacaataatataatttgattttattattaattttcactataa
ataatacacaaaattaaaatgataagatgaaaaattaatttaaaaatacaatacataatatga
taatttaaatacaggataacaatacaatacatgacataataatttaaatacaagataaaataca
atacataacataataatcaattcgtgatgaaacaagaatcatgccaaaaagatattgaacgtgc
attcgaagttttgcaatcacgttttgcaattattgcaagaccgtcacgttttggagaaaggaa
gtgtgacatgatataatgactacatgtattatactgcacaccatgataattgaggatgaacatg

Figure 2a - continued

*atcttaatgcaccaaatcaagatgccgtagaggctccaactccaacgacaaaaatgatggtaga
tgaaaatcttcggtttgaacaatttttagctagacataaaaaagttaaggacaaaaatgctcat
tttgaactccgtaatgcattaatagagcatttatgacagcaacgtgataatttcgaaacttgag
tgtttatgtaattatatttcacttttatttgaatttgtccattaatttgtatattatataatat
ttatttacaatttatgttatttaaaaatttagaataaaataaaattttgaaaaaataaaatttt
atatcacatgaaaattatataaagtaattattggggaaaaaaataaaggatttatattatgaaa
taagaaaataagaatgaatagaaataataataatataatattgaggagaaataattatttttttt
tagagagtgaaatagagaattgggttgaaattgattgtctgaaaaaataaaaaactctatattt
ggagagtaaaatagagtgtagggttggagacgtca*

Figure 2b of 5

**Insert without flanking region - SEQ-ID-No. 2**

**Upper case italic-underlined – Blb2 expression cassette (promoter – gene –terminator)**
**Upper case bold – Blb1 expression cassette (promoter – gene –terminator)**
**Upper case bold italic – AHAS expression cassette (promoter – gene –terminator)**

CAATTTCACACAGGAAACAGCTATGAC*CATGATTACGCCAAGCTGGCGCGCCAAGCTTGCATGC*
*CTGCAGGTCGACTCTAGAGGATCTAGAATCACCGAACCTCCCCTCGGTACAGCTCCTCCAGTTC*
*TACCATGAATTTCATCCACTGATTCCTCTTCAATCGCCATTGCAGATTCTCTCGATCTATGCTC*
*AAAAAATCCCGAGATAAAACCCTAGATCTGCTTCAAATGCTCTGATACCATGTAATTTCAGTGA*
*ATTCTAACTAAACAATGGAGAGAATTAACTATTTTAGAAAGACTGATTGAAGGAGAAGAAGAGA*
*GAAAAATTCTATATTGAACTCATGAACCAAAATGAATGAAAAAAATAATGAGAAGAACTATACT*
*ATTACAATCTATATATCTCTATTTATATTCTAATCTGAAGCAGTTAATTTAACTGACTCTAACA*
*ACTAGACTGATAGGTGTACATTTTCTGTTAGTGCACTGCAGTGCATTTAACTAACTGCTTAACA*
*TAAAGAATGTTGTTCGAACTTCATTCGAATAGCTTCAATGAGAAGCAAACATGTGTACCTGTAA*
*AGACACACAGTAAAAGTGTTAATAATGAATAAATATGAATAAATCAAATAATAAATTAAAAATA*
*AAAACACATCCAATTAACATTGGAGGTCTTGAAAATCGATGGTAATTAACAAAGACCCTTGTGA*
*AATTTAAGTCTGTAATTGAAAATTTGAGTATAGGTTAGGGGACATTTGACTATTTTCTCATTTT*
*CTTTATCTTTTTCCTAATTTGTGGCAGACAAGTGAGGAGGCCCCACTGTAATTGATTCATGCTT*
*TTGCTTTCTTGACTTTTTGGAACAATACTATGCATCATATTTGGTCTTAATTATTCCTCTGTTT*
*ATTTCCAGAATTTTGAGCTCTATACATCTAATAACAAAGCAAGCAGAGGATATATAGTTTCATC*
*AACTAAAAAGGTTAGTCAACTCATCTAATATTTGCTACTCTCATCTCTATTGAAGTACAGTTAT*
*GGAAAAGTAGAAGTGATGTAAGAAAAATGAAAGAACTTTAGTAGGTTAGTTGGATCTAACAAAG*
*AGAAAGGGAAATAAATTGCAGGAGAAAGAGAGAGGTTAAATACTTACTCACACCACCGATTTAC*
*AACAAATCACTTAATTGTGGTTAGTTAATGTATACTTTCACCTCATTAAATTATTACTTACCCA*
*TGATAAGTTGTATTAATTTGGTATTAATATCCGGTGCGGGTGAATTCTTACCGGGTGAGAGGGA*
*TGGGGTTGGAGAGTGTGGAGTGAACAGAAGCAGATGTTTTAGATTTTTTCTAAGATGACGAAAG*
*ATTCCCCTCACTAATGAAAATATATTACTATACGCTATTAGAGATAGAAAGGTTCGGTACCAGT*
*TGGTCTCGTTTCTGGATGAACCCCATTTTTACAAGTCATTTTCTTCAATTCAAATCGCAAGTGT*
*ACCTTTATCATCTTCCACTAATTAAGTCCTCTTAAGTTCGCGTGAAAATAGTGAAATTATTGAT*
*TATTCTTATCATTTCATCTTCTTTCTCCTGATAAAGTTTTATGTACTTTTTATGCATCAGGTCT*
*TGAGAACTTGGAAAGGAAAAGTAGAATCATGGAAAAACGAAAAGATAATGAAGAAGCAAACAAC*
*TCATTGGTATGTTATTTGATAGAGTGAACTGTAAAGTATTGAATTGTAGATATCATGTGGCTTT*
*AAAAATTTGATATGTGTTATTTTGGCAGGAGTCATTTTCTGCTCTTCGCAAGGATGCTGCCAAT*
*GTTCTGGATTTCCTAGAGAGATTAAAGAATGAAGAAGATCAAAAGGCTGTTGATGTGGATCTGA*
*TTGAAAGCCTGAAATTGAAGCTGACATTTATTTGTACATATGTCCAGCTTTCTTATTCCGATTT*
*GGAGAAGTTTGAAGATATAATGACTAGAAAAAGACAAGAGGTTGAGAATCTGCTTCAACCAATT*
*TTGGATGATGATGGCAAAGACGTCGGGTGTAAATATGTCCTTACTAGCCTCGCCGGTAATATGG*
*ATGACTGTATAAGCTTGTATCATCGTTCTAAATCAGATGCCACCATGATGGATGAGCAATTGGG*
*CTTCCTCCTCTTGAATCTCTCTCATCTATCCAAGCATCGTGCTGAAAAGATGTTTCCTGGAGTG*
*ACTCAATATGAGGTTCTTCAGAATGTATGTGGCAACATAAGAGATTTCCATGGATTGATAGTGA*
*ATTGTTGCATTAAGCATGAGATGGTTGAGAATGTCTTATCTCTGTTTCAACTGATGGCTGAGAG*
*AGTAGGACGCTTCCTTTGGGAGGATCAGGCTGATGAAGACTCTCAACTCTCCGAGCTAGATGAG*
*GATGATCAGAATGATAAAGACCCTCAACTCTTCAAGCTAGCACATCTACTCTTGAAGATTGTTC*
*CAACTGAATTGGAGGTTATGCACATATGTTATAAAACTTTGAAAGCTTCAACTTCAACAGAAAT*
*TGGACGCTTCATTAAGAAGCTCCTGGAAACCTCTCCGGACATTCTCAGAGAATATCTGATTCAT*
*CTACAAGAGCATATGATAACTGTTATTACCCCTAACACTTCAGGGGCTCGAAACATTCATGTCA*
*TGATGGAATTCCTATTGATTATTCTTTCTGATATGCCGCCCAAGGACTTTATTCATCATGACAA*

Figure 2b - continued

*ACTTTTTGATCTCTTGGCTCGTGTTGTAGCACTTACCAGGGAGGTATCAACTCTTGTACGCGAC*
*TTGGAAGAGAAATTAAGGATTAAAGAGAGTACTGACGAAACAAATTGTGCAACCCTAAAGTTTC*
*TGGAAAATATTGAACTCCTTAAGGAAGATCTCAAACATGTTTATCTGAAAGTCCCGGATTCATC*
*TCAATATTGCTTCCCCATGAGTGATGGACCTCTCTTCATGCATCTGCTACAGAGACACTTAGAT*
*GATTTGCTGGATTCCAATGCTTATTCAATTGCTTTGATAAAGGAACAAATTGGGCTGGTGAAAG*
*AAGACTTGGAATTCATAAGATCTTTTTTCGCGAATATTGAGCAAGGATTGTATAAAGATCTCTG*
*GGAACGTGTTCTAGATGTGGCATATGAGGCAAAAGATGTCATAGATTCAATTATTGTTCGAGAT*
*AATGGTCTCTTACATCTTATTTTCTCACTTCCCATTACCAGAAAGAAGATGATGCTTATCAAAG*
*AAGAGGTCTCTGATTTACATGAGAACATTTCCAAGAACAGAGGTCTCATCGTTGTGAACTCTCC*
*CAAGAAACCAGTTGAGAGCAAGTCATTGACAACTGATAAAATAATTGTAGGTTTTGGTGAGGAG*
*ACAAACTTGATACTTAGAAAGCTCACCAGTGGACCGGCAGATCTAGATGTCATTTCGATCATTG*
*GTATGCCGGGTTTAGGTAAAACTACTTTGGCGTACAAAGTATACAATGATAAATCAGTTTCTAG*
*CCATTTCGACCTTCGTGCATGGTGCACGGTCGACCAAGTATATGACGAGAAGAAGTTGTTGGAT*
*AAAATTTTCAATCAAGTTAGTGACTCAAATTCAAAATTGAGTGAGAATATTGATGTTGCTGATA*
*AACTACGGAAACAATTGTTTGGAAAGAGGTATCTTATTGTCTTAGATGACGTGTGGGATACTAA*
*TACATGGGATGAGCTAACAAGACCTTTTCCTGATGGTATGAAAGGAAGTAGAATTATTTTGACA*
*ACTCGAGAAAAGAAAGTTGCTTTGCATGGAAAGCTCTACACTGATCCTCTTAACCTTCGATTGC*
*TAAGATCAGAAGAAAGTTGGGAGTTATTAGAGAAAAGGGCATTTGGAAACGAGAGTTGCCCTGA*
*TGAACTATTGGATGTTGGTAAAGAAATAGCCGAAAATTGTAAAGGGCTTCCTTTGGTGGTGGAT*
*CTGATTGCTGGAATCATTGCTGGGAGGGAAAAGAAAAAGAGTGTGTGGCTTGAAGTTGTAAATA*
*ATTTGCATTCCTTTATTTTGAAGAATGAAGTGGAAGTGATGAAAGTTATAGAAATAAGTTATGA*
*CCACTTACCTGATCACCTGAAGCCATGCTTGCTGTACTTTGCAAGTGCGCCGAAGGACTGGGTA*
*ACGACAATCCATGAGTTGAAACTTATTTGGGGTTTTGAAGGATTTGTGGAAAAGACAGATATGA*
*AGAGTCTGGAAGAAGTGGTGAAAATTTATTTGGATGATTTAATTTCCAGTAGCTTGGTAATTTG*
*TTTCAATGAGATAGGTGATTACCCTACTTGCCAACTTCATGATCTTGTGCATGACTTTTGTTTG*
*ATAAAAGCAAGAAAGGAAAAGTTGTGTGATCGGATAAGTTCAAGTGCTCCATCAGATTTGTTGC*
*CACGTCAAATTAGCATTGATTATGATGATGATGAAGAGCACTTTGGGCTTAATTTTGTCCTGTT*
*CGGTTCAAATAAGAAAAGGCATTCCGGTAAACACCTCTATTCTTTGACCATAAATGGAGATGAG*
*CTGGACGACCATCTTTCTGATACATTTCATCTAAGACACTTGAGGCTTCTTAGAACCTTGCACC*
*TGGAATCCTCTTTTATCATGGTTAAAGATTCTTTGCTGAATGAAATATGCATGTTGAATCATTT*
*GAGGTACTTAAGCATTGGGACAGAAGTTAAATCTCTGCCTTTGTCTTTCTCAAACCTCTGGAAT*
*CTAGAAATCTTGTTTGTGGATAACAAAGAATCAACCTTGATACTATTACCGAGAATTTGGGATC*
*TTGTAAAGTTGCAAGTGCTGTTCACGACTGCTTGTTCTTTCTTTGATATGGATGCAGATGAATC*
*AATACTGATAGCAGAGGACACAAAGTTAGAGAACTTGACAGCATTAGGGGAACTCGTGCTTTCC*
*TATTGGAAAGATACAGAGGATATTTTCAAAAGGCTTCCCAATCTTCAAGTGCTTCATTTCAAAC*
*TCAAGGAGTCATGGGATTATTCAACAGAGCAATATTGGTTCCCGAAATTGGATTTCCTAACTGA*
*ACTAGAAAAACTCACTGTAGATTTTGAAAGATCAAACACAAATGACAGTGGGTCCTCTGCAGCC*
*ATAAATCGGCCATGGGATTTTCACTTTCCTTCGAGTTTGAAAAGATTGCAATTGCATGAATTTC*
*CTCTGACATCCGATTCACTATCAACAATAGCGAGACTGCTGAACCTTGAAGAGTTGTACCTTTA*
*TCGTACAATCATCCATGGGGAAGAATGGAACATGGGAGAAGAAGACACCTTTGAGAATCTCAAA*
*TGTTTGATGTTGAGTCAAGTGATTCTTTCCAAGTGGGAGGTTGGAGAGGAATCTTTTCCCACGC*
*TTGAGAAATTAGAACTGTCGGACTGTCATAATCTTGAGGAGATTCCGTCTAGTTTTGGGGATAT*
*TTATTCCTTGAAAATTATCGAACTTGTAAGGAGCCCTCAACTTGAAAATTCCGCTCTCAAGATT*
*AAGGAATATGCTGAAGATATGAGGGGAGGGGACGAGCTTCAGATCCTTGGCCAGAAGGATATCC*
*CGTTATTTAAGTAGTTTTTGAGCATTATGGTTGAAAAGTAGATTGCACTTTGCTGGGTAGATTG*
*TATATGGTTAAGAAAATTCTGTTACAGTTGTTATGAAACATTTTTATTTGACTTTTCTGAGTTT*
*CTTTTAGAAAACTCAGAAGTTTTTAACAAAAATTATAGTTTTTATAAATACAATGTGGATTTGC*
*CTTTGGCTGTCCAACTTGGTCTGAAGTCTCATATGCTCAGAGCACTATCGTTCAACCTCAATCA*
*AGGTACTGATTTAAAATGACATCTATACTACTTTATCACAAACCCAACGAACTTTCATCTCAAA*

Figure 2b - continued

*AGCTAGGCCAGGAAGTGAAGAGGTTGTAGAGAGCTTATAAGCACTCATGACTTCCTTTTCTCGA*
*ACATTCAACCAACGTAGGCTGAAATCCCACTCTGAACGAAAATAAGTGTTTGTTTATCAAATTA*
*ACTCTCGTAGTAGAACACTGAAATACCTTCTTCTAAACGTTCAACAAATGGGATTTCCAGCACT*
*CAAAGTGAATGAAAGGTTCACATTAATCTTCAAAAAGAATTACGACAATTCATGACCACAAGTA*
*CATTGACAGCACCATTTCAACAGAAGAACAAGTCAATGCTGCATCTTCATCAATAATCCGAGTG*
*TCGAACCTCCTTCCTGACACTGTCCTGTATATGTAAAGTTTCTCAACAGGGCAACTTTCTGGTC*
*TCGTATCTGGATGACCCCTCTCGTCTATAACTTCAACATTAAGCCCTGGCAACTTCTGGACCAA*
*CAGCTTACATGCTTCAAAACTTACTGAACAATTAGACATCCAAAGGGATCGCATTGTCTCCAGC*
*TTTGCAGCATTAGCCAACAGAGCCTCATCGCCAAAGGGGCAGTCTCTAATCTCGAATTTGAAAA*
*AATTGTTGTTGTATGACTTTCCTCTGACATCCGATGCACTATCAACAATAGCAAGACTGGAGGT*
*TGGAGAGGAATCCTTTATTATACAATCATTCAGGGAGAAGAATGGAACATGGGGGAGGAAGACA*
*CTTTTGAGAATCTGAAATGTGTTAGAGCCACAAGCTACAGAAGTATTGAATTTGTCATGAATAT*
*CAACATTCTTCATCCTAGTTAATTCTTTTTCAATTTTTAATAGACTCTCATTTTAATCACTAAT*
*ATTCTTCTATTTGTGACTTCTTTTCTGCAGGTGGCAACTTTAAATTCATAAAGTATAGGATTGA*
*TGACAAACTCGAAAAATATCTTAATGAGGTGAAGTTTGAGCAGTCAGCAGATGGTGGTTCCAAC*
*TCTAAGTTGACAAGCACATACTATCCCGGAGGGCGATTTCAAGCCTGATGCATATGGTTAGTGT*
*GGCTAGAGCAGACAGGATGTATTACCTGGATATCTACCAAGACGAATCCACAATCAGTTTTATG*
*TCAAGCAATACATGAAGTAACTCCCGATAGAACAGTAAAAGCAAGATGTGTAGGTGTATCTCGA*
*CTCTAAGAGATTGTACATTCCTCTTTGAGATTTTTACTGCTAATACAAATTTACACCTCAGAAG*
*CGAATCTAGAATTTCTAGAGCATGAATGCACCACTAATGAAAGGAGAAAAAAGGAAGTATGAAG*
*TGGGAATTTGATCCTTGTTTCTAGGTATATAAAATTTATCATTCAACTATACTTCATTTAGCAA*
*ACAACTCTCTTTGCCATTATTTCTCAAACAAGGGCTTCTAATATTGCTAAACTAAAGACTGTCA*
*AAAGGTAAGTTCATCTTCAAACTCTCTTGTTTACTTTATCTAAAGGGGAACTATGAAAAACAAG*
*AAACATCAGGAATGTCCCGTAAACAAAGCAGCCTCATGCACAAAACATCCAACGTTGGTAGGAT*
*TAATGGAGGGATCGCATCCCAGGAGGATACTGTAGAAAAATTAGTGGCTTCTTTCACCGCTCAA*
*ACCCATGATCTATAGGTTACATGGAGACAACTTTATGGTTGCTCGTAGGCTCCCGTCAATTCTC*
*ATAAACCACAACACCAAAGTTGCATCAGACATCATCTTCATTCACAAGCTGACAATCTCCACAA*
*GTCTTAGTCAACTTGTAATATGAATATTAGCCAGGTAGACGTACATATTTACAAAATTGAGTTT*
*CCTATATAATATGGTTTGAAGGAATGAAACATGATGGGGAGGGTAGATAAAATAATATATGAGG*
*CATAAAAATAGGAAAGATATTTGTAGTGAGAGGTTTTGACTTTTTATGCTGCTTTTGATCTTCA*
*GTTTCTTGTATTCTTTTTCTACTGCTTTCCTCTTCTTTCTCCTGAGTAAAGTTTTATGTAGGTA*
*CTTTTTATACGTCCGATCGTGAGAACTTGAAAGAAAGCTCTCTATAGCTATGTTAGGTGCCCAC*
*ATAAAAAAATGAAATATTACAAAAACCCTGATAATAAAATACACTAATCTAAGATATTCACTGC*
*AACATACATGCAAAATATATATATATAAATTTTCATGAAAATTATAACAAATAATAGATGTGAA*
*CATATAACTTTAAAAATAATATTACATCCATAAAGCTTAAATTCTAGATCCCCGGTCGACTCTA*
*GAGGATCCCCACTCCATCCGTTCACT***TTGATTTGTCATGTTGCACTTTTCGAAAGTCAATTTGA**
**CTAATTTTTAAAGCTAAATTAGATTACACTAATTCAATATTTTAAACAGAAAAATTAGATATTC**
**AAAAACTATACAAAAAATATTATACATTGCAATTTTTTGCATATCAATATGATAAAAAAATATA**
**TCGTAAAATATTAGTCAAAATTTTTATAATTTGACTCAAATCATGAAAAGTATAATAATTAATA**
**GTGGACGGAGGAAGTATTGTCTTTCCAGATTTGTGGCCATTTTTGGTCCAAGGGCCATTAGCAG**
**TTCTCTTCATTTTCTACTTCTGTCTCATATTAGATGGGCATCTTACTAAAAATATTTGTCTCAT**
**ATTACTTGATTATTTATTAAATCAAAAAGAATTAATTAATTTTTTCTCATTTTACCCCTACAAT**
**TAATATAGTTTTAAAAGTTTTAAACAAATTTTGAAGAATCAAAATTTCTTTTTGCAAGAGACTT**
**ATTAATATAAACAAAGGATAAAATAATAAAATTTGTCAATTTATTGACGATCACTTAATAATCA**
**TATAAAATAGAAAATGTTTATCTAATATGAGACGGAGAAAATATATCCTAAAATATTTTTGGAC**
**AGATATGTGATATTCTAACCATTCACTAGACTATATTATGCATTTTAGCCGCCAATGACTTATT**
**TCAGCTTTAATTAATTAGGAAAGAGGAAACTGCCAATGAGGAAGAGTAGGGGCGTAGTTGCTGT**
**CGACGAAAAAAAGATAATACTCACTCTTTTCGATTTTTATTTTTATTTATCACTTTTAACCTAT**
**CATGTAAAAAGATAATTATTTTTTTCATGCTTTATCCTTAGTATTAAACAATTTAATAGGGATT**

Figure 2b - continued

ATTTTGTAAAATATTTATATGAATAATTGTTTTCGTAATGAATTTGTCCGGTCAAACAATGATA
AATAAAAATGAATGAAGAGAGTAGAAAACAAAACAAAAGAACAAGTTGACAACTTGAGAGATTA
AAAGGGTCCAAAACGCCTTGGATTTTGAGATTCCATATGTGAAATTTCCATGAAATAATTGAAT
TTGTATTATTACAAGTCAAACTTTCCATTTCATTCCAACTAGCCATCTTGGTTTCAAAATTACA
CATTCATTCATTCACAGATCTAATATTCTTAATAGTGATTTCCACATATGGCTGAAGCTTTCAT
TCAAGTTCTGCTAGACAATCTCACTTCTTTCCTCAAAGGGGAACTTGTATTGCTTTTCGGTTTT
CAAGATGAGTTCCAAAGGCTTTCAAGCATGTTTTCTACAATTCAAGCCGTCCTTGAAGATGCTC
AGGAGAAGCAACTCAACAACAAGCCTCTAGAAAATTGGTTGCAAAAACTCAATGCTGCTACATA
TGAAGTCGATGACATCTTGGATGAATATAAAACCAAGGCCACAAGATTCTCCCAGTCTGAATAT
GGCCGTTATCATCCAAAGGTTATCCCTTTCCGTCACAAGGTCGGGAAAAGGATGGACCAAGTGA
TGAAAAAACTAAAGGCAATTGCTGAGGAAAGAAAGAATTTTCATTTGCACGAAAAAATTGTAGA
GAGACAAGCTGTTAGACGGGAAACAGGTACTCATCTTAAATTAGTATTACAACAACTAAGTTTA
TATTCATTTTTTTGGCAATTATCAAATTCAGAAAAGGGTTAAATATACTCATGTCCTATCGTAA
ATAGTGTATATATACCTCTCGTTGTACTTTCGATCTGAATATACTTGTCAAATCTGGCAAGCTC
AGAATCAAATTATCCACCCCAACTTTTAAATACTCGATATCTTTAGAAATCCACCTGTCTAACT
CATCCACTACCCATTCCCTTTGCTTTGAATTCTTTTCTTTACCTATAAACTTGGAACACTCGAT
CCGTTTTGCTTTTCTTAACAAAGCAGCTCAGAGAAAAGAGGTTTTCTTCTATTCTGTTTCTCTG
TGTGCTGCACTTGGGTCCTTAATCCCATTAAAAACAGGGCATGTTAATCCCAACGACGGTAGCC
TTTCCTGACAGCTGACTGTAAATTTTGTCTAACAAAGAAAAAAAAAGATTAGACATGTTTTTCC
TTGTCATTGATTAGGCTGGATTTCTTTCAGAGTGGAACATAGGGGATATATTGGACCAAAAGTA
GAATGGGTATATATTTAAAGTATTTCTGATAGAACAGGAGTATATTGTGCGAAAATATCCTCTA
TTTTCTGTTGTCTCCTAATGAGTTTGAATGTAATAATATTCTCATGTGGACATTGCTTGCACCA
GGTTCTGTATTAACCGAACCGCAGGTTTATGGAAGAGACAAAGAGAAAGATGAGATAGTGAAAA
TCCTAATAAACAATGTTAGTGATGCCCAACACCTTTCAGTCCTCCCAATACTTGGTATGGGGG
ATTAGGAAAAACGACTCTTGCCCAAATGGTCTTCAATGACCAGAGAGTTACTGAGCATTTCCAT
TCCAAAATATGGATTTGTGTCTCGGAAGATTTTGATGAGAAGAGGTTAATAAAGGCAATTGTAG
AATCTATTGAAGGAAGGCCACTACTTGGTGAGATGGACTTGGCTCCACTTCAAAAGAAGCTTCA
GGAGTTGCTGAATGGAAAAAGATACTTGCTTGTCTTAGATGATGTTTGGAATGAAGATCAACAG
AAGTGGGCTAATTTAAGAGCAGTCTTGAAGGTTGGAGCAAGTGGTGCTTCTGTTCTAACCACTA
CTCGTCTTGAAAAGGTTGGATCAATTATGGGAACATTGCAACCATATGAACTGTCAAATCTGTC
TCAAGAAGATTGTTGGTTGTTGTTCATGCAACGTGCATTTGGACACCAAGAAGAAATAAATCCA
AACCTTGTGGCAATCGGAAAGGAGATTGTGAAAAAAAGTGGTGGTGTGCCTCTAGCAGCCAAAA
CTCTTGGAGGTATTTTGTGCTTCAAGAGAGAAGAAAGAGCATGGGAACATGTGAGAGACAGTCC
GATTTGGAATTTGCCTCAAGATGAAAGTTCTATTCTGCCTGCCCTGAGGCTTAGTTACCATCAA
CTTCCACTTGATTTGAAACAATGCTTTGCGTATTGTGCGGTGTTCCCAAAGGATGCCAAAATGG
AAAAAGAAAAGCTAATCTCTCTCTGGATGGCGCATGGTTTTCTTTTATCAAAAGGAAACATGGA
GCTAGAGGATGTGGGCGATGAAGTATGGAAAGAATTATACTTGAGGTCTTTTTTCCAAGAGATT
GAAGTTAAAGATGGTAAAACTTATTTCAAGATGCATGATCTCATCCATGATTTGGCAACATCTC
TGTTTTCAGCAAACACATCAAGCAGCAATATCCGTGAAATAAATAAACACAGTTACACACATAT
GATGTCCATTGGTTTCGCCGAAGTGGTGTTTTTTTACACTCTTCCCCCCTTGGAAAAGTTTATC
TCGTTAAGAGTGCTTAATCTAGGTGATTCGACATTTAATAAGTTACCATCTTCCATTGGAGATC
TAGTACATTTAAGATACTTGAACCTGTATGGCAGTGGCATGCGTAGTCTTCCAAAGCAGTTATG
CAAGCTTCAAAATCTGCAAACTCTTGATCTACAATATTGCACCAAGCTTTGTTGTTTGCCAAAA
GAAACAAGTAAACTTGGTAGTCTCCGAAATCTTTTACTTGATGGTAGCCAGTCATTGACTTGTA
TGCCACCAAGGATAGGATCATTGACATGCCTTAAGACTCTAGGTCAATTTGTTGTTGGAAGGAA
GAAAGGTTATCAACTTGGTGAACTAGGAAACCTAAATCTCTATGGCTCAATTAAAATCTCGCAT
CTTGAGAGAGTGAAGAATGATAAGGACGCAAAAGAAGCCAATTTATCTGCAAAAGGGAATCTGC
ATTCTTTAAGCATGAGTTGGAATAACTTTGGACCACATATATATGAATCAGAAGAAGTTAAAGT
GCTTGAAGCCCTCAAACCACACTCCAATCTGACTTCTTTAAAAATCTATGGCTTCAGAGGAATC

Figure 2b - continued

**CATCTCCCAGAGTGGATGAATCACTCAGTATTGAAAAATATTGTCTCTATTCTAATTAGCAACT**
**TCAGAAACTGCTCATGCTTACCACCCTTTGGTGATCTGCCTTGTCTAGAAAGTCTAGAGTTACA**
**CTGGGGGTCTGCGGATGTGGAGTATGTTGAAGAAGTGGATATTGATGTTCATTCTGGATTCCCC**
**ACAAGAATAAGGTTTCCATCCTTGAGGAAACTTGATATATGGGACTTTGGTAGTCTGAAAGGAT**
**TGCTGAAAAAGGAAGGAGAAGAGCAATTCCCTGTGCTTGAAGAGATGATAATTCACGAGTGCCC**
**TTTTCTGACCCTTTCTTCTAATCTTAGGGCTCTTACTTCCCTCAGAATTTGCTATAATAAAGTA**
**GCTACTTCATTCCCAGAAGAGATGTTCAAAAACCTTGCAAATCTCAAATACTTGACAATCTCTC**
**GGTGCAATAATCTCAAAGAGCTGCCTACCAGCTTGGCTAGTCTGAATGCTTTGAAAAGTCTAAA**
**AATTCAATTGTGTTGCGCACTAGAGAGTCTCCCTGAGGAAGGGCTGGAAGGTTTATCTTCACTC**
**ACAGAGTTATTTGTTGAACACTGTAACATGCTAAAATGTTTACCAGAGGGATTGCAGCACCTAA**
**CAACCCTCACAAGTTTAAAAATTCGGGGATGTCCACAACTGATCAAGCGGTGTGAGAAGGGAAT**
**AGGAGAAGACTGGCACAAAATTTCTCACATTCCTAATGTGAATATATATATTTAAGTTATTTGC**
**TATTGTTTCTTTGTTTGTGAGTCTTTTTGGTTCCTGCCATTGTGATTGCATGTAATTTTTTTCT**
**AGGGTTGTTTCTTTATGAGTCTCTCTCTCATTGGATGTAATTTTCTTTTGGAAACAAATCTGTC**
**AATTGATTTGTATTATACGCTTTCAGAATCTATTACTTATTTGTAATTGTTTCTTTGTTTGTAA**
**ATTGTGAGTATCTTATTTTATGGAATTTTCTGATTTTATTTTGAAAACAAATCAATGATTTGTA**
**AGATCCATCTGTATTATACTCCCTTCGTCTCATTTTATGTGTCACCTGTCGGATTTCGAGATTC**
**AAACAAATCTATCTTTGATCGTAAATTTTTAATAGATCTTTTAAACATTTTGAATTATCAATTA**
**TTGTGACTTTAGT**_GGCTAGACTAGTGGATCCGATATCGCCCAGCTTCACGCTGCCGCAAGCACT_
_CAGGGCGCAAGGGCTGCTAAAGGAAGCGGAACACGTAGAAAGCCAGTCCGCAGAAACGGTGCTG_
_ACCCCGGATGAATGTCAGCTACTGGGCTATCTGGACAAGGGAAAACGCAAGCGCAAAGAGAAAG_
_CAGGTAGCTTGCAGTGGGCTTACATGGCGATAGCTAGACTGGGCGGTTTTATGGACAGCAAGCG_
_AACCGGAATTGCCAGCTGGGGCGCCCTCTGGTAAGGTTGGGAAGCCCTGCAAAGTAAACTGGAT_
_GGCTTTCTTGCCGCCAAGGATCTGATGGCGCAGGGGATCAA_***GATCATGAGCGGAGAATTAAGGG***
***AGTCACGTTATGACCCCCGCCGATGACGCGGACAAGCCGTTTTACGTTTGGAACTGACAGAAC***
***CGCAACGTTGAAGGAGCCACTCAGCCGCGGGTTTCTGGAGTTTAATGAGCTAAGCACATACGTC***
***AGAAACCATTATTGCGCGTTCAAAAGTCGCCTAAGGTCACTATCAGCTAGCAAATATTTCTTGT***
***CAAAAATGCTCCACTGACGTTCCATAAATTCCCCTCGGTATCCAATTAGAGTCTCATATTCACT***
***CTCAATCCAGATCCCCGGGTACCATGGCGGCGGCAACAACAACAACAACAACATCTTCTTCGAT***
***CTCCTTCTCCACCAAACCATCTCCTTCCTCCTCCAAATCACCATTACCAATCTCCAGATTCTCC***
***CTCCCATTCTCCCTAAACCCCAACAAATCATCCTCCTCCTCCCGCCGCCGCGGTATCAAATCCA***
***GCTCTCCCTCCTCCATCTCCGCCGTGCTCAACACAACCACCAATGTCACAACCACTCCCTCTCC***
***AACCAAACCTACCAAACCCGAAACATTCATCTCCCGATTCGCTCCAGATCAACCCCGCAAAGGC***
***GCTGATATTCTCGTCGAGGCTTTAGAACGTCAAGGCGTAGAAACCGTATTCGCTTACCCTGGAG***
***GTGCATCAATGGAGATTCACCAAGCCTTAACCCGCTCTTCCTCAATCCGTAACGTCCTTCCTCG***
***TCACGAACAAGGAGGTGTATTCGCAGCAGAAGGATACGCTCGATCCTCAGGTAAACCAGGTATC***
***TGTATAGCCACTTCAGGTCCCGGAGCTACAAATCTCGTTAGCGGATTAGCCGATGCGTTGTTAG***
***ATAGTGTTCCTCTTGTAGCAATCACAGGACAAGTCCCTCGTCGTATGATTGGTACAGATGCGTT***
***TCAAGAGACTCCGATTGTTGAGGTAACGCGTTCGATTACGAAGCATAACTATCTTGTGATGGAT***
***GTTGAAGATATTCCTAGGATTATTGAGGAGGCTTTCTTTTTAGCTACTTCTGGTAGACCTGGAC***
***CTGTTTTGGTTGATGTTCCTAAAGATATTCAACAACAGCTTGCGATTCCTAATTGGGAACAGGC***
***TATGAGATTACCTGGTTATATGTCTAGGATGCCTAAACCTCCGGAAGATTCTCATTTGGAGCAG***
***ATTGTTAGGTTGATTTCTGAGTCTAAGAAGCCTGTGTTGTATGTTGGTGGTGGTTGTTTGAACT***
***CTAGCGATGAATTGGGTAGGTTTGTTGAGCTTACGGGAATCCCTGTTGCGAGTACGTTGATGGG***
***GCTGGGATCTTATCCTTGTGATGATGAGTTGTCGTTACATATGCTTGGAATGCATGGGACTGTG***
***TATGCAAATTACGCTGTGGAGCATAGTGATTTGTTGTTGGCGTTTGGGGTAAGGTTTGATGATC***
***GTGTCACGGGTAAACTTGAGGCTTTTGCTAGTAGGGCTAAGATTGTTCATATTGATATTGACTC***
***GGCTGAGATTGGGAAGAATAAGACTCCTCATGTGTCTGTGTGTGGTGATGTTAAGCTGGCTTTG***
***CAAGGGATGAATAAGGTTCTTGAGAACCGAGCGGAGGAGCTTAAACTTGATTTTGGAGTTTGGA***

Figure 2b - continued

***GGAATGAGTTGAACGTACAGAAACAGAAGTTTCCGTTGAGCTTTAAGACGTTTGGGGAAGCTAT***
***TCCTCCACAGTATGCGATTAAGGTCCTTGATGAGTTGACTGATGGAAAAGCCATAATAAGTACT***
***GGTGTCGGGCAACATCAAATGTGGGCGGCGCAGTTCTACAATTACAAGAAACCAAGGCAGTGGC***
***TATCATCAGGAGGCCTTGGAGCTATGGGATTTGGACTTCCTGCTGCGATTGGAGCGTCTGTTGC***
***TAACCCTGATGCGATAGTTGTGGATATTGACGGAGATGGAAGTTTTATAATGAATGTGCAAGAG***
***CTAGCCACTATTCGTGTAGAGAATCTTCCAGTGAAGGTACTTTTATTAAACAACCAGCATCTTG***
***GCATGGTTATGCAATGGGAAGATCGGTTCTACAAAGCTAACCGAGCACACACATTTCTCGGAGA***
***TCCGGCTCAGGAGGACGAGATATTCCCGAACATGTTGCTGTTTGCAGCAGCTTGCGGATTCCA***
***GCGGCGAGGGTGACAAAGAAAGCAGATCTCCGAGAAGCTATTCAGACAATGCTGGATACACCAG***
***GACCTTACCTGTTGGATGTGATTTGTCCGCACCAAGAACATGTGTTGCCGATGATCCCGAATGG***
***TGGCACTTTCAACGATGTCATAACGGAAGGAGATGGCCGGATTAAATACTGAGAGCTCGAATTT***
***CCCCGATCGTTCAAACATTTGGCAATAAAGTTTCTTAAGATTGAATCCTGTTGCCGGTCTTGCG***
***ATGATTATCATATAATTTCTGTTGAATTACGTTAAGCATGTAATAATTAACATGTAATGCATGA***
***CGTTATTTATGAGATGGGTTTTTATGATTAGAGTCCCGCAATTATACATTTAATACGCGATAGA***
***AAACAAAATATAGCGCGCAAACTAGGATAAATTATCGCGCGCGGTGTCATCTATGTTACTAGAT***
***C***_GGGAATTCACTGGCCGTCGTTTTACAACGACTCAGCTGCTTGGTAATAATTGTCATTAGATTG_
_TTTTTATGCATAGATGCACTCGAAATCAGCCAATTTTAGACAAGTATCAAACGGATGTTAATTC_
_AGTACATTAAAGACGTCCGCAATGTGTTATTAAGTTGTCTAAG_

Figure 2c of 5

**Insert without flanking region without ahas marker- SEQ-ID-No. 3**

**Upper case italic-underlined – Blb2 expression cassette (promoter – gene –terminator)**
**Upper case bold – Blb1 expression cassette (promoter – gene –terminator)**

CAATTTCACACAGGAAACAGCTATGAC*CATGATTACGCCAAGCTGGCGCGCCAAGCTTGCATGC*
*CTGCAGGTCGACTCTAGAGGATCTAGAATCACCGAACCTCCCCTCGGTACAGCTCCTCCAGTTC*
*TACCATGAATTTCATCCACTGATTCCTCTTCAATCGCCATTGCAGATTCTCTCGATCTATGCTC*
*AAAAAATCCCGAGATAAAACCCTAGATCTGCTTCAAATGCTCTGATACCATGTAATTTCAGTGA*
*ATTCTAACTAAACAATGGAGAGAATTAACTATTTTAGAAAGACTGATTGAAGGAGAAGAAGAGA*
*GAAAAATTCTATATTGAACTCATGAACCAAAATGAATGAAAAAAATAATGAGAAGAACTATACT*
*ATTACAATCTATATATCTCTATTTATATTCTAATCTGAAGCAGTTAATTTAACTGACTCTAACA*
*ACTAGACTGATAGGTGTACATTTTCTGTTAGTGCACTGCAGTGCATTTAACTAACTGCTTAACA*
*TAAAGAATGTTGTTCGAACTTCATTCGAATAGCTTCAATGAGAAGCAAACATGTGTACCTGTAA*
*AGACACACAGTAAAAGTGTTAATAATGAATAAATATGAATAAATCAAATAATAAATTAAAAATA*
*AAAACACATCCAATTAACATTGGAGGTCTTGAAAATCGATGGTAATTAACAAAGACCCTTGTGA*
*AATTTAAGTCTGTAATTGAAAATTTGAGTATAGGTTAGGGGACATTTGACTATTTTCTCATTTT*
*CTTTATCTTTTTCCTAATTTGTGGCAGACAAGTGAGGAGGCCCCACTGTAATTGATTCATGCTT*
*TTGCTTTCTTGACTTTTTGGAACAATACTATGCATCATATTTGGTCTTAATTATTCCTCTGTTT*
*ATTTCCAGAATTTTGAGCTCTATACATCTAATAACAAAGCAAGCAGAGGATATATAGTTTCATC*
*AACTAAAAAGGTTAGTCAACTCATCTAATATTTGCTACTCTCATCTCTATTGAAGTACAGTTAT*
*GGAAAAGTAGAAGTGATGTAAGAAAAATGAAAGAACTTTAGTAGGTTAGTTGGATCTAACAAAG*
*AGAAAGGGAAATAAATTGCAGGAGAAAGAGAGAGGTTAAATACTTACTCACACCACCGATTTAC*
*AACAAATCACTTAATTGTGGTTAGTTAATGTATACTTTCACCTCATTAAATTATTACTTACCCA*
*TGATAAGTTGTATTAATTTGGTATTAATATCCGGTGCGGGTGAATTCTTACCGGGTGAGAGGGA*
*TGGGGTTGGAGAGTGTGGAGTGAACAGAAGCAGATGTTTTAGATTTTTTCTAAGATGACGAAAG*
*ATTCCCCTCACTAATGAAAATATATTACTATACGCTATTAGAGATAGAAAGGTTCGGTACCAGT*
*TGGTCTCGTTTCTGGATGAACCCCATTTTTACAAGTCATTTTCTTCAATTCAAATCGCAAGTGT*
*ACCTTTATCATCTTCCACTAATTAAGTCCTCTTAAGTTCGCGTGAAAATAGTGAAATTATTGAT*
*TATTCTTATCATTTCATCTTCTTTCTCCTGATAAAGTTTTATGTACTTTTTATGCATCAGGTCT*
*TGAGAACTTGGAAAGGAAAAGTAGAATCATGGAAAAACGAAAAGATAATGAAGAAGCAAACAAC*
*TCATTGGTATGTTATTTGATAGAGTGAACTGTAAAGTATTGAATTGTAGATATCATGTGGCTTT*
*AAAAATTTGATATGTGTTATTTTGGCAGGAGTCATTTTCTGCTCTTCGCAAGGATGCTGCCAAT*
*GTTCTGGATTTCCTAGAGAGATTAAAGAATGAAGAAGATCAAAAGGCTGTTGATGTGGATCTGA*
*TTGAAAGCCTGAAATTGAAGCTGACATTTATTTGTACATATGTCCAGCTTTCTTATTCCGATTT*
*GGAGAAGTTTGAAGATATAATGACTAGAAAAAGACAAGAGGTTGAGAATCTGCTTCAACCAATT*
*TTGGATGATGATGGCAAAGACGTCGGGTGTAAATATGTCCTTACTAGCCTCGCCGGTAATATGG*
*ATGACTGTATAAGCTTGTATCATCGTTCTAAATCAGATGCCACCATGATGGATGAGCAATTGGG*
*CTTCCTCCTCTTGAATCTCTCTCATCTATCCAAGCATCGTGCTGAAAAGATGTTTCCTGGAGTG*
*ACTCAATATGAGGTTCTTCAGAATGTATGTGGCAACATAAGAGATTTCCATGGATTGATAGTGA*
*ATTGTTGCATTAAGCATGAGATGGTTGAGAATGTCTTATCTCTGTTTCAACTGATGGCTGAGAG*
*AGTAGGACGCTTCCTTTGGGAGGATCAGGCTGATGAAGACTCTCAACTCTCCGAGCTAGATGAG*
*GATGATCAGAATGATAAAGACCCTCAACTCTTCAAGCTAGCACATCTACTCTTGAAGATTGTTC*
*CAACTGAATTGGAGGTTATGCACATATGTTATAAAACTTTGAAAGCTTCAACTTCAACAGAAAT*
*TGGACGCTTCATTAAGAAGCTCCTGGAAACCTCTCCGGACATTCTCAGAGAATATCTGATTCAT*
*CTACAAGAGCATATGATAACTGTTATTACCCCTAACACTTCAGGGGCTCGAAACATTCATGTCA*
*TGATGGAATTCCTATTGATTATTCTTTCTGATATGCCGCCCAAGGACTTTATTCATCATGACAA*
*ACTTTTTGATCTCTTGGCTCGTGTTGTAGCACTTACCAGGGAGGTATCAACTCTTGTACGCGAC*

*TTGGAAGAGAAATTAAGGATTAAAGAGAGTACTGACGAAACAAATTGTGCAACCCTAAAGTTTC*
*TGGAAAATATTGAACTCCTTAAGGAAGATCTCAAACATGTTTATCTGAAAGTCCCGGATTCATC*
*TCAATATTGCTTCCCCATGAGTGATGGACCTCTCTTCATGCATCTGCTACAGAGACACTTAGAT*
*GATTTGCTGGATTCCAATGCTTATTCAATTGCTTTGATAAAGGAACAAATTGGGCTGGTGAAAG*
*AAGACTTGGAATTCATAAGATCTTTTTTCGCGAATATTGAGCAAGGATTGTATAAAGATCTCTG*
*GGAACGTGTTCTAGATGTGGCATATGAGGCAAAAGATGTCATAGATTCAATTATTGTTCGAGAT*
*AATGGTCTCTTACATCTTATTTTCTCACTTCCCATTACCAGAAAGAAGATGATGCTTATCAAAG*
*AAGAGGTCTCTGATTTACATGAGAACATTTCCAAGAACAGAGGTCTCATCGTTGTGAACTCTCC*
*CAAGAAACCAGTTGAGAGCAAGTCATTGACAACTGATAAAATAATTGTAGGTTTTGGTGAGGAG*
*ACAAACTTGATACTTAGAAAGCTCACCAGTGGACCGGCAGATCTAGATGTCATTTCGATCATTG*
*GTATGCCGGGTTTAGGTAAAACTACTTTGGCGTACAAAGTATACAATGATAAATCAGTTTCTAG*
*CCATTTCGACCTTCGTGCATGGTGCACGGTCGACCAAGTATATGACGAGAAGAAGTTGTTGGAT*
*AAAATTTTCAATCAAGTTAGTGACTCAAATTCAAAATTGAGTGAGAATATTGATGTTGCTGATA*
*AACTACGGAAACAATTGTTTGGAAAGAGGTATCTTATTGTCTTAGATGACGTGTGGGATACTAA*
*TACATGGGATGAGCTAACAAGACCTTTTCCTGATGGTATGAAAGGAAGTAGAATTATTTTGACA*
*ACTCGAGAAAAGAAAGTTGCTTTGCATGGAAAGCTCTACACTGATCCTCTTAACCTTCGATTGC*
*TAAGATCAGAAGAAAGTTGGGAGTTATTAGAGAAAAGGGCATTTGGAAACGAGAGTTGCCCTGA*
*TGAACTATTGGATGTTGGTAAAGAAATAGCCGAAAATTGTAAAGGGCTTCCTTTGGTGGTGGAT*
*CTGATTGCTGGAATCATTGCTGGGAGGGAAAAGAAAAAGAGTGTGTGGCTTGAAGTTGTAAATA*
*ATTTGCATTCCTTTATTTTGAAGAATGAAGTGGAAGTGATGAAAGTTATAGAAATAAGTTATGA*
*CCACTTACCTGATCACCTGAAGCCATGCTTGCTGTACTTTGCAAGTGCGCCGAAGGACTGGGTA*
*ACGACAATCCATGAGTTGAAACTTATTTGGGGTTTTGAAGGATTTGTGGAAAAGACAGATATGA*
*AGAGTCTGGAAGAAGTGGTGAAAATTTATTTGGATGATTTAATTTCCAGTAGCTTGGTAATTTG*
*TTTCAATGAGATAGGTGATTACCCTACTTGCCAACTTCATGATCTTGTGCATGACTTTTGTTTG*
*ATAAAAGCAAGAAAGGAAAAGTTGTGTGATCGGATAAGTTCAAGTGCTCCATCAGATTTGTTGC*
*CACGTCAAATTAGCATTGATTATGATGATGATGAAGAGCACTTTGGGCTTAATTTTGTCCTGTT*
*CGGTTCAAATAAGAAAAGGCATTCCGGTAAACACCTCTATTCTTTGACCATAAATGGAGATGAG*
*CTGGACGACCATCTTTCTGATACATTTCATCTAAGACACTTGAGGCTTCTTAGAACCTTGCACC*
*TGGAATCCTCTTTTATCATGGTTAAAGATTCTTTGCTGAATGAAATATGCATGTTGAATCATTT*
*GAGGTACTTAAGCATTGGGACAGAAGTTAAATCTCTGCCTTTGTCTTTCTCAAACCTCTGGAAT*
*CTAGAAATCTTGTTTGTGGATAACAAAGAATCAACCTTGATACTATTACCGAGAATTTGGGATC*
*TTGTAAAGTTGCAAGTGCTGTTCACGACTGCTTGTTCTTTCTTTGATATGGATGCAGATGAATC*
*AATACTGATAGCAGAGGACACAAAGTTAGAGAACTTGACAGCATTAGGGGAACTCGTGCTTTCC*
*TATTGGAAAGATACAGAGGATATTTTCAAAAGGCTTCCCAATCTTCAAGTGCTTCATTTCAAAC*
*TCAAGGAGTCATGGGATTATTCAACAGAGCAATATTGGTTCCCGAAATTGGATTTCCTAACTGA*
*ACTAGAAAAACTCACTGTAGATTTTGAAAGATCAAACACAAATGACAGTGGGTCCTCTGCAGCC*
*ATAAATCGGCCATGGGATTTTCACTTTCCTTCGAGTTTGAAAAGATTGCAATTGCATGAATTTC*
*CTCTGACATCCGATTCACTATCAACAATAGCGAGACTGCTGAACCTTGAAGAGTTGTACCTTTA*
*TCGTACAATCATCCATGGGGAAGAATGGAACATGGGAGAAGAAGACACCTTTGAGAATCTCAAA*
*TGTTTGATGTTGAGTCAAGTGATTCTTTCCAAGTGGGAGGTTGGAGAGGAATCTTTTCCCACGC*
*TTGAGAAATTAGAACTGTCGGACTGTCATAATCTTGAGGAGATTCCGTCTAGTTTTGGGGATAT*
*TTATTCCTTGAAAATTATCGAACTTGTAAGGAGCCCTCAACTTGAAAATTCCGCTCTCAAGATT*
*AAGGAATATGCTGAAGATATGAGGGGAGGGGACGAGCTTCAGATCCTTGGCCAGAAGGATATCC*
*CGTTATTTAAGTAGTTTTTGAGCATTATGGTTGAAAAGTAGATTGCACTTTGCTGGGTAGATTG*
*TATATGGTTAAGAAAATTCTGTTACAGTTGTTATGAAACATTTTTATTTGACTTTTCTGAGTTT*
*CTTTTAGAAAACTCAGAAGTTTTTAACAAAAATTATAGTTTTTATAAATACAATGTGGATTTGC*
*CTTTGGCTGTCCAACTTGGTCTGAAGTCTCATATGCTCAGAGCACTATCGTTCAACCTCAATCA*
*AGGTACTGATTTAAAATGACATCTATACTACTTTATCACAAACCCAACGAACTTTCATCTCAAA*
*AGCTAGGCCAGGAAGTGAAGAGGTTGTAGAGAGCTTATAAGCACTCATGACTTCCTTTTCTCGA*
*ACATTCAACCAACGTAGGCTGAAATCCCACTCTGAACGAAAATAAGTGTTTGTTTATCAAATTA*
*ACTCTCGTAGTAGAACACTGAAATACCTTCTTCTAAACGTTCAACAAATGGGATTTCCAGCACT*

*CAAAGTGAATGAAAGGTTCACATTAATCTTCAAAAAGAATTACGACAATTCATGACCACAAGTA*
*CATTGACAGCACCATTTCAACAGAAGAACAAGTCAATGCTGCATCTTCATCAATAATCCGAGTG*
*TCGAACCTCCTTCCTGACACTGTCCTGTATATGTAAAGTTTCTCAACAGGGCAACTTTCTGGTC*
*TCGTATCTGGATGACCCCTCTCGTCTATAACTTCAACATTAAGCCCTGGCAACTTCTGGACCAA*
*CAGCTTACATGCTTCAAAACTTACTGAACAATTAGACATCCAAAGGGATCGCATTGTCTCCAGC*
*TTTGCAGCATTAGCCAACAGAGCCTCATCGCCAAAGGGGCAGTCTCTAATCTCGAATTTGAAAA*
*AATTGTTGTTGTATGACTTTCCTCTGACATCCGATGCACTATCAACAATAGCAAGACTGGAGGT*
*TGGAGAGGAATCCTTTATTATACAATCATTCAGGGAGAAGAATGGAACATGGGGGAGGAAGACA*
*CTTTTGAGAATCTGAAATGTGTTAGAGCCACAAGCTACAGAAGTATTGAATTTGTCATGAATAT*
*CAACATTCTTCATCCTAGTTAATTCTTTTTCAATTTTTAATAGACTCTCATTTTAATCACTAAT*
*ATTCTTCTATTTGTGACTTCTTTTCTGCAGGTGGCAACTTTAAATTCATAAAGTATAGGATTGA*
*TGACAAACTCGAAAAATATCTTAATGAGGTGAAGTTTGAGCAGTCAGCAGATGGTGGTTCCAAC*
*TCTAAGTTGACAAGCACATACTATCCCGGAGGGCGATTTCAAGCCTGATGCATATGGTTAGTGT*
*GGCTAGAGCAGACAGGATGTATTACCTGGATATCTACCAAGACGAATCCACAATCAGTTTTATG*
*TCAAGCAATACATGAAGTAACTCCCGATAGAACAGTAAAAGCAAGATGTGTAGGTGTATCTCGA*
*CTCTAAGAGATTGTACATTCCTCTTTGAGATTTTTACTGCTAATACAAATTTACACCTCAGAAG*
*CGAATCTAGAATTTCTAGAGCATGAATGCACCACTAATGAAAGGAGAAAAAAGGAAGTATGAAG*
*TGGGAATTTGATCCTTGTTTCTAGGTATATAAAATTTATCATTCAACTATACTTCATTTAGCAA*
*ACAACTCTCTTTGCCATTATTTCTCAAACAAGGGCTTCTAATATTGCTAAACTAAAGACTGTCA*
*AAAGGTAAGTTCATCTTCAAACTCTCTTGTTTACTTTATCTAAAGGGGAACTATGAAAAACAAG*
*AAACATCAGGAATGTCCCGTAAACAAAGCAGCCTCATGCACAAAACATCCAACGTTGGTAGGAT*
*TAATGGAGGGATCGCATCCCAGGAGGATACTGTAGAAAAATTAGTGGCTTCTTTCACCGCTCAA*
*ACCCATGATCTATAGGTTACATGGAGACAACTTTATGGTTGCTCGTAGGCTCCCGTCAATTCTC*
*ATAAACCACAACACCAAAGTTGCATCAGACATCATCTTCATTCACAAGCTGACAATCTCCACAA*
*GTCTTAGTCAACTTGTAATATGAATATTAGCCAGGTAGACGTACATATTTACAAAATTGAGTTT*
*CCTATATAATATGGTTTGAAGGAATGAAACATGATGGGGAGGGTAGATAAAATAATATATGAGG*
*CATAAAAATAGGAAAGATATTTGTAGTGAGAGGTTTTGACTTTTTATGCTGCTTTTGATCTTCA*
*GTTTCTTGTATTCTTTTTCTACTGCTTTCCTCTTCTTTCTCCTGAGTAAAGTTTTATGTAGGTA*
*CTTTTTATACGTCCGATCGTGAGAACTTGAAAGAAAGCTCTCTATAGCTATGTTAGGTGCCCAC*
*ATAAAAAAATGAAATATTACAAAAACCCTGATAATAAAATACACTAATCTAAGATATTCACTGC*
*AACATACATGCAAAATATATATATATAAATTTTCATGAAAATTATAACAAATAATAGATGTGAA*
*CATATAACTTTAAAAATAATATTACATCCATAAAGCTTAAATTCTAGATCCCCGGTCGACTCTA*
*GAGGATCCCCACTCCATCCGTTCACT***TTGATTTGTCATGTTGCACTTTTCGAAAGTCAATTTGA**
**CTAATTTTTAAAGCTAAATTAGATTACACTAATTCAATATTTTAAACAGAAAAATTAGATATTC**
**AAAAACTATACAAAAAATATTATACATTGCAATTTTTTGCATATCAATATGATAAAAAAATATA**
**TCGTAAAATATTAGTCAAAATTTTTATAATTTGACTCAAATCATGAAAAGTATAATAATTAATA**
**GTGGACGGAGGAAGTATTGTCTTTCCAGATTTGTGGCCATTTTTGGTCCAAGGGCCATTAGCAG**
**TTCTCTTCATTTTCTACTTCTGTCTCATATTAGATGGGCATCTTACTAAAAATATTTGTCTCAT**
**ATTACTTGATTATTTATTAAATCAAAAAGAATTAATTAATTTTTTCTCATTTTACCCCTACAAT**
**TAATATAGTTTTAAAAGTTTTAAACAAATTTTGAAGAATCAAAATTTCTTTTTGCAAGAGACTT**
**ATTAATATAAACAAAGGATAAAATAATAAAATTTGTCAATTTATTGACGATCACTTAATAATCA**
**TATAAAATAGAAAATGTTTATCTAATATGAGACGGAGAAAATATATCCTAAAATATTTTTGGAC**
**AGATATGTGATATTCTAACCATTCACTAGACTATATTATGCATTTTAGCCGCCAATGACTTATT**
**TCAGCTTTAATTAATTAGGAAAGAGGAAACTGCCAATGAGGAAGAGTAGGGGCGTAGTTGCTGT**
**CGACGAAAAAAAGATAATACTCACTCTTTTCGATTTTTATTTTTATTTATCACTTTTAACCTAT**
**CATGTAAAAAGATAATTATTTTTTTCATGCTTTATCCTTAGTATTAAACAATTTAATAGGGATT**
**ATTTTGTAAAATATTTATATGAATAATTGTTTTCGTAATGAATTTGTCCGGTCAAACAATGATA**
**AATAAAAATGAATGAAGAGAGTAGAAAACAAAACAAAAGAACAAGTTGACAACTTGAGAGATTA**
**AAAGGGTCCAAAACGCCTTGGATTTTGAGATTCCATATGTGAAATTTCCATGAAATAATTGAAT**
**TTGTATTATTACAAGTCAAACTTTCCATTTCATTCCAACTAGCCATCTTGGTTTCAAAATTACA**
**CATTCATTCATTCACAGATCTAATATTCTTAATAGTGATTTCCACATATGGCTGAAGCTTTCAT**

TCAAGTTCTGCTAGACAATCTCACTTCTTTCCTCAAAGGGGAACTTGTATTGCTTTTCGGTTTT
CAAGATGAGTTCCAAAGGCTTTCAAGCATGTTTTCTACAATTCAAGCCGTCCTTGAAGATGCTC
AGGAGAAGCAACTCAACAACAAGCCTCTAGAAAATTGGTTGCAAAAACTCAATGCTGCTACATA
TGAAGTCGATGACATCTTGGATGAATATAAAACCAAGGCCACAAGATTCTCCCAGTCTGAATAT
GGCCGTTATCATCCAAAGGTTATCCCTTTCCGTCACAAGGTCGGAAAAGGATGGACCAAGTGA
TGAAAAAACTAAAGGCAATTGCTGAGGAAAGAAAGAATTTTCATTTGCACGAAAAAATTGTAGA
GAGACAAGCTGTTAGACGGGAAACAGGTACTCATCTTAAATTAGTATTACAACAACTAAGTTTA
TATTCATTTTTTTGGCAATTATCAAATTCAGAAAAGGGTTAAATATACTCATGTCCTATCGTAA
ATAGTGTATATATACCTCTCGTTGTACTTTCGATCTGAATATACTTGTCAAATCTGGCAAGCTC
AGAATCAAATTATCCACCCCAACTTTTAAATACTCGATATCTTTAGAAATCCACCTGTCTAACT
CATCCACTACCCATTCCCTTTGCTTTGAATTCTTTTCTTTACCTATAAACTTGGAACACTCGAT
CCGTTTTGCTTTTCTTAACAAAGCAGCTCAGAGAAAAGAGGTTTTCTTCTATTCTGTTTCTCTG
TGTGCTGCACTTGGGTCCTTAATCCCATTAAAAACAGGGCATGTTAATCCCAACGACGGTAGCC
TTTCCTGACAGCTGACTGTAAATTTTGTCTAACAAAGAAAAAAAAAGATTAGACATGTTTTTCC
TTGTCATTGATTAGGCTGGATTTCTTTCAGAGTGGAACATAGGGGATATATTGGACCAAAAGTA
GAATGGGTATATATTTAAAGTATTTCTGATAGAACAGGAGTATATTGTGCGAAAATATCCTCTA
TTTTCTGTTGTCTCCTAATGAGTTTGAATGTAATAATATTCTCATGTGGACATTGCTTGCACCA
GGTTCTGTATTAACCGAACCGCAGGTTTATGGAAGAGACAAAGAGAAAGATGAGATAGTGAAAA
TCCTAATAAACAATGTTAGTGATGCCCAACACCTTTCAGTCCTCCCAATACTTGGTATGGGGGG
ATTAGGAAAAACGACTCTTGCCCAAATGGTCTTCAATGACCAGAGAGTTACTGAGCATTTCCAT
TCCAAAATATGGATTTGTGTCTCGGAAGATTTTGATGAGAAGAGGTTAATAAAGGCAATTGTAG
AATCTATTGAAGGAAGGCCACTACTTGGTGAGATGGACTTGGCTCCACTTCAAAAGAAGCTTCA
GGAGTTGCTGAATGGAAAAAGATACTTGCTTGTCTTAGATGATGTTTGGAATGAAGATCAACAG
AAGTGGGCTAATTTAAGAGCAGTCTTGAAGGTTGGAGCAAGTGGTGCTTCTGTTCTAACCACTA
CTCGTCTTGAAAAGGTTGGATCAATTATGGGAACATTGCAACCATATGAACTGTCAAATCTGTC
TCAAGAAGATTGTTGGTTGTTGTTCATGCAACGTGCATTTGGACACCAAGAAGAAATAAATCCA
AACCTTGTGGCAATCGGAAAGGAGATTGTGAAAAAAAGTGGTGGTGTGCCTCTAGCAGCCAAAA
CTCTTGGAGGTATTTTGTGCTTCAAGAGAGAAGAAAGAGCATGGGAACATGTGAGAGACAGTCC
GATTTGGAATTTGCCTCAAGATGAAAGTTCTATTCTGCCTGCCCTGAGGCTTAGTTACCATCAA
CTTCCACTTGATTTGAAACAATGCTTTGCGTATTGTGCGGTGTTCCCAAAGGATGCCAAAATGG
AAAAAGAAAAGCTAATCTCTCTCTGGATGGCGCATGGTTTCTTTTATCAAAAGGAAACATGGA
GCTAGAGGATGTGGGCGATGAAGTATGGAAAGAATTATACTTGAGGTCTTTTTTCCAAGAGATT
GAAGTTAAAGATGGTAAAACTTATTTCAAGATGCATGATCTCATCCATGATTTGGCAACATCTC
TGTTTTCAGCAAACACATCAAGCAGCAATATCCGTGAAATAAATAAACACAGTTACACACATAT
GATGTCCATTGGTTTCGCCGAAGTGGTGTTTTTTACACTCTTCCCCCCTTGGAAAAGTTTATC
TCGTTAAGAGTGCTTAATCTAGGTGATTCGACATTTAATAAGTTACCATCTTCCATTGGAGATC
TAGTACATTTAAGATACTTGAACCTGTATGGCAGTGGCATGCGTAGTCTTCCAAAGCAGTTATG
CAAGCTTCAAAATCTGCAAACTCTTGATCTACAATATTGCACCAAGCTTTGTTGTTTGCCAAAA
GAAACAAGTAAACTTGGTAGTCTCCGAAATCTTTTACTTGATGGTAGCCAGTCATTGACTTGTA
TGCCACCAAGGATAGGATCATTGACATGCCTTAAGACTCTAGGTCAATTTGTTGTTGGAAGGAA
GAAAGGTTATCAACTTGGTGAACTAGGAAACCTAAATCTCTATGGCTCAATTAAAATCTCGCAT
CTTGAGAGAGTGAAGAATGATAAGGACGCAAAAGAAGCCAATTTATCTGCAAAAGGGAATCTGC
ATTCTTTAAGCATGAGTTGGAATAACTTTGGACCACATATATATGAATCAGAAGAAGTTAAAGT
GCTTGAAGCCCTCAAACCACACTCCAATCTGACTTCTTTAAAAATCTATGGCTTCAGAGGAATC
CATCTCCCAGAGTGGATGAATCACTCAGTATTGAAAAATATTGTCTCTATTCTAATTAGCAACT
TCAGAAACTGCTCATGCTTACCACCCTTTGGTGATCTGCCTTGTCTAGAAAGTCTAGAGTTACA
CTGGGGGTCTGCGGATGTGGAGTATGTTGAAGAAGTGGATATTGATGTTCATTCTGGATTCCCC
ACAAGAATAAGGTTTCCATCCTTGAGGAAACTTGATATATGGGACTTTGGTAGTCTGAAAGGAT
TGCTGAAAAAGGAAGGAGAAGAGCAATTCCCTGTGCTTGAAGAGATGATAATTCACGAGTGCCC
TTTTCTGACCCTTTCTTCTAATCTTAGGGCTCTTACTTCCCTCAGAATTTGCTATAATAAAGTA
GCTACTTCATTCCCAGAAGAGATGTTCAAAAACCTTGCAAATCTCAAATACTTGACAATCTCTC

**GGTGCAATAATCTCAAAGAGCTGCCTACCAGCTTGGCTAGTCTGAATGCTTTGAAAAGTCTAAA**
**AATTCAATTGTGTTGCGCACTAGAGAGTCTCCCTGAGGAAGGGCTGGAAGGTTTATCTTCACTC**
**ACAGAGTTATTTGTTGAACACTGTAACATGCTAAAATGTTTACCAGAGGGATTGCAGCACCTAA**
**CAACCCTCACAAGTTTAAAAATTCGGGGATGTCCACAACTGATCAAGCGGTGTGAGAAGGGAAT**
**AGGAGAAGACTGGCACAAAATTTCTCACATTCCTAATGTGAATATATATATTTAAGTTATTTGC**
**TATTGTTTCTTTGTTTGTGAGTCTTTTTGGTTCCTGCCATTGTGATTGCATGTAATTTTTTTCT**
**AGGGTTGTTTCTTTATGAGTCTCTCTCTCATTGGATGTAATTTTCTTTTGGAAACAAATCTGTC**
**AATTGATTTGTATTATACGCTTTCAGAATCTATTACTTATTTGTAATTGTTTCTTTGTTTGTAA**
**ATTGTGAGTATCTTATTTTATGGAATTTTCTGATTTTATTTTGAAAACAAATCAATGATTTGTA**
**AGATCCATCTGTATTATACTCCCTTCGTCTCATTTTATGTGTCACCTGTCGGATTTCGAGATTC**
**AAACAAATCTATCTTTGATCGTAAATTTTTAATAGATCTTTTAAACATTTTGAATTATCAATTA**
**TTGTGACTTTAGT***GGCTAGACTAGTGGATCCGATATCGCCCAGCTTCACGCTGCCGCAAGCACT*
*CAGGGCGCAAGGGCTGCTAAAGGAAGCGGAACACGTAGAAAGCCAGTCCGCAGAAACGGTGCTG*
*ACCCCGGATGAATGTCAGCTACTGGGCTATCTGGACAAGGGAAAACGCAAGCGCAAAGAGAAAG*
*CAGGTAGCTTGCAGTGGGCTTACATGGCGATAGCTAGACTGGGCGGTTTTATGGACAGCAAGCG*
*AACCGGAATTGCCAGCTGGGGCGCCCTCTGGTAAGGTTGGGAAGCCCTGCAAAGTAAACTGGAT*
*GGCTTTCTTGCCGCCAAGGATCTGATGGCGCAGGGGATCAAGGGAATTCACTGGCCGTCGTTTT*
*ACAACGACTCAGCTGCTTGGTAATAATTGTCATTAGATTGTTTTTATGCATAGATGCACTCGAA*
*ATCAGCCAATTTTAGACAAGTATCAAACGGATGTTAATTCAGTACATTAAAGACGTCCGCAATG*
*TGTTATTAAGTTGTCTAAG*

Figure 2d of 5

| Event D | Border | Sense primer | SEQ-ID-No. | Antisense primer | SEQ-ID-No. | Annealig temperature [°C] | Product length (bp) (range) |
|---|---|---|---|---|---|---|---|
| | LB | TCAAACGGATGTTAATTCAGTACATT | 4 | CCAGTTCCCAATTGACTACTAGAAA | 5 | 52 | 131 (126 to 136) |
| | LB | TCTGTTGAATTACGTTAAGC | 6 | CTCAGAAGAAAGAATTGTTC | 7 | 48 | 510 (505 to 515) |
| | LB | GTTTCTTAAGATTGAATCCTGTTGC | 8 | GCCCATTCTCTATTTTACTCACTAA | 9 | 51 | 630 (625 to 635) |
| | RB | CCAAGATAGTGTTTCAGGAAAGTTATT | 12 | AAATTCATGGTAGAACTGGAGGAG | 13 | 52 | 287 (282 to 292) |
| | RB | AACTGAATTTTGGGATTGAG | 14 | GAGTCAGTTAAATTAACTGCTTCAG | 15 | 49 | 882 (877 to 887) |
| | RB | ACAAGAATAGCAAGGATTATCC | 16 | GAAGTTCGAACAACATTCTT | 17 | 49 | 832 (827 to 837) |
| Left-flanking region to blb1 | LB | GTGAACTAGGAAACCTAAATC | 10 | CAACTAATAAAACCAAGGAC | 11 | 52 | 4757 (4752 to 4762) |
| Right flanking region to Blb1 | RB | AACTGAATTTTGGGATTGAG | 18 | ATGTAGCAGCATTGAGTTTT | 19 | 50 | 9910 (9905 to 9915) |

Figure 2 e of 5

**Flanking genomic sequence left border**

GAAACTCAGTTGGAGATTACGTTTAAGGGATGGCCTACATGGATAAGGTTATCTACAATGATGC
AATGGAGATAATAATAGTACTCCTATGTGATTTCTTCGTTTTAATTTGTTTATTTTATTTTCAT
TTAATTTGATTTGATACGTAGTTTAAAAAAATAAAAAAGATATTTAAATTTTGTGATGTTAGAT
GAAACATACGTTAAATGCATCAAAATGTCATTTAAATTTGTCTTGAACATATAGTATTAGAAAG
TTGAAATTAAAGAGCTATCAAAAAAGAAGAAGATATGACTAAAAAAAAATAGCACAAGCAAATTG
AAACTAAAAGAGTAATTAACAATAACAATTTTTTTTAAAATGATACATAATACTCACTCTTTC
TCAATATGTGGCACAAAAATATCAAGAATCAAACTATTTAATTTTGACTATAAATTTAGAAATA
AATTTTTTAAATTTCTTAAAAAACAAAATTTACAAAGCAAATACTATATAAAAAATATTATAAA
TTATAATAATTAATAATTCAAAATAGTTAACCATACAAACTGAATTTTGGGATTGAGTTTCTAT
TTAAATTTTCGTGAGTAAAGATTAAAGTTCAAATTAACTTTTTATAAAATGAGTTTTCAATTTT
AAAAAGTCAAACAAAATATATATAGAATATAAAGTGAGAAACCAAATAAAACAAGAATAGCAAG
GATTATCCATTTAGGAAATAAGATGAAAGAATCAAATCAACTAATAAAAACTTTAATCCTATAA
GATAAGCTTGCCAATAATTTAAAATAGCATGATGCGTCTAAGAGGGTATCAATATATTGTTATC
CTTTAAAGACCAAGATAGTGTTTCAGGAAAGTTATTTCATTTCCAAATTTAAATAAAAGAAAAT
AATTATTCAAAATTCTTATGTCTTTTTTATTGATTTATAGTCGAAGATCTTTCAAAAATAATTT
TTCTATAGGTAAAAAAGGATAATTT (SEQ-ID-No. 20)

**Flanking genomic sequence right border**

TTGTCCTTGGTTTTATTAGTTGTTATTGTTGTTTTGTCTTTTCAATTTCTAGTAGTCAATTGGG
AACTGGAAGTACAAAGTTGAATTTTTAGACTTATGTATACAAATTGAAAATGTATCAAAAAGTA
GTGACACTTATAAAAAGAACAATTCTTTCTTCTGAGAACATCTTCAACCCACCTCTATTTTACT
CTCCATTCTCTATATTTAGTGAGTAAAATAGAGAATGGGCACTCCAACCCACCTCCATATCACT
CTCCATTCTTCATATTTAGAGAGTCATATTATTTTTATTATTATTTTTATTACTTTCTAATTAA
TATATTATTTTACATATAATGTCATTAATTAAATATCTAATATTCATAATTCTTTTTAAATGTC
ATATTATATTTTAAAAAATATATTATTTAATATATACTACTTTCATCCCGAATTAATAGTATTT
TAATTTTTTCTAATTTTCGACAATAATATAATTTGATTTTATTATTAATTTTCACTATAAATAA
TACACAAAATTAAAATGATAAGATGAAAAAATTAATTTAAAAATACAATACATAATATGATAAT
TTAAATACAGGATAACAATACAATACATGACATAATAATTTAAATACAAGATAAAATACAATAC
ATAACATAATAATCAATTCGTGATGAAACAAGAATCATGCCAAAAAGATATTGAACGTGCATTC
GAAGTTTTGCAATCACGTTTTGCAATTATTGCAAGACCGTCACGTTTTTGGAGAAAGGAAGTGT
GACATGATATAATGACTACATGTATTATACTGCACACCATGATAATTGAGGATGAACATGATCT
TAATGCACCAAATCAAGATGCCGTAGAGGCTCCAACTCCAACGACAAAAATGATGGTAGATGAA
AATCTTCGGTTTGAACAATTTTTAGCTAGACATAAAAAAGTTAAGGACAAAAATGCTCATTTTG
AACTCCGTAATGCATTAATAGAGCATTTATGACAGCAACGTGATAATTTCGAAACTTGAGTGTT
TATGTAATTATATTTCACTTTTATTTGAATTTGTCCATTAATTTGTATATTATATAATATTTAT
TTACAATTTATGTTATTTAAAAATTTAGAATAAAATAAAATTTTGAAAAAATAAAATTTTATAT
CACATGAAAATTATATAAAGTAATTATTGGGGAAAAAAATAAAGGATTTATATTATGAAATAAG
AAAATAAGAATGAATAGAAATAATAATAATATAATATTGAGGAGAAATAATTATTTTTTTAGA
GAGTGAAATAGAGAATTGGGTTGAAATTGATTGTCTGAAAAAATAAAAAACTCTATATTTGGAG
AGTAAAATAGAGTGTAGGGTTGGAGACGTCA (SEQ-ID-NO.21)

Figure 2 e - continued

**Part of flanking genomic sequence left border including part of insert**
Partial insertion sequence is bold (T-DNA, Vector), Primer-binding sites are underlined:

**Gtttcttaagattgaatcctgttgccggtcttgcgatgattatcatataatttctgttgaattacgttaagcatgtaataattaacatgta
atgcatgacgttatttatgagatgggtttttatgattagagtcccgcaattatacatttaatacgcgatagaaaacaaaatatagcgc
gcaaactaggataaattatcgcgcgcggtgtcatctatgttactagatcgggaattcactggccgtcgttttacaacgactcagctgc
ttggtaataattgtcattagattgtttttatgcatagatgcactcgaaatcagccaattttagacaagtatcaaacggatgttaattca
gtacattaaagacgtccgcaatgtgttattaagttgtctaag**ttgtccttggttttattagttgttattgttgttttgtcttttcaatttctagtagtc
aattgggaactggaagtacaaagttgaatttttagacttatgtatacaaattgaaaatgtatcaaaaagtagtgacacttataaaaagaacaattc
tttcttctgagaacatcttcaacccacctctattttactctccattctctatatttagtgagtaaaatagagaatgggcactccaacccacctccata
tcactctccattcttcatatttagagagtcatattatttttattattatttttattactttctaattaatatattattttacatataatgtcattaattaaatatcta
atattcataattctttttaaatgtcatattatattttaaaaaatatattatttaatatatactactttcatcccgaattaatagtattttaattttttctaattttc
gacaataatataatttgattttattattaattttcactataaataatacacaaaattaaaatgataagatgaaaaaattaatttaaaaatacaatacata
atatgataatttaaatacaggataacaatacaatacatgacataataatttaaatacaagataaaatacaatacataacataataatcaattcgtga
tgaaacaagaatcatgccaaaaagatattgaacgtgcattcgaagttttgcaatcacgttttgcaattattgcaagaccgtcacgtttttggaga
aaggaagtgtgacatgatataatgactacatgtattatactgcacaccatgataattgaggatgaacatgatcttaatgcaccaaatcaagatg
ccgtagaggctccaactccaacgacaaaaatgatggtagatgaaaatcttcggtttgaacaatttttagctagacataaaaaagttaaggaca
aaaatgctcattttgaactccgtaatgcattaatagagcatttatgacagcaacgtgataatttcgaaacttgagtgtttatgtaattatatttcacttt
tatttgaatttgtccattaatttgtatattatataatatttatttacaatttatgttatttaaaaatttagaataaaataaaattttgaaaaaataaaattttat
atcacatgaaaattatataaagtaattattggggaaaaaaataaaggatttatattatgaaataagaaaataagaatgaatagaaataataataat
ataatattgaggagaaataattatttttttagagagtgaaatagagaattgggttgaaattgattgtctgaaaaaataaaaaactctatatttgga
gagtaaaatagagtgtagggttggagacgtca (SEQ-ID-No. 22)

**Part of flanking genomic sequence right border including part of insert**
Partial insertion sequence is bold (T-DNA, Vector), Primer-binding sites are underlined:

gaaactcagttggagattacgtttaagggatggcctacatggataaggttatctacaatgatgcaatggagataataatagtactcctatgtgat
ttcttcgttttaatttgtttattttattttcatttaatttgatttgatacgtagtttaaaaaaataaaaaagatatttaaattttgtgatgttagatgaaacata
cgttaaatgcatcaaaatgtcatttaaatttgtcttgaacatatagtattagaaagttgaaattaaagagctatcaaaaaagaagaagatatgact
aaaaaaaatagcacaagcaaattgaaactaaaagagtaattaacaataacaattttttttaaaatgatacataatactcactctttctcaatatgtg
gcacaaaaatatcaagaatcaaactatttaattttgactataaatttagaaataaatttttaaatttcttaaaaaacaaaatttacaaagcaaatact
atataaaaaatattataaattataataattaataattcaaaatagttaaccatacaaactgaattttgggattgagtttctatttaaattttcgtgagtaa
agattaaagttcaaattaactttttataaaatgagttttcaattttaaaaagtcaaacaaaatatatatagaatataaagtgagaaaccaaataaaa
caagaatagcaaggattatccatttaggaaataagatgaaagaatcaaatcaactaataaaaactttaatcctataagataagcttgccaataat
ttaaaatagcatgatgcgtctaagagggtatcaatatattgttatcctttaaagaccaagatagtgtttcaggaaagttatttcatttccaaatttaa
ataaaagaaaataattattcaaaattcttatgtctttttattgatttatagtcgaagatctttcaaaaataatttttctataggtaaaaaaggataattt**c
aatttcacacaggaaacagctatgaccatgattacgccaagctggcgcgccaagcttgcatgcctgcaggtcgactctagaggatc
tagaatcaccgaacctcccctcggtacagctcctccagttctaccatgaatttcatccactgattcctcttcaatcgccattgcagattc
tctcgatctatgctcaaaaaatcccgagataaaaccctagatctgcttcaaatgctctgataccatgtaatttcagtgaattctaacta
aacaatggagagaattaactattttagaaagactgattgaaggagaagaagagagaaaaattctatattgaactcatgaaccaaa
atgaatgaaaaaaataatgagaagaactatactattacaatctatatatctctatttatattctaatctgaagcagttaatttaactga
ctctaacaactagactgataggtgtacattttctgttagtgcactgcagtgcatttaactaactgcttaacataaagaatgttgttcga
acttc** (SEQ-ID-No. 23)

**Figure 2 f of 5**

**Partial-sequences of the insert with flanking region**
Insertion sequence is bold (T-DNA, Vector), Primer-binding sites are underlined:

**Event: A**

Left border - PMA16
**Agcatgtaataattaacatgtaatgcatgacgttatttatgagatgggtttttatgattagagtcccgcaattatacatttaatacgcgatagaa**
**aacaaaatatagcgcgcaaactaggataaattatcgcgcgcggtgtcatctatgttactagatcgggaattcactggccgtcgttttacaa**
**cgactcagctgcttggtaataattgtcattagattgtttttatgcatagatgcactcgaaatcagccaattttagacaagtatcaaacggatgtt**
**aattcagtacattaaagacgtccgcaatgtg**aactcatttgatcaagaaatgactatgggacattaaagtaaaaatcataaagacatttttttcata
ttccataataaaagaataataacagaatattagtggaaaaaaatgaaaaatttctaaatgtaggggtgggggtggggaaggagatacattaggtttc
taaaaataaataaaaaacgaactaaaccaagattgatcaaatcgaaaaaatgcgttaattggtttgattttaataattttaaaatgaattaaattgatttg
attttagtggcaaaaagttgatctaaattgatctctgagtacctttagccccactaattccttttaagaggataaacgttttcaattgtaaaagacaattagg
actaaatagctgaaagaaaggatagttttaacccaataatcaatgttaagacaatttaaatttaatataaagaagaaaaattaaaactattttcaatatg
ttagggacaagggaataaatcttaaaacaatcgcaatatcaaaattgggcgctaataataataacttaattaattaactattctagtaaagagtcgata
tatttctaattaatcctctcagctttcctcgataagaagtagtaatggatataggattgtaaggtcatgcgagctct (SEQ-ID-No. 24)

#1: Product of length 63 (rating: 171)
Contains region of the molecule from 281 to 343
Tm: 64.2 C TaOpt: 45.0 C GC: 36.5
Sense Primer:
TAATTCAGTACATTAAAGACGTCCG (SEQ-ID-No. 25)
Similarity: 100.0%
Length: 25 Tm: 51.8 C GC: 36.0
dH: -185.5 kcal/mol dS: -488.6 cal/mol dG: -38.0 kcal/mol
Antisense Primer:
GTCCCATAGTCATTTCTTGATCA(SEQ-ID-No. 26)
Similarity: 100.0%
Length: 23 Tm: 50.1 C GC: 39.1
dH: -160.9 kcal/mol dS: -419.9 cal/mol dG: -33.9 kcal/mol
Tm Difference: 1.7
GC Difference: 3.1

Right border-PMA16
gattttattttattaataaaaattgaaaaaaattaaatcaaactaaaacaataaattattcatataatttttataattatatatatacacacataatatattaatt
ttacaattatttataatcatttatatactttttcaccattatcatatttgtctctgataggctaataaactatgtatttaatgttgctgtacatttt**ctgatagtttaaa**
**ctgaaggcgggaaacgacaatcagatctagtaggaaacagctatgaccatgattacgccaagcttgcatgccctggcacgacaggttt**
**cccgactggaaagcgggcagtgagcgcaacgcaattaatgtgagttagctcactcattaggcaccccaggctttacactttatgcttccg**
**gctcgtatgttgtgtggaattgtgagcggataacaatttcacacaggaaacagctatgaccatgattacgccaagctggcgcgccaagct**
**tgcatgcctgcaggtcgactctagaggatctagaatcaccgaacctcccctcggtacagctcctccagttctaccatgaatttcatccact**
**gattcctcttcaatcgccattgca**(SEQ-ID-No. 27)

#1: Product of length 91 (rating: 171)
Contains region of the molecule from 155 to 245
Tm: 66.5 C TaOpt: 46.1 C GC: 34.1
Sense Primer:
TGTCTCTGATAGGCTAATAAACTATG
Similarity: 100.0%(SEQ-ID-No. 28)
Length: 26 Tm: 48.7 C GC: 34.6
dH: -185.4 kcal/mol dS: -493.2 cal/mol dG: -36.6 kcal/mol
Antisense Primer:
TAGATCTGATTGTCGTTTCCC(SEQ-ID-No. 29)
Similarity: 100.0%
Length: 21 Tm: 48.4 C GC: 42.9
dH: -152.6 kcal/mol dS: -398.0 cal/mol dG: -32.2 kcal/mol
Tm Difference: 0.2
GC Difference: 8.2

**Figure 2 g of 5**

**partial-sequences of the insert with flanking region**
Insertion sequence is bold (T-DNA, Vector), Primer-binding sites are underlined:

**Event B**

Left border - PMA16

**Ggattaaatactgagagctcgaatttccccgatcgttcaaacatttggcaataaagtttcttaagattgaatcctgttgccggtcttgcgatg attatcatataatttctgttgaattacgttaagcatgtaataattaacatgtaatgcatgacgttatttatgagatgggttttatgattagagtccc gcaatt**ggatttgtaccagcaacaagaagacataaaattgcacgttttgccttttaaatgagttagtttttatttttttatccttcaaattgggtgatgtttaattt tgttcttttctaatcgaacttatacctagtgatacataagttctttaaaattaaaatttagtccgagaataacttatgaaatattacgatataaggataaaact tcaaatgaccaacacaaaactcg (SEQ-ID-No.30)

#1: Product of length 100 (rating: 171)
Contains region of the molecule from 151 to 250
Tm: 67.1 C TaOpt: 46.8 C GC: 34.0
Sense Primer:
ATGACGTTATTTATGAGATGGGT(SEQ-ID-No. 31)
Similarity: 100.0%
Length: 23 Tm: 49.1 C GC: 34.8
dH: -169.1 kcal/mol dS: -445.1 cal/mol dG: -34.6 kcal/mol
Antisense Primer:
ATTTAAAAGGCAAAACGTGC(SEQ-ID-No. 32)
Similarity: 100.0%
Length: 20 Tm: 49.3 C GC: 35.0
dH: -164.9 kcal/mol dS: -432.6 cal/mol dG: -34.1 kcal/mol
Tm Difference: 0.2
GC Difference: 0.2

Right-border - PMA16

**T**ttttttgttatggagaaagcagagataatagttatagaattgttggttaccctttagatttcaagagtatagaatgatgtttagttcatttgagaatagcgct atagcagtatatttgcatacataaaccagaaacatacaaccatcacctatctcgaatgatcaagccaaactttccccttgcatagtgtcttggcttgttctt agtttaaaacaataatataaacaacaataaattactaatggccgaataatttatctgaattttaacaaattttattctcggcaaaaatatgatcaactttcc caacttagggctccttcccaccgtgaaatcatgtcaacaccttctcccaagggcaataacttagtttctacggcttaagcaataatgcacaaggttgga gagtcaaacttttgcctttatcgataaattcaatgggaaaactaatgcaaactgtccaaaatcacccctcccaagtgtaagaactcaatgtacagaaa ataggtgttttgggttattttctgcataaaattctgattcagctcaaaatagtagtccaacccgtgatagcgacaactagtctcactatagcgatctccaca ttctgctatagcgagacaccttttgccacctaaactccagaaaaatgtcatttgtacaacacaaactctccttcatgtcaagttcaatttcaggagtt**cat gccctggcacgacaggtttcccgactggaaagcgggcagtgagcgcaacgcaattaatgtgagttagctcactcattaggcaccccag gctttacactttatgcttccggctcgtatgttgtgtggaagttg** (SEQ-ID-No. 33)

#1: Product of length 94 (rating: 171)
Contains region of the molecule from 681 to 774
Tm: 73.2 C TaOpt: 51.7 C GC: 50.0
Sense Primer:
TTCATGTCAAGTTCAATTTCAGG(SEQ-ID-No. 34)
Similarity: 100.0%
Length: 23 Tm: 51.2 C GC: 34.8
dH: -162.8 kcal/mol dS: -423.9 cal/mol dG: -34.6 kcal/mol
Antisense Primer:
ACTCACATTAATTGCGTTGCG(SEQ-ID-No. 35)
Similarity: 100.0%
Length: 21 Tm: 53.0 C GC: 42.9
dH: -161.7 kcal/mol dS: -418.3 cal/mol dG: -35.2 kcal/mol
Tm Difference: 1.8
GC Difference: 8.1

Figure 2 h of 5

**partial-sequences of the insert with flanking region**
Insertion sequence is bold (T-DNA, Vector), Primer-binding sites are underlined:

**Event C**
Left-border - PMA16
**Tgttgccggtcttgcgatgattatcatataatttctgttgaattacgttaagcatgtaataattaacatgtaatgcatgacgttatttatgagatg ggtttttatgattagagtcccgcaattatacatttaatacgcgatagaaaacaaaatatagcgcgcaaactaggataaattatcgcgcgcg gtgtcatctatgttactagatcgggaattcactggccgtcgttttacaacgactcagctgcttggtaataattgtcattagattgtttttatgcat agatgcactc**caaagaatgtaatattaatgtaaataattcaaagaatgtaatattaatgtaaataattcaaaggtcaaggcggcttgcttggagaaat gagagattgtttagttgactaatgatatgtttaattaagaagaccattttagcattcatttttacattttcttactatgaatagcagaacaaacgacgcaaat agaacctttttcttcttccattttctcttaattttgacttagcaaaatgatgaggtaccacaaatcacaataattaatacttttcaaagatatcaataaattaat gatgatccttaaattttaggtaaaagtgttggaactgtcatgattcagaccgtcgtgattgacacccacactagccctccggtgggagaaccattacta caacccaaactaacaaattttatgaaaactaaagcatttaaagatatagaagcaggtataaatgacaataaaaatggagtccccataaactccaa ggtttttaacaaacaactaatcaaactaatgcggaagaacaaccccaaaactaaacaacaccttaagcaaagtctgagtccgaaaagagtggac ttaaacaagaaagatccatggcagcctgaaagaactgactcacccttgaatccggtcacgctcaatatccgcctaaagatgccctgtactcaacaa aaaaacaagcaagtacagtatcagtacacaaccacagagtactggtaggatcacgcgactatcctaataagtgaaacacatgcaagtaaccac aacattataaaacaagcatataccgaacatatacagtattagtcatcttttcaagatcaatgtagcatcacacgttctcaataatacacattggttatcatt ttagagcagcatacagtcttccaatatcaatcatcattag (SEQ-ID-No. 36)

#1: Product of length 118 (rating: 171)
Contains region of the molecule from 247 to 364
Tm: 64.6 C TaOpt: 45.0 C GC: 25.4
Sense Primer:
GCTTGGTAATAATTGTCATTAGATTG (SEQ-ID-No. 37)
Similarity: 100.0%
Length: 26 Tm: 50.8 C GC: 30.8
dH: -192.1 kcal/mol dS: -509.2 cal/mol dG: -38.5 kcal/mol
Antisense Primer:
GCCTTGACCTTTGAATTATTTAC (SEQ-ID-No. 38)
Similarity: 100.0%
Length: 23 Tm: 49.1 C GC: 34.8
dH: -177.4 kcal/mol dS: -469.3 cal/mol dG: -35.7 kcal/mol
Tm Difference: 1.8
GC Difference: 4.0

Figure 2 h - continued

Right-border - PMA16
Atacaccataccttaggagaaaaacacaactaacgttatttttatcctgcaaatggaaaggtctcaaccatagcaacggataggatcattgcagcctt
taccagcagactgctttgagccaaaccgaccccccttttgcaaggtactgctggtgatcttcctcagctcgataaaatctcacatcaaatgactcccaa
gtgtttagatataaattgcgtcatatttgaggggttctttaaagttgaattaacaataatatttagttgatattgtttgaacacccattccacttggccttttttaaa
agttagcaaggttaattttcttttacttgaaacaattggaataaaaaaataattatttcctttgtctatatctcaatttatatgatatagtttaactatattagaaa
agttaatcgtaataactcatataattgtcaaattattatacgctttgacctcaatctctccgaattacctcaagcgtttgggccctcatattttgaactcttcgg
ctttttaacctaaaaggttcgttataattgaattatgaactcacttttatgtgaccctaacttttcctctcgttctgatgcagaattttctaactcaaatttcataat
attcgattcttgttattgagtccaaatcataatatttgtttcttctattaaatctgattatgaaatctctagtcgcctcgtgaatttataggctacaatctcattttcc
ctcaatgaatattcaattcttgttattgagttcaaatcataatattcgtttcttctattaaatctgattatgaaatctctagtcgcctcgtgaatttataggctaca
atctcattttc**caaacactgatagtttaaactgaaggcgggaaacgacaatcagatctagtaggaaacagctatgaccatgattacgccaa**
**gcttgcatgccctggcacgacaggtttcccgactggaaagcgggcagtgagcgcaacgcaattaatgtgagttagctcactcattaggc**
**accccaggctttacactttatgcttccggctcgtatgttgtgtggaattgtgagcggataacaatttcacacaggaaacagctatgaccatg**
**attacgccaagctggcgcgccaagcttgcatgcctgcaggtcgactctagaggatctagaatcaccgaacctcccctcggtacagctc**
**ctc** (SEQ-ID-No. 39)

Contains region of the molecule from 589 to 905
Tm: 70.4 C TaOpt: 50.1 C GC: 30.9
Sense Primer:
TCTGATGCAGAATTTTCTAACTCAA (SEQ-ID-No. 40)
Similarity: 100.0%
Length: 25 Tm: 52.2 C GC: 32.0
dH: -177.8 kcal/mol dS: -465.8 cal/mol dG: -37.1 kcal/mol
Antisense Primer:
TTCCTACTAGATCTGATTGTCGTTTC(SEQ-ID-No. 41)
Similarity: 100.0%
Length: 26 Tm: 52.3 C GC: 38.5
dH: -184.4 kcal/mol dS: -484.7 cal/mol dG: -38.1 kcal/mol
Tm Difference: 0.1
GC Difference: 6.5

Figure 2 i of 5

**partial-sequences of the insert with partial flanking region**
Insertion sequence is bold (T-DNA, Vector), Primer-binding sites are underlined:

**Elite-Event D**
Left-border - PMA16
**Attaaatactgagagctcgaatttccccgatcgttcaaacatttggcaataaagtttcttaagattgaatcctgttgccggtcttgcga**
**tgattatcatataatttctgttgaattacgttaagcatgtaataattaacatgtaatgcatgacgttatttatgagatgggtttttatgatt**
**agagtcccgcaattatacatttaatacgcgatagaaaacaaaatatagcgcgcaaactaggataaattatcgcgcgcggtgtcatc**
**tatgttactagatcgggaattcactggccgtcgttttacaacgactcagctgcttggtaataattgtcattagattgtttttatgcatag**
**atgcactcgaaatcagccaattttagacaagtatcaaacggatgttaattcagtacattaaagacgtccgcaatgtgttattaagttg**
**tctaag**ttgtccttggttttattagttgttattgttgttttgtcttttcaatttctagtagtcaattgggaactggaagtacaaagttgaatttttagactta
tgtatacaaattgaaaatgtatcaaaaagtagtgacacttataaaaagaacaattctttcttctgagaacatcttcaacccacctctattttactctc
cattctctatatttagtgagtaaaatagagaatgggcactccaacccaccttcatc (SEQ-ID-No. 42)

Primer pair1
Sense Primer:
TCAAACGGATGTTAATTCAGTACATT (SEQ-ID-No. 4)
Similarity: 100.0%
Length: 26 Tm: 53.2 C GC: 30.8
dH: -189.1 kcal/mol dS: -496.8 cal/mol dG: -39.2 kcal/mol
Antisense Primer:
CCAGTTCCCAATTGACTACTAGAAA (SEQ-ID-No.5)
Similarity: 100.0%
Length: 25 Tm: 52.9 C GC: 40.0
dH: -184.0 kcal/mol dS: -482.6 cal/mol dG: -38.3 kcal/mol
Tm Difference: 0.3
GC Difference: 9.2

Primer pair2
Tm: 72.1 C TaOpt: 47.7 C GC: 32.9
Sense Primer:
TCTGTTGAATTACGTTAAGC (SEQ-ID-No. 6)
Similarity: 100.0%
Length: 20 Tm: 42.4 C GC: 35.0
dH: -147.0 kcal/mol dS: -389.3 cal/mol dG: -29.2 kcal/mol
Antisense Primer:
CTCAGAAGAAAGAATTGTTC SEQ-ID-No. 7)
Similarity: 100.0%
Length: 20 Tm: 40.4 C GC: 35.0
dH: -140.5 kcal/mol dS: -372.5 cal/mol dG: -27.7 kcal/mol
Tm Difference: 2.0
GC Difference: 0.0

**Figure 2i – continued**

Primer pair3

Contains region of the molecule from 54 to 683
Tm: 72.5 C TaOpt: 51.0 C GC: 33.3
Sense Primer:
GTTTCTTAAGATTGAATCCTGTTGC (SEQ-ID-No. 8)
Similarity: 100.0%
Length: 25 Tm: 52.6 C GC: 36.0
dH: -185.2 kcal/mol dS: -486.5 cal/mol dG: -38.4 kcal/mol
Antisense Primer:
GCCCATTCTCTATTTTACTCACTAA (SEQ-ID-No. 9)
Similarity: 100.0%
Length: 25 Tm: 50.4 C GC: 36.0
dH: -186.4 kcal/mol dS: -493.3 cal/mol dG: -37.5 kcal/mol
Tm Difference: 2.2
GC Difference: 0.0

Right-border - PMA16

Aatagttaaccatacaaactgaattttgggattgagtttctatttaaattttcgtgagtaaagattaaagttcaaattaactttttataaaatgagtttt
caattttaaaaagtcaaacaaaatatatatagaatataaagtgagaaaccaaataaaacaagaatagcaaggattatccatttaggaaataaga
tgaaagaatcaaatcaactaataaaaactttaatcctataagataagcttgccaataatttaaaatagcatgatgcgtctaagagggtatcaatat
attgttatcctttaaagaccaagatagtgtttcaggaaagttatttcatttccaaatttaaataaaagaaaataattattcaaaattcttatgtcttttta
ttgatttatagtcgaagatctttcaaaaataatttttctataggtaaaaaaggataattt**caatttcacacaggaaacagctatgaccatgatta**
**cgccaagctggcgcgccaagcttgcatgcctgcaggtcgactctagaggatctagaatcaccgaacctcccctcggtacagctcct**
**ccagttctaccatgaatttcatccactgattcctcttcaatcgccattgcagattctctcgatctatgctcaaaaaatcccgagataaa**
**accctagatctgcttcaaatgctctgataccatgtaatttcagtgaattctaactaaacaatggagagaattaactattttagaaaga**
**ctgattgaaggagaagaagagagaaaaattctatattgaactcatgaaccaaaatgaatgaaaaaaataatgagaagaactata**
**ctattacaatctatatatctctatttatattctaatctgaagcagttaatttaactgactctaacaactagactgataggtgtacattttct**
**gttagtgcactgcagtgcatttaactaactgcttaacataaagaatgttgttcgaacttcattcgaatagcttcaatgagaagcaaac**
**atgtgtacctgtaaagacacacagtaaaagtgttaataatgaataaatatgaataaatcaaataataaattaaaaataaaaacac**
**atccaattaacattggaggtcttgaaaatcgatggtaa (SEQ-ID-NO. 43)**

Primer pair 1:

Contains region of the molecule from 309 to 595
Tm: 72.0 C TaOpt: 51.3 C GC: 35.2
Sense Primer:
CCAAGATAGTGTTTCAGGAAAGTTATT (SEQ-ID-No. 12)
Similarity: 100.0%
Length: 27 Tm: 53.0 C GC: 33.3
dH: -199.8 kcal/mol dS: -527.9 cal/mol dG: -40.6 kcal/mol
Antisense Primer:
AAATTCATGGTAGAACTGGAGGAG (SEQ-ID-No. 13)
Similarity: 100.0%
Length: 24 Tm: 52.6 C GC: 41.7
dH: -176.6 kcal/mol dS: -461.8 cal/mol dG: -37.1 kcal/mol
Tm Difference: 0.4
GC Difference: 8.3

## Figure 2i – continued

### Primer pair 2

Contains region of the molecule from 17 to 898
Tm: 71.5 C TaOpt: 48.9 C GC: 30.0
Sense Primer:
AACTGAATTTTGGGATTGAG (SEQ-ID-No. 14)
Similarity: 100.0%
Length: 20 Tm: 45.9 C GC: 35.0
dH: -150.1 kcal/mol dS: -393.9 cal/mol dG: -30.9 kcal/mol
Antisense Primer:
GAGTCAGTTAAATTAACTGCTTCAG (SEQ-ID-No. 15)
Similarity: 100.0%
Length: 25 Tm: 48.8 C GC: 36.0
dH: -179.0 kcal/mol dS: -474.3 cal/mol dG: -35.8 kcal/mol
Tm Difference: 2.9
GC Difference: 1.0

### Primer pair 3

Contains region of the molecule from 118 to 949
Tm: 72.3 C TaOpt: 48.8 C GC: 32.3
Sense Primer:
ACAAGAATAGCAAGGATTATCC (SEQ-ID-No. 16)
Similarity: 100.0%
Length: 22 Tm: 46.8 C GC: 36.4
dH: -165.6 kcal/mol dS: -438.2 cal/mol dG: -33.2 kcal/mol
Antisense Primer:
GAAGTTCGAACAACATTCTT (SEQ-ID-No. 17)
Similarity: 100.0%
Length: 20 Tm: 43.4 C GC: 35.0
dH: -144.2 kcal/mol dS: -379.8 cal/mol dG: -29.2 kcal/mol
Tm Difference: 3.4
GC Difference: 1.4

Figure 2 j of 5

**Elite Event D**
**partial-sequences of the insert with partial flanking region**
Insertion sequence is bold (T-DNA, Vector), Primer-binding sites are underlined:

**Upper case bold – Blb1 expression cassette (promoter – gene –terminator)**
**Upper case bold italic – AHAS expression cassette (promoter – gene –terminator)**
**Lower case italic – flanking regions at left border**

Extra-primer for flanking region (left border) to BLB1 (over AHAS cassette):
Part of blb1 - blb1 terminator - ahas-cassette - flanking region left border

**GTGAACTAGGAAACCTAAATCTCTATGGCTCAATTAAAATCTCGCATCTTGAGAGAGTGAAGAA**
**TGATAAGGACGCAAAAGAAGCCAATTTATCTGCAAAAGGGAATCTGCATTCTTTAAGCATGAGT**
**TGGAATAACTTTGGACCACATATATATGAATCAGAAGAAGTTAAAGTGCTTGAAGCCCTCAAAC**
**CACACTCCAATCTGACTTCTTTAAAAATCTATGGCTTCAGAGGAATCCATCTCCCAGAGTGGAT**
**GAATCACTCAGTATTGAAAAATATTGTCTCTATTCTAATTAGCAACTTCAGAAACTGCTCATGC**
**TTACCACCCTTTGGTGATCTGCCTTGTCTAGAAAGTCTAGAGTTACACTGGGGGTCTGCGGATG**
**TGGAGTATGTTGAAGAAGTGGATATTGATGTTCATTCTGGATTCCCCACAAGAATAAGGTTTCC**
**ATCCTTGAGGAAACTTGATATATGGGACTTTGGTAGTCTGAAAGGATTGCTGAAAAAGGAAGGA**
**GAAGAGCAATTCCCTGTGCTTGAAGAGATGATAATTCACGAGTGCCCTTTTCTGACCCTTTCTT**
**CTAATCTTAGGGCTCTTACTTCCCTCAGAATTTGCTATAATAAAGTAGCTACTTCATTCCCAGA**
**AGAGATGTTCAAAAACCTTGCAAATCTCAAATACTTGACAATCTCTCGGTGCAATAATCTCAAA**
**GAGCTGCCTACCAGCTTGGCTAGTCTGAATGCTTTGAAAAGTCTAAAAATTCAATTGTGTTGCG**
**CACTAGAGAGTCTCCCTGAGGAAGGGCTGGAAGGTTTATCTTCACTCACAGAGTTATTTGTTGA**
**ACACTGTAACATGCTAAAATGTTTACCAGAGGGATTGCAGCACCTAACAACCCTCACAAGTTTA**
**AAAATTCGGGGATGTCCACAACTGATCAAGCGGTGTGAGAAGGGAATAGGAGAAGACTGGCACA**
**AAATTTCTCACATTCCTAATGTGAATATATATATTTAAGTTATTTGCTATTGTTTCTTTGTTTG**
**TGAGTCTTTTTGGTTCCTGCCATTGTGATTGCATGTAATTTTTTTCTAGGGTTGTTTCTTTATG**
**AGTCTCTCTCTCATTGGATGTAATTTTCTTTTGGAAACAAATCTGTCAATTGATTTGTATTATA**
**CGCTTTCAGAATCTATTACTTATTTGTAATTGTTTCTTTGTTTGTAAATTGTGAGTATCTTATT**
**TTATGGAATTTTCTGATTTTATTTTGAAAACAAATCAATGATTTGTAAGATCCATCTGTATTAT**
**ACTCCCTTCGTCTCATTTTATGTGTCACCTGTCGGATTTCGAGATTCAAACAAATCTATCTTTG**
**ATCGTAAATTTTTAATAGATCTTTTAAACATTTTGAATTATCAATTATTGTGACTTTAGT***GGCT*
*AGACTAGTGGATCCGATATCGCCCAGCTTCACGCTGCCGCAAGCACTCAGGGCGCAAGGGCTGC*
*TAAAGGAAGCGGAACACGTAGAAAGCCAGTCCGCAGAAACGGTGCTGACCCCGGATGAATGTCA*
*GCTACTGGGCTATCTGGACAAGGGAAAACGCAAGCGCAAAGAGAAAGCAGGTAGCTTGCAGTGG*
*GCTTACATGGCGATAGCTAGACTGGGCGGTTTTATGGACAGCAAGCGAACCGGAATTGCCAGCT*
*GGGGCGCCCTCTGGTAAGGTTGGGAAGCCCTGCAAAGTAAACTGGATGGCTTTCTTGCCGCCAA*
*GGATCTGATGGCGCAGGGGATCAA****GATCATGAGCGGAGAATTAAGGGAGTCACGTTATGACCCC***
***CGCCGATGACGCGGGACAAGCCGTTTTACGTTTGGAACTGACAGAACCGCAACGTTGAAGGAGC***
***CACTCAGCCGCGGGTTTCTGGAGTTTAATGAGCTAAGCACATACGTCAGAAACCATTATTGCGC***
***GTTCAAAAGTCGCCTAAGGTCACTATCAGCTAGCAAATATTTCTTGTCAAAAATGCTCCACTGA***
***CGTTCCATAAATTCCCCTCGGTATCCAATTAGAGTCTCATATTCACTCTCAATCCAGATCCCCG***
***GGTACCATGGCGGCGGCAACAACAACAACAACAACATCTTCTTCGATCTCCTTCTCCACCAAAC***
***CATCTCCTTCCTCCTCCAAATCACCATTACCAATCTCCAGATTCTCCCTCCCATTCTCCCTAAA***
***CCCCAACAAATCATCCTCCTCCTCCCGCCGCCGCGGTATCAAATCCAGCTCTCCCTCCTCCATC***
***TCCGCCGTGCTCAACACAACCACCAATGTCACAACCACTCCCTCTCCAACCAAACCTACCAAAC***
***CCGAAACATTCATCTCCCGATTCGCTCCAGATCAACCCCGCAAAGGCGCTGATATTCTCGTCGA***

*GGCTTTAGAACGTCAAGGCGTAGAAACCGTATTCGCTTACCCTGGAGGTGCATCAATGGAGATT*
*CACCAAGCCTTAACCCGCTCTTCCTCAATCCGTAACGTCCTTCCTCGTCACGAACAAGGAGGTG*

Figure 2j - continued

TATTCGCAGCAGAAGGATACGCTCGATCCTCAGGTAAACCAGGTATCTGTATAGCCACTTCAGG
TCCCGGAGCTACAAATCTCGTTAGCGGATTAGCCGATGCGTTGTTAGATAGTGTTCCTCTTGTA
GCAATCACAGGACAAGTCCCTCGTCGTATGATTGGTACAGATGCGTTTCAAGAGACTCCGATTG
TTGAGGTAACGCGTTCGATTACGAAGCATAACTATCTTGTGATGGATGTTGAAGATATTCCTAG
GATTATTGAGGAGGCTTTCTTTTTAGCTACTTCTGGTAGACCTGGACCTGTTTTGGTTGATGTT
CCTAAAGATATTCAACAACAGCTTGCGATTCCTAATTGGGAACAGGCTATGAGATTACCTGGTT
ATATGTCTAGGATGCCTAAACCTCCGGAAGATTCTCATTTGGAGCAGATTGTTAGGTTGATTTC
TGAGTCTAAGAAGCCTGTGTTGTATGTTGGTGGTGGTTGTTTGAACTCTAGCGATGAATTGGGT
AGGTTTGTTGAGCTTACGGGAATCCCTGTTGCGAGTACGTTGATGGGGCTGGGATCTTATCCTT
GTGATGATGAGTTGTCGTTACATATGCTTGGAATGCATGGGACTGTGTATGCAAATTACGCTGT
GGAGCATAGTGATTTGTTGTTGGCGTTTGGGGTAAGGTTTGATGATCGTGTCACGGGTAAACTT
GAGGCTTTTGCTAGTAGGGCTAAGATTGTTCATATTGATATTGACTCGGCTGAGATTGGGAAGA
ATAAGACTCCTCATGTGTCTGTGTGTGGTGATGTTAAGCTGGCTTTGCAAGGGATGAATAAGGT
TCTTGAGAACCGAGCGGAGGAGCTTAAACTTGATTTTGGAGTTTGGAGGAATGAGTTGAACGTA
CAGAAACAGAAGTTTCCGTTGAGCTTTAAGACGTTTGGGGAAGCTATTCCTCCACAGTATGCGA
TTAAGGTCCTTGATGAGTTGACTGATGGAAAAGCCATAATAAGTACTGGTGTCGGGCAACATCA
AATGTGGGCGGCGCAGTTCTACAATTACAAGAAACCAAGGCAGTGGCTATCATCAGGAGGCCTT
GGAGCTATGGGATTTGGACTTCCTGCTGCGATTGGAGCGTCTGTTGCTAACCCTGATGCGATAG
TTGTGGATATTGACGGAGATGGAAGTTTTATAATGAATGTGCAAGAGCTAGCCACTATTCGTGT
AGAGAATCTTCCAGTGAAGGTACTTTTATTAAACAACCAGCATCTTGGCATGGTTATGCAATGG
GAAGATCGGTTCTACAAAGCTAACCGAGCACACACATTTCTCGGAGATCCGGCTCAGGAGGACG
AGATATTCCCGAACATGTTGCTGTTTGCAGCAGCTTGCGGGATTCCAGCGGCGAGGGTGACAAA
GAAAGCAGATCTCCGAGAAGCTATTCAGACAATGCTGGATACACCAGGACCTTACCTGTTGGAT
GTGATTTGTCCGCACCAAGAACATGTGTTGCCGATGATCCCGAATGGTGGCACTTTCAACGATG
TCATAACGGAAGGAGATGGCCGGATTAAATACTGAGAGCTCGAATTTCCCCGATCGTTCAAACA
TTTGGCAATAAAGTTTCTTAAGATTGAATCCTGTTGCCGGTCTTGCGATGATTATCATATAATT
TCTGTTGAATTACGTTAAGCATGTAATAATTAACATGTAATGCATGACGTTATTTATGAGATGG
GTTTTTATGATTAGAGTCCCGCAATTATACATTTAATACGCGATAGAAAACAAAATATAGCGCG
CAAACTAGGATAAATTATCGCGCGCGGTGTCATCTATGTTACTAGATCGGGAATTCACTGGCCG
TCGTTTTACAACGACTCAGCTGCTTGGTAATAATTGTCATTAGATTGTTTTTATGCATAGATGC
ACTCGAAATCAGCCAATTTTAGACAAGTATCAAACGGATGTTAATTCAGTACATTAAAGACGTC
CGCAATGTGTTATTAAGTTGTCTAAGttgtccttggttttattagttgttattgttgttttgtc
ttttcaatttctagtagtcaattgggaactggaagtacaaagttgaattttagacttatgtat
acaaattgaaaatgtatcaaaaagtagtgacacttataaaagaacaattctttcttctgagaa
catcttcaacccacctctattttactctccattctctatatttagtgagtaaaatagagaatgg
gcactccaacccacctccatatcactctccattcttcatatttagagagtcatattattttat
tattatttttattactttctaattaatatattattttacatataatgtcattaattaaatatct
aatattcataattctttttaaatgtcatattatattttaaaaatatattatttaatatatact
actttcatcccgaattaatagtattttaatttttctaattttcgacaataatataatttgatt
ttattattaattttcactataaataatacacaaaattaaaatgataagatgaaaaaattaattt
aaaaatacaatacataatatgataatttaaatacaggataacaatacaatacatgacataataa
tttaaatacaagataaaatacaatacataacataataatcaattcgtgatgaaacaagaatcat
gccaaaaagatattgaacgtgcattcgaagttttgcaatcacgttttgcaattattgcaagacc
gtcacgtttttggagaaaggaagtgtgacatgatataatgactacatgtattatactgcacacc
atgataattgaggatgaacatgatcttaatgcaccaaatcaagatgccgtagaggctccaactc
caacgacaaaaatgatggtagatgaaaatcttcggtttgaacaattttagctagacataaaaa
agttaaggacaaaaatgctcatttgaactccgtaatgcattaatagagcatttatgacagcaa

*cgtgataatttcgaaacttgagtgtttatgtaattatatttcacttttatttgaatttgtccat*
*taatttgtatattatataatatttatttacaatttatgttatttaaaatttagaataaaataa*

Figure2j - continued

*aattttgaaaaaataaaattttatatcacatgaaaattatataaagtaattattggggaaaaaa*
*ataaaggatttatattatgaaataagaaaataagaatgaatagaaataataataatataatatt*
*gaggagaaataattatttttttagagagtgaaatagagaattgggttgaaattgattgtctga*
*aaaaataaaaaactctatatttggagagtaaaatagagtgtagggttggagacgtca* **(SEQ-ID-No.44)**

Tm: 77.5 C TaOpt: 51.6 C GC: 43.2
Sense Primer:
GTGAACTAGGAAACCTAAATC (SEQ-ID-No. 10)
Similarity: 100.0%
Length: 21 Tm: 42.0 C GC: 38.1
dH: -153.6 kcal/mol dS: -409.4 cal/mol dG: -29.8 kcal/mol
Antisense Primer:
CAACTAATAAAACCAAGGAC (SEQ-ID-No. 11)
Similarity: 100.0%
Length: 20 Tm: 41.1 C GC: 35.0
dH: -149.5 kcal/mol dS: -398.5 cal/mol dG: -28.9 kcal/mol
Tm Difference: 1.0
GC Difference: 3.1

Extra-primer for flanking region (right border) to BLB1 (over blb2.cassette):
Part of blb1 - blb1 terminator - ahas-cassette - flanking region left border (SEQ-ID-No.45)

**Lower case bold – flanking regions at right border**
**Upper case italic–– Blb2 expression cassette (promoter – gene –terminator)**
**Upper case bold – Blb1 expression cassette (promoter – gene –terminator)**

**gaaactcagttggagattacgtttaagggatggcctacatggataaggttatctacaatgatgc**
**aatggagataataatagtactcctatgtgatttcttcgttttaatttgtttattttattttcat**
**ttaatttgatttgatacgtagtttaaaaaaataaaaaagatatttaaattttgtgatgttagat**
**gaaacatacgttaaatgcatcaaaatgtcatttaaatttgtcttgaacatatagtattagaaag**
**ttgaaattaaagagctatcaaaaaagaagaagatatgactaaaaaaaatagcacaagcaaattg**
**aaactaaaagagtaattaacaataacaatttttttttaaaatgatacataatactcactctttc**
**tcaatatgtggcacaaaaatatcaagaatcaaactatttaattttgactataaatttagaaata**
**aatttttttaaatttcttaaaaaacaaaatttacaaagcaaatactatataaaaaatattataaa**
**ttataataattaataattcaaaatagttaaccatacaaactgaattttgggattgagtttctat**
**ttaaattttcgtgagtaaagattaaagttcaaattaactttttataaaatgagttttcaatttt**
**aaaaagtcaaacaaaatatatatagaatataaagtgagaaaccaaataaaacaagaatagcaag**
**gattatccatttaggaaataagatgaaagaatcaaatcaactaataaaaactttaatcctataa**
**gataagcttgccaataatttaaaatagcatgatgcgtctaagagggtatcaatatattgttatc**
**ctttaaagaccaagatagtgtttcaggaaagttatttcatttccaatttaaataaaagaaaat**
**aattattcaaaattcttatgtctttttttattgatttatagtcgaagatctttcaaaaataattt**
**ttctataggtaaaaaaggataattt**CAATTTCACACAGGAAACAGCTATGACCATGATTACGCC
AAGCTGGCGCGCCAAGCTTGCATGCCTGCAGGTCGACTCTAGAGGATCTAGAATCACCG*AACCT*
*CCCCTCGGTACAGCTCCTCCAGTTCTACCATGAATTTCATCCACTGATTCCTCTTCAATCGCCA*
*TTGCAGATTCTCTCGATCTATGCTCAAAAAATCCCGAGATAAAACCCTAGATCTGCTTCAAATG*

Figure2j - continued

CTCTGATACCATGTAATTTCAGTGAATTCTAACTAAACAATGGAGAGAATTAACTATTTTAGAA
AGACTGATTGAAGGAGAAGAAGAGAGAAAAATTCTATATTGAACTCATGAACCAAAATGAATGA
AAAAAATAATGAGAAGAACTATACTATTACAATCTATATATCTCTATTTATATTCTAATCTGAA
GCAGTTAATTTAACTGACTCTAACAACTAGACTGATAGGTGTACATTTTCTGTTAGTGCACTGC
AGTGCATTTAACTAACTGCTTAACATAAAGAATGTTGTTCGAACTTCATTCGAATAGCTTCAAT
GAGAAGCAAACATGTGTACCTGTAAAGACACACAGTAAAAGTGTTAATAATGAATAAATATGAA
TAAATCAAATAATAAATTAAAAATAAAAACACATCCAATTAACATTGGAGGTCTTGAAAATCGA
TGGTAATTAACAAAGACCCTTGTGAAATTTAAGTCTGTAATTGAAAATTTGAGTATAGGTTAGG
GGACATTTGACTATTTTCTCATTTTCTTTATCTTTTTCCTAATTTGTGGCAGACAAGTGAGGAG
GCCCCACTGTAATTGATTCATGCTTTTGCTTTCTTGACTTTTTGGAACAATACTATGCATCATA
TTTGGTCTTAATTATTCCTCTGTTTATTTCCAGAATTTTGAGCTCTATACATCTAATAACAAAG
CAAGCAGAGGATATATAGTTTCATCAACTAAAAAGGTTAGTCAACTCATCTAATATTTGCTACT
CTCATCTCTATTGAAGTACAGTTATGGAAAAGTAGAAGTGATGTAAGAAAAATGAAAGAACTTT
AGTAGGTTAGTTGGATCTAACAAAGAGAAAGGGAAATAAATTGCAGGAGAAAGAGAGAGGTTAA
ATACTTACTCACACCACCGATTTACAACAAATCACTTAATTGTGGTTAGTTAATGTATACTTTC
ACCTCATTAAATTATTACTTACCCATGATAAGTTGTATTAATTTGGTATTAATATCCGGTGCGG
GTGAATTCTTACCGGGTGAGAGGGATGGGGTTGGAGAGTGTGGAGTGAACAGAAGCAGATGTTT
TAGATTTTTTCTAAGATGACGAAAGATTCCCCTCACTAATGAAAATATATTACTATACGCTATT
AGAGATAGAAAGGTTCGGTACCAGTTGGTCTCGTTTCTGGATGAACCCCATTTTTACAAGTCAT
TTTCTTCAATTCAAATCGCAAGTGTACCTTTATCATCTTCCACTAATTAAGTCCTCTTAAGTTC
GCGTGAAAATAGTGAAATTATTGATTATTCTTATCATTTCATCTTCTTTCTCCTGATAAAGTTT
TATGTACTTTTTATGCATCAGGTCTTGAGAACTTGGAAAGGAAAAGTAGAATCATGGAAAAACG
AAAAGATAATGAAGAAGCAAACAACTCATTGGTATGTTATTTGATAGAGTGAACTGTAAAGTAT
TGAATTGTAGATATCATGTGGCTTTAAAAATTTGATATGTGTTATTTTGGCAGGAGTCATTTTC
TGCTCTTCGCAAGGATGCTGCCAATGTTCTGGATTTCCTAGAGAGATTAAAGAATGAAGAAGAT
CAAAAGGCTGTTGATGTGGATCTGATTGAAAGCCTGAAATTGAAGCTGACATTTATTTGTACAT
ATGTCCAGCTTTCTTATTCCGATTTGGAGAAGTTTGAAGATATAATGACTAGAAAAAGACAAGA
GGTTGAGAATCTGCTTCAACCAATTTTGGATGATGATGGCAAAGACGTCGGGTGTAAATATGTC
CTTACTAGCCTCGCCGGTAATATGGATGACTGTATAAGCTTGTATCATCGTTCTAAATCAGATG
CCACCATGATGGATGAGCAATTGGGCTTCCTCCTCTTGAATCTCTCTCATCTATCCAAGCATCG
TGCTGAAAAGATGTTTCCTGGAGTGACTCAATATGAGGTTCTTCAGAATGTATGTGGCAACATA
AGAGATTTCCATGGATTGATAGTGAATTGTTGCATTAAGCATGAGATGGTTGAGAATGTCTTAT
CTCTGTTTCAACTGATGGCTGAGAGAGTAGGACGCTTCCTTTGGGAGGATCAGGCTGATGAAGA
CTCTCAACTCTCCGAGCTAGATGAGGATGATCAGAATGATAAAGACCCTCAACTCTTCAAGCTA
GCACATCTACTCTTGAAGATTGTTCCAACTGAATTGGAGGTTATGCACATATGTTATAAAACTT
TGAAAGCTTCAACTTCAACAGAAATTGGACGCTTCATTAAGAAGCTCCTGGAAACCTCTCCGGA
CATTCTCAGAGAATATCTGATTCATCTACAAGAGCATATGATAACTGTTATTACCCCTAACACT
TCAGGGGCTCGAAACATTCATGTCATGATGGAATTCCTATTGATTATTCTTTCTGATATGCCGC
CCAAGGACTTTATTCATCATGACAAACTTTTTGATCTCTTGGCTCGTGTTGTAGCACTTACCAG
GGAGGTATCAACTCTTGTACGCGACTTGGAAGAGAAATTAAGGATTAAAGAGAGTACTGACGAA
ACAAATTGTGCAACCCTAAAGTTTCTGGAAAATATTGAACTCCTTAAGGAAGATCTCAAACATG
TTTATCTGAAAGTCCCGGATTCATCTCAATATTGCTTCCCCATGAGTGATGGACCTCTCTTCAT
GCATCTGCTACAGAGACACTTAGATGATTTGCTGGATTCCAATGCTTATTCAATTGCTTTGATA
AAGGAACAAATTGGGCTGGTGAAAGAAGACTTGGAATTCATAAGATCTTTTTTCGCGAATATTG
AGCAAGGATTGTATAAAGATCTCTGGGAACGTGTTCTAGATGTGGCATATGAGGCAAAAGATGT
CATAGATTCAATTATTGTTCGAGATAATGGTCTCTTACATCTTATTTTCTCACTTCCCATTACC
AGAAAGAAGATGATGCTTATCAAAGAAGAGGTCTCTGATTTACATGAGAACATTTCCAAGAACA
GAGGTCTCATCGTTGTGAACTCTCCCAAGAAACCAGTTGAGAGCAAGTCATTGACAACTGATAA

*AATAATTGTAGGTTTTGGTGAGGAGACAAACTTGATACTTAGAAAGCTCACCAGTGGACCGGCA*
*GATCTAGATGTCATTTCGATCATTGGTATGCCGGGTTTAGGTAAAACTACTTTGGCGTACAAAG*
*TATACAATGATAAATCAGTTTCTAGCCATTTCGACCTTCGTGCATGGTGCACGGTCGACCAAGT*

Figure2j - continued

*ATATGACGAGAAGAAGTTGTTGGATAAAATTTTCAATCAAGTTAGTGACTCAAATTCAAAATTG
AGTGAGAATATTGATGTTGCTGATAAACTACGGAAACAATTGTTTGGAAAGAGGTATCTTATTG
TCTTAGATGACGTGTGGGATACTAATACATGGGATGAGCTAACAAGACCTTTTCCTGATGGTAT
GAAAGGAAGTAGAATTATTTTGACAACTCGAGAAAAGAAAGTTGCTTTGCATGGAAAGCTCTAC
ACTGATCCTCTTAACCTTCGATTGCTAAGATCAGAAGAAAGTTGGGAGTTATTAGAGAAAAGGG
CATTTGGAAACGAGAGTTGCCCTGATGAACTATTGGATGTTGGTAAAGAAATAGCCGAAAATTG
TAAAGGGCTTCCTTTGGTGGTGGATCTGATTGCTGGAATCATTGCTGGGAGGGAAAAGAAAAAG
AGTGTGTGGCTTGAAGTTGTAAATAATTTGCATTCCTTTATTTTGAAGAATGAAGTGGAAGTGA
TGAAAGTTATAGAAATAAGTTATGACCACTTACCTGATCACCTGAAGCCATGCTTGCTGTACTT
TGCAAGTGCGCCGAAGGACTGGGTAACGACAATCCATGAGTTGAAACTTATTTGGGGTTTTGAA
GGATTTGTGGAAAAGACAGATATGAAGAGTCTGGAAGAAGTGGTGAAAATTTATTTGGATGATT
TAATTTCCAGTAGCTTGGTAATTTGTTTCAATGAGATAGGTGATTACCCTACTTGCCAACTTCA
TGATCTTGTGCATGACTTTTGTTTGATAAAAGCAAGAAAGGAAAAGTTGTGTGATCGGATAAGT
TCAAGTGCTCCATCAGATTTGTTGCCACGTCAAATTAGCATTGATTATGATGATGATGAAGAGC
ACTTTGGGCTTAATTTTGTCCTGTTCGGTTCAAATAAGAAAAGGCATTCCGGTAAACACCTCTA
TTCTTTGACCATAAATGGAGATGAGCTGGACGACCATCTTTCTGATACATTTCATCTAAGACAC
TTGAGGCTTCTTAGAACCTTGCACCTGGAATCCTCTTTTATCATGGTTAAAGATTCTTTGCTGA
ATGAAATATGCATGTTGAATCATTTGAGGTACTTAAGCATTGGGACAGAAGTTAAATCTCTGCC
TTTGTCTTTCTCAAACCTCTGGAATCTAGAAATCTTGTTTGTGGATAACAAAGAATCAACCTTG
ATACTATTACCGAGAATTTGGGATCTTGTAAAGTTGCAAGTGCTGTTCACGACTGCTTGTTCTT
TCTTTGATATGGATGCAGATGAATCAATACTGATAGCAGAGGACACAAAGTTAGAGAACTTGAC
AGCATTAGGGGAACTCGTGCTTTCCTATTGGAAAGATACAGAGGATATTTTCAAAAGGCTTCCC
AATCTTCAAGTGCTTCATTTCAAACTCAAGGAGTCATGGGATTATTCAACAGAGCAATATTGGT
TCCCGAAATTGGATTTCCTAACTGAACTAGAAAAACTCACTGTAGATTTTGAAAGATCAAACAC
AAATGACAGTGGGTCCTCTGCAGCCATAAATCGGCCATGGGATTTTCACTTTCCTTCGAGTTTG
AAAAGATTGCAATTGCATGAATTTCCTCTGACATCCGATTCACTATCAACAATAGCGAGACTGC
TGAACCTTGAAGAGTTGTACCTTTATCGTACAATCATCCATGGGGAAGAATGGAACATGGGAGA
AGAAGACACCTTTGAGAATCTCAAATGTTTGATGTTGAGTCAAGTGATTCTTTCCAAGTGGGAG
GTTGGAGAGGAATCTTTTCCCACGCTTGAGAAATTAGAACTGTCGGACTGTCATAATCTTGAGG
AGATTCCGTCTAGTTTTGGGGATATTTATTCCTTGAAAATTATCGAACTTGTAAGGAGCCCTCA
ACTTGAAAATTCCGCTCTCAAGATTAAGGAATATGCTGAAGATATGAGGGGAGGGGACGAGCTT
CAGATCCTTGGCCAGAAGGATATCCCGTTATTTAAGTAGTTTTTGAGCATTATGGTTGAAAAGT
AGATTGCACTTTGCTGGGTAGATTGTATATGGTTAAGAAAATTCTGTTACAGTTGTTATGAAAC
ATTTTTATTTGACTTTTCTGAGTTTCTTTTAGAAAACTCAGAAGTTTTTAACAAAAATTATAGT
TTTTATAAATACAATGTGGATTTGCCTTTGGCTGTCCAACTTGGTCTGAAGTCTCATATGCTCA
GAGCACTATCGTTCAACCTCAATCAAGGTACTGATTTAAAATGACATCTATACTACTTTATCAC
AAACCCAACGAACTTTCATCTCAAAAGCTAGGCCAGGAAGTGAAGAGGTTGTAGAGAGCTTATA
AGCACTCATGACTTCCTTTTCTCGAACATTCAACCAACGTAGGCTGAAATCCCACTCTGAACGA
AAATAAGTGTTTGTTTATCAAATTAACTCTCGTAGTAGAACACTGAAATACCTTCTTCTAAACG
TTCAACAAATGGGATTTCCAGCACTCAAAGTGAATGAAAGGTTCACATTAATCTTCAAAAAGAA
TTACGACAATTCATGACCACAAGTACATTGACAGCACCATTTCAACAGAAGAACAAGTCAATGC
TGCATCTTCATCAATAATCCGAGTGTCGAACCTCCTTCCTGACACTGTCCTGTATATGTAAAGT
TTCTCAACAGGGCAACTTTCTGGTCTCGTATCTGGATGACCCCTCTCGTCTATAACTTCAACAT
TAAGCCCTGGCAACTTCTGGACCAACAGCTTACATGCTTCAAAACTTACTGAACAATTAGACAT
CCAAAGGGATCGCATTGTCTCCAGCTTTGCAGCATTAGCCAACAGAGCCTCATCGCCAAAGGGG
CAGTCTCTAATCTCGAATTTGAAAAAATTGTTGTTGTATGACTTTCCTCTGACATCCGATGCAC
TATCAACAATAGCAAGACTGGAGGTTGGAGAGGAATCCTTTATTATACAATCATTCAGGGAGAA
GAATGGAACATGGGGGAGGAAGACACTTTTGAGAATCTGAAATGTGTTAGAGCCACAAGCTACA*

*GAAGTATTGAATTTGTCATGAATATCAACATTCTTCATCCTAGTTAATTCTTTTTCAATTTTTA*
*ATAGACTCTCATTTTAATCACTAATATTCTTCTATTTGTGACTTCTTTTCTGCAGGTGGCAACT*
*TTAAATTCATAAAGTATAGGATTGATGACAAACTCGAAAAATATCTTAATGAGGTGAAGTTTGA*

Figure2j - continued

*GCAGTCAGCAGATGGTGGTTCCAACTCTAAGTTGACAAGCACATACTATCCCGGAGGGCGATTT*
*CAAGCCTGATGCATATGGTTAGTGTGGCTAGAGCAGACAGGATGTATTACCTGGATATCTACCA*
*AGACGAATCCACAATCAGTTTTATGTCAAGCAATACATGAAGTAACTCCCGATAGAACAGTAAA*
*AGCAAGATGTGTAGGTGTATCTCGACTCTAAGAGATTGTACATTCCTCTTTGAGATTTTTACTG*
*CTAATACAAATTTACACCTCAGAAGCGAATCTAGAATTTCTAGAGCATGAATGCACCACTAATG*
*AAAGGAGAAAAAAGGAAGTATGAAGTGGGAATTTGATCCTTGTTTCTAGGTATATAAAATTTAT*
*CATTCAACTATACTTCATTTAGCAAACAACTCTCTTTGCCATTATTTCTCAAACAAGGGCTTCT*
*AATATTGCTAAACTAAAGACTGTCAAAAGGTAAGTTCATCTTCAAACTCTCTTGTTTACTTTAT*
*CTAAAGGGGAACTATGAAAAACAAGAAACATCAGGAATGTCCCGTAAACAAAGCAGCCTCATGC*
*ACAAAACATCCAACGTTGGTAGGATTAATGGAGGGATCGCATCCCAGGAGGATACTGTAGAAAA*
*ATTAGTGGCTTCTTTCACCGCTCAAACCCATGATCTATAGGTTACATGGAGACAACTTTATGGT*
*TGCTCGTAGGCTCCCGTCAATTCTCATAAACCACAACACCAAAGTTGCATCAGACATCATCTTC*
*ATTCACAAGCTGACAATCTCCACAAGTCTTAGTCAACTTGTAATATGAATATTAGCCAGGTAGA*
*CGTACATATTTACAAAATTGAGTTTCCTATATAATATGGTTTGAAGGAATGAAACATGATGGGG*
*AGGGTAGATAAAATAATATATGAGGCATAAAAATAGGAAAGATATTTGTAGTGAGAGGTTTTGA*
*CTTTTTATGCTGCTTTTGATCTTCAGTTTCTTGTATTCTTTTTCTACTGCTTTCCTCTTCTTTC*
*TCCTGAGTAAAGTTTTATGTAGGTACTTTTTATACGTCCGATCGTGAGAACTTGAAAGAAAGCT*
*CTCTATAGCTATGTTAGGTGCCCACATAAAAAAATGAAATATTACAAAAACCCTGATAATAAAA*
*TACACTAATCTAAGATATTCACTGCAACATACATGCAAAATATATATATATAAATTTTCATGAA*
*AATTATAACAAATAATAGATGTGAACATATAACTTTAAAAATAATATTACATCCATAAAGCTTA*
*AATTCTAGA*TCCCCGGTCGACTCTAGAGGATCCCCACTCCATCCGTTCACT**TTGATTTGTCATG**
**TTGCACTTTTCGAAAGTCAATTTGACTAATTTTTAAAGCTAAATTAGATTACACTAATTCAATA**
**TTTTAAACAGAAAAATTAGATATTCAAAAACTATACAAAAAATATTATACATTGCAATTTTTTG**
**CATATCAATATGATAAAAAAATATATCGTAAAATATTAGTCAAAATTTTTATAATTTGACTCAA**
**ATCATGAAAAGTATAATAATTAATAGTGGACGGAGGAAGTATTGTCTTTCCAGATTTGTGGCCA**
**TTTTTGGTCCAAGGGCCATTAGCAGTTCTCTTCATTTTCTACTTCTGTCTCATATTAGATGGGC**
**ATCTTACTAAAAATATTTGTCTCATATTACTTGATTATTTATTAAATCAAAAAGAATTAATTAA**
**TTTTTTCTCATTTTACCCCTACAATTAATATAGTTTTAAAAGTTTTAAACAAATTTTGAAGAAT**
**CAAAATTTCTTTTTGCAAGAGACTTATTAATATAAACAAAGGATAAAATAATAAAATTTGTCAA**
**TTTATTGACGATCACTTAATAATCATATAAAATAGAAAATGTTTATCTAATATGAGACGGAGAA**
**AATATATCCTAAAATATTTTTGGACAGATATGTGATATTCTAACCATTCACTAGACTATATTAT**
**GCATTTTAGCCGCCAATGACTTATTTCAGCTTTAATTAATTAGGAAAGAGGAAACTGCCAATGA**
**GGAAGAGTAGGGGCGTAGTTGCTGTCGACGAAAAAAAGATAATACTCACTCTTTTCGATTTTTA**
**TTTTTATTTATCACTTTTAACCTATCATGTAAAAAGATAATTATTTTTTTCATGCTTTATCCTT**
**AGTATTAAACAATTTAATAGGGATTATTTTGTAAAATATTTATATGAATAATTGTTTTCGTAAT**
**GAATTTGTCCGGTCAAACAATGATAAATAAAAATGAATGAAGAGAGTAGAAAACAAAACAAAAG**
**AACAAGTTGACAACTTGAGAGATTAAAAGGGTCCAAAACGCCTTGGATTTTGAGATTCCATATG**
**TGAAATTTCCATGAAATAATTGAATTTGTATTATTACAAGTCAAACTTTCCATTTCATTCCAAC**
**TAGCCATCTTGGTTTCAAAATTACACATTCATTCATTCACAGATCTAATATTCTTAATAGTGAT**
**TTCCACATATGGCTGAAGCTTTCATTCAAGTTCTGCTAGACAATCTCACTTCTTTCCTCAAAGG**
**GGAACTTGTATTGCTTTTCGGTTTTCAAGATGAGTTCCAAAGGCTTTCAAGCATGTTTTCTACA**
**ATTCAAGCCGTCCTTGAAGATGCTCAGGAGAAGCAACTCAACAACAAGCCTCTAGAAAATTGGT**
**TGCAAAAACTCAATGCTGCTACATATGAAGTCGATGACATCTTGGATGAATATAAAACCAAGGC**
**CACAAGATTCTCCCAGTCTGAATATGGCCGTTATCATCCAAAGGTTATCCCTTTCCGTCACAAG**
**GTCGGGAAAAGGATGGACCAAGTGATGAAAAAACTAAAGGCAATTGCTGAGGAAAGAAAGAATT**
**TTCATTTGCACGAAAAAATTGTAGAGAGACAAGCTGTTAGACGGGAAACAGGTACTCATCTTAA**
**ATTAGTATTACAACAACTAAGTTTATATTCATTTTTTTGGCAATTATCAAATTCAGAAAAGGGT**
**TAAATATACTCATGTCCTATCGTAAATAGTGTATATATACCTCTCGTTGTACTTTCGATCTGAA**

TATACTTGTCAAATCTGGCAAGCTCAGAATCAAATTATCCACCCCAACTTTTAAATACTCGATA
TCTTTAGAAATCCACCTGTCTAACTCATCCACTACCCATTCCCTTTGCTTTGAATTCTTTTCTT
TACCTATAAACTTGGAACACTCGATCCGTTTTGCTTTTCTTAACAAAGCAGCTCAGAGAAAAGA

Figure 2j continued

GGTTTTCTTCTATTCTGTTTCTCTGTGTGCTGCACTTGGGTCCTTAATCCCATTAAAAACAGGG
CATGTTAATCCCAACGACGGTAGCCTTTCCTGACAGCTGACTGTAAATTTTGTCTAACAAAGAA
AAAAAAAGATTAGACATGTTTTTCCTTGTCATTGATTAGGCTGGATTTCTTTCAGAGTGGAACA
TAGGGGATATATTGGACCAAAAGTAGAATGGGTATATATTTAAAGTATTTCTGATAGAACAGGA
GTATATTGTGCGAAAATATCCTCTATTTTCTGTTGTCTCCTAATGAGTTTGAATGTAATAATAT
TCTCATGTGGACATTGCTTGCACCAGGTTCTGTATTAACCGAACCGCAGGTTTATGGAAGAGAC
AAAGAGAAAGATGAGATAGTGAAAATCCTAATAAACAATGTTAGTGATGCCCAACACCTTTCAG
TCCTCCCAATACTTGGTATGGGGGGATTAGGAAAAACGACTCTTGCCCAAATGGTCTTCAATGA
CCAGAGAGTTACTGAGCATTTCCATTCCAAAATATGGATTTGTGTCTCGGAAGATTTTGATGAG
AAGAGGTTAATAAAGGCAATTGTAGAATCTATTGAAGGAAGGCCACTACTTGGTGAGATGGACT
TGGCTCCACTTCAAAAGAAGCTTCAGGAGTTGCTGAATGGAAAAAGATACTTGCTTGTCTTAGA
TGATGTTTGGAATGAAGATCAACAGAAGTGGGCTAATTTAAGAGCAGTCTTGAAGGTTGGAGCA
AGTGGTGCTTCTGTTCTAACCACTACTCGTCTTGAAAAGGTTGGATCAATTATGGGAACATTGC
AACCATATGAACTGTCAAATCTGTCTCAAGAAGATTGTTGGTTGTTGTTCATGCAACGTGCATT
TGGACACCAAGAAGAAATAAATCCAAACCTTGTGGCAATCGGAAAGGAGATTGTGAAAAAAAGT
GGTGGTGTGCCTCTAGCAGCCAAAACTCTTGGAGGTATTTTGTGCTTCAAGAGAGAAGAAAGAG
CATGGGAACATGTGAGAGACAGTCCGATTTGGAATTTGCCTCAAGATGAAAGTTCTATTCTGCC
TGCCCTGAGGCTTAGTTACCATCAACTTCCACTTGATTTGAAACAATGCTTTGCGTATTGTGCG
GTGTTCCCAAAGGATGCCAAAATGGAAAAAGAAAAGCTAATCTCTCTCTGGATGGCGCATGGTT
TTCTTTTATCAAAAGGAAACATGGAGCTAGAGGATGTGGGCGATGAAGTATGGAAAGAATTATA
CTTGAGGTCTTTTTTCCAAGAGATTGAAGTTAAAGATGGTAAAACTTATTTCAAGATGCATGAT
CTCATCCATGATTTGGCAACATCTCTGTTTTCAGCAAACACATCAAGCAGCAATATCCGTGAAA
TAAATAAACACAGTTACACACATATGATGTCCATTGGTTTCGCCGAAGTGGTGTTTTTTTACAC
TCTTCCCCCCTTGGAAAAGTTTATCTCGTTAAGAGTGCTTAATCTAGGTGATTCGACATTTAAT
AAGTTACCATCTTCCATTGGAGATCTAGTACATTTAAGATACTTGAACCTGTATGGCAGTGGCA
TGCGTAGTCTTCCAAAGCAGTTATGCAAGCTTCAAAATCTGCAAACTCTTGATCTACAATATTG
CACCAAGCTTTGTTGTTTGCCAAAAGAAACAAGTAAACTTGGTAGTCTCCGAAATCTTTTACTT
GATGGTAGCCAGTCATTGACTTGTATGCCACCAAGGATAGGATCATTGACATGCCTTAAGACTC
TAGGTCAATTTGTTGTTGGAAGGAAGAAAGGTTATCAACTTGGTGAACTAGGAAACCTAAATCT
CTATGGCTCAATTAAAATCTCGCATCTTGAGAGAGTGAAGAATGATAAGGACGCAAAAGAAGCC
AATTTATCTGCAAAAGGGAATCTGCATTCTTTAAGCATGAGTTGGAATAACTTTGGACCACATA
TATATGAATCAGAAGAAGTTAAAGTGCTTGAAGCCCTCAAACCACACTCCAATCTGACTTCTTT
AAAAATCTATGGCTTCAGAGGAATCCATCTCCCAGAGTGGATGAATCACTCAGTATTGAAAAAT
ATTGTCTCTATTCTAATTAGCAACTTCAGAAACTGCTCATGCTTACCACCCTTTGGTGATCTGC
CTTGTCTAGAAAGTCTAGAGTTACACTGGGGGTCTGCGGATGTGGAGTATGTTGAAGAAGTGGA
TATTGATGTTCATTCTGGATTCCCCACAAGAATAAGGTTTCCATCCTTGAGGAAACTTGATATA
TGGGACTTTGGTAGTCTGAAAGGATTGCTGAAAAAGGAAGGAGAAGAGCAATTCCCTGTGCTTG
AAGAGATGATAATTCACGAGTGCCCTTTTCTGACCCTTTCTTCTAATCTTAGGGCTCTTACTTC
CCTCAGAATTTGCTATAATAAAGTAGCTACTTCATTCCCAGAAGAGATGTTCAAAAACCTTGCA
AATCTCAAATACTTGACAATCTCTCGGTGCAATAATCTCAAAGAGCTGCCTACCAGCTTGGCTA
GTCTGAATGCTTTGAAAAGTCTAAAAATTCAATTGTGTTGCGCACTAGAGAGTCTCCCTGAGGA
AGGGCTGGAAGGTTTATCTTCACTCACAGAGTTATTTGTTGAACACTGTAACATGCTAAAATGT
TTACCAGAGGGATTGCAGCACCTAACAACCCTCACAAGTTTAAAAATTCGGGGATGTCCACAAC
TGATCAAGCGGTGTGAGAAGGGAATAGGAGAAGACTGGCACAAAATTTCTCACATTCCTAATGT
GAATATATATATTTAAGTTATTTGCTATTGTTTCTTTGTTTGTGAGTCTTTTTGGTTCCTGCCA
TTGTGATTGCATGTAATTTTTTTCTAGGGTTGTTTCTTTATGAGTCTCTCTCTCATTGGATGTA
ATTTTCTTTTGGAAACAAATCTGTCAATTGATTTGTATTATACGCTTTCAGAATCTATTACTTA
TTTGTAATTGTTTCTTTGTTTGTAAATTGTGAGTATCTTATTTTATGGAATTTTCTGATTTTAT

TTTGAAAACAAATCAATGATTTGTAAGATCCATCTGTATTATACTCCCTTCGTCTCATTTTATG
TGTCACCTGTCGGATTTCGAGATTCAAACAAATCTATCTTTGATCGTAAATTTTTAATAGATCT
TTTAAACATTTTGAATTATCAATTATTGTGACTTTAGT

## Figure2j - continued

Tm: 74.2 C  TaOpt: 50.3 C  GC: 35.0
Sense Primer:
AACTGAATTTTGGGATTGAG(SEQ-ID-No. 18)
Similarity: 100.0%
Length: 20  Tm: 45.9 C  GC: 35.0
dH: -150.1 kcal/mol  dS: -393.9 cal/mol  dG: -30.9 kcal/mol
Antisense Primer:
ATGTAGCAGCATTGAGTTTT(SEQ-ID-No. 19)
Similarity: 100.0%
Length: 20  Tm: 44.2 C  GC: 35.0
dH: -147.0 kcal/mol  dS: -387.0 cal/mol  dG: -29.8 kcal/mol
Tm Difference: 1.7
GC Difference: 0.0

Figure 2k of 5

Blb1-expression casette (SEQ-ID-No. 46)

**Upper case italic–- promoter**
**Upper case bold – Blb1**
**Upper case italic underlined – terminator**

*TTGATTTGTCATGTTGCACTTTTCGAAAGTCAATTTGACTAATTTTTAAAGCTAAATTAGATTA*
*CACTAATTCAATATTTTAAACAGAAAAATTAGATATTCAAAAACTATACAAAAAATATTATACA*
*TTGCAATTTTTTGCATATCAATATGATAAAAAAATATATCGTAAAATATTAGTCAAAATTTTTA*
*TAATTTGACTCAAATCATGAAAAGTATAATAATTAATAGTGGACGGAGGAAGTATTGTCTTTCC*
*AGATTTGTGGCCATTTTTGGTCCAAGGGCCATTAGCAGTTCTCTTCATTTTCTACTTCTGTCTC*
*ATATTAGATGGGCATCTTACTAAAAATATTTGTCTCATATTACTTGATTATTTATTAAATCAAA*
*AAGAATTAATTAATTTTTTCTCATTTTACCCCTACAATTAATATAGTTTTAAAAGTTTTAAACA*
*AATTTTGAAGAATCAAAATTTCTTTTTGCAAGAGACTTATTAATATAAACAAAGGATAAAATAA*
*TAAAATTTGTCAATTTATTGACGATCACTTAATAATCATATAAAATAGAAAATGTTTATCTAAT*
*ATGAGACGGAGAAAATATATCCTAAAATATTTTTGGACAGATATGTGATATTCTAACCATTCAC*
*TAGACTATATTATGCATTTTAGCCGCCAATGACTTATTTCAGCTTTAATTAATTAGGAAAGAGG*
*AAACTGCCAATGAGGAAGAGTAGGGGCGTAGTTGCTGTCGACGAAAAAAAGATAATACTCACTC*
*TTTTCGATTTTTATTTTTATTTATCACTTTTAACCTATCATGTAAAAAGATAATTATTTTTTTC*
*ATGCTTTATCCTTAGTATTAAACAATTTAATAGGGATTATTTTGTAAAATATTTATATGAATAA*
*TTGTTTTCGTAATGAATTTGTCCGGTCAAACAATGATAAATAAAAATGAATGAAGAGAGTAGAA*
*AACAAAACAAAAGAACAAGTTGACAACTTGAGAGATTAAAAGGGTCCAAAACGCCTTGGATTTT*
*GAGATTCCATATGTGAAATTTCCATGAAATAATTGAATTTGTATTATTACAAGTCAAACTTTCC*
*ATTTCATTCCAACTAGCCATCTTGGTTTCAAAATTACACATTCATTCATTCACAGATCTAATAT*
_TCTTAATAGTGATTTCCACAT_**ATGGCTGAAGCTTTCATTCAAGTTCTGCTAGACAATCTCACTT**
**CTTTCCTCAAAGGGGAACTTGTATTGCTTTTCGGTTTTCAAGATGAGTTCCAAAGGCTTTCAAG**
**CATGTTTTCTACAATTCAAGCCGTCCTTGAAGATGCTCAGGAGAAGCAACTCAACAACAAGCCT**
**CTAGAAAATTGGTTGCAAAAACTCAATGCTGCTACATATGAAGTCGATGACATCTTGGATGAAT**
**ATAAAACCAAGGCCACAAGATTCTCCCAGTCTGAATATGGCCGTTATCATCCAAAGGTTATCCC**
**TTTCCGTCACAAGGTCGGGAAAAGGATGGACCAAGTGATGAAAAAACTAAAGGCAATTGCTGAG**
**GAAAGAAAGAATTTTCATTTGCACGAAAAAATTGTAGAGAGACAAGCTGTTAGACGGAAACAG**
**GTACTCATCTTAAATTAGTATTACAACAACTAAGTTTATATTCATTTTTTTGGCAATTATCAAA**
**TTCAGAAAAGGGTTAAATATACTCATGTCCTATCGTAAATAGTGTATATATACCTCTCGTTGTA**
**CTTTCGATCTGAATATACTTGTCAAATCTGGCAAGCTCAGAATCAAATTATCCACCCCAACTTT**
**TAAATACTCGATATCTTTAGAAATCCACCTGTCTAACTCATCCACTACCCATTCCCTTTGCTTT**
**GAATTCTTTTCTTTACCTATAAACTTGGAACACTCGATCCGTTTTGCTTTTCTTAACAAAGCAG**
**CTCAGAGAAAAGAGGTTTTCTTCTATTCTGTTTCTCTGTGTGCTGCACTTGGGTCCTTAATCCC**
**ATTAAAAACAGGGCATGTTAATCCCAACGACGGTAGCCTTTCCTGACAGCTGACTGTAAATTTT**
**GTCTAACAAAGAAAAAAAAAGATTAGACATGTTTTTCCTTGTCATTGATTAGGCTGGATTTCTT**
**TCAGAGTGGAACATAGGGGATATATTGGACCAAAAGTAGAATGGGTATATATTTAAAGTATTTC**
**TGATAGAACAGGAGTATATTGTGCGAAAATATCCTCTATTTTCTGTTGTCTCCTAATGAGTTTG**
**AATGTAATAATATTCTCATGTGGACATTGCTTGCACCAGGTTCTGTATTAACCGAACCGCAGGT**
**TTATGGAAGAGACAAAGAGAAAGATGAGATAGTGAAAATCCTAATAAACAATGTTAGTGATGCC**
**CAACACCTTTCAGTCCTCCCAATACTTGGTATGGGGGATTAGGAAAAACGACTCTTGCCCAAA**
**TGGTCTTCAATGACCAGAGAGTTACTGAGCATTTCCATTCCAAAATATGGATTTGTGTCTCGGA**
**AGATTTTGATGAGAAGAGGTTAATAAAGGCAATTGTAGAATCTATTGAAGGAAGGCCACTACTT**
**GGTGAGATGGACTTGGCTCCACTTCAAAAGAAGCTTCAGGAGTTGCTGAATGGAAAAAGATACT**
**TGCTTGTCTTAGATGATGTTTGGAATGAAGATCAACAGAAGTGGGCTAATTTAAGAGCAGTCTT**
**GAAGGTTGGAGCAAGTGGTGCTTCTGTTCTAACCACTACTCGTCTTGAAAAGGTTGGATCAATT**

Figure 2k - continued

**ATGGGAACATTGCAACCATATGAACTGTCAAATCTGTCTCAAGAAGATTGTTGGTTGTTGTTCA**
**TGCAACGTGCATTTGGACACCAAGAAGAAATAAATCCAAACCTTGTGGCAATCGGAAAGGAGAT**
**TGTGAAAAAAAGTGGTGGTGTGCCTCTAGCAGCCAAAACTCTTGGAGGTATTTTGTGCTTCAAG**
**AGAGAAGAAAGAGCATGGGAACATGTGAGAGACAGTCCGATTTGGAATTTGCCTCAAGATGAAA**
**GTTCTATTCTGCCTGCCCTGAGGCTTAGTTACCATCAACTTCCACTTGATTTGAAACAATGCTT**
**TGCGTATTGTGCGGTGTTCCCAAAGGATGCCAAAATGGAAAAAGAAAAGCTAATCTCTCTCTGG**
**ATGGCGCATGGTTTTCTTTTATCAAAAGGAAACATGGAGCTAGAGGATGTGGGCGATGAAGTAT**
**GGAAAGAATTATACTTGAGGTCTTTTTTCCAAGAGATTGAAGTTAAAGATGGTAAAACTTATTT**
**CAAGATGCATGATCTCATCCATGATTTGGCAACATCTCTGTTTTCAGCAAACACATCAAGCAGC**
**AATATCCGTGAAATAAATAAACACAGTTACACACATATGATGTCCATTGGTTTCGCCGAAGTGG**
**TGTTTTTTTACACTCTTCCCCCCTTGGAAAAGTTTATCTCGTTAAGAGTGCTTAATCTAGGTGA**
**TTCGACATTTAATAAGTTACCATCTTCCATTGGAGATCTAGTACATTTAAGATACTTGAACCTG**
**TATGGCAGTGGCATGCGTAGTCTTCCAAAGCAGTTATGCAAGCTTCAAAATCTGCAAACTCTTG**
**ATCTACAATATTGCACCAAGCTTTGTTGTTTGCCAAAAGAAACAAGTAAACTTGGTAGTCTCCG**
**AAATCTTTTACTTGATGGTAGCCAGTCATTGACTTGTATGCCACCAAGGATAGGATCATTGACA**
**TGCCTTAAGACTCTAGGTCAATTTGTTGTTGGAAGGAAGAAAGGTTATCAACTTGGTGAACTAG**
**GAAACCTAAATCTCTATGGCTCAATTAAAATCTCGCATCTTGAGAGAGTGAAGAATGATAAGGA**
**CGCAAAAGAAGCCAATTTATCTGCAAAAGGGAATCTGCATTCTTTAAGCATGAGTTGGAATAAC**
**TTTGGACCACATATATATGAATCAGAAGAAGTTAAAGTGCTTGAAGCCCTCAAACCACACTCCA**
**ATCTGACTTCTTTAAAAATCTATGGCTTCAGAGGAATCCATCTCCCAGAGTGGATGAATCACTC**
**AGTATTGAAAAATATTGTCTCTATTCTAATTAGCAACTTCAGAAACTGCTCATGCTTACCACCC**
**TTTGGTGATCTGCCTTGTCTAGAAAGTCTAGAGTTACACTGGGGGTCTGCGGATGTGGAGTATG**
**TTGAAGAAGTGGATATTGATGTTCATTCTGGATTCCCCACAAGAATAAGGTTTCCATCCTTGAG**
**GAAACTTGATATATGGGACTTTGGTAGTCTGAAAGGATTGCTGAAAAAGGAAGGAGAAGAGCAA**
**TTCCCTGTGCTTGAAGAGATGATAATTCACGAGTGCCCTTTTCTGACCCTTTCTTCTAATCTTA**
**GGGCTCTTACTTCCCTCAGAATTTGCTATAATAAAGTAGCTACTTCATTCCCAGAAGAGATGTT**
**CAAAAACCTTGCAAATCTCAAATACTTGACAATCTCTCGGTGCAATAATCTCAAAGAGCTGCCT**
**ACCAGCTTGGCTAGTCTGAATGCTTTGAAAAGTCTAAAAATTCAATTGTGTTGCGCACTAGAGA**
**GTCTCCCTGAGGAAGGGCTGGAAGGTTTATCTTCACTCACAGAGTTATTTGTTGAACACTGTAA**
**CATGCTAAAATGTTTACCAGAGGGATTGCAGCACCTAACAACCCTCACAAGTTTAAAAATTCGG**
**GGATGTCCACAACTGATCAAGCGGTGTGAGAAGGGAATAGGAGAAGACTGGCACAAAATTTCTC**
**ACATTCCTAATGTGAATATATATATTTAA***GTTATTTGCTATTGTTTCTTTGTTTGTGAGTCTTT*
*TTGGTTCCTGCCATTGTGATTGCATGTAATTTTTTTCTAGGGTTGTTTCTTTATGAGTCTCTCT*
*CTCATTGGATGTAATTTTCTTTTGGAAACAAATCTGTCAATTGATTTGTATTATACGCTTTCAG*
*AATCTATTACTTATTTGTAATTGTTTCTTTGTTTGTAAATTGTGAGTATCTTATTTTATGGAAT*
*TTTCTGATTTTATTTTGAAAACAAATCAATGATTTGTAAGATCCATCTGTATTATACTCCCTTC*
*GTCTCATTTTATGTGTCACCTGTCGGATTTCGAGATTCAAACAAATCTATCTTTGATCGTAAAT*
*TTTTAATAGATCTTTTAAACATTTTGAATTATCAATTATTGTGACTTTAGT*

Figure 2k - continued
Blb2-expression casette (SEQ-ID-No. 47)

**Upper case italic— promoter**
**Upper case bold – Blb2**
**Upper case italic underlined – terminator**

*AACCTCCCCTCGGTACAGCTCCTCCAGTTCTACCATGAATTTCATCCACTGATTCCTCTTCAAT*
*CGCCATTGCAGATTCTCTCGATCTATGCTCAAAAAATCCCGAGATAAAACCCTAGATCTGCTTC*
*AAATGCTCTGATACCATGTAATTTCAGTGAATTCTAACTAAACAATGGAGAGAATTAACTATTT*
*TAGAAAGACTGATTGAAGGAGAAGAAGAGAGAAAAATTCTATATTGAACTCATGAACCAAAATG*
*AATGAAAAAAATAATGAGAAGAACTATACTATTACAATCTATATATCTCTATTTATATTCTAAT*
*CTGAAGCAGTTAATTTAACTGACTCTAACAACTAGACTGATAGGTGTACATTTTCTGTTAGTGC*
*ACTGCAGTGCATTTAACTAACTGCTTAACATAAAGAATGTTGTTCGAACTTCATTCGAATAGCT*
*TCAATGAGAAGCAAACATGTGTACCTGTAAAGACACACAGTAAAAGTGTTAATAATGAATAAAT*
*ATGAATAAATCAAATAATAAATTAAAAATAAAAACACATCCAATTAACATTGGAGGTCTTGAAA*
*ATCGATGGTAATTAACAAAGACCCTTGTGAAATTTAAGTCTGTAATTGAAAATTTGAGTATAGG*
*TTAGGGGACATTTGACTATTTTCTCATTTTCTTTATCTTTTTCCTAATTTGTGGCAGACAAGTG*
*AGGAGGCCCCACTGTAATTGATTCATGCTTTTGCTTTCTTGACTTTTTGGAACAATACTATGCA*
*TCATATTTGGTCTTAATTATTCCTCTGTTTATTTCCAGAATTTTGAGCTCTATACATCTAATAA*
*CAAAGCAAGCAGAGGATATATAGTTTCATCAACTAAAAAGGTTAGTCAACTCATCTAATATTTG*
*CTACTCTCATCTCTATTGAAGTACAGTTATGGAAAAGTAGAAGTGATGTAAGAAAAATGAAAGA*
*ACTTTAGTAGGTTAGTTGGATCTAACAAAGAGAAAGGGAAATAAATTGCAGGAGAAAGAGAGAG*
*GTTAAATACTTACTCACACCACCGATTTACAACAAATCACTTAATTGTGGTTAGTTAATGTATA*
*CTTTCACCTCATTAAATTATTACTTACCCATGATAAGTTGTATTAATTTGGTATTAATATCCGG*
*TGCGGGTGAATTCTTACCGGGTGAGAGGGATGGGGTTGGAGAGTGTGGAGTGAACAGAAGCAGA*
*TGTTTTAGATTTTTTCTAAGATGACGAAAGATTCCCCTCACTAATGAAAATATATTACTATACG*
*CTATTAGAGATAGAAAGGTTCGGTACCAGTTGGTCTCGTTTCTGGATGAACCCCATTTTTACAA*
*GTCATTTTCTTCAATTCAAATCGCAAGTGTACCTTTATCATCTTCCACTAATTAAGTCCTCTTA*
*AGTTCGCGTGAAAATAGTGAAATTATTGATTATTCTTATCATTTCATCTTCTTTCTCCTGATAA*
*AGTTTTATGTACTTTTTATGCATCAGGTCTTGAGAACTTGGAAAGGAAAAGTAGAATC***ATGGAA**
**AAACGAAAAGATAATGAAGAAGCAAACAACTCATTGGTATGTTATTTGATAGAGTGAACTGTAA**
**AGTATTGAATTGTAGATATCATGTGGCTTTAAAAATTTGATATGTGTTATTTTGGCAGGAGTCA**
**TTTTCTGCTCTTCGCAAGGATGCTGCCAATGTTCTGGATTTCCTAGAGAGATTAAAGAATGAAG**
**AAGATCAAAAGGCTGTTGATGTGGATCTGATTGAAAGCCTGAAATTGAAGCTGACATTTATTTG**
**TACATATGTCCAGCTTTCTTATTCCGATTTGGAGAAGTTTGAAGATATAATGACTAGAAAAAGA**
**CAAGAGGTTGAGAATCTGCTTCAACCAATTTTGGATGATGATGGCAAAGACGTCGGGTGTAAAT**
**ATGTCCTTACTAGCCTCGCCGGTAATATGGATGACTGTATAAGCTTGTATCATCGTTCTAAATC**
**AGATGCCACCATGATGGATGAGCAATTGGGCTTCCTCCTCTTGAATCTCTCTCATCTATCCAAG**
**CATCGTGCTGAAAAGATGTTTCCTGGAGTGACTCAATATGAGGTTCTTCAGAATGTATGTGGCA**
**ACATAAGAGATTTCCATGGATTGATAGTGAATTGTTGCATTAAGCATGAGATGGTTGAGAATGT**
**CTTATCTCTGTTTCAACTGATGGCTGAGAGAGTAGGACGCTTCCTTTGGGAGGATCAGGCTGAT**
**GAAGACTCTCAACTCTCCGAGCTAGATGAGGATGATCAGAATGATAAAGACCCTCAACTCTTCA**
**AGCTAGCACATCTACTCTTGAAGATTGTTCCAACTGAATTGGAGGTTATGCACATATGTTATAA**
**AACTTTGAAAGCTTCAACTTCAACAGAAATTGGACGCTTCATTAAGAAGCTCCTGGAAACCTCT**
**CCGGACATTCTCAGAGAATATCTGATTCATCTACAAGAGCATATGATAACTGTTATTACCCCTA**
**ACACTTCAGGGGCTCGAAACATTCATGTCATGATGGAATTCCTATTGATTATTCTTTCTGATAT**
**GCCGCCCAAGGACTTTATTCATCATGACAAACTTTTTGATCTCTTGGCTCGTGTTGTAGCACTT**
**ACCAGGGAGGTATCAACTCTTGTACGCGACTTGGAAGAGAAATTAAGGATTAAAGAGAGTACTG**
**ACGAAACAAATTGTGCAACCCTAAAGTTTCTGGAAAATATTGAACTCCTTAAGGAAGATCTCAA**

Figure 2k - continued

ACATGTTTATCTGAAAGTCCCGGATTCATCTCAATATTGCTTCCCCATGAGTGATGGACCTCTC
TTCATGCATCTGCTACAGAGACACTTAGATGATTTGCTGGATTCCAATGCTTATTCAATTGCTT
TGATAAAGGAACAAATTGGGCTGGTGAAAGAAGACTTGGAATTCATAAGATCTTTTTTCGCGAA
TATTGAGCAAGGATTGTATAAAGATCTCTGGGAACGTGTTCTAGATGTGGCATATGAGGCAAAA
GATGTCATAGATTCAATTATTGTTCGAGATAATGGTCTCTTACATCTTATTTTCTCACTTCCCA
TTACCAGAAAGAAGATGATGCTTATCAAAGAAGAGGTCTCTGATTTACATGAGAACATTTCCAA
GAACAGAGGTCTCATCGTTGTGAACTCTCCCAAGAAACCAGTTGAGAGCAAGTCATTGACAACT
GATAAAATAATTGTAGGTTTTGGTGAGGAGACAAACTTGATACTTAGAAAGCTCACCAGTGGAC
CGGCAGATCTAGATGTCATTTCGATCATTGGTATGCCGGGTTTAGGTAAAACTACTTTGGCGTA
CAAAGTATACAATGATAAATCAGTTTCTAGCCATTTCGACCTTCGTGCATGGTGCACGGTCGAC
CAAGTATATGACGAGAAGAAGTTGTTGGATAAAATTTTCAATCAAGTTAGTGACTCAAATTCAA
AATTGAGTGAGAATATTGATGTTGCTGATAAACTACGGAAACAATTGTTTGGAAAGAGGTATCT
TATTGTCTTAGATGACGTGTGGGATACTAATACATGGGATGAGCTAACAAGACCTTTTCCTGAT
GGTATGAAAGGAAGTAGAATTATTTTGACAACTCGAGAAAAGAAAGTTGCTTTGCATGGAAAGC
TCTACACTGATCCTCTTAACCTTCGATTGCTAAGATCAGAAGAAAGTTGGGAGTTATTAGAGAA
AAGGGCATTTGGAAACGAGAGTTGCCCTGATGAACTATTGGATGTTGGTAAAGAAATAGCCGAA
AATTGTAAAGGGCTTCCTTTGGTGGTGGATCTGATTGCTGGAATCATTGCTGGGAGGGAAAAGA
AAAAGAGTGTGTGGCTTGAAGTTGTAAATAATTTGCATTCCTTTATTTTGAAGAATGAAGTGGA
AGTGATGAAAGTTATAGAAATAAGTTATGACCACTTACCTGATCACCTGAAGCCATGCTTGCTG
TACTTTGCAAGTGCGCCGAAGGACTGGGTAACGACAATCCATGAGTTGAAACTTATTTGGGGTT
TTGAAGGATTTGTGGAAAAGACAGATATGAAGAGTCTGGAAGAAGTGGTGAAAATTTATTTGGA
TGATTTAATTTCCAGTAGCTTGGTAATTTGTTTCAATGAGATAGGTGATTACCCTACTTGCCAA
CTTCATGATCTTGTGCATGACTTTTGTTTGATAAAAGCAAGAAAGGAAAAGTTGTGTGATCGGA
TAAGTTCAAGTGCTCCATCAGATTTGTTGCCACGTCAAATTAGCATTGATTATGATGATGATGA
AGAGCACTTTGGGCTTAATTTTGTCCTGTTCGGTTCAAATAAGAAAAGGCATTCCGGTAAACAC
CTCTATTCTTTGACCATAAATGGAGATGAGCTGGACGACCATCTTTCTGATACATTTCATCTAA
GACACTTGAGGCTTCTTAGAACCTTGCACCTGGAATCCTCTTTTATCATGGTTAAAGATTCTTT
GCTGAATGAAATATGCATGTTGAATCATTTGAGGTACTTAAGCATTGGGACAGAAGTTAAATCT
CTGCCTTTGTCTTTCTCAAACCTCTGGAATCTAGAAATCTTGTTTGTGGATAACAAAGAATCAA
CCTTGATACTATTACCGAGAATTTGGGATCTTGTAAAGTTGCAAGTGCTGTTCACGACTGCTTG
TTCTTTCTTTGATATGGATGCAGATGAATCAATACTGATAGCAGAGGACACAAAGTTAGAGAAC
TTGACAGCATTAGGGGAACTCGTGCTTTCCTATTGGAAAGATACAGAGGATATTTTCAAAAGGC
TTCCCAATCTTCAAGTGCTTCATTTCAAACTCAAGGAGTCATGGGATTATTCAACAGAGCAATA
TTGGTTCCCGAAATTGGATTTCCTAACTGAACTAGAAAAACTCACTGTAGATTTTGAAAGATCA
AACACAAATGACAGTGGGTCCTCTGCAGCCATAAATCGGCCATGGGATTTTCACTTTCCTTCGA
GTTTGAAAAGATTGCAATTGCATGAATTTCCTCTGACATCCGATTCACTATCAACAATAGCGAG
ACTGCTGAACCTTGAAGAGTTGTACCTTTATCGTACAATCATCCATGGGGAAGAATGGAACATG
GGAGAAGAAGACACCTTTGAGAATCTCAAATGTTTGATGTTGAGTCAAGTGATTCTTTCCAAGT
GGGAGGTTGGAGAGGAATCTTTTCCCACGCTTGAGAAATTAGAACTGTCGGACTGTCATAATCT
TGAGGAGATTCCGTCTAGTTTTGGGGATATTTATTCCTTGAAAATTATCGAACTTGTAAGGAGC
CCTCAACTTGAAAATTCCGCTCTCAAGATTAAGGAATATGCTGAAGATATGAGGGGAGGGGACG
AGCTTCAGATCCTTGGCCAGAAGGATATCCCGTTATTTAAGTAG*TTTTTGAGCATTATGGTTGA*
*AAAGTAGATTGCACTTTGCTGGGTAGATTGTATATGGTTAAGAAAATTCTGTTACAGTTGTTAT*
*GAAACATTTTTATTTGACTTTTCTGAGTTTCTTTTAGAAAACTCAGAAGTTTTTAACAAAAATT*
*ATAGTTTTTATAAATACAATGTGGATTTGCCTTTGGCTGTCCAACTTGGTCTGAAGTCTCATAT*
*GCTCAGAGCACTATCGTTCAACCTCAATCAAGGTACTGATTTAAAATGACATCTATACTACTTT*
*ATCACAAACCCAACGAACTTTCATCTCAAAAGCTAGGCCAGGAAGTGAAGAGGTTGTAGAGAGC*

*TTATAAGCACTCATGACTTCCTTTTCTCGAACATTCAACCAACGTAGGCTGAAATCCCACTCTG*
*AACGAAAATAAGTGTTTGTTTATCAAATTAACTCTCGTAGTAGAACACTGAAATACCTTCTTCT*

Figure 2k - continued

AAACGTTCAACAAATGGGATTTCCAGCACTCAAAGTGAATGAAAGGTTCACATTAATCTTCAAA
AAGAATTACGACAATTCATGACCACAAGTACATTGACAGCACCATTTCAACAGAAGAACAAGTC
AATGCTGCATCTTCATCAATAATCCGAGTGTCGAACCTCCTTCCTGACACTGTCCTGTATATGT
AAAGTTTCTCAACAGGGCAACTTTCTGGTCTCGTATCTGGATGACCCCTCTCGTCTATAACTTC
AACATTAAGCCCTGGCAACTTCTGGACCAACAGCTTACATGCTTCAAAACTTACTGAACAATTA
GACATCCAAAGGGATCGCATTGTCTCCAGCTTTGCAGCATTAGCCAACAGAGCCTCATCGCCAA
AGGGGCAGTCTCTAATCTCGAATTTGAAAAAATTGTTGTTGTATGACTTTCCTCTGACATCCGA
TGCACTATCAACAATAGCAAGACTGGAGGTTGGAGAGGAATCCTTTATTATACAATCATTCAGG
GAGAAGAATGGAACATGGGGGAGGAAGACACTTTTGAGAATCTGAAATGTGTTAGAGCCACAAG
CTACAGAAGTATTGAATTTGTCATGAATATCAACATTCTTCATCCTAGTTAATTCTTTTTCAAT
TTTTAATAGACTCTCATTTTAATCACTAATATTCTTCTATTTGTGACTTCTTTTCTGCAGGTGG
CAACTTTAAATTCATAAAGTATAGGATTGATGACAAACTCGAAAAATATCTTAATGAGGTGAAG
TTTGAGCAGTCAGCAGATGGTGGTTCCAACTCTAAGTTGACAAGCACATACTATCCCGGAGGGC
GATTTCAAGCCTGATGCATATGGTTAGTGTGGCTAGAGCAGACAGGATGTATTACCTGGATATC
TACCAAGACGAATCCACAATCAGTTTTATGTCAAGCAATACATGAAGTAACTCCCGATAGAACA
GTAAAAGCAAGATGTGTAGGTGTATCTCGACTCTAAGAGATTGTACATTCCTCTTTGAGATTTT
TACTGCTAATACAAATTTACACCTCAGAAGCGAATCTAGAATTTCTAGAGCATGAATGCACCAC
TAATGAAAGGAGAAAAAAGGAAGTATGAAGTGGGAATTTGATCCTTGTTTCTAGGTATATAAAA
TTTATCATTCAACTATACTTCATTTAGCAAACAACTCTCTTTGCCATTATTTCTCAAACAAGGG
CTTCTAATATTGCTAAACTAAAGACTGTCAAAAGGTAAGTTCATCTTCAAACTCTCTTGTTTAC
TTTATCTAAAGGGGAACTATGAAAAACAAGAAACATCAGGAATGTCCCGTAAACAAAGCAGCCT
CATGCACAAAACATCCAACGTTGGTAGGATTAATGGAGGGATCGCATCCCAGGAGGATACTGTA
GAAAAATTAGTGGCTTCTTTCACCGCTCAAACCCATGATCTATAGGTTACATGGAGACAACTTT
ATGGTTGCTCGTAGGCTCCCGTCAATTCTCATAAACCACAACACCAAAGTTGCATCAGACATCA
TCTTCATTCACAAGCTGACAATCTCCACAAGTCTTAGTCAACTTGTAATATGAATATTAGCCAG
GTAGACGTACATATTTACAAAATTGAGTTTCCTATATAATATGGTTTGAAGGAATGAAACATGA
TGGGGAGGGTAGATAAAATAATATATGAGGCATAAAAATAGGAAAGATATTTGTAGTGAGAGGT
TTTGACTTTTTATGCTGCTTTTGATCTTCAGTTTCTTGTATTCTTTTTCTACTGCTTTCCTCTT
CTTTCTCCTGAGTAAAGTTTTATGTAGGTACTTTTTATACGTCCGATCGTGAGAACTTGAAAGA
AAGCTCTCTATAGCTATGTTAGGTGCCCACATAAAAAAATGAAATATTACAAAAACCCTGATAA
TAAAATACACTAATCTAAGATATTCACTGCAACATACATGCAAAATATATATATATAAATTTTC
ATGAAAATTATAACAAATAATAGATGTGAACATATAACTTTAAAAATAATATTACATCCATAAA
GCTTAAATTCTAGA

Figure 2k - continued

Ahas expression casette (SEQ-ID-No. 48)

**Upper case italic— promoter**
**Upper case bold – AHAS**
**Upper case italic underlined – terminator**

*GATCATGAGCGGAGAATTAAGGGAGTCACGTTATGACCCCCGCCGATGACGCGGGACAAGCCGT*
*TTTACGTTTGGAACTGACAGAACCGCAACGTTGAAGGAGCCACTCAGCCGCGGGTTTCTGGAGT*
*TTAATGAGCTAAGCACATACGTCAGAAACCATTATTGCGCGTTCAAAAGTCGCCTAAGGTCACT*
*ATCAGCTAGCAAATATTTCTTGTCAAAAATGCTCCACTGACGTTCCATAAATTCCCCTCGGTAT*
*CCAATTAGAGTCTCATATTCACTCTCAATCCA*GATCCCCGGGTACC**ATGGCGGCGGCAACAACA**
**ACAACAACAACATCTTCTTCGATCTCCTTCTCCACCAAACCATCTCCTTCCTCCTCCAAATCAC**
**CATTACCAATCTCCAGATTCTCCCTCCCATTCTCCCTAAACCCCAACAAATCATCCTCCTCCTC**
**CCGCCGCCGCGGTATCAAATCCAGCTCTCCCTCCTCCATCTCCGCCGTGCTCAACACAACCACC**
**AATGTCACAACCACTCCCTCTCCAACCAAACCTACCAAACCCGAAACATTCATCTCCCGATTCG**
**CTCCAGATCAACCCCGCAAAGGCGCTGATATTCTCGTCGAGGCTTTAGAACGTCAAGGCGTAGA**
**AACCGTATTCGCTTACCCTGGAGGTGCATCAATGGAGATTCACCAAGCCTTAACCCGCTCTTCC**
**TCAATCCGTAACGTCCTTCCTCGTCACGAACAAGGAGGTGTATTCGCAGCAGAAGGATACGCTC**
**GATCCTCAGGTAAACCAGGTATCTGTATAGCCACTTCAGGTCCCGGAGCTACAAATCTCGTTAG**
**CGGATTAGCCGATGCGTTGTTAGATAGTGTTCCTCTTGTAGCAATCACAGGACAAGTCCCTCGT**
**CGTATGATTGGTACAGATGCGTTTCAAGAGACTCCGATTGTTGAGGTAACGCGTTCGATTACGA**
**AGCATAACTATCTTGTGATGGATGTTGAAGATATTCCTAGGATTATTGAGGAGGCTTTCTTTTT**
**AGCTACTTCTGGTAGACCTGGACCTGTTTTGGTTGATGTTCCTAAAGATATTCAACAACAGCTT**
**GCGATTCCTAATTGGGAACAGGCTATGAGATTACCTGGTTATATGTCTAGGATGCCTAAACCTC**
**CGGAAGATTCTCATTTGGAGCAGATTGTTAGGTTGATTTCTGAGTCTAAGAAGCCTGTGTTGTA**
**TGTTGGTGGTGGTTGTTTGAACTCTAGCGATGAATTGGGTAGGTTTGTTGAGCTTACGGGAATC**
**CCTGTTGCGAGTACGTTGATGGGGCTGGGATCTTATCCTTGTGATGATGAGTTGTCGTTACATA**
**TGCTTGGAATGCATGGGACTGTGTATGCAAATTACGCTGTGGAGCATAGTGATTTGTTGTTGGC**
**GTTTGGGGTAAGGTTTGATGATCGTGTCACGGGTAAACTTGAGGCTTTTGCTAGTAGGGCTAAG**
**ATTGTTCATATTGATATTGACTCGGCTGAGATTGGGAAGAATAAGACTCCTCATGTGTCTGTGT**
**GTGGTGATGTTAAGCTGGCTTTGCAAGGGATGAATAAGGTTCTTGAGAACCGAGCGGAGGAGCT**
**TAAACTTGATTTTGGAGTTTGGAGGAATGAGTTGAACGTACAGAAACAGAAGTTTCCGTTGAGC**
**TTTAAGACGTTTGGGGAAGCTATTCCTCCACAGTATGCGATTAAGGTCCTTGATGAGTTGACTG**
**ATGGAAAAGCCATAATAAGTACTGGTGTCGGGCAACATCAAATGTGGGCGGCGCAGTTCTACAA**
**TTACAAGAAACCAAGGCAGTGGCTATCATCAGGAGGCCTTGGAGCTATGGGATTTGGACTTCCT**
**GCTGCGATTGGAGCGTCTGTTGCTAACCCTGATGCGATAGTTGTGGATATTGACGGAGATGGAA**
**GTTTTATAATGAATGTGCAAGAGCTAGCCACTATTCGTGTAGAGAATCTTCCAGTGAAGGTACT**
**TTTATTAAACAACCAGCATCTTGGCATGGTTATGCAATGGGAAGATCGGTTCTACAAAGCTAAC**
**CGAGCACACACATTTCTCGGAGATCCGGCTCAGGAGGACGAGATATTCCCGAACATGTTGCTGT**
**TTGCAGCAGCTTGCGGGATTCCAGCGGCGAGGGTGACAAAGAAAGCAGATCTCCGAGAAGCTAT**
**TCAGACAATGCTGGATACACCAGGACCTTACCTGTTGGATGTGATTTGTCCGCACCAAGAACAT**
**GTGTTGCCGATGATCCCGAATGGTGGCACTTTCAACGATGTCATAACGGAAGGAGATGGCCGGA**
**TTAAATACTGA**GAGCTCGAATTTCCCC*GATCGTTCAAACATTTGGCAATAAAGTTTCTTAAGAT*
*TGAATCCTGTTGCCGGTCTTGCGATGATTATCATATAATTTCTGTTGAATTACGTTAAGCATGT*
*AATAATTAACATGTAATGCATGACGTTATTTATGAGATGGGTTTTTATGATTAGAGTCCCGCAA*
*TTATACATTTAATACGCGATAGAAAACAAAATATAGCGCGCAAACTAGGATAAATTATCGCGCG*
*CGGTGTCATCTATGTTACTAGATC*

Figure 2 I of 5

**Primer for detection blb1 and blb-2 in transformed potato plants using real time-PCR**

5´-TGT TGA ACA CTG TAA CAT GCT AAA ATG-3´ (forward Primer; SEQ ID No. 49)

5´-AGT TGT GGA CAT CCC CGA ATT-3´ (backward Primer; SEQ ID No. 50)

5´-AGA GGG ATT GCA GCA CCT AAC AAC CCT C-3´ (Probe; SEQ ID No. 51)

Rpi-blb2:

5´-TTC AAA ACC CCA AAT AAG TTT CAA C-3´ (forward Primer; SEQ ID No. 52)

5´-CCA TGC TTG CTG TAC TTT GCA-3´ (backward Primer; SEQ ID No. 53)

5´-CGT TAC CCA GTC CTT CGG CG-3´ (Probe; SEQ ID No. 54).

**Figure 2 m of 5**

Primers for Tail PCR-Amplification of flanking Sequences

| Name | Sequence | Position in VC-PMA16 | Comment |
|---|---|---|---|
| 07-038_P25 | AACGATGTCATAACGGAAGG (SEQ-ID-No. 55) | 16136 | Specific primer for tail PCR (LB1) |
| 07-038_P26 | AGAGCATTTGAAGCAGATCTAGGGT (SEQ-ID-No. 56) | 464 | Specific primer for tail PCR (RB1) |
| 07-038_P27 | CGGATTAAATACTGAGAGCTCGAAT (SEQ-ID-No. 57) | 16163 | Specific primer for tail PCR (LB2) |
| 07-038_P28 | CAGATCTAGGGTTTTATCTCGG (SEQ-ID-No. 58) | 454 | Specific primer for tail PCR (RB2) |
| 07-038_P29 | TGCCGGTCTTGCGATGATTA (SEQ-ID-No. 59) | 16241 | Specific primer for tail PCR (LB3) |
| 07-038_P30 | AGATCTAGGGTTTTATCTCGGGATT (SEQ-ID-No. 60) | 450 | Specific primer for tail PCR (RB3) |

In addition, four arbitrary degenerate (AD) primers were synthesized according to Liu et al 1995):

TGWGNAGWANCASAGA-3’ (ADI) (SEQ-ID-No. 61),

W may by A or T

S may be G or C

N may be A,T, C, or G

AGWGNAGWANCAWAGG-3’ (AD2) (SEQ-ID-No. 62),

W may by A or T

N may be A,T, C, or G

CAWCGICNGAIASGAA-3’ (AD3,) (SEQ-ID-No. 63),

W may by A or T

S may be G or C

I is inosine

N may be A,T, C, or G

TCSTICGNACITWGGA-3’ (AD4) (SEQ-ID-No. 64)

W may by A or T

S may be G or C

I is inosine

N may be A,T, C, or G

**Figure 2 n of 5**

Primers for detection of specific events A, B, and C

| Event | Border | Sense primer | SEQ ID No. | Antisense primer | SEQ ID No. | Annealig temperature [°C] | Product length (bp) |
|---|---|---|---|---|---|---|---|
| A | LB | TAATTCAGTACATT AAAGACGTCCG | 65 | GTCCCATAGTCAT TTCTTGATCA | 66 | 50 | 63 |
| | RB | TGTCTCTGATAGGC TAATAAACTATG | 67 | TAGATCTGATTGT CGTTTCCC | 68 | 48 | 91 |
| B | LB | ATGACGTTATTTAT GAGATGGGT | 69 | ATTTAAAAGGCAA AACGTGC | 70 | 49 | 100 |
| | RB | TTCATGTCAAGTTC AATTTCAGG | 71 | ACTCACATTAATT GCGTTGCG | 72 | 51 | 94 |
| C | LB | GCTTGGTAATAATT GTCATTAGATTG | 73 | GCCTTGACCTTTG AATTATTTAC | 74 | 49 | 118 |
| | RB | TCTGATGCAGAATT TTCTAACTCAA | 75 | TTCCTACTAGATC TGATTGTCGTTTC | 76 | 52 | 317 |

Figure 3 of 5

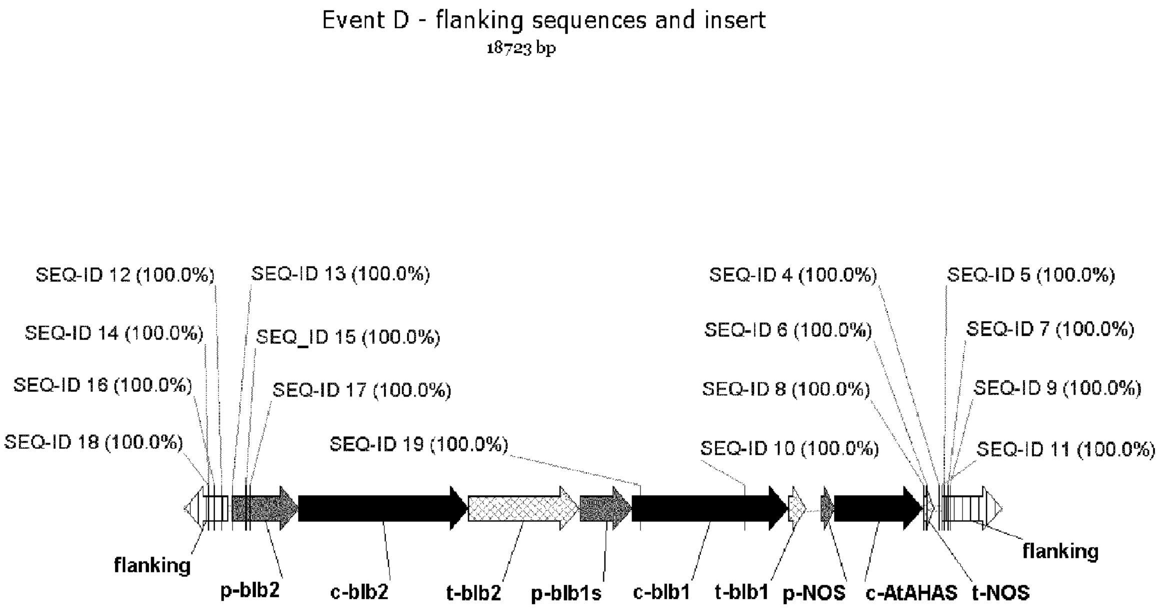
Event D - flanking sequences and insert
18723 bp
SEQ-ID 12 (100.0%)
SEQ-ID 13 (100.0%)
SEQ-ID 14 (100.0%)
SEQ_ID 15 (100.0%)
SEQ-ID 16 (100.0%)
SEQ-ID 17 (100.0%)
SEQ-ID 18 (100.0%)
SEQ-ID 19 (100.0%)
SEQ-ID 4 (100.0%)
SEQ-ID 5 (100.0%)
SEQ-ID 6 (100.0%)
SEQ-ID 7 (100.0%)
SEQ-ID 8 (100.0%)
SEQ-ID 9 (100.0%)
SEQ-ID 10 (100.0%)
SEQ-ID 11 (100.0%)
flanking
p-blb2
c-blb2
t-blb2
p-blb1s
c-blb1
t-blb1
p-NOS
c-AtAHAS
t-NOS
flanking

Figure 4 of 5

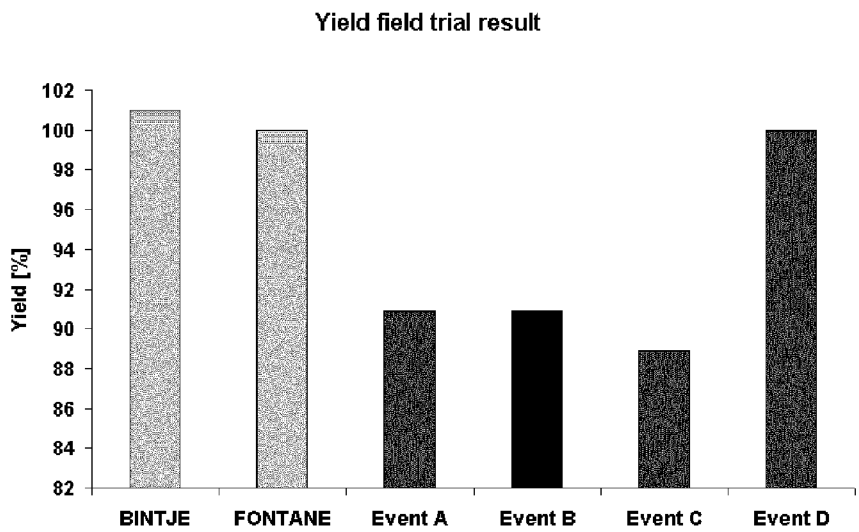
Yield field trial result
Yield [%]
102
100
98
96
94
92
90
88
86
84
82
BINTJE
FONTANE
Event A
Event B
Event C
Event D

Figure 5 of 5

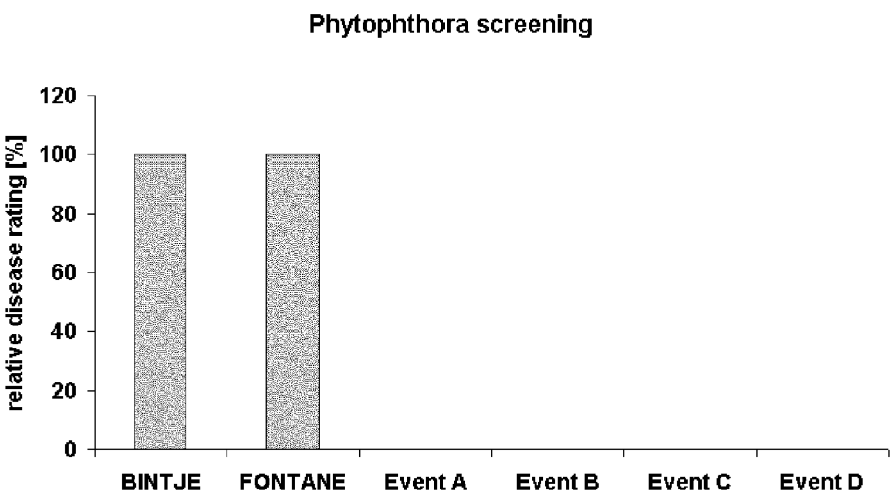
Phytophthora screening
relative disease rating [%]
120
100
80
60
40
20
0
BINTJE
FONTANE
Event A
Event B
Event C
Event D

Europäisches Patentamt
European Patent Office
Office européen des brevets

# EUROPEAN SEARCH REPORT

Application Number
EP 12 16 8070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/034876 A1 (BASF PLANT SCIENCE GMBH [DE]; FRANK MARKUS [DE]; SPEAKMAN JOHN-BRYAN [) 27 March 2008 (2008-03-27)<br>* abstract *<br>* page 8, line 21 - page 9, line 7 *<br>* page 22, line 11 - line 19 *<br>* page 28, line 1 - line 2 *<br>* page 39, line 9 - line 11 *<br>* page 57, line 10 - line 17 *<br>----- | 1,9-11 | INV.<br>C12N15/82 |
| X | MCLOUGHLIN T: "INSPECTORS REPORT ON A LICENCE APPLICATION",<br>INTERNET CITATION<br>,<br>13 April 2006 (2006-04-13), XP002466181,<br>Retrieved from the Internet:<br>URL:http://www.epa.ie/downloads/pubs/other/gmo/field/epa_gm_trial_inspection_report.pdf<br>[retrieved on 2008-01-25]<br>* page 10 *<br>----- | 1,9-11 | |
| | | | TECHNICAL FIELDS SEARCHED (IPC) |
| X | ANONYMOUS: "Notification Number B/NL/05/03",<br>INTERNET CITATION<br>,<br>30 August 2005 (2005-08-30), XP002466222,<br>Retrieved from the Internet:<br>URL:http://gmoinfo.jrc.ec.europa.eu/gmp_report.aspx?CurNot=B/NL/05/03<br>[retrieved on 2008-01-24]<br>* the whole document *<br>-----<br>-/-- | 1,9-11 | C12N |

The present search report has been drawn up for all claims

| Place of search | Date of completion of the search | Examiner |
|---|---|---|
| Munich | 8 November 2012 | Mundel, Christophe |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 8070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Pavel Forint: "Decision - Reference Number: 4697/ENV/07", The Ministry of the Environment of the Czech Republic , 10 May 2007 (2007-05-10), XP002655466, Retrieved from the Internet: URL:http://www.mzp.cz/www/webdav_biosafety.nsf/biosafety/pdf/decision_4697_ENV_07.pdf [retrieved on 2011-08-01] * page 2 * * Purpose of the release; page 7 * ----- | 1,9-11 | |

TECHNICAL FIELDS SEARCHED (IPC)

The present search report has been drawn up for all claims

1

| Place of search | Date of completion of the search | Examiner |
|---|---|---|
| Munich | 8 November 2012 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 16 8070

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2012

| Patent document cited in search report | Publication date | Patent family member(s) | Publication date |
|---|---|---|---|
| WO 2008034876 A1 | 27-03-2008 | CL 27132007 A1 | 23-05-2008 |
| | | WO 2008034876 A1 | 27-03-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008034876 A
- US 5656493 A
- EP 0116718 A
- EP 0067553 A
- US 4407956 A
- WO 9534668 A
- WO 9303161 A
- EP 0270356 A
- WO 8501856 A
- US 4684611 A
- WO 07047859 A
- WO 07049156 A
- WO 08021207 A
- WO 2010079430 A
- WO 0200900 A
- WO 2004005516 A


### Non-patent literature cited in the description


- **GISI U ; COHEN Y.** Resistance to phenylamide fungicides: A case study with Phytophthora infestans involving mating type and race structure. *Annual Rev Phytopathol.,* 1996, vol. 34, 549-572
- **RIEGER et al.** Glossary of genetics: classical and molecular. Springer-Verlag, 1991
- Current Protocols in Molecular Biology. Greene Publishing Associates, Inc. and John Wiley & Sons, Inc, 1998
- **SAMBROOK et al.** Molecular Cloning. Cold Spring Harbor Laboratory, 1989
- **MANIATIS et al.** Molecular Cloning. Cold Spring Harbor Laboratory, 1982
- Meth. Enzymol. 1993, vol. 218
- Meth Enzymol. 1979, vol. 68
- Meth. Enzymol. 1983, vol. 100, 101
- Meth. Enzymol. 1980, vol. 65
- Experiments in Molecular Genetics. Cold Spring Harbor Laboratory, 1972
- **OLD ; PRIMROSE.** Principles of Gene Manipulation, University of California Press, Berkeley. 1981
- **SCHLEIF ; WENSINK.** *Practical Methods in Molecular Biology,* 1982
- DNA Cloning. IRL Press, 1985, vol. I, II
- Nucleic Acid Hybridization. IRL Press, 1985
- **SETLOW ; HOLLAENDER.** Genetic Engineering: Principles and Methods. Plenum Press, 1979, vol. 1-4
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500, http://www.ebi.ac.uk/Tools/clustalw/index.html
- **VOET.** Voet. Wiley Press, 896-897
- **GRIBSKOV ; DEVEREUX.** Sequence Analysis Primer. Stockton Press, 1991
- **LIORENTE et al.** A quantitative real-time PCR method for in planta monitoring of Phytophthora infestans growth. *Lett Appl Microbiol.,* 2010, vol. 51 (6), 603-10
- **HORSCH RB et al.** *Science,* 1985, vol. 225, 1229
- Vectors for Gene Transfer in Higher Plants. **WHITE FF.** Transgenic Plants, Vol. 1, Engineering and Utilization. Academic Press, 1993, vol. 1, 15-38
- Techniques for Gene Transfer, Transgenic Plants. **JENES B et al.** Engineering and Utilization. Academic Press, 1993, vol. 1, 128-143
- **POTRYKUS.** *Annu Rev Plant Physiol Plant Molec Biol,* 1991, vol. 42, 205-225
- **HÖFGEN R ; WILLMITZER L.** Storage of competent cells for Agrobacterium transformation. *Nucleic Acids Res,* 1988, vol. 16, 9877
- **DUGGLEBY, R.G. ; PANG, S.S.** *Journal of Biochemistry and Molecular Biology,* 2000, vol. 33 (1), 1-36
- **HANIN ; PASZKOWSKI.** *Current Opinion Plant Biol.,* 2003, vol. 6 (2), 157-62
- **SHAKED et al.** *Proc Natl Acad Sci USA,* 2005, vol. 102 (34), 12265-9
- **IIDA ; TERADA.** *Plant Mol. Biol,* 2005, vol. 59, 205-219
- *Trends Biotechnol.,* 2005, vol. 23 (12), 567-9
- *Cell Mol Life Sci.,* 2007, vol. 64 (22), 2933-44
- **MAHFOUZ et al.** De novo-engineered transcription activator-like effector (TALE) hybrid nuclease with novel DNA binding specificity creates double-strand breaks. *PNAS,* 2011, vol. 108 (6), 2623-2628
- Voet, Voet. Wiley Press, 896-897

- **SANGER et al.** *Proc. Natl. Acad. Sci. USA,* 1977, vol. 74, 5463
- **ANDERSSON et al.** *Plant Cell Rep,* 2003, vol. 22, 261-267
- **BECKER et al.** *Molecular plant Biology,* vol. 20, 1195-1197
- **BECKER et al.** *Molecular plant Biology,* 1992, vol. 20, 1195-1197
- Agrobacterium-mediated gene transfer to plant cells: co-integrate and binary vector systems. **WALKERPEACH ; VELTEN.** Plant Molecular Biology Manual. Kluwer Academic Publishers, 1994, B1, 1-B119
- Regeneration and transformation of potato by Agrobacterium tumefaciens. **VISSER RGF.** Plant Culture Manual. Kluwer Academic Publishers, 1991, B5, 1-B59
- **ANDERSSON et al.** *plant Cell Rep,* 2003, vol. 22, 2261-267
- **SAMBROOK et al.** Molecular Cloning: A laboratory manual. Cold Spring Harbor Laboratory Press, 1989

SEQUENCE LISTING

<110> BASF Plant Science Company

<120> Development of Phytophthora resistant potato with increased yield

<130> PF71652

<140>
<141>

<150> EP11167250.7
<151> 20110524

<150> US61/489270
<151> 20110524

<160> 76

<170> PatentIn version 3.5

<210> 1
<211> 18723
<212> DNA
<213> artificial

<220>
<223> synthetic construct sequence

<400> 1
gaaactcagt tggagattac gtttaaggga tggcctacat ggataaggtt atctacaatg 60

atgcaatgga gataataata gtactcctat gtgatttctt cgttttaatt tgtttatttt 120

attttcattt aatttgattt gatacgtagt ttaaaaaaat aaaaaagata tttaaatttt 180

gtgatgttag atgaaacata cgttaaatgc atcaaaatgt catttaaatt tgtcttgaac 240

atatagtatt agaaagttga aattaaagag ctatcaaaaa agaagaagat atgactaaaa 300

aaaatagcac aagcaaattg aaactaaaag agtaattaac aataacaatt ttttttaaa 360

atgatacata atactcactc tttctcaata tgtggcacaa aaatatcaag aatcaaacta 420

tttaattttg actataaatt tagaaataaa ttttttaaat ttcttaaaaa acaaaattta 480

caaagcaaat actatataaa aaatattata aattataata attaataatt caaaatagtt 540

aaccatacaa actgaatttt gggattgagt ttctatttaa attttcgtga gtaaagatta 600

aagttcaaat taacttttta taaaatgagt tttcaatttt aaaaagtcaa acaaaatata 660

tatagaatat aaagtgagaa accaaataaa acaagaatag caaggattat ccatttagga 720

aataagatga aagaatcaaa tcaactaata aaaactttaa tcctataaga taagcttgcc 780

aataatttaa aatagcatga tgcgtctaag agggtatcaa tatattgtta tcctttaaag 840

accaagatag tgtttcagga aagttatttc atttccaaat ttaaataaaa gaaaataatt 900

attcaaaatt cttatgtctt ttttattgat ttatagtcga agatctttca aaaataattt 960

ttctataggt aaaaaaggat aatttcaatt tcacacagga aacagctatg accatgatta 1020

cgccaagctg gcgcgccaag cttgcatgcc tgcaggtcga ctctagagga tctagaatca 1080

ccgaacctcc cctcggtaca gctcctccag ttctaccatg aatttcatcc actgattcct 1140
cttcaatcgc cattgcagat tctctcgatc tatgctcaaa aaatcccgag ataaaaccct 1200
agatctgctt caaatgctct gataccatgt aatttcagtg aattctaact aaacaatgga 1260
gagaattaac tattttagaa agactgattg aaggagaaga agagagaaaa attctatatt 1320
gaactcatga accaaaatga atgaaaaaaa taatgagaag aactatacta ttacaatcta 1380
tatatctcta tttatattct aatctgaagc agttaattta actgactcta acaactagac 1440
tgataggtgt acattttctg ttagtgcact gcagtgcatt taactaactg cttaacataa 1500
agaatgttgt tcgaacttca ttcgaatagc ttcaatgaga agcaaacatg tgtacctgta 1560
aagacacaca gtaaaagtgt taataatgaa taaatatgaa taaatcaaat aataaattaa 1620
aaataaaaac acatccaatt aacattggag gtcttgaaaa tcgatggtaa ttaacaaaga 1680
cccttgtgaa atttaagtct gtaattgaaa atttgagtat aggttagggg acatttgact 1740
attttctcat tttctttatc tttttcctaa tttgtggcag acaagtgagg aggccccact 1800
gtaattgatt catgcttttg ctttcttgac tttttggaac aatactatgc atcatatttg 1860
gtcttaatta ttcctctgtt tatttccaga attttgagct ctatacatct aataacaaag 1920
caagcagagg atatatagtt tcatcaacta aaaaggttag tcaactcatc taatatttgc 1980
tactctcatc tctattgaag tacagttatg gaaaagtaga agtgatgtaa gaaaaatgaa 2040
agaactttag taggttagtt ggatctaaca aagagaaagg gaaataaatt gcaggagaaa 2100
gagagaggtt aaatacttac tcacaccacc gatttacaac aaatcactta attgtggtta 2160
gttaatgtat actttcacct cattaaatta ttacttaccc atgataagtt gtattaattt 2220
ggtattaata tccggtgcgg gtgaattctt accgggtgag agggatgggg ttggagagtg 2280
tggagtgaac agaagcagat gttttagatt ttttctaaga tgacgaaaga ttcccctcac 2340
taatgaaaat atattactat acgctattag agatagaaag gttcggtacc agttggtctc 2400
gtttctggat gaaccccatt tttacaagtc attttcttca attcaaatcg caagtgtacc 2460
tttatcatct tccactaatt aagtcctctt aagttcgcgt gaaaatagtg aaattattga 2520
ttattcttat catttcatct tctttctcct gataaagttt tatgtacttt ttatgcatca 2580
ggtcttgaga acttggaaag gaaaagtaga atcatggaaa aacgaaaaga taatgaagaa 2640
gcaaacaact cattggtatg ttatttgata gagtgaactg taaagtattg aattgtagat 2700
atcatgtggc tttaaaaatt tgatatgtgt tattttggca ggagtcattt tctgctcttc 2760
gcaaggatgc tgccaatgtt ctggatttcc tagagagatt aaagaatgaa gaagatcaaa 2820
aggctgttga tgtggatctg attgaaagcc tgaaattgaa gctgacattt atttgtacat 2880
atgtccagct ttcttattcc gatttggaga agtttgaaga tataatgact agaaaaagac 2940
aagaggttga gaatctgctt caaccaattt tggatgatga tggcaaagac gtcgggtgta 3000

aatatgtcct tactagcctc gccggtaata tggatgactg tataagcttg tatcatcgtt 3060
ctaaatcaga tgccaccatg atggatgagc aattgggctt cctcctcttg aatctctctc 3120
atctatccaa gcatcgtgct gaaaagatgt ttcctggagt gactcaatat gaggttcttc 3180
agaatgtatg tggcaacata agagatttcc atggattgat agtgaattgt tgcattaagc 3240
atgagatggt tgagaatgtc ttatctctgt ttcaactgat ggctgagaga gtaggacgct 3300
tcctttggga ggatcaggct gatgaagact ctcaactctc cgagctagat gaggatgatc 3360
agaatgataa agaccctcaa ctcttcaagc tagcacatct actcttgaag attgttccaa 3420
ctgaattgga ggttatgcac atatgttata aaactttgaa agcttcaact tcaacagaaa 3480
ttggacgctt cattaagaag ctcctggaaa cctctccgga cattctcaga gaatatctga 3540
ttcatctaca agagcatatg ataactgtta ttacccctaa cacttcaggg gctcgaaaca 3600
ttcatgtcat gatggaattc ctattgatta ttctttctga tatgccgccc aaggacttta 3660
ttcatcatga caaacttttt gatctcttgg ctcgtgttgt agcacttacc agggaggtat 3720
caactcttgt acgcgacttg gaagagaaat taaggattaa agagagtact gacgaaacaa 3780
attgtgcaac cctaaagttt ctggaaaata ttgaactcct taaggaagat ctcaaacatg 3840
tttatctgaa agtcccggat tcatctcaat attgcttccc catgagtgat ggacctctct 3900
tcatgcatct gctacagaga cacttagatg atttgctgga ttccaatgct tattcaattg 3960
ctttgataaa ggaacaaatt gggctggtga aagaagactt ggaattcata agatcttttt 4020
tcgcgaatat tgagcaagga ttgtataaag atctctggga acgtgttcta gatgtggcat 4080
atgaggcaaa agatgtcata gattcaatta ttgttcgaga taatggtctc ttacatctta 4140
ttttctcact tcccattacc agaaagaaga tgatgcttat caaagaagag gtctctgatt 4200
tacatgagaa catttccaag aacagaggtc tcatcgttgt gaactctccc aagaaaccag 4260
ttgagagcaa gtcattgaca actgataaaa taattgtagg ttttggtgag gagacaaact 4320
tgatacttag aaagctcacc agtggaccgg cagatctaga tgtcatttcg atcattggta 4380
tgccgggttt aggtaaaact actttggcgt acaaagtata caatgataaa tcagtttcta 4440
gccatttcga ccttcgtgca tggtgcacgg tcgaccaagt atatgacgag aagaagttgt 4500
tggataaaat tttcaatcaa gttagtgact caaattcaaa attgagtgag aatattgatg 4560
ttgctgataa actacggaaa caattgtttg gaaagaggta tcttattgtc ttagatgacg 4620
tgtgggatac taatacatgg gatgagctaa caagaccttt tcctgatggt atgaaaggaa 4680
gtagaattat tttgacaact cgagaaaaga aagttgcttt gcatggaaag ctctacactg 4740
atcctcttaa ccttcgattg ctaagatcag aagaaagttg ggagttatta gagaaaaggg 4800
catttggaaa cgagagttgc cctgatgaac tattggatgt tggtaaagaa atagccgaaa 4860
attgtaaagg gcttcctttg gtggtggatc tgattgctgg aatcattgct gggagggaaa 4920
agaaaaagag tgtgtggctt gaagttgtaa ataatttgca ttcctttatt ttgaagaatg 4980

aagtggaagt gatgaaagtt atagaaataa gttatgacca cttacctgat cacctgaagc 5040
catgcttgct gtactttgca agtgcgccga aggactgggt aacgacaatc catgagttga 5100
aacttatttg gggttttgaa ggatttgtgg aaaagacaga tatgaagagt ctggaagaag 5160
tggtgaaaat ttatttggat gatttaattt ccagtagctt ggtaatttgt ttcaatgaga 5220
taggtgatta ccctacttgc caacttcatg atcttgtgca tgacttttgt ttgataaaag 5280
caagaaagga aaagttgtgt gatcggataa gttcaagtgc tccatcagat ttgttgccac 5340
gtcaaattag cattgattat gatgatgatg aagagcactt tgggcttaat tttgtcctgt 5400
tcggttcaaa taagaaaagg cattccggta aacacctcta ttctttgacc ataaatggag 5460
atgagctgga cgaccatctt tctgatacat ttcatctaag acacttgagg cttcttagaa 5520
ccttgcacct ggaatcctct tttatcatgg ttaaagattc tttgctgaat gaaatatgca 5580
tgttgaatca tttgaggtac ttaagcattg ggacagaagt taaatctctg cctttgtctt 5640
tctcaaacct ctggaatcta gaaatcttgt ttgtggataa caaagaatca accttgatac 5700
tattaccgag aatttgggat cttgtaaagt tgcaagtgct gttcacgact gcttgttctt 5760
tctttgatat ggatgcagat gaatcaatac tgatagcaga ggacacaaag ttagagaact 5820
tgacagcatt aggggaactc gtgctttcct attggaaaga tacagaggat attttcaaaa 5880
ggcttcccaa tcttcaagtg cttcatttca aactcaagga gtcatgggat tattcaacag 5940
agcaatattg gttcccgaaa ttggatttcc taactgaact agaaaaactc actgtagatt 6000
ttgaaagatc aaacacaaat gacagtgggt cctctgcagc cataaatcgg ccatgggatt 6060
ttcactttcc ttcgagtttg aaaagattgc aattgcatga atttcctctg acatccgatt 6120
cactatcaac aatagcgaga ctgctgaacc ttgaagagtt gtacctttat cgtacaatca 6180
tccatgggga agaatggaac atgggagaag aagacacctt tgagaatctc aaatgtttga 6240
tgttgagtca agtgattctt tccaagtggg aggttggaga ggaatctttt cccacgcttg 6300
agaaattaga actgtcggac tgtcataatc ttgaggagat tccgtctagt tttggggata 6360
tttattcctt gaaaattatc gaacttgtaa ggagccctca acttgaaaat tccgctctca 6420
agattaagga atatgctgaa gatatgaggg gaggggacga gcttcagatc cttggccaga 6480
aggatatccc gttatttaag tagtttttga gcattatggt tgaaaagtag attgcacttt 6540
gctgggtaga ttgtatatgg ttaagaaaat tctgttacag ttgttatgaa acatttttat 6600
ttgacttttc tgagtttctt ttagaaaact cagaagtttt taacaaaaat tatagttttt 6660
ataaatacaa tgtggatttg cctttggctg tccaacttgg tctgaagtct catatgctca 6720
gagcactatc gttcaacctc aatcaaggta ctgatttaaa atgacatcta tactacttta 6780
tcacaaaccc aacgaacttt catctcaaaa gctaggccag gaagtgaaga ggttgtagag 6840
agcttataag cactcatgac ttccttttct cgaacattca accaacgtag gctgaaatcc 6900

cactctgaac gaaaataagt gtttgtttat caaattaact ctcgtagtag aacactgaaa 6960
taccttcttc taaacgttca acaaatggga tttccagcac tcaaagtgaa tgaaaggttc 7020
acattaatct tcaaaaagaa ttacgacaat tcatgaccac aagtacattg acagcaccat 7080
ttcaacagaa gaacaagtca atgctgcatc ttcatcaata atccgagtgt cgaacctcct 7140
tcctgacact gtcctgtata tgtaaagttt ctcaacaggg caactttctg gtctcgtatc 7200
tggatgaccc ctctcgtcta taacttcaac attaagccct ggcaacttct ggaccaacag 7260
cttacatgct tcaaaactta ctgaacaatt agacatccaa agggatcgca ttgtctccag 7320
ctttgcagca ttagccaaca gagcctcatc gccaaagggg cagtctctaa tctcgaattt 7380
gaaaaaattg ttgttgtatg actttcctct gacatccgat gcactatcaa caatagcaag 7440
actggaggtt ggagaggaat cctttattat acaatcattc agggagaaga atggaacatg 7500
ggggaggaag acacttttga gaatctgaaa tgtgttagag ccacaagcta cagaagtatt 7560
gaatttgtca tgaatatcaa cattcttcat cctagttaat tctttttcaa tttttaatag 7620
actctcattt taatcactaa tattcttcta tttgtgactt cttttctgca ggtggcaact 7680
ttaaattcat aaagtatagg attgatgaca aactcgaaaa atatcttaat gaggtgaagt 7740
ttgagcagtc agcagatggt ggttccaact ctaagttgac aagcacatac tatcccggag 7800
ggcgatttca agcctgatgc atatggttag tgtggctaga gcagacagga tgtattacct 7860
ggatatctac caagacgaat ccacaatcag ttttatgtca agcaatacat gaagtaactc 7920
ccgatagaac agtaaaagca agatgtgtag gtgtatctcg actctaagag attgtacatt 7980
cctctttgag atttttactg ctaatacaaa tttacacctc agaagcgaat ctagaatttc 8040
tagagcatga atgcaccact aatgaaagga gaaaaaagga agtatgaagt gggaatttga 8100
tccttgtttc taggtatata aaatttatca ttcaactata cttcatttag caaacaactc 8160
tctttgccat tatttctcaa acaagggctt ctaatattgc taaactaaag actgtcaaaa 8220
ggtaagttca tcttcaaact ctcttgttta ctttatctaa aggggaacta tgaaaaacaa 8280
gaaacatcag gaatgtcccg taaacaaagc agcctcatgc acaaaacatc caacgttggt 8340
aggattaatg gagggatcgc atcccaggag gatactgtag aaaaattagt ggcttctttc 8400
accgctcaaa cccatgatct ataggttaca tggagacaac tttatggttg ctcgtaggct 8460
cccgtcaatt ctcataaacc acaacaccaa agttgcatca gacatcatct tcattcacaa 8520
gctgacaatc tccacaagtc ttagtcaact tgtaatatga atattagcca ggtagacgta 8580
catatttaca aaattgagtt tcctatataa tatggtttga aggaatgaaa catgatgggg 8640
agggtagata aaataatata tgaggcataa aaataggaaa gatatttgta gtgagaggtt 8700
ttgacttttt atgctgcttt tgatcttcag tttcttgtat tctttttcta ctgctttcct 8760
cttctttctc ctgagtaaag ttttatgtag gtacttttta tacgtccgat cgtgagaact 8820
tgaaagaaag ctctctatag ctatgttagg tgcccacata aaaaaatgaa atattacaaa 8880

aaccctgata ataaaataca ctaatctaag atattcactg caacatacat gcaaaatata 8940
tatatataaa ttttcatgaa aattataaca aataatagat gtgaacatat aactttaaaa 9000
ataatattac atccataaag cttaaattct agatccccgg tcgactctag aggatcccca 9060
ctccatccgt tcactttgat ttgtcatgtt gcacttttcg aaagtcaatt tgactaattt 9120
ttaaagctaa attagattac actaattcaa tattttaaac agaaaaatta gatattcaaa 9180
aactatacaa aaaatattat acattgcaat tttttgcata tcaatatgat aaaaaaatat 9240
atcgtaaaat attagtcaaa atttttataa tttgactcaa atcatgaaaa gtataataat 9300
taatagtgga cggaggaagt attgtctttc cagatttgtg gccatttttg gtccaagggc 9360
cattagcagt tctcttcatt ttctacttct gtctcatatt agatgggcat cttactaaaa 9420
atatttgtct catattactt gattatttat taaatcaaaa agaattaatt aatttttttct 9480
cattttaccc ctacaattaa tatagtttta aaagttttaa acaaattttg aagaatcaaa 9540
atttcttttt gcaagagact tattaatata aacaaaggat aaaataataa aatttgtcaa 9600
tttattgacg atcacttaat aatcatataa aatagaaaat gtttatctaa tatgagacgg 9660
agaaaatata tcctaaaata tttttggaca gatatgtgat attctaacca ttcactagac 9720
tatattatgc attttagccg ccaatgactt atttcagctt taattaatta ggaaagagga 9780
aactgccaat gaggaagagt aggggcgtag ttgctgtcga cgaaaaaaag ataatactca 9840
ctcttttcga tttttatttt tatttatcac ttttaaccta tcatgtaaaa agataattat 9900
ttttttcatg ctttatcctt agtattaaac aatttaatag ggattatttt gtaaaatatt 9960
tatatgaata attgttttcg taatgaattt gtccggtcaa acaatgataa ataaaaatga 10020
atgaagagag tagaaaacaa aacaaaagaa caagttgaca acttgagaga ttaaaagggt 10080
ccaaaacgcc ttggattttg agattccata tgtgaaattt ccatgaaata attgaatttg 10140
tattattaca agtcaaactt tccatttcat tccaactagc catcttggtt tcaaaattac 10200
acattcattc attcacagat ctaatattct taatagtgat ttccacatat ggctgaagct 10260
ttcattcaag ttctgctaga caatctcact tctttcctca aaggggaact tgtattgctt 10320
ttcggttttc aagatgagtt ccaaaggctt tcaagcatgt tttctacaat tcaagccgtc 10380
cttgaagatg ctcaggagaa gcaactcaac aacaagcctc tagaaaattg gttgcaaaaa 10440
ctcaatgctg ctacatatga agtcgatgac atcttggatg aatataaaac caaggccaca 10500
agattctccc agtctgaata tggccgttat catccaaagg ttatcccttt ccgtcacaag 10560
gtcgggaaaa ggatggacca agtgatgaaa aaactaaagg caattgctga ggaaagaaag 10620
aattttcatt tgcacgaaaa aattgtagag agacaagctg ttagacggga aacaggtact 10680
catcttaaat tagtattaca acaactaagt ttatattcat ttttttggca attatcaaat 10740
tcagaaaagg gttaaatata ctcatgtcct atcgtaaata gtgtatatat acctctcgtt 10800

gtactttcga tctgaatata cttgtcaaat ctggcaagct cagaatcaaa ttatccaccc 10860
caacttttaa atactcgata tctttagaaa tccacctgtc taactcatcc actacccatt 10920
ccctttgctt tgaattcttt tctttaccta taaacttgga acactcgatc cgttttgctt 10980
ttcttaacaa agcagctcag agaaaagagg ttttcttcta ttctgtttct ctgtgtgctg 11040
cacttgggtc cttaatccca ttaaaaacag ggcatgttaa tcccaacgac ggtagccttt 11100
cctgacagct gactgtaaat tttgtctaac aaagaaaaaa aaagattaga catgtttttc 11160
cttgtcattg attaggctgg atttctttca gagtggaaca taggggatat attggaccaa 11220
aagtagaatg ggtatatatt taaagtattt ctgatagaac aggagtatat tgtgcgaaaa 11280
tatcctctat tttctgttgt ctcctaatga gtttgaatgt aataatattc tcatgtggac 11340
attgcttgca ccaggttctg tattaaccga accgcaggtt tatggaagag acaaagagaa 11400
agatgagata gtgaaaatcc taataaacaa tgttagtgat gcccaacacc tttcagtcct 11460
cccaatactt ggtatggggg gattaggaaa aacgactctt gcccaaatgg tcttcaatga 11520
ccagagagtt actgagcatt tccattccaa aatatggatt tgtgtctcgg aagattttga 11580
tgagaagagg ttaataaagg caattgtaga atctattgaa ggaaggccac tacttggtga 11640
gatggacttg gctccacttc aaaagaagct tcaggagttg ctgaatggaa aaagatactt 11700
gcttgtctta gatgatgttt ggaatgaaga tcaacagaag tgggctaatt taagagcagt 11760
cttgaaggtt ggagcaagtg gtgcttctgt tctaaccact actcgtcttg aaaaggttgg 11820
atcaattatg ggaacattgc aaccatatga actgtcaaat ctgtctcaag aagattgttg 11880
gttgttgttc atgcaacgtg catttggaca ccaagaagaa ataaatccaa accttgtggc 11940
aatcggaaag gagattgtga aaaaaagtgg tggtgtgcct ctagcagcca aaactcttgg 12000
aggtattttg tgcttcaaga gagaagaaag agcatgggaa catgtgagag acagtccgat 12060
ttggaatttg cctcaagatg aaagttctat tctgcctgcc ctgaggctta gttaccatca 12120
acttccactt gatttgaaac aatgctttgc gtattgtgcg gtgttcccaa aggatgccaa 12180
aatggaaaaa gaaaagctaa tctctctctg gatggcgcat ggttttcttt tatcaaaagg 12240
aaacatggag ctagaggatg tgggcgatga agtatggaaa gaattatact tgaggtcttt 12300
tttccaagag attgaagtta aagatggtaa aacttatttc aagatgcatg atctcatcca 12360
tgatttggca acatctctgt tttcagcaaa cacatcaagc agcaatatcc gtgaaataaa 12420
taaacacagt tacacacata tgatgtccat tggtttcgcc gaagtggtgt ttttttacac 12480
tcttcccccc ttggaaaagt ttatctcgtt aagagtgctt aatctaggtg attcgacatt 12540
taataagtta ccatcttcca ttggagatct agtacattta agatacttga acctgtatgg 12600
cagtggcatg cgtagtcttc caaagcagtt atgcaagctt caaaatctgc aaactcttga 12660
tctacaatat tgcaccaagc tttgttgttt gccaaaagaa acaagtaaac ttggtagtct 12720
ccgaaatctt ttacttgatg gtagccagtc attgacttgt atgccaccaa ggataggatc 12780

attgacatgc cttaagactc taggtcaatt tgttgttgga aggaagaaag gttatcaact 12840
tggtgaacta ggaaacctaa atctctatgg ctcaattaaa atctcgcatc ttgagagagt 12900
gaagaatgat aaggacgcaa aagaagccaa tttatctgca aaagggaatc tgcattcttt 12960
aagcatgagt tggaataact ttggaccaca tatatatgaa tcagaagaag ttaaagtgct 13020
tgaagccctc aaaccacact ccaatctgac ttctttaaaa atctatggct tcagaggaat 13080
ccatctccca gagtggatga atcactcagt attgaaaaat attgtctcta ttctaattag 13140
caacttcaga aactgctcat gcttaccacc ctttggtgat ctgccttgtc tagaaagtct 13200
agagttacac tgggggtctg cggatgtgga gtatgttgaa gaagtggata ttgatgttca 13260
ttctggattc ccccacaagaa taaggtttcc atccttgagg aaacttgata tatgggactt 13320
tggtagtctg aaaggattgc tgaaaaagga aggagaagag caattccctg tgcttgaaga 13380
gatgataatt cacgagtgcc cttttctgac cctttcttct aatcttaggg ctcttacttc 13440
cctcagaatt tgctataata aagtagctac ttcattccca gaagagatgt tcaaaaacct 13500
tgcaaatctc aaatacttga caatctctcg gtgcaataat ctcaaagagc tgcctaccag 13560
cttggctagt ctgaatgctt tgaaaagtct aaaaattcaa ttgtgttgcg cactagagag 13620
tctccctgag gaagggctgg aaggtttatc ttcactcaca gagttatttg ttgaacactg 13680
taacatgcta aaatgtttac cagagggatt gcagcaccta acaaccctca caagtttaaa 13740
aattcgggga tgtccacaac tgatcaagcg gtgtgagaag ggaataggag aagactggca 13800
caaaatttct cacattccta atgtgaatat atatatttaa gttatttgct attgtttctt 13860
tgtttgtgag tctttttggt tcctgccatt gtgattgcat gtaatttttt tctagggttg 13920
tttctttatg agtctctctc tcattggatg taattttctt ttggaaacaa atctgtcaat 13980
tgatttgtat tatacgcttt cagaatctat tacttatttg taattgtttc tttgtttgta 14040
aattgtgagt atcttatttt atggaatttt ctgattttat tttgaaaaca aatcaatgat 14100
ttgtaagatc catctgtatt atactccctt cgtctcattt tatgtgtcac ctgtcggatt 14160
tcgagattca aacaaatcta tctttgatcg taaattttta atagatcttt taaacatttt 14220
gaattatcaa ttattgtgac tttagtggct agactagtgg atccgatatc gcccagcttc 14280
acgctgccgc aagcactcag ggcgcaaggg ctgctaaagg aagcggaaca cgtagaaagc 14340
cagtccgcag aaacggtgct gaccccggat gaatgtcagc tactgggcta tctggacaag 14400
ggaaaacgca agcgcaaaga gaaagcaggt agcttgcagt gggcttacat ggcgatagct 14460
agactgggcg gttttatgga cagcaagcga accggaattg ccagctgggg cgccctctgg 14520
taaggttggg aagccctgca aagtaaactg gatggctttc ttgccgccaa ggatctgatg 14580
gcgcagggga tcaagatcat gagcggagaa ttaagggagt cacgttatga cccccgccga 14640
tgacgcggga caagccgttt tacgtttgga actgacagaa ccgcaacgtt gaaggagcca 14700

ctcagccgcg ggtttctgga gtttaatgag ctaagcacat acgtcagaaa ccattattgc 14760

gcgttcaaaa gtcgcctaag gtcactatca gctagcaaat atttcttgtc aaaaatgctc 14820

cactgacgtt ccataaattc ccctcggtat ccaattagag tctcatattc actctcaatc 14880

cagatccccg ggtaccatgg cggcggcaac aacaacaaca acaacatctt cttcgatctc 14940

cttctccacc aaaccatctc cttcctcctc caaatcacca ttaccaatct ccagattctc 15000

cctcccattc tccctaaacc ccaacaaatc atcctcctcc tcccgccgcc gcggtatcaa 15060

atccagctct ccctcctcca tctccgccgt gctcaacaca accaccaatg tcacaaccac 15120

tccctctcca accaaaccta ccaaacccga aacattcatc tcccgattcg ctccagatca 15180

accccgcaaa ggcgctgata ttctcgtcga ggctttagaa cgtcaaggcg tagaaaccgt 15240

attcgcttac cctggaggtg catcaatgga gattcaccaa gccttaaccc gctcttcctc 15300

aatccgtaac gtccttcctc gtcacgaaca aggaggtgta ttcgcagcag aaggatacgc 15360

tcgatcctca ggtaaaccag gtatctgtat agccacttca ggtcccggag ctacaaatct 15420

cgttagcgga ttagccgatg cgttgttaga tagtgttcct cttgtagcaa tcacaggaca 15480

agtccctcgt cgtatgattg gtacagatgc gtttcaagag actccgattg ttgaggtaac 15540

gcgttcgatt acgaagcata actatcttgt gatggatgtt gaagatattc ctaggattat 15600

tgaggaggct ttctttttag ctacttctgg tagacctgga cctgttttgg ttgatgttcc 15660

taaagatatt caacaacagc ttgcgattcc taattgggaa caggctatga gattacctgg 15720

ttatatgtct aggatgccta aacctccgga agattctcat ttggagcaga ttgttaggtt 15780

gatttctgag tctaagaagc ctgtgttgta tgttggtggt ggttgtttga actctagcga 15840

tgaattgggt aggtttgttg agcttacggg aatccctgtt gcgagtacgt tgatggggct 15900

gggatcttat ccttgtgatg atgagttgtc gttacatatg cttggaatgc atgggactgt 15960

gtatgcaaat tacgctgtgg agcatagtga tttgttgttg gcgtttgggg taaggtttga 16020

tgatcgtgtc acgggtaaac ttgaggcttt tgctagtagg gctaagattg ttcatattga 16080

tattgactcg gctgagattg ggaagaataa gactcctcat gtgtctgtgt gtggtgatgt 16140

taagctggct ttgcaaggga tgaataaggt tcttgagaac cgagcggagg agcttaaact 16200

tgattttgga gtttggagga atgagttgaa cgtacagaaa cagaagtttc cgttgagctt 16260

taagacgttt ggggaagcta ttcctccaca gtatgcgatt aaggtccttg atgagttgac 16320

tgatggaaaa gccataataa gtactggtgt cgggcaacat caaatgtggg cggcgcagtt 16380

ctacaattac aagaaaccaa ggcagtggct atcatcagga ggccttggag ctatgggatt 16440

tggacttcct gctgcgattg gagcgtctgt tgctaaccct gatgcgatag ttgtggatat 16500

tgacggagat ggaagtttta taatgaatgt gcaagagcta gccactattc gtgtagagaa 16560

tcttccagtg aaggtacttt tattaaacaa ccagcatctt ggcatggtta tgcaatggga 16620

agatcggttc tacaaagcta accgagcaca cacatttctc ggagatccgg ctcaggagga 16680

cgagatattc ccgaacatgt tgctgtttgc agcagcttgc gggattccag cggcgagggt 16740
gacaaagaaa gcagatctcc gagaagctat tcagacaatg ctggatacac caggacctta 16800
cctgttggat gtgatttgtc cgcaccaaga acatgtgttg ccgatgatcc cgaatggtgg 16860
cactttcaac gatgtcataa cggaaggaga tggccggatt aaatactgag agctcgaatt 16920
tccccgatcg ttcaaacatt tggcaataaa gtttcttaag attgaatcct gttgccggtc 16980
ttgcgatgat tatcatataa tttctgttga attacgttaa gcatgtaata attaacatgt 17040
aatgcatgac gttatttatg agatgggttt ttatgattag agtcccgcaa ttatacattt 17100
aatacgcgat agaaaacaaa atatagcgcg caaactagga taaattatcg cgcgcggtgt 17160
catctatgtt actagatcgg gaattcactg gccgtcgttt tacaacgact cagctgcttg 17220
gtaataattg tcattagatt gtttttatgc atagatgcac tcgaaatcag ccaattttag 17280
acaagtatca aacggatgtt aattcagtac attaaagacg tccgcaatgt gttattaagt 17340
tgtctaagtt gtccttggtt ttattagttg ttattgttgt tttgtctttt caatttctag 17400
tagtcaattg ggaactggaa gtacaaagtt gaatttttag acttatgtat acaaattgaa 17460
aatgtatcaa aaagtagtga cacttataaa aagaacaatt ctttcttctg agaacatctt 17520
caacccacct ctattttact ctccattctc tatatttagt gagtaaaata gagaatgggc 17580
actccaaccc acctccatat cactctccat tcttcatatt tagagagtca tattattttt 17640
attattattt ttattacttt ctaattaata tattatttta catataatgt cattaattaa 17700
atatctaata ttcataattc tttttaaatg tcatattata ttttaaaaaa tatattattt 17760
aatatatact actttcatcc cgaattaata gtattttaat tttttctaat tttcgacaat 17820
aatataattt gattttatta ttaattttca ctataaataa tacacaaaat taaaatgata 17880
agatgaaaaa attaatttaa aaatacaata cataatatga taatttaaat acaggataac 17940
aatacaatac atgacataat aatttaaata caagataaaa tacaatacat aacataataa 18000
tcaattcgtg atgaaacaag aatcatgcca aaaagatatt gaacgtgcat tcgaagtttt 18060
gcaatcacgt tttgcaatta ttgcaagacc gtcacgtttt tggagaaagg aagtgtgaca 18120
tgatataatg actacatgta ttatactgca caccatgata attgaggatg aacatgatct 18180
taatgcacca aatcaagatg ccgtagaggc tccaactcca acgacaaaaa tgatggtaga 18240
tgaaaatctt cggtttgaac aatttttagc tagacataaa aaagttaagg acaaaaatgc 18300
tcattttgaa ctccgtaatg cattaataga gcatttatga cagcaacgtg ataatttcga 18360
aacttgagtg tttatgtaat tatatttcac ttttatttga atttgtccat taatttgtat 18420
attatataat atttatttac aatttatgtt atttaaaaat ttagaataaa ataaaatttt 18480
gaaaaaataa aattttatat cacatgaaaa ttatataaag taattattgg ggaaaaaaat 18540
aaaggattta tattatgaaa taagaaaata agaatgaata gaaataataa taatataata 18600

ttgaggagaa ataattattt tttttagaga gtgaaataga gaattgggtt gaaattgatt 18660

gtctgaaaaa ataaaaaact ctatatttgg agagtaaaat agagtgtagg gttggagacg 18720

tca 18723

<210> 2
<211> 16363
<212> DNA
<213> artificial

<220>
<223> synthetic construct sequence

<400> 2
caatttcaca caggaaacag ctatgaccat gattacgcca agctggcgcg ccaagcttgc 60

atgcctgcag gtcgactcta gaggatctag aatcaccgaa cctcccctcg gtacagctcc 120

tccagttcta ccatgaattt catccactga ttcctcttca atcgccattg cagattctct 180

cgatctatgc tcaaaaaatc ccgagataaa accctagatc tgcttcaaat gctctgatac 240

catgtaattt cagtgaattc taactaaaca atggagagaa ttaactattt tagaaagact 300

gattgaagga gaagaagaga gaaaaattct atattgaact catgaaccaa aatgaatgaa 360

aaaaataatg agaagaacta tactattaca atctatatat ctctatttat attctaatct 420

gaagcagtta atttaactga ctctaacaac tagactgata ggtgtacatt ttctgttagt 480

gcactgcagt gcatttaact aactgcttaa cataaagaat gttgttcgaa cttcattcga 540

atagcttcaa tgagaagcaa acatgtgtac ctgtaaagac acacagtaaa agtgttaata 600

atgaataaat atgaataaat caaataataa attaaaaata aaaacacatc caattaacat 660

tggaggtctt gaaaatcgat ggtaattaac aaagaccctt gtgaaattta agtctgtaat 720

tgaaaatttg agtataggtt aggggacatt tgactatttt ctcattttct ttatcttttt 780

cctaatttgt ggcagacaag tgaggaggcc ccactgtaat tgattcatgc ttttgctttc 840

ttgacttttt ggaacaatac tatgcatcat atttggtctt aattattcct ctgtttattt 900

ccagaatttt gagctctata catctaataa caaagcaagc agaggatata tagtttcatc 960

aactaaaaag gttagtcaac tcatctaata tttgctactc tcatctctat tgaagtacag 1020

ttatggaaaa gtagaagtga tgtaagaaaa atgaaagaac tttagtaggt tagttggatc 1080

taacaaagag aaagggaaat aaattgcagg agaaagagag aggttaaata cttactcaca 1140

ccaccgattt acaacaaatc acttaattgt ggttagttaa tgtatacttt cacctcatta 1200

aattattact tacccatgat aagttgtatt aatttggtat taatatccgg tgcgggtgaa 1260

ttcttaccgg gtgagaggga tggggttgga gagtgtggag tgaacagaag cagatgtttt 1320

agattttttc taagatgacg aaagattccc ctcactaatg aaaatatatt actatacgct 1380

attagagata gaaaggttcg gtaccagttg gtctcgtttc tggatgaacc ccatttttac 1440

aagtcatttt cttcaattca aatcgcaagt gtacctttat catcttccac taattaagtc 1500

ctcttaagtt cgcgtgaaaa tagtgaaatt attgattatt cttatcattt catcttcttt 1560
ctcctgataa agttttatgt actttttatg catcaggtct tgagaacttg gaaaggaaaa 1620
gtagaatcat ggaaaaacga aaagataatg aagaagcaaa caactcattg gtatgttatt 1680
tgatagagtg aactgtaaag tattgaattg tagatatcat gtggctttaa aaatttgata 1740
tgtgttattt tggcaggagt cattttctgc tcttcgcaag gatgctgcca atgttctgga 1800
tttcctagag agattaaaga atgaagaaga tcaaaaggct gttgatgtgg atctgattga 1860
aagcctgaaa ttgaagctga catttatttg tacatatgtc cagctttctt attccgattt 1920
ggagaagttt gaagatataa tgactagaaa aagacaagag gttgagaatc tgcttcaacc 1980
aattttggat gatgatggca aagacgtcgg gtgtaaatat gtccttacta gcctcgccgg 2040
taatatggat gactgtataa gcttgtatca tcgttctaaa tcagatgcca ccatgatgga 2100
tgagcaattg ggcttcctcc tcttgaatct ctctcatcta tccaagcatc gtgctgaaaa 2160
gatgtttcct ggagtgactc aatatgaggt tcttcagaat gtatgtggca acataagaga 2220
tttccatgga ttgatagtga attgttgcat taagcatgag atggttgaga atgtcttatc 2280
tctgtttcaa ctgatggctg agagagtagg acgcttcctt tgggaggatc aggctgatga 2340
agactctcaa ctctccgagc tagatgagga tgatcagaat gataaagacc ctcaactctt 2400
caagctagca catctactct tgaagattgt tccaactgaa ttggaggtta tgcacatatg 2460
ttataaaact ttgaaagctt caacttcaac agaaattgga cgcttcatta agaagctcct 2520
ggaaacctct ccggacattc tcagagaata tctgattcat ctacaagagc atatgataac 2580
tgttattacc cctaacactt caggggctcg aaacattcat gtcatgatgg aattcctatt 2640
gattattctt tctgatatgc cgcccaagga ctttattcat catgacaaac tttttgatct 2700
cttggctcgt gttgtagcac ttaccaggga ggtatcaact cttgtacgcg acttggaaga 2760
gaaattaagg attaaagaga gtactgacga aacaaattgt gcaaccctaa agtttctgga 2820
aaatattgaa ctccttaagg aagatctcaa acatgtttat ctgaaagtcc cggattcatc 2880
tcaatattgc ttccccatga gtgatggacc tctcttcatg catctgctac agagacactt 2940
agatgatttg ctggattcca atgcttattc aattgctttg ataaaggaac aaattgggct 3000
ggtgaaagaa gacttggaat tcataagatc ttttttcgcg aatattgagc aaggattgta 3060
taaagatctc tgggaacgtg ttctagatgt ggcatatgag gcaaaagatg tcatagattc 3120
aattattgtt cgagataatg gtctcttaca tcttattttc tcacttccca ttaccagaaa 3180
gaagatgatg cttatcaaag aagaggtctc tgatttacat gagaacattt ccaagaacag 3240
aggtctcatc gttgtgaact ctcccaagaa accagttgag agcaagtcat tgacaactga 3300
taaaataatt gtaggttttg gtgaggagac aaacttgata cttagaaagc tcaccagtgg 3360
accggcagat ctagatgtca tttcgatcat tggtatgccg ggtttaggta aaactacttt 3420

ggcgtacaaa gtatacaatg ataaatcagt ttctagccat ttcgaccttc gtgcatggtg 3480

cacggtcgac caagtatatg acgagaagaa gttgttggat aaaattttca atcaagttag 3540

tgactcaaat tcaaaattga gtgagaatat tgatgttgct gataaactac ggaaacaatt 3600

gtttggaaag aggtatctta ttgtcttaga tgacgtgtgg gatactaata catgggatga 3660

gctaacaaga ccttttcctg atggtatgaa aggaagtaga attattttga caactcgaga 3720

aaagaaagtt gctttgcatg gaaagctcta cactgatcct cttaaccttc gattgctaag 3780

atcagaagaa agttgggagt tattagagaa aagggcattt ggaaacgaga gttgccctga 3840

tgaactattg gatgttggta aagaaatagc cgaaaattgt aaagggcttc ctttggtggt 3900

ggatctgatt gctggaatca ttgctgggag ggaaaagaaa aagagtgtgt ggcttgaagt 3960

tgtaaataat ttgcattcct ttattttgaa gaatgaagtg gaagtgatga aagttataga 4020

aataagttat gaccacttac ctgatcacct gaagccatgc ttgctgtact ttgcaagtgc 4080

gccgaaggac tgggtaacga caatccatga gttgaaactt atttggggtt ttgaaggatt 4140

tgtggaaaag acagatatga agagtctgga agaagtggtg aaaatttatt tggatgattt 4200

aatttccagt agcttggtaa tttgtttcaa tgagataggt gattacccta cttgccaact 4260

tcatgatctt gtgcatgact tttgtttgat aaaagcaaga aaggaaaagt tgtgtgatcg 4320

gataagttca agtgctccat cagatttgtt gccacgtcaa attagcattg attatgatga 4380

tgatgaagag cactttgggc ttaattttgt cctgttcggt tcaaataaga aaaggcattc 4440

cggtaaacac ctctattctt tgaccataaa tggagatgag ctggacgacc atctttctga 4500

tacatttcat ctaagacact tgaggcttct tagaaccttg cacctggaat cctcttttat 4560

catggttaaa gattctttgc tgaatgaaat atgcatgttg aatcatttga ggtacttaag 4620

cattgggaca gaagttaaat ctctgccttt gtctttctca aacctctgga atctagaaat 4680

cttgtttgtg gataacaaag aatcaacctt gatactatta ccgagaattt gggatcttgt 4740

aaagttgcaa gtgctgttca cgactgcttg ttctttcttt gatatggatg cagatgaatc 4800

aatactgata gcagaggaca caaagttaga gaacttgaca gcattagggg aactcgtgct 4860

ttcctattgg aaagatacag aggatatttt caaaaggctt cccaatcttc aagtgcttca 4920

tttcaaactc aaggagtcat gggattattc aacagagcaa tattggttcc cgaaattgga 4980

tttcctaact gaactagaaa aactcactgt agattttgaa agatcaaaca caaatgacag 5040

tgggtcctct gcagccataa atcggccatg ggattttcac tttccttcga gtttgaaaag 5100

attgcaattg catgaatttc ctctgacatc cgattcacta tcaacaatag cgagactgct 5160

gaaccttgaa gagttgtacc tttatcgtac aatcatccat ggggaagaat ggaacatggg 5220

agaagaagac acctttgaga atctcaaatg tttgatgttg agtcaagtga ttctttccaa 5280

gtgggaggtt ggagaggaat cttttcccac gcttgagaaa ttagaactgt cggactgtca 5340

taatcttgag gagattccgt ctagttttgg ggatatttat tccttgaaaa ttatcgaact 5400

tgtaaggagc cctcaacttg aaaattccgc tctcaagatt aaggaatatg ctgaagatat 5460
gaggggaggg gacgagcttc agatccttgg ccagaaggat atcccgttat ttaagtagtt 5520
tttgagcatt atggttgaaa agtagattgc actttgctgg gtagattgta tatggttaag 5580
aaaattctgt tacagttgtt atgaaacatt tttatttgac ttttctgagt ttcttttaga 5640
aaactcagaa gtttttaaca aaaattatag tttttataaa tacaatgtgg atttgccttt 5700
ggctgtccaa cttggtctga agtctcatat gctcagagca ctatcgttca acctcaatca 5760
aggtactgat ttaaaatgac atctatacta ctttatcaca aacccaacga actttcatct 5820
caaaagctag gccaggaagt gaagaggttg tagagagctt ataagcactc atgacttcct 5880
tttctcgaac attcaaccaa cgtaggctga aatcccactc tgaacgaaaa taagtgtttg 5940
tttatcaaat taactctcgt agtagaacac tgaaatacct tcttctaaac gttcaacaaa 6000
tgggatttcc agcactcaaa gtgaatgaaa ggttcacatt aatcttcaaa aagaattacg 6060
acaattcatg accacaagta cattgacagc accatttcaa cagaagaaca agtcaatgct 6120
gcatcttcat caataatccg agtgtcgaac ctccttcctg acactgtcct gtatatgtaa 6180
agtttctcaa cagggcaact ttctggtctc gtatctggat gacccctctc gtctataact 6240
tcaacattaa gccctggcaa cttctggacc aacagcttac atgcttcaaa acttactgaa 6300
caattagaca tccaaaggga tcgcattgtc tccagctttg cagcattagc caacagagcc 6360
tcatcgccaa aggggcagtc tctaatctcg aatttgaaaa aattgttgtt gtatgacttt 6420
cctctgacat ccgatgcact atcaacaata gcaagactgg aggttggaga ggaatccttt 6480
attatacaat cattcaggga gaagaatgga acatggggga ggaagacact tttgagaatc 6540
tgaaatgtgt tagagccaca agctacagaa gtattgaatt tgtcatgaat atcaacattc 6600
ttcatcctag ttaattcttt ttcaattttt aatagactct cattttaatc actaatattc 6660
ttctatttgt gacttctttt ctgcaggtgg caactttaaa ttcataaagt ataggattga 6720
tgacaaactc gaaaaatatc ttaatgaggt gaagtttgag cagtcagcag atggtggttc 6780
caactctaag ttgacaagca catactatcc cggagggcga tttcaagcct gatgcatatg 6840
gttagtgtgg ctagagcaga caggatgtat tacctggata tctaccaaga cgaatccaca 6900
atcagtttta tgtcaagcaa tacatgaagt aactcccgat agaacagtaa aagcaagatg 6960
tgtaggtgta tctcgactct aagagattgt acattcctct ttgagatttt tactgctaat 7020
acaaatttac acctcagaag cgaatctaga atttctagag catgaatgca ccactaatga 7080
aaggagaaaa aaggaagtat gaagtgggaa tttgatcctt gtttctaggt atataaaatt 7140
tatcattcaa ctatacttca tttagcaaac aactctcttt gccattattt ctcaaacaag 7200
ggcttctaat attgctaaac taaagactgt caaaaggtaa gttcatcttc aaactctctt 7260
gtttacttta tctaaagggg aactatgaaa aacaagaaac atcaggaatg tcccgtaaac 7320

aaagcagcct catgcacaaa acatccaacg ttggtaggat taatggaggg atcgcatccc 7380
aggaggatac tgtagaaaaa ttagtggctt ctttcaccgc tcaaacccat gatctatagg 7440
ttacatggag acaactttat ggttgctcgt aggctcccgt caattctcat aaaccacaac 7500
accaaagttg catcagacat catcttcatt cacaagctga caatctccac aagtcttagt 7560
caacttgtaa tatgaatatt agccaggtag acgtacatat ttacaaaatt gagtttccta 7620
tataatatgg tttgaaggaa tgaaacatga tggggagggt agataaaata atatatgagg 7680
cataaaaata ggaaagatat ttgtagtgag aggttttgac tttttatgct gcttttgatc 7740
ttcagtttct tgtattcttt ttctactgct ttcctcttct ttctcctgag taaagtttta 7800
tgtaggtact ttttatacgt ccgatcgtga gaacttgaaa gaaagctctc tatagctatg 7860
ttaggtgccc acataaaaaa atgaaatatt acaaaaaccc tgataataaa atacactaat 7920
ctaagatatt cactgcaaca tacatgcaaa atatatatat ataaattttc atgaaaatta 7980
taacaaataa tagatgtgaa catataactt taaaaataat attacatcca taaagcttaa 8040
attctagatc cccggtcgac tctagaggat ccccactcca tccgttcact ttgatttgtc 8100
atgttgcact tttcgaaagt caatttgact aatttttaaa gctaaattag attacactaa 8160
ttcaatattt taaacagaaa aattagatat tcaaaaacta tacaaaaaat attatacatt 8220
gcaatttttt gcatatcaat atgataaaaa aatatatcgt aaaatattag tcaaaatttt 8280
tataatttga ctcaaatcat gaaaagtata ataattaata gtggacggag gaagtattgt 8340
ctttccagat ttgtggccat ttttggtcca agggccatta gcagttctct tcattttcta 8400
cttctgtctc atattagatg ggcatcttac taaaaatatt tgtctcatat tacttgatta 8460
tttattaaat caaaaagaat taattaattt tttctcattt taccccctaca attaatatag 8520
ttttaaaagt tttaaacaaa ttttgaagaa tcaaaatttc tttttgcaag agacttatta 8580
atataaacaa aggataaaat aataaaattt gtcaatttat tgacgatcac ttaataatca 8640
tataaaatag aaaatgttta tctaatatga gacggagaaa atatatccta aaatattttt 8700
ggacagatat gtgatattct aaccattcac tagactatat tatgcatttt agccgccaat 8760
gacttatttc agctttaatt aattaggaaa gaggaaactg ccaatgagga agagtagggg 8820
cgtagttgct gtcgacgaaa aaaagataat actcactctt ttcgattttt atttttattt 8880
atcactttta acctatcatg taaaaagata attatttttt tcatgcttta tccttagtat 8940
taaacaattt aataggggatt attttgtaaa atatttatat gaataattgt tttcgtaatg 9000
aatttgtccg gtcaaacaat gataaataaa aatgaatgaa gagagtagaa aacaaaacaa 9060
aagaacaagt tgacaacttg agagattaaa agggtccaaa acgccttgga ttttgagatt 9120
ccatatgtga aatttccatg aaataattga atttgtatta ttacaagtca aactttccat 9180
ttcattccaa ctagccatct tggtttcaaa attacacatt cattcattca cagatctaat 9240
attcttaata gtgatttcca catatggctg aagctttcat tcaagttctg ctagacaatc 9300

tcacttcttt cctcaaaggg gaacttgtat tgcttttcgg ttttcaagat gagttccaaa 9360
ggctttcaag catgttttct acaattcaag ccgtccttga agatgctcag gagaagcaac 9420
tcaacaacaa gcctctagaa aattggttgc aaaaactcaa tgctgctaca tatgaagtcg 9480
atgacatctt ggatgaatat aaaaccaagg ccacaagatt ctcccagtct gaatatggcc 9540
gttatcatcc aaaggttatc cctttccgtc acaaggtcgg gaaaaggatg gaccaagtga 9600
tgaaaaaact aaaggcaatt gctgaggaaa gaaagaattt tcatttgcac gaaaaaattg 9660
tagagagaca agctgttaga cgggaaacag gtactcatct taaattagta ttacaacaac 9720
taagtttata ttcatttttt tggcaattat caaattcaga aaagggttaa atatactcat 9780
gtcctatcgt aaatagtgta tatatacctc tcgttgtact ttcgatctga atatacttgt 9840
caaatctggc aagctcagaa tcaaattatc caccccaact tttaaatact cgatatcttt 9900
agaaatccac ctgtctaact catccactac ccattccctt tgctttgaat tcttttcttt 9960
acctataaac ttggaacact cgatccgttt tgcttttctt aacaaagcag ctcagagaaa 10020
agaggttttc ttctattctg tttctctgtg tgctgcactt gggtccttaa tcccattaaa 10080
aacagggcat gttaatccca acgacggtag cctttcctga cagctgactg taaattttgt 10140
ctaacaaaga aaaaaaaaga ttagacatgt ttttccttgt cattgattag gctggatttc 10200
tttcagagtg gaacataggg gatatattgg accaaaagta gaatgggtat atatttaaag 10260
tatttctgat agaacaggag tatattgtgc gaaaatatcc tctattttct gttgtctcct 10320
aatgagtttg aatgtaataa tattctcatg tggacattgc ttgcaccagg ttctgtatta 10380
accgaaccgc aggtttatgg aagagacaaa gagaaagatg agatagtgaa aatcctaata 10440
aacaatgtta gtgatgccca acacctttca gtcctcccaa tacttggtat gggggatta 10500
ggaaaaacga ctcttgccca aatggtcttc aatgaccaga gagttactga gcatttccat 10560
tccaaaatat ggatttgtgt ctcggaagat tttgatgaga agaggttaat aaaggcaatt 10620
gtagaatcta ttgaaggaag gccactactt ggtgagatgg acttggctcc acttcaaaag 10680
aagcttcagg agttgctgaa tggaaaaaga tacttgcttg tcttagatga tgtttggaat 10740
gaagatcaac agaagtgggc taatttaaga gcagtcttga aggttggagc aagtggtgct 10800
tctgttctaa ccactactcg tcttgaaaag gttggatcaa ttatgggaac attgcaacca 10860
tatgaactgt caaatctgtc tcaagaagat tgttggttgt tgttcatgca acgtgcattt 10920
ggacaccaag aagaaataaa tccaaacctt gtggcaatcg gaaaggagat tgtgaaaaaa 10980
agtggtggtg tgcctctagc agccaaaact cttggaggta ttttgtgctt caagagagaa 11040
gaaagagcat gggaacatgt gagagacagt ccgatttgga atttgcctca agatgaaagt 11100
tctattctgc ctgccctgag gcttagttac catcaacttc cacttgattt gaaacaatgc 11160
tttgcgtatt gtgcggtgtt cccaaaggat gccaaaatgg aaaaagaaaa gctaatctct 11220

ctctggatgg cgcatggttt tcttttatca aaaggaaaca tggagctaga ggatgtgggc 11280
gatgaagtat ggaaagaatt atacttgagg tcttttttcc aagagattga agttaaagat 11340
ggtaaaactt atttcaagat gcatgatctc atccatgatt tggcaacatc tctgttttca 11400
gcaaacacat caagcagcaa tatccgtgaa ataaataaac acagttacac acatatgatg 11460
tccattggtt tcgccgaagt ggtgtttttt tacactcttc ccccttgga aaagtttatc 11520
tcgttaagag tgcttaatct aggtgattcg acatttaata agttaccatc ttccattgga 11580
gatctagtac atttaagata cttgaacctg tatggcagtg gcatgcgtag tcttccaaag 11640
cagttatgca agcttcaaaa tctgcaaact cttgatctac aatattgcac caagctttgt 11700
tgtttgccaa aagaaacaag taaacttggt agtctccgaa atcttttact tgatggtagc 11760
cagtcattga cttgtatgcc accaaggata ggatcattga catgccttaa gactctaggt 11820
caatttgttg ttggaaggaa gaaaggttat caacttggtg aactaggaaa cctaaatctc 11880
tatggctcaa ttaaaatctc gcatcttgag agagtgaaga atgataagga cgcaaaagaa 11940
gccaatttat ctgcaaaagg gaatctgcat tctttaagca tgagttggaa taactttgga 12000
ccacatatat atgaatcaga agaagttaaa gtgcttgaag ccctcaaacc acactccaat 12060
ctgacttctt taaaaatcta tggcttcaga ggaatccatc tcccagagtg gatgaatcac 12120
tcagtattga aaaatattgt ctctattcta attagcaact tcagaaactg ctcatgctta 12180
ccaccctttg gtgatctgcc ttgtctagaa agtctagagt tacactgggg gtctgcggat 12240
gtggagtatg ttgaagaagt ggatattgat gttcattctg gattccccac aagaataagg 12300
tttccatcct tgaggaaact tgatatatgg gactttggta gtctgaaagg attgctgaaa 12360
aaggaaggag aagagcaatt ccctgtgctt gaagagatga taattcacga gtgccctttt 12420
ctgacccttt cttctaatct tagggctctt acttccctca gaatttgcta taataaagta 12480
gctacttcat tcccagaaga gatgttcaaa aaccttgcaa atctcaaata cttgacaatc 12540
tctcggtgca ataatctcaa agagctgcct accagcttgg ctagtctgaa tgctttgaaa 12600
agtctaaaaa ttcaattgtg ttgcgcacta gagagtctcc ctgaggaagg gctggaaggt 12660
ttatcttcac tcacagagtt atttgttgaa cactgtaaca tgctaaaatg tttaccagag 12720
ggattgcagc acctaacaac cctcacaagt ttaaaaattc ggggatgtcc acaactgatc 12780
aagcggtgtg agaagggaat aggagaagac tggcacaaaa tttctcacat tcctaatgtg 12840
aatatatata tttaagttat ttgctattgt ttctttgttt gtgagtcttt ttggttcctg 12900
ccattgtgat tgcatgtaat ttttttctag ggttgtttct ttatgagtct ctctctcatt 12960
ggatgtaatt ttcttttgga aacaaatctg tcaattgatt tgtattatac gctttcagaa 13020
tctattactt atttgtaatt gtttctttgt ttgtaaattg tgagtatctt attttatgga 13080
attttctgat tttattttga aaacaaatca atgatttgta agatccatct gtattatact 13140
cccttcgtct cattttatgt gtcacctgtc ggatttcgag attcaaacaa atctatcttt 13200

gatcgtaaat ttttaataga tcttttaaac attttgaatt atcaattatt gtgactttag 13260

tggctagact agtggatccg atatcgccca gcttcacgct gccgcaagca ctcagggcgc 13320

aagggctgct aaaggaagcg gaacacgtag aaagccagtc cgcagaaacg gtgctgaccc 13380

cggatgaatg tcagctactg ggctatctgg acaagggaaa acgcaagcgc aaagagaaag 13440

caggtagctt gcagtgggct tacatggcga tagctagact gggcggtttt atggacagca 13500

agcgaaccgg aattgccagc tggggcgccc tctggtaagg ttgggaagcc ctgcaaagta 13560

aactggatgg ctttcttgcc gccaaggatc tgatggcgca ggggatcaag atcatgagcg 13620

gagaattaag ggagtcacgt tatgaccccc gccgatgacg cgggacaagc cgttttacgt 13680

ttggaactga cagaaccgca acgttgaagg agccactcag ccgcgggttt ctggagttta 13740

atgagctaag cacatacgtc agaaaccatt attgcgcgtt caaaagtcgc ctaaggtcac 13800

tatcagctag caaatatttc ttgtcaaaaa tgctccactg acgttccata aattcccctc 13860

ggtatccaat tagagtctca tattcactct caatccagat ccccgggtac catggcggcg 13920

gcaacaacaa caacaacaac atcttcttcg atctccttct ccaccaaacc atctccttcc 13980

tcctccaaat caccattacc aatctccaga ttctccctcc cattctccct aaaccccaac 14040

aaatcatcct cctcctcccg ccgccgcggt atcaaatcca gctctccctc ctccatctcc 14100

gccgtgctca acacaaccac caatgtcaca accactccct ctccaaccaa acctaccaaa 14160

cccgaaacat tcatctcccg attcgctcca gatcaacccc gcaaaggcgc tgatattctc 14220

gtcgaggctt tagaacgtca aggcgtagaa accgtattcg cttaccctgg aggtgcatca 14280

atggagattc accaagcctt aacccgctct tcctcaatcc gtaacgtcct tcctcgtcac 14340

gaacaaggag gtgtattcgc agcagaagga tacgctcgat cctcaggtaa accaggtatc 14400

tgtatagcca cttcaggtcc cggagctaca aatctcgtta gcggattagc cgatgcgttg 14460

ttagatagtg ttcctcttgt agcaatcaca ggacaagtcc ctcgtcgtat gattggtaca 14520

gatgcgtttc aagagactcc gattgttgag gtaacgcgtt cgattacgaa gcataactat 14580

cttgtgatgg atgttgaaga tattcctagg attattgagg aggctttctt tttagctact 14640

tctggtagac ctggacctgt tttggttgat gttcctaaag atattcaaca acagcttgcg 14700

attcctaatt gggaacaggc tatgagatta cctggttata tgtctaggat gcctaaacct 14760

ccggaagatt ctcatttgga gcagattgtt aggttgattt ctgagtctaa gaagcctgtg 14820

ttgtatgttg gtggtggttg tttgaactct agcgatgaat tgggtaggtt tgttgagctt 14880

acgggaatcc ctgttgcgag tacgttgatg ggctgggat cttatccttg tgatgatgag 14940

ttgtcgttac atatgcttgg aatgcatggg actgtgtatg caaattacgc tgtggagcat 15000

agtgatttgt tgttggcgtt tggggtaagg tttgatgatc gtgtcacggg taaacttgag 15060

gcttttgcta gtagggctaa gattgttcat attgatattg actcggctga gattgggaag 15120

aataagactc ctcatgtgtc tgtgtgtggt gatgttaagc tggctttgca agggatgaat 15180

aaggttcttg agaaccgagc ggaggagctt aaacttgatt ttggagtttg gaggaatgag 15240

ttgaacgtac agaaacagaa gtttccgttg agctttaaga cgtttgggga agctattcct 15300

ccacagtatg cgattaaggt ccttgatgag ttgactgatg gaaaagccat aataagtact 15360

ggtgtcgggc aacatcaaat gtgggcggcg cagttctaca attacaagaa accaaggcag 15420

tggctatcat caggaggcct tggagctatg ggatttggac ttcctgctgc gattggagcg 15480

tctgttgcta accctgatgc gatagttgtg gatattgacg gagatggaag ttttataatg 15540

aatgtgcaag agctagccac tattcgtgta gagaatcttc cagtgaaggt acttttatta 15600

aacaaccagc atcttggcat ggttatgcaa tgggaagatc ggttctacaa agctaaccga 15660

gcacacacat ttctcggaga tccggctcag gaggacgaga tattcccgaa catgttgctg 15720

tttgcagcag cttgcgggat tccagcggcg agggtgacaa agaaagcaga tctccgagaa 15780

gctattcaga caatgctgga tacaccagga ccttacctgt tggatgtgat ttgtccgcac 15840

caagaacatg tgttgccgat gatcccgaat ggtggcactt tcaacgatgt cataacggaa 15900

ggagatggcc ggattaaata ctgagagctc gaatttcccc gatcgttcaa acatttggca 15960

ataaagtttc ttaagattga atcctgttgc cggtcttgcg atgattatca tataatttct 16020

gttgaattac gttaagcatg taataattaa catgtaatgc atgacgttat ttatgagatg 16080

ggtttttatg attagagtcc cgcaattata catttaatac gcgatagaaa acaaaatata 16140

gcgcgcaaac taggataaat tatcgcgcgc ggtgtcatct atgttactag atcgggaatt 16200

cactggccgt cgttttacaa cgactcagct gcttggtaat aattgtcatt agattgtttt 16260

tatgcataga tgcactcgaa atcagccaat tttagacaag tatcaaacgg atgttaattc 16320

agtacattaa agacgtccgc aatgtgttat taagttgtct aag 16363

<210> 3
<211> 13779
<212> DNA
<213> artificial

<220>
<223> synthetic construct sequence

<400> 3
caatttcaca caggaaacag ctatgaccat gattacgcca agctggcgcg ccaagcttgc 60

atgcctgcag gtcgactcta gaggatctag aatcaccgaa cctcccctcg gtacagctcc 120

tccagttcta ccatgaattt catccactga ttcctcttca atcgccattg cagattctct 180

cgatctatgc tcaaaaaatc ccgagataaa accctagatc tgcttcaaat gctctgatac 240

catgtaattt cagtgaattc taactaaaca atggagagaa ttaactattt tagaaagact 300

gattgaagga gaagaagaga gaaaaattct atattgaact catgaaccaa aatgaatgaa 360

aaaaataatg agaagaacta tactattaca atctatatat ctctatttat attctaatct 420

gaagcagtta atttaactga ctctaacaac tagactgata ggtgtacatt ttctgttagt 480
gcactgcagt gcatttaact aactgcttaa cataaagaat gttgttcgaa cttcattcga 540
atagcttcaa tgagaagcaa acatgtgtac ctgtaaagac acacagtaaa agtgttaata 600
atgaataaat atgaataaat caaataataa attaaaaata aaaacacatc caattaacat 660
tggaggtctt gaaaatcgat ggtaattaac aaagaccctt gtgaaattta agtctgtaat 720
tgaaaatttg agtataggtt aggggacatt tgactatttt ctcattttct ttatcttttt 780
cctaatttgt ggcagacaag tgaggaggcc ccactgtaat tgattcatgc ttttgctttc 840
ttgacttttt ggaacaatac tatgcatcat atttggtctt aattattcct ctgtttattt 900
ccagaatttt gagctctata catctaataa caaagcaagc agaggatata tagtttcatc 960
aactaaaaag gttagtcaac tcatctaata tttgctactc tcatctctat tgaagtacag 1020
ttatggaaaa gtagaagtga tgtaagaaaa atgaaagaac tttagtaggt tagttggatc 1080
taacaaagag aaagggaaat aaattgcagg agaaagagag aggttaaata cttactcaca 1140
ccaccgattt acaacaaatc acttaattgt ggttagttaa tgtatacttt cacctcatta 1200
aattattact tacccatgat aagttgtatt aatttggtat taatatccgg tgcgggtgaa 1260
ttcttaccgg gtgagaggga tggggttgga gagtgtggag tgaacagaag cagatgtttt 1320
agattttttc taagatgacg aaagattccc ctcactaatg aaaatatatt actatacgct 1380
attagagata gaaaggttcg gtaccagttg gtctcgtttc tggatgaacc ccatttttac 1440
aagtcatttt cttcaattca aatcgcaagt gtacctttat catcttccac taattaagtc 1500
ctcttaagtt cgcgtgaaaa tagtgaaatt attgattatt cttatcattt catcttcttt 1560
ctcctgataa agttttatgt actttttatg catcaggtct tgagaacttg gaaaggaaaa 1620
gtagaatcat ggaaaaacga aaagataatg aagaagcaaa caactcattg gtatgttatt 1680
tgatagagtg aactgtaaag tattgaattg tagatatcat gtggctttaa aaatttgata 1740
tgtgttattt tggcaggagt cattttctgc tcttcgcaag gatgctgcca atgttctgga 1800
tttcctagag agattaaaga atgaagaaga tcaaaaggct gttgatgtgg atctgattga 1860
aagcctgaaa ttgaagctga catttatttg tacatatgtc cagctttctt attccgattt 1920
ggagaagttt gaagatataa tgactagaaa aagacaagag gttgagaatc tgcttcaacc 1980
aattttggat gatgatggca aagacgtcgg gtgtaaatat gtccttacta gcctcgccgg 2040
taatatggat gactgtataa gcttgtatca tcgttctaaa tcagatgcca ccatgatgga 2100
tgagcaattg ggcttcctcc tcttgaatct ctctcatcta tccaagcatc gtgctgaaaa 2160
gatgtttcct ggagtgactc aatatgaggt tcttcagaat gtatgtggca acataagaga 2220
tttccatgga ttgatagtga attgttgcat taagcatgag atggttgaga atgtcttatc 2280
tctgtttcaa ctgatggctg agagagtagg acgcttcctt tgggaggatc aggctgatga 2340

agactctcaa ctctccgagc tagatgagga tgatcagaat gataaagacc ctcaactctt 2400
caagctagca catctactct tgaagattgt tccaactgaa ttggaggtta tgcacatatg 2460
ttataaaact ttgaaagctt caacttcaac agaaattgga cgcttcatta agaagctcct 2520
ggaaacctct ccggacattc tcagagaata tctgattcat ctacaagagc atatgataac 2580
tgttattacc cctaacactt caggggctcg aaacattcat gtcatgatgg aattcctatt 2640
gattattctt tctgatatgc cgcccaagga ctttattcat catgacaaac tttttgatct 2700
cttggctcgt gttgtagcac ttaccaggga ggtatcaact cttgtacgcg acttggaaga 2760
gaaattaagg attaaagaga gtactgacga aacaaattgt gcaaccctaa agtttctgga 2820
aaatattgaa ctccttaagg aagatctcaa acatgtttat ctgaaagtcc cggattcatc 2880
tcaatattgc ttccccatga gtgatggacc tctcttcatg catctgctac agagacactt 2940
agatgatttg ctggattcca atgcttattc aattgctttg ataaaggaac aaattgggct 3000
ggtgaaagaa gacttggaat tcataagatc tttttcgcg aatattgagc aaggattgta 3060
taaagatctc tgggaacgtg ttctagatgt ggcatatgag gcaaaagatg tcatagattc 3120
aattattgtt cgagataatg gtctcttaca tcttattttc tcacttccca ttaccagaaa 3180
gaagatgatg cttatcaaag aagaggtctc tgatttacat gagaacattt ccaagaacag 3240
aggtctcatc gttgtgaact ctcccaagaa accagttgag agcaagtcat tgacaactga 3300
taaaataatt gtaggttttg gtgaggagac aaacttgata cttagaaagc tcaccagtgg 3360
accggcagat ctagatgtca tttcgatcat tggtatgccg ggtttaggta aaactacttt 3420
ggcgtacaaa gtatacaatg ataaatcagt ttctagccat ttcgaccttc gtgcatggtg 3480
cacggtcgac caagtatatg acgagaagaa gttgttggat aaaattttca atcaagttag 3540
tgactcaaat tcaaaattga gtgagaatat tgatgttgct gataaactac ggaaacaatt 3600
gtttggaaag aggtatctta ttgtcttaga tgacgtgtgg gatactaata catgggatga 3660
gctaacaaga ccttttcctg atggtatgaa aggaagtaga attattttga caactcgaga 3720
aaagaaagtt gctttgcatg gaaagctcta cactgatcct cttaaccttc gattgctaag 3780
atcagaagaa agttgggagt tattagagaa aagggcattt ggaaacgaga gttgccctga 3840
tgaactattg gatgttggta aagaaatagc cgaaaattgt aaagggcttc ctttggtggt 3900
ggatctgatt gctggaatca ttgctgggag ggaaaagaaa aagagtgtgt ggcttgaagt 3960
tgtaaataat ttgcattcct ttattttgaa gaatgaagtg gaagtgatga aagttataga 4020
aataagttat gaccacttac ctgatcacct gaagccatgc ttgctgtact ttgcaagtgc 4080
gccgaaggac tgggtaacga caatccatga gttgaaactt atttggggtt ttgaaggatt 4140
tgtggaaaag acagatatga agagtctgga agaagtggtg aaaatttatt tggatgattt 4200
aatttccagt agcttggtaa tttgtttcaa tgagataggt gattacccta cttgccaact 4260
tcatgatctt gtgcatgact tttgtttgat aaaagcaaga aaggaaaagt tgtgtgatcg 4320

gataagttca agtgctccat cagatttgtt gccacgtcaa attagcattg attatgatga 4380
tgatgaagag cactttgggc ttaattttgt cctgttcggt tcaaataaga aaaggcattc 4440
cggtaaacac ctctattctt tgaccataaa tggagatgag ctggacgacc atctttctga 4500
tacatttcat ctaagacact tgaggcttct tagaaccttg cacctggaat cctcttttat 4560
catggttaaa gattctttgc tgaatgaaat atgcatgttg aatcatttga ggtacttaag 4620
cattgggaca gaagttaaat ctctgccttt gtctttctca aacctctgga atctagaaat 4680
cttgtttgtg gataacaaag aatcaacctt gatactatta ccgagaattt gggatcttgt 4740
aaagttgcaa gtgctgttca cgactgcttg ttctttcttt gatatggatg cagatgaatc 4800
aatactgata gcagaggaca caaagttaga gaacttgaca gcattagggg aactcgtgct 4860
ttcctattgg aaagatacag aggatatttt caaaaggctt cccaatcttc aagtgcttca 4920
tttcaaactc aaggagtcat gggattattc aacagagcaa tattggttcc cgaaattgga 4980
tttcctaact gaactagaaa aactcactgt agattttgaa agatcaaaca caaatgacag 5040
tgggtcctct gcagccataa atcggccatg ggattttcac tttccttcga gtttgaaaag 5100
attgcaattg catgaatttc ctctgacatc cgattcacta tcaacaatag cgagactgct 5160
gaaccttgaa gagttgtacc tttatcgtac aatcatccat ggggaagaat ggaacatggg 5220
agaagaagac acctttgaga atctcaaatg tttgatgttg agtcaagtga ttctttccaa 5280
gtgggaggtt ggagaggaat cttttcccac gcttgagaaa ttagaactgt cggactgtca 5340
taatcttgag gagattccgt ctagttttgg ggatatttat tccttgaaaa ttatcgaact 5400
tgtaaggagc cctcaacttg aaaattccgc tctcaagatt aaggaatatg ctgaagatat 5460
gaggggaggg gacgagcttc agatccttgg ccagaaggat atcccgttat ttaagtagtt 5520
tttgagcatt atggttgaaa agtagattgc actttgctgg gtagattgta tatggttaag 5580
aaaattctgt tacagttgtt atgaaacatt tttatttgac ttttctgagt ttcttttaga 5640
aaactcagaa gtttttaaca aaaattatag tttttataaa tacaatgtgg atttgccttt 5700
ggctgtccaa cttggtctga agtctcatat gctcagagca ctatcgttca acctcaatca 5760
aggtactgat ttaaaatgac atctatacta ctttatcaca aacccaacga actttcatct 5820
caaaagctag gccaggaagt gaagaggttg tagagagctt ataagcactc atgacttcct 5880
tttctcgaac attcaaccaa cgtaggctga aatcccactc tgaacgaaaa taagtgtttg 5940
tttatcaaat taactctcgt agtagaacac tgaaatacct tcttctaaac gttcaacaaa 6000
tgggatttcc agcactcaaa gtgaatgaaa ggttcacatt aatcttcaaa aagaattacg 6060
acaattcatg accacaagta cattgacagc accatttcaa cagaagaaca agtcaatgct 6120
gcatcttcat caataatccg agtgtcgaac ctccttcctg acactgtcct gtatatgtaa 6180
agtttctcaa cagggcaact ttctggtctc gtatctggat gacccctctc gtctataact 6240

tcaacattaa gccctggcaa cttctggacc aacagcttac atgcttcaaa acttactgaa 6300
caattagaca tccaaaggga tcgcattgtc tccagctttg cagcattagc caacagagcc 6360
tcatcgccaa aggggcagtc tctaatctcg aatttgaaaa aattgttgtt gtatgacttt 6420
cctctgacat ccgatgcact atcaacaata gcaagactgg aggttggaga ggaatccttt 6480
attatacaat cattcaggga gaagaatgga acatgggggga ggaagacact tttgagaatc 6540
tgaaatgtgt tagagccaca agctacagaa gtattgaatt tgtcatgaat atcaacattc 6600
ttcatcctag ttaattcttt ttcaattttt aatagactct cattttaatc actaatattc 6660
ttctatttgt gacttctttt ctgcaggtgg caactttaaa ttcataaagt ataggattga 6720
tgacaaactc gaaaaatatc ttaatgaggt gaagtttgag cagtcagcag atggtggttc 6780
caactctaag ttgacaagca catactatcc cggagggcga tttcaagcct gatgcatatg 6840
gttagtgtgg ctagagcaga caggatgtat tacctggata tctaccaaga cgaatccaca 6900
atcagtttta tgtcaagcaa tacatgaagt aactcccgat agaacagtaa aagcaagatg 6960
tgtaggtgta tctcgactct aagagattgt acattcctct ttgagatttt tactgctaat 7020
acaaatttac acctcagaag cgaatctaga atttctagag catgaatgca ccactaatga 7080
aaggagaaaa aaggaagtat gaagtgggaa tttgatcctt gtttctaggt atataaaatt 7140
tatcattcaa ctatacttca tttagcaaac aactctcttt gccattattt ctcaaacaag 7200
ggcttctaat attgctaaac taaagactgt caaaaggtaa gttcatcttc aaactctctt 7260
gtttacttta tctaaagggg aactatgaaa aacaagaaac atcaggaatg tcccgtaaac 7320
aaagcagcct catgcacaaa acatccaacg ttggtaggat taatggaggg atcgcatccc 7380
aggaggatac tgtagaaaaa ttagtggctt ctttcaccgc tcaaacccat gatctatagg 7440
ttacatggag acaactttat ggttgctcgt aggctcccgt caattctcat aaaccacaac 7500
accaaagttg catcagacat catcttcatt cacaagctga caatctccac aagtcttagt 7560
caacttgtaa tatgaatatt agccaggtag acgtacatat ttacaaaatt gagtttccta 7620
tataatatgg tttgaaggaa tgaaacatga tggggagggt agataaaata atatatgagg 7680
cataaaaata ggaaagatat ttgtagtgag aggttttgac tttttatgct gcttttgatc 7740
ttcagtttct tgtattcttt ttctactgct ttcctcttct ttctcctgag taaagtttta 7800
tgtaggtact ttttatacgt ccgatcgtga gaacttgaaa gaaagctctc tatagctatg 7860
ttaggtgccc acataaaaaa atgaaatatt acaaaaaccc tgataataaa atacactaat 7920
ctaagatatt cactgcaaca tacatgcaaa atatatatat ataaattttc atgaaaatta 7980
taacaaataa tagatgtgaa catataactt taaaaataat attacatcca taaagcttaa 8040
attctagatc cccggtcgac tctagaggat ccccactcca tccgttcact ttgatttgtc 8100
atgttgcact tttcgaaagt caatttgact aatttttaaa gctaaattag attacactaa 8160
ttcaatattt taaacagaaa aattagatat tcaaaaacta tacaaaaaat attatacatt 8220

gcaatttttt gcatatcaat atgataaaaa aatatatcgt aaaatattag tcaaaatttt 8280
tataatttga ctcaaatcat gaaaagtata ataattaata gtggacggag gaagtattgt 8340
ctttccagat ttgtggccat ttttggtcca agggccatta gcagttctct tcattttcta 8400
cttctgtctc atattagatg ggcatcttac taaaaatatt tgtctcatat tacttgatta 8460
tttattaaat caaaaagaat taattaattt tttctcattt taccccctaca attaatatag 8520
ttttaaaagt tttaaacaaa ttttgaagaa tcaaaatttc tttttgcaag agacttatta 8580
atataaacaa aggataaaat aataaaattt gtcaatttat tgacgatcac ttaataatca 8640
tataaaatag aaaatgttta tctaatatga gacggagaaa atatatccta aaatattttt 8700
ggacagatat gtgatattct aaccattcac tagactatat tatgcatttt agccgccaat 8760
gacttatttc agctttaatt aattaggaaa gaggaaactg ccaatgagga agagtagggg 8820
cgtagttgct gtcgacgaaa aaaagataat actcactctt ttcgattttt atttttattt 8880
atcactttta acctatcatg taaaaagata attatttttt tcatgcttta tccttagtat 8940
taaacaattt aatagggatt attttgtaaa atatttatat gaataattgt tttcgtaatg 9000
aatttgtccg gtcaaacaat gataaataaa aatgaatgaa gagagtagaa aacaaaacaa 9060
aagaacaagt tgacaacttg agagattaaa agggtccaaa acgccttgga ttttgagatt 9120
ccatatgtga aatttccatg aaataattga atttgtatta ttacaagtca aactttccat 9180
ttcattccaa ctagccatct tggtttcaaa attacacatt cattcattca cagatctaat 9240
attcttaata gtgatttcca catatggctg aagctttcat tcaagttctg ctagacaatc 9300
tcacttcttt cctcaaaggg gaacttgtat tgcttttcgg ttttcaagat gagttccaaa 9360
ggctttcaag catgttttct acaattcaag ccgtccttga agatgctcag gagaagcaac 9420
tcaacaacaa gcctctagaa aattggttgc aaaaactcaa tgctgctaca tatgaagtcg 9480
atgacatctt ggatgaatat aaaaccaagg ccacaagatt ctcccagtct gaatatggcc 9540
gttatcatcc aaaggttatc cctttccgtc acaaggtcgg gaaaaggatg gaccaagtga 9600
tgaaaaaact aaaggcaatt gctgaggaaa gaaagaattt tcatttgcac gaaaaaattg 9660
tagagagaca agctgttaga cgggaaacag gtactcatct taaattagta ttacaacaac 9720
taagtttata ttcatttttt tggcaattat caaattcaga aaagggttaa atatactcat 9780
gtcctatcgt aaatagtgta tatatacctc tcgttgtact ttcgatctga atatacttgt 9840
caaatctggc aagctcagaa tcaaattatc caccccaact tttaaatact cgatatcttt 9900
agaaatccac ctgtctaact catccactac ccattccctt tgctttgaat tcttttcttt 9960
acctataaac ttggaacact cgatccgttt tgcttttctt aacaaagcag ctcagagaaa 10020
agaggttttc ttctattctg tttctctgtg tgctgcactt gggtccttaa tcccattaaa 10080
aacagggcat gttaatccca acgacggtag cctttcctga cagctgactg taaattttgt 10140

ctaacaaaga aaaaaaaaga ttagacatgt ttttccttgt cattgattag gctggatttc 10200
tttcagagtg gaacataggg gatatattgg accaaaagta gaatgggtat atatttaaag 10260
tatttctgat agaacaggag tatattgtgc gaaaatatcc tctattttct gttgtctcct 10320
aatgagtttg aatgtaataa tattctcatg tggacattgc ttgcaccagg ttctgtatta 10380
accgaaccgc aggtttatgg aagagacaaa gagaaagatg agatagtgaa aatcctaata 10440
aacaatgtta gtgatgccca acacctttca gtcctcccaa tacttggtat gggggggatta 10500
ggaaaaacga ctcttgccca aatggtcttc aatgaccaga gagttactga gcatttccat 10560
tccaaaatat ggatttgtgt ctcggaagat tttgatgaga agaggttaat aaaggcaatt 10620
gtagaatcta ttgaaggaag gccactactt ggtgagatgg acttggctcc acttcaaaag 10680
aagcttcagg agttgctgaa tggaaaaaga tacttgcttg tcttagatga tgtttggaat 10740
gaagatcaac agaagtgggc taatttaaga gcagtcttga aggttggagc aagtggtgct 10800
tctgttctaa ccactactcg tcttgaaaag gttggatcaa ttatgggaac attgcaacca 10860
tatgaactgt caaatctgtc tcaagaagat tgttggttgt tgttcatgca acgtgcattt 10920
ggacaccaag aagaaataaa tccaaacctt gtggcaatcg gaaaggagat tgtgaaaaaa 10980
agtggtggtg tgcctctagc agccaaaact cttggaggta ttttgtgctt caagagagaa 11040
gaaagagcat gggaacatgt gagagacagt ccgatttgga atttgcctca agatgaaagt 11100
tctattctgc ctgccctgag gcttagttac catcaacttc cacttgattt gaaacaatgc 11160
tttgcgtatt gtgcggtgtt cccaaaggat gccaaaatgg aaaaagaaaa gctaatctct 11220
ctctggatgg cgcatggttt tcttttatca aaaggaaaca tggagctaga ggatgtgggc 11280
gatgaagtat ggaaagaatt atacttgagg tctttttttcc aagagattga agttaaagat 11340
ggtaaaactt atttcaagat gcatgatctc atccatgatt tggcaacatc tctgttttca 11400
gcaaacacat caagcagcaa tatccgtgaa ataaataaac acagttacac acatatgatg 11460
tccattggtt tcgccgaagt ggtgtttttt tacactcttc ccccccttgga aaagtttatc 11520
tcgttaagag tgcttaatct aggtgattcg acatttaata agttaccatc ttccattgga 11580
gatctagtac atttaagata cttgaacctg tatggcagtg gcatgcgtag tcttccaaag 11640
cagttatgca agcttcaaaa tctgcaaact cttgatctac aatattgcac caagctttgt 11700
tgtttgccaa aagaaacaag taaacttggt agtctccgaa atcttttact tgatggtagc 11760
cagtcattga cttgtatgcc accaaggata ggatcattga catgccttaa gactctaggt 11820
caatttgttg ttggaaggaa gaaaggttat caacttggtg aactaggaaa cctaaatctc 11880
tatggctcaa ttaaaatctc gcatcttgag agagtgaaga atgataagga cgcaaaagaa 11940
gccaatttat ctgcaaaagg gaatctgcat tctttaagca tgagttggaa taactttgga 12000
ccacatatat atgaatcaga agaagttaaa gtgcttgaag ccctcaaacc acactccaat 12060
ctgacttctt taaaaatcta tggcttcaga ggaatccatc tcccagagtg gatgaatcac 12120

tcagtattga aaaatattgt ctctattcta attagcaact tcagaaactg ctcatgctta 12180

ccaccctttg gtgatctgcc ttgtctagaa agtctagagt tacactgggg gtctgcggat 12240

gtggagtatg ttgaagaagt ggatattgat gttcattctg gattccccac aagaataagg 12300

tttccatcct tgaggaaact tgatatatgg gactttggta gtctgaaagg attgctgaaa 12360

aaggaaggag aagagcaatt ccctgtgctt gaagagatga taattcacga gtgccctttt 12420

ctgacccttt cttctaatct tagggctctt acttccctca gaatttgcta taataaagta 12480

gctacttcat tcccagaaga gatgttcaaa aaccttgcaa atctcaaata cttgacaatc 12540

tctcggtgca ataatctcaa agagctgcct accagcttgg ctagtctgaa tgctttgaaa 12600

agtctaaaaa ttcaattgtg ttgcgcacta gagagtctcc ctgaggaagg gctggaaggt 12660

ttatcttcac tcacagagtt atttgttgaa cactgtaaca tgctaaaatg tttaccagag 12720

ggattgcagc acctaacaac cctcacaagt ttaaaaattc ggggatgtcc acaactgatc 12780

aagcggtgtg agaagggaat aggagaagac tggcacaaaa tttctcacat tcctaatgtg 12840

aatatatata tttaagttat ttgctattgt ttctttgttt gtgagtcttt ttggttcctg 12900

ccattgtgat tgcatgtaat ttttttctag ggttgtttct ttatgagtct ctctctcatt 12960

ggatgtaatt ttcttttgga aacaaatctg tcaattgatt tgtattatac gctttcagaa 13020

tctattactt atttgtaatt gtttctttgt ttgtaaattg tgagtatctt attttatgga 13080

attttctgat tttattttga aaacaaatca atgatttgta agatccatct gtattatact 13140

cccttcgtct cattttatgt gtcacctgtc ggatttcgag attcaaacaa atctatcttt 13200

gatcgtaaat ttttaataga tcttttaaac attttgaatt atcaattatt gtgactttag 13260

tggctagact agtggatccg atatcgccca gcttcacgct gccgcaagca ctcagggcgc 13320

aagggctgct aaaggaagcg gaacacgtag aaagccagtc cgcagaaacg gtgctgaccc 13380

cggatgaatg tcagctactg ggctatctgg acaagggaaa acgcaagcgc aaagagaaag 13440

caggtagctt gcagtgggct tacatggcga tagctagact gggcggtttt atggacagca 13500

agcgaaccgg aattgccagc tggggcgccc tctggtaagg ttgggaagcc ctgcaaagta 13560

aactggatgg ctttcttgcc gccaaggatc tgatggcgca ggggatcaag ggaattcact 13620

ggccgtcgtt ttacaacgac tcagctgctt ggtaataatt gtcattagat tgtttttatg 13680

catagatgca ctcgaaatca gccaatttta gacaagtatc aaacggatgt taattcagta 13740

cattaaagac gtccgcaatg tgttattaag ttgtctaag 13779

<210> 4
<211> 26
<212> DNA
<213> artificial

<220>
<223> Primer

<400> 4
tcaaacggat gttaattcag tacatt 26

<210> 5
<211> 25
<212> DNA
<213> artificial

<220>
<223> Primer

<400> 5
ccagttccca attgactact agaaa 25

<210> 6
<211> 20
<212> DNA
<213> artificial

<220>
<223> Primer

<400> 6
tctgttgaat tacgttaagc 20

<210> 7
<211> 20
<212> DNA
<213> artificial

<220>
<223> Primer

<400> 7
ctcagaagaa agaattgttc 20

<210> 8
<211> 25
<212> DNA
<213> artificial

<220>
<223> Primer

<400> 8
gtttcttaag attgaatcct gttgc 25

<210> 9
<211> 25
<212> DNA
<213> artificial

<220>
<223> Primer

<400> 9
gcccattctc tattttactc actaa 25

<210> 10
<211> 21
<212> DNA
<213> artificial

<220>
<223> Primer

<400> 10
gtgaactagg aaacctaaat c 21

<210> 11
<211> 20
<212> DNA
<213> artificial

<220>
<223> Primer

<400> 11
caactaataa aaccaaggac 20

<210> 12
<211> 27
<212> DNA
<213> artificial

<220>
<223> Primer

<400> 12
ccaagatagt gtttcaggaa agttatt 27

<210> 13
<211> 24
<212> DNA
<213> artificial

<220>
<223> Primer

<400> 13
aaattcatgg tagaactgga ggag 24

<210> 14
<211> 20
<212> DNA
<213> artificial

<220>
<223> Primer

<400> 14
aactgaattt tgggattgag 20

<210> 15
<211> 25
<212> DNA
<213> artificial

<220>
<223> Primer

<400> 15
gagtcagtta aattaactgc ttcag 25

<210> 16
<211> 22
<212> DNA
<213> artificial

<220>
<223> Primer

<400> 16
acaagaatag caaggattat cc 22

<210> 17
<211> 20
<212> DNA
<213> artificial

<220>
<223> Primer

<400> 17
gaagttcgaa caacattctt 20

<210> 18
<211> 20
<212> DNA
<213> artificial

<220>
<223> Primer

<400> 18
aactgaattt tgggattgag 20

<210> 19
<211> 20
<212> DNA
<213> artificial

<220>
<223> Primer

<400> 19
atgtagcagc attgagtttt 20

<210> 20
<211> 985
<212> DNA
<213> Solanum tuberosum

<400> 20
gaaactcagt tggagattac gtttaaggga tggcctacat ggataaggtt atctacaatg 60

atgcaatgga gataataata gtactcctat gtgatttctt cgttttaatt tgtttatttt 120

attttcattt aatttgattt gatacgtagt ttaaaaaaat aaaaaagata tttaaatttt 180

gtgatgttag atgaaacata cgttaaatgc atcaaaatgt catttaaatt tgtcttgaac 240

atatagtatt agaaagttga aattaaagag ctatcaaaaa agaagaagat atgactaaaa 300

aaaatagcac aagcaaattg aaactaaaag agtaattaac aataacaatt ttttttaaa 360

atgatacata atactcactc tttctcaata tgtggcacaa aaatatcaag aatcaaacta 420

tttaattttg actataaatt tagaaataaa ttttttaaat ttcttaaaaa acaaaattta 480

caaagcaaat actatataaa aaatattata aattataata attaataatt caaaatagtt 540

aaccatacaa actgaatttt gggattgagt ttctatttaa attttcgtga gtaaagatta 600

aagttcaaat taacttttta taaaatgagt tttcaatttt aaaaagtcaa acaaaatata 660

tatagaatat aaagtgagaa accaaataaa acaagaatag caaggattat ccatttagga 720

aataagatga aagaatcaaa tcaactaata aaaactttaa tcctataaga taagcttgcc 780

aataatttaa aatagcatga tgcgtctaag agggtatcaa tatattgtta tcctttaaag 840

accaagatag tgtttcagga aagttatttc atttccaaat ttaaataaaa gaaaataatt 900

attcaaaatt cttatgtctt ttttattgat ttatagtcga agatctttca aaaataattt 960

ttctataggt aaaaaaggat aattt 985

<210> 21
<211> 1375
<212> DNA
<213> Solanum tuberosum

<400> 21
ttgtccttgg ttttattagt tgttattgtt gttttgtctt ttcaatttct agtagtcaat 60

tgggaactgg aagtacaaag ttgaattttt agacttatgt atacaaattg aaaatgtatc 120

aaaaagtagt gacacttata aaaagaacaa ttctttcttc tgagaacatc ttcaacccac 180

ctctatttta ctctccattc tctatattta gtgagtaaaa tagagaatgg gcactccaac 240

ccacctccat atcactctcc attcttcata tttagagagt catattattt ttattattat 300

ttttattact ttctaattaa tatattattt tacatataat gtcattaatt aaatatctaa 360

tattcataat tctttttaaa tgtcatatta tattttaaaa aatatattat ttaatatata 420

ctactttcat cccgaattaa tagtatttta attttttcta attttcgaca ataatataat 480

ttgattttat tattaatttt cactataaat aatacacaaa attaaaatga taagatgaaa 540

aaattaattt aaaaatacaa tacataatat gataatttaa atacaggata acaatacaat 600

acatgacata ataatttaaa tacaagataa aatacaatac ataacataat aatcaattcg 660

tgatgaaaca agaatcatgc caaaaagata ttgaacgtgc attcgaagtt ttgcaatcac 720

gttttgcaat tattgcaaga ccgtcacgtt tttggagaaa ggaagtgtga catgatataa 780

tgactacatg tattatactg cacaccatga taattgagga tgaacatgat cttaatgcac 840

caaatcaaga tgccgtagag gctccaactc caacgacaaa aatgatggta gatgaaaatc 900

ttcggtttga acaattttta gctagacata aaaaagttaa ggacaaaaat gctcattttg 960

aactccgtaa tgcattaata gagcatttat gacagcaacg tgataatttc gaaacttgag 1020

tgtttatgta attatatttc acttttattt gaatttgtcc attaatttgt atattatata 1080

atatttattt acaatttatg ttatttaaaa atttagaata aaataaaatt ttgaaaaaat 1140

aaaattttat atcacatgaa aattatataa agtaattatt ggggaaaaaa ataaaggatt 1200

tatattatga aataagaaaa taagaatgaa tagaaataat aataatataa tattgaggag 1260

aaataattat tttttttaga gagtgaaata gagaattggg ttgaaattga ttgtctgaaa 1320

aaataaaaaa ctctatattt ggagagtaaa atagagtgta gggttggaga cgtca 1375

<210> 22
<211> 1773
<212> DNA
<213> artificial

<220>
<223> synthetic construct sequence

<400> 22
gtttcttaag attgaatcct gttgccggtc ttgcgatgat tatcatataa tttctgttga 60

attacgttaa gcatgtaata attaacatgt aatgcatgac gttatttatg agatgggttt 120

ttatgattag agtcccgcaa ttatacattt aatacgcgat agaaaacaaa atatagcgcg 180

caaactagga taaattatcg cgcgcggtgt catctatgtt actagatcgg gaattcactg 240

gccgtcgttt tacaacgact cagctgcttg gtaataattg tcattagatt gtttttatgc 300

atagatgcac tcgaaatcag ccaattttag acaagtatca aacggatgtt aattcagtac 360

attaaagacg tccgcaatgt gttattaagt tgtctaagtt gtccttggtt ttattagttg 420

ttattgttgt tttgtctttt caatttctag tagtcaattg ggaactggaa gtacaaagtt 480

gaatttttag acttatgtat acaaattgaa aatgtatcaa aaagtagtga cacttataaa 540

aagaacaatt ctttcttctg agaacatctt caacccacct ctattttact ctccattctc 600

tatatttagt gagtaaaata gagaatgggc actccaaccc acctccatat cactctccat 660

tcttcatatt tagagagtca tattattttt attattattt ttattacttt ctaattaata 720

tattatttta catataatgt cattaattaa atatctaata ttcataattc tttttaaatg 780

tcatattata ttttaaaaaa tatattattt aatatatact actttcatcc cgaattaata 840

gtattttaat tttttctaat tttcgacaat aatataattt gattttatta ttaattttca 900

ctataaataa tacacaaaat taaaatgata agatgaaaaa attaatttaa aaatacaata 960

cataatatga taatttaaat acaggataac aatacaatac atgacataat aatttaaata 1020

caagataaaa tacaatacat aacataataa tcaattcgtg atgaaacaag aatcatgcca 1080

aaaagatatt gaacgtgcat tcgaagtttt gcaatcacgt tttgcaatta ttgcaagacc 1140

gtcacgtttt tggagaaagg aagtgtgaca tgatataatg actacatgta ttatactgca 1200

caccatgata attgaggatg aacatgatct taatgcacca aatcaagatg ccgtagaggc 1260

tccaactcca acgacaaaaa tgatggtaga tgaaaatctt cggtttgaac aatttttagc 1320

tagacataaa aaagttaagg acaaaaatgc tcattttgaa ctccgtaatg cattaataga 1380

gcatttatga cagcaacgtg ataatttcga aacttgagtg tttatgtaat tatatttcac 1440

ttttatttga atttgtccat taatttgtat attatataat atttatttac aatttatgtt 1500

atttaaaaat ttagaataaa ataaaatttt gaaaaaataa aattttatat cacatgaaaa 1560

ttatataaag taattattgg ggaaaaaaat aaaggattta tattatgaaa taagaaaata 1620

agaatgaata gaaataataa taatataata ttgaggagaa ataattattt tttttagaga 1680

gtgaaataga gaattgggtt gaaattgatt gtctgaaaaa ataaaaaact ctatatttgg 1740

agagtaaaat agagtgtagg gttggagacg tca 1773

<210> 23
<211> 1519
<212> DNA
<213> artificial

<220>
<223> synthetic construct sequence

<400> 23
gaaactcagt tggagattac gtttaaggga tggcctacat ggataaggtt atctacaatg 60

atgcaatgga gataataata gtactcctat gtgatttctt cgttttaatt tgtttatttt 120

attttcattt aatttgattt gatacgtagt ttaaaaaaat aaaaaagata tttaaatttt 180

gtgatgttag atgaaacata cgttaaatgc atcaaaatgt catttaaatt tgtcttgaac 240

atatagtatt agaaagttga aattaaagag ctatcaaaaa agaagaagat atgactaaaa 300

aaaatagcac aagcaaattg aaactaaaag agtaattaac aataacaatt ttttttaaa 360

atgatacata atactcactc tttctcaata tgtggcacaa aaatatcaag aatcaaacta 420

tttaattttg actataaatt tagaaataaa ttttttaaat ttcttaaaaa acaaaattta 480

caaagcaaat actatataaa aaatattata aattataata attaataatt caaaatagtt 540

aaccatacaa actgaatttt gggattgagt ttctatttaa attttcgtga gtaaagatta 600

aagttcaaat taacttttta taaaatgagt tttcaatttt aaaaagtcaa acaaaatata 660

tatagaatat aaagtgagaa accaaataaa acaagaatag caaggattat ccatttagga 720

aataagatga aagaatcaaa tcaactaata aaaactttaa tcctataaga taagcttgcc 780

aataatttaa aatagcatga tgcgtctaag agggtatcaa tatattgtta tcctttaaag 840

accaagatag tgtttcagga aagttatttc atttccaaat ttaaataaaa gaaaataatt 900

attcaaaatt cttatgtctt ttttattgat ttatagtcga agatctttca aaaataattt 960

ttctataggt aaaaaaggat aatttcaatt tcacacagga aacagctatg accatgatta 1020

cgccaagctg gcgcgccaag cttgcatgcc tgcaggtcga ctctagagga tctagaatca 1080

ccgaacctcc cctcggtaca gctcctccag ttctaccatg aatttcatcc actgattcct 1140

cttcaatcgc cattgcagat tctctcgatc tatgctcaaa aaatcccgag ataaaaccct 1200

agatctgctt caaatgctct gataccatgt aatttcagtg aattctaact aaacaatgga 1260

gagaattaac tattttagaa agactgattg aaggagaaga agagagaaaa attctatatt 1320

gaactcatga accaaaatga atgaaaaaaa taatgagaag aactatacta ttacaatcta 1380

tatatctcta tttatattct aatctgaagc agttaattta actgactcta acaactagac 1440

tgataggtgt acattttctg ttagtgcact gcagtgcatt taactaactg cttaacataa 1500

agaatgttgt tcgaacttc 1519

<210> 24
<211> 957
<212> DNA
<213> artificial

<220>
<223> synthetic construct sequence

<400> 24
agcatgtaat aattaacatg taatgcatga cgttatttat gagatgggtt tttatgatta 60

gagtcccgca attatacatt taatacgcga tagaaaacaa aatatagcgc gcaaactagg 120

ataaattatc gcgcgcggtg tcatctatgt tactagatcg ggaattcact ggccgtcgtt 180

ttacaacgac tcagctgctt ggtaataatt gtcattagat tgtttttatg catagatgca 240

ctcgaaatca gccaatttta gacaagtatc aaacggatgt taattcagta cattaaagac 300

gtccgcaatg tgaactcatt tgatcaagaa atgactatgg gacattaaag taaaaatcat 360

aaagacattt ttttttcata ttccataata aaagaataat aacagaatat tagtggaaaa 420

aaatgaaaaa tttctaaatg taggggtggg ggtggggaag gagatacatt aggtttctaa 480

aaataaataa aaaacgaact aaaccaagat tgatcaaatc gaaaaaatgc gttaattggt 540

ttgattttaa taattttaaa atgaattaaa ttgatttgat tttagtggca aaaagttgat 600

ctaaattgat ctctgagtac ctttagcccc actaattcct tttaagagga taaacgtttt 660

caattgtaaa agacaattag gactaaatag ctgaaagaaa ggatagtttt aacccaataa 720

tcaatgttaa gacaatttaa atttaatata aagaagaaaa attaaaacta ttttcaatat 780

gttagggaca agggaataaa tcttaaaaca atcgcaatat caaaattggg cgctaataat 840

aataacttaa ttaattaact attctagtaa agagtcgata tatttctaat taatcctctc 900

agctttcctc gataagaagt agtaatggat ataggattgt aaggtcatgc gagctct 957

<210> 25
<211> 25

<212> DNA
<213> artificial

<220>
<223> Primer

<400> 25
taattcagta cattaaagac gtccg 25

<210> 26
<211> 23
<212> DNA
<213> artificial

<220>
<223> Primer

<400> 26
gtcccatagt catttcttga tca 23

<210> 27
<211> 599
<212> DNA
<213> artificial

<220>
<223> synthetic construct sequence

<400> 27
gattttattt tattaataaa aattgaaaaa aattaaatca aactaaaaca ataaattatt 60

catataattt ttataattat atatatacac acataatata ttaattttac aattatttat 120

aatcatttat atactttttc accattatca tatttgtctc tgataggcta ataaactatg 180

tatttaatgt tgctgtacat tttctgatag tttaaactga aggcgggaaa cgacaatcag 240

atctagtagg aaacagctat gaccatgatt acgccaagct tgcatgccct ggcacgacag 300

gtttcccgac tggaaagcgg gcagtgagcg caacgcaatt aatgtgagtt agctcactca 360

ttaggcaccc caggctttac actttatgct tccggctcgt atgttgtgtg gaattgtgag 420

cggataacaa tttcacacag gaaacagcta tgaccatgat tacgccaagc tggcgcgcca 480

agcttgcatg cctgcaggtc gactctagag gatctagaat caccgaacct cccctcggta 540

cagctcctcc agttctacca tgaatttcat ccactgattc ctcttcaatc gccattgca 599

<210> 28
<211> 26
<212> DNA
<213> artificial

<220>
<223> Primer

<400> 28
tgtctctgat aggctaataa actatg 26

<210> 29

<211> 21
<212> DNA
<213> artificial

<220>
<223> Primer

<400> 29
tagatctgat tgtcgtttcc c 21

<210> 30
<211> 426
<212> DNA
<213> artificial

<220>
<223> synthetic construct sequence

<400> 30
ggattaaata ctgagagctc gaatttcccc gatcgttcaa acatttggca ataaagtttc 60

ttaagattga atcctgttgc cggtcttgcg atgattatca tataatttct gttgaattac 120

gttaagcatg taataattaa catgtaatgc atgacgttat ttatgagatg ggtttttatg 180

attagagtcc cgcaattgga tttgtaccag caacaagaag acataaaatt gcacgttttg 240

ccttttaaat gagttagttt tttatttttt tatccttcaa attgggtgat gtttaatttt 300

gttcttttct aatcgaactt atacctagtg atacataagt tctttaaaat taaaatttag 360

tccgagaata acttatgaaa tattacgata taaggataaa acttcaaatg accaacacaa 420

aactcg 426

<210> 31
<211> 23
<212> DNA
<213> artificial

<220>
<223> Primer

<400> 31
atgacgttat ttatgagatg ggt 23

<210> 32
<211> 20
<212> DNA
<213> artificial

<220>
<223> Primer

<400> 32
atttaaaagg caaaacgtgc 20

<210> 33
<211> 842
<212> DNA
<213> artificial

<220>
<223> synthetic construct sequence

<400> 33
tttttttttgt tatggagaaa gcagagataa tagttataga attgttggtt accctttaga 60

tttcaagagt atagaatgat gtttagttca tttgagaata gcgctatagc agtatatttg 120

catacataaa ccagaaacat acaaccatca cctatctcga atgatcaagc caaactttcc 180

ccttgcatag tgtcttggct tgttcttagt ttaaaacaat aatataaaca acaataaatt 240

actaatggcc gaataattta tctgaatttt aacaaatttt attctcggca aaaatatgat 300

caactttccc aacttagggc tccttcccac cgtgaaatca tgtcaacacc ttctcccaag 360

ggcaataact tagtttctac ggcttaagca ataatgcaca aggttggaga gtcaaacttt 420

tgcctttatc gataaattca atgggaaaac taatgcaaac tgtccaaaat cacccctccc 480

aagtgtaaga actcaatgta cagaaaatag gtgttttggg ttattttctg cataaaattc 540

tgattcagct caaaatagta gtccaacccg tgatagcgac aactagtctc actatagcga 600

tctccacatt ctgctatagc gagacacctt ttgccaccta aactccagaa aaatgtcatt 660

tgtacaacac aaactctcct ttcatgtcaa gttcaatttc aggagttcat gccctggcac 720

gacaggtttc ccgactggaa agcgggcagt gagcgcaacg caattaatgt gagttagctc 780

actcattagg caccccaggc tttacacttt atgcttccgg ctcgtatgtt gtgtggaagt 840

tg 842

<210> 34
<211> 23
<212> DNA
<213> artificial

<220>
<223> Primer

<400> 34
ttcatgtcaa gttcaatttc agg 23

<210> 35
<211> 21
<212> DNA
<213> artificial

<220>
<223> Primer

<400> 35
actcacatta attgcgttgc g 21

<210> 36
<211> 1209
<212> DNA
<213> artificial

<220>
<223> synthetic construct sequence

<400> 36
tgttgccggt cttgcgatga ttatcatata atttctgttg aattacgtta agcatgtaat 60

aattaacatg taatgcatga cgttatttat gagatgggtt tttatgatta gagtcccgca 120

attatacatt taatacgcga tagaaaacaa aatatagcgc gcaaactagg ataaattatc 180

gcgcgcggtg tcatctatgt tactagatcg ggaattcact ggccgtcgtt ttacaacgac 240

tcagctgctt ggtaataatt gtcattagat tgtttttatg catagatgca ctccaaagaa 300

tgtaatatta atgtaaataa ttcaaagaat gtaatattaa tgtaaataat tcaaaggtca 360

aggcggcttg cttggagaaa tgagagattg tttagttgac taatgatatg tttaattaag 420

aagaccattt tagcattcat ttttacattt tcttactatg aatagcagaa caaacgacgc 480

aaatagaacc ttttttcttc ttccattttc tcttaatttt gacttagcaa aatgatgagg 540

taccacaaat cacaataatt aatacttttc aaagatatca ataaattaat gatgatcctt 600

aaattttagg taaaagtgtt ggaactgtca tgattcagac cgtcgtgatt gacacccaca 660

ctagccctcc ggtgggagaa ccattactac aacccaaact aacaaatttt atgaaaacta 720

aagcatttaa agatatagaa gcaggtataa atgacaataa aaatggagtc cccataaact 780

ccaaggtttt taacaaacaa ctaatcaaac taatgcggaa gaacaacccc aaaactaaac 840

aacaccttaa gcaaagtctg agtccgaaaa gagtggactt aaacaagaaa gatccatggc 900

agcctgaaag aactgactca cccttgaatc cggtcacgct caatatccgc ctaaagatgc 960

cctgtactca acaaaaaaac aagcaagtac agtatcagta cacaaccaca gagtactggt 1020

aggatcacgc gactatccta ataagtgaaa cacatgcaag taaccacaac attataaaac 1080

aagcatatac cgaacatata cagtattagt catcttttca agatcaatgt agcatcacac 1140

gttctcaata atacacattg gttatcattt tagagcagca tacagtcttc caatatcaat 1200

catcattag 1209

<210> 37
<211> 26
<212> DNA
<213> artificial

<220>
<223> Primer

<400> 37
gcttggtaat aattgtcatt agattg 26

<210> 38
<211> 23
<212> DNA
<213> artificial

<220>

<223> Primer

<400> 38
gccttgacct ttgaattatt tac 23

<210> 39
<211> 1202
<212> DNA
<213> artificial

<220>
<223> synthetic construct sequence

<400> 39
atacaccata ccttaggaga aaaacacaac taacgttatt tttatcctgc aaatggaaag 60

gtctcaacca tagcaacgga taggatcatt gcagccttta ccagcagact gctttgagcc 120

aaaccgaccc ccctttgcaa ggtactgctg gtgatcttcc tcagctcgat aaaatctcac 180

atcaaatgac tcccaagtgt ttagatataa attgcgtcat atttgagggg ttctttaaag 240

ttgaattaac aataatattt agttgatatt gtttgaacac ccattccact tggccttttt 300

aaaagttagc aaggttaatt ttcttttact tgaaacaatt ggaataaaaa aataattatt 360

tcctttgtct atatctcaat ttatatgata tagtttaact atattagaaa agttaatcgt 420

aataactcat ataattgtca aattattata cgctttgacc tcaatctctc cgaattacct 480

caagcgtttg ggccctcata ttttgaactc ttcggctttt taacctaaaa ggttcgttat 540

aattgaatta tgaactcact tttatgtgac cctaactttt cctctcgttc tgatgcagaa 600

ttttctaact caaatttcat aatattcgat tcttgttatt gagtccaaat cataatattt 660

gtttcttcta ttaaatctga ttatgaaatc tctagtcgcc tcgtgaattt ataggctaca 720

atctcatttt ccctcaatga atattcaatt cttgttattg agttcaaatc ataatattcg 780

tttcttctat taaatctgat tatgaaatct ctagtcgcct cgtgaattta taggctacaa 840

tctcattttc caaacactga tagtttaaac tgaaggcggg aaacgacaat cagatctagt 900

aggaaacagc tatgaccatg attacgccaa gcttgcatgc cctggcacga caggtttccc 960

gactggaaag cgggcagtga gcgcaacgca attaatgtga gttagctcac tcattaggca 1020

ccccaggctt tacactttat gcttccggct cgtatgttgt gtggaattgt gagcggataa 1080

caatttcaca caggaaacag ctatgaccat gattacgcca agctggcgcg ccaagcttgc 1140

atgcctgcag gtcgactcta gaggatctag aatcaccgaa cctcccctcg gtacagctcc 1200

tc 1202

<210> 40
<211> 25
<212> DNA
<213> artificial

<220>
<223> Primer

<400> 40
tctgatgcag aattttctaa ctcaa 25

<210> 41
<211> 26
<212> DNA
<213> artificial

<220>
<223> Primer

<400> 41
ttcctactag atctgattgt cgtttc 26

<210> 42
<211> 702
<212> DNA
<213> artificial

<220>
<223> synthetic construct sequence

<400> 42
attaaatact gagagctcga atttccccga tcgttcaaac atttggcaat aaagtttctt 60

aagattgaat cctgttgccg gtcttgcgat gattatcata taatttctgt tgaattacgt 120

taagcatgta ataattaaca tgtaatgcat gacgttattt atgagatggg tttttatgat 180

tagagtcccg caattataca tttaatacgc gatagaaaac aaaatatagc gcgcaaacta 240

ggataaatta tcgcgcgcgg tgtcatctat gttactagat cgggaattca ctggccgtcg 300

ttttacaacg actcagctgc ttggtaataa ttgtcattag attgttttta tgcatagatg 360

cactcgaaat cagccaattt tagacaagta tcaaacggat gttaattcag tacattaaag 420

acgtccgcaa tgtgttatta agttgtctaa gttgtccttg gttttattag ttgttattgt 480

tgttttgtct tttcaatttc tagtagtcaa ttgggaactg gaagtacaaa gttgaatttt 540

tagacttatg tatacaaatt gaaaatgtat caaaaagtag tgacacttat aaaaagaaca 600

attctttctt ctgagaacat cttcaaccca cctctatttt actctccatt ctctatattt 660

agtgagtaaa atagagaatg ggcactccaa cccaccttca tc 702

<210> 43
<211> 1137
<212> DNA
<213> artificial

<220>
<223> synthetic construct sequence

<400> 43
aatagttaac catacaaact gaattttggg attgagtttc tatttaaatt ttcgtgagta 60

aagattaaag ttcaaattaa ctttttataa aatgagtttt caattttaaa aagtcaaaca 120

aaatatatat agaatataaa gtgagaaacc aaataaaaca agaatagcaa ggattatcca 180

tttaggaaat aagatgaaag aatcaaatca actaataaaa actttaatcc tataagataa 240

gcttgccaat aatttaaaat agcatgatgc gtctaagagg gtatcaatat attgttatcc 300

tttaaagacc aagatagtgt ttcaggaaag ttatttcatt tccaaattta aataaaagaa 360

aataattatt caaaattctt atgtcttttt tattgattta tagtcgaaga tctttcaaaa 420

ataatttttc tataggtaaa aaaggataat ttcaatttca cacaggaaac agctatgacc 480

atgattacgc caagctggcg cgccaagctt gcatgcctgc aggtcgactc tagaggatct 540

agaatcaccg aacctcccct cggtacagct cctccagttc taccatgaat ttcatccact 600

gattcctctt caatcgccat tgcagattct ctcgatctat gctcaaaaaa tcccgagata 660

aaaccctaga tctgcttcaa atgctctgat accatgtaat ttcagtgaat tctaactaaa 720

caatggagag aattaactat tttagaaaga ctgattgaag gagaagaaga gagaaaaatt 780

ctatattgaa ctcatgaacc aaaatgaatg aaaaaaataa tgagaagaac tatactatta 840

caatctatat atctctattt atattctaat ctgaagcagt taatttaact gactctaaca 900

actagactga taggtgtaca ttttctgtta gtgcactgca gtgcatttaa ctaactgctt 960

aacataaaga atgttgttcg aacttcattc gaatagcttc aatgagaagc aaacatgtgt 1020

acctgtaaag acacacagta aaagtgttaa taatgaataa atatgaataa atcaaataat 1080

aaattaaaaa taaaaacaca tccaattaac attggaggtc ttgaaaatcg atggtaa 1137

<210> 44
<211> 5881
<212> DNA
<213> artificial

<220>
<223> synthetic construct sequence

<400> 44
gtgaactagg aaacctaaat ctctatggct caattaaaat ctcgcatctt gagagagtga 60

agaatgataa ggacgcaaaa gaagccaatt tatctgcaaa agggaatctg cattctttaa 120

gcatgagttg gaataacttt ggaccacata tatatgaatc agaagaagtt aaagtgcttg 180

aagccctcaa accacactcc aatctgactt ctttaaaaat ctatggcttc agaggaatcc 240

atctcccaga gtggatgaat cactcagtat tgaaaaatat tgtctctatt ctaattagca 300

acttcagaaa ctgctcatgc ttaccaccct ttggtgatct gccttgtcta gaaagtctag 360

agttacactg ggggtctgcg gatgtggagt atgttgaaga agtggatatt gatgttcatt 420

ctggattccc cacaagaata aggtttccat ccttgaggaa acttgatata tgggactttg 480

gtagtctgaa aggattgctg aaaaaggaag gagaagagca attccctgtg cttgaagaga 540

tgataattca cgagtgccct tttctgaccc tttcttctaa tcttagggct cttacttccc 600

tcagaatttg ctataataaa gtagctactt cattcccaga agagatgttc aaaaaccttg 660

caaatctcaa atacttgaca atctctcggt gcaataatct caaagagctg cctaccagct 720
tggctagtct gaatgctttg aaaagtctaa aaattcaatt gtgttgcgca ctagagagtc 780
tccctgagga agggctggaa ggtttatctt cactcacaga gttatttgtt gaacactgta 840
acatgctaaa atgtttacca gagggattgc agcacctaac aaccctcaca agtttaaaaa 900
ttcggggatg tccacaactg atcaagcggt gtgagaaggg aataggagaa gactggcaca 960
aaatttctca cattcctaat gtgaatatat atatttaagt tatttgctat tgtttctttg 1020
tttgtgagtc tttttggttc ctgccattgt gattgcatgt aatttttttc tagggttgtt 1080
tctttatgag tctctctctc attggatgta attttctttt ggaaacaaat ctgtcaattg 1140
atttgtatta tacgctttca gaatctatta cttatttgta attgtttctt tgtttgtaaa 1200
ttgtgagtat cttattttat ggaattttct gattttattt tgaaaacaaa tcaatgattt 1260
gtaagatcca tctgtattat actcccttcg tctcatttta tgtgtcacct gtcggatttc 1320
gagattcaaa caaatctatc tttgatcgta aatttttaat agatctttta aacattttga 1380
attatcaatt attgtgactt tagtggctag actagtggat ccgatatcgc ccagcttcac 1440
gctgccgcaa gcactcaggg cgcaagggct gctaaaggaa gcggaacacg tagaaagcca 1500
gtccgcagaa acggtgctga ccccggatga atgtcagcta ctgggctatc tggacaaggg 1560
aaaacgcaag cgcaaagaga aagcaggtag cttgcagtgg gcttacatgg cgatagctag 1620
actgggcggt tttatggaca gcaagcgaac cggaattgcc agctggggcg ccctctggta 1680
aggttgggaa gccctgcaaa gtaaactgga tggctttctt gccgccaagg atctgatggc 1740
gcaggggatc aagatcatga gcggagaatt aagggagtca cgttatgacc cccgccgatg 1800
acgcgggaca agccgtttta cgtttggaac tgacagaacc gcaacgttga aggagccact 1860
cagccgcggg tttctggagt ttaatgagct aagcacatac gtcagaaacc attattgcgc 1920
gttcaaaagt cgcctaaggt cactatcagc tagcaaatat ttcttgtcaa aaatgctcca 1980
ctgacgttcc ataaattccc ctcggtatcc aattagagtc tcatattcac tctcaatcca 2040
gatccccggg taccatggcg gcggcaacaa caacaacaac aacatcttct tcgatctcct 2100
tctccaccaa accatctcct tcctcctcca aatcaccatt accaatctcc agattctccc 2160
tcccattctc cctaaacccc aacaaatcat cctcctcctc ccgccgccgc ggtatcaaat 2220
ccagctctcc ctcctccatc tccgccgtgc tcaacacaac caccaatgtc acaaccactc 2280
cctctccaac caaacctacc aaacccgaaa cattcatctc ccgattcgct ccagatcaac 2340
cccgcaaagg cgctgatatt ctcgtcgagg ctttagaacg tcaaggcgta gaaaccgtat 2400
tcgcttaccc tggaggtgca tcaatggaga ttcaccaagc cttaacccgc tcttcctcaa 2460
tccgtaacgt ccttcctcgt cacgaacaag gaggtgtatt cgcagcagaa ggatacgctc 2520
gatcctcagg taaaccaggt atctgtatag ccacttcagg tcccggagct acaaatctcg 2580
ttagcggatt agccgatgcg ttgttagata gtgttcctct tgtagcaatc acaggacaag 2640

tccctcgtcg tatgattggt acagatgcgt ttcaagagac tccgattgtt gaggtaacgc 2700
gttcgattac gaagcataac tatcttgtga tggatgttga agatattcct aggattattg 2760
aggaggcttt ctttttagct acttctggta gacctggacc tgttttggtt gatgttccta 2820
aagatattca acaacagctt gcgattccta attgggaaca ggctatgaga ttacctggtt 2880
atatgtctag gatgcctaaa cctccggaag attctcattt ggagcagatt gttaggttga 2940
tttctgagtc taagaagcct gtgttgtatg ttggtggtgg ttgtttgaac tctagcgatg 3000
aattgggtag gtttgttgag cttacgggaa tccctgttgc gagtacgttg atggggctgg 3060
gatcttatcc ttgtgatgat gagttgtcgt tacatatgct tggaatgcat gggactgtgt 3120
atgcaaatta cgctgtggag catagtgatt tgttgttggc gtttggggta aggtttgatg 3180
atcgtgtcac gggtaaactt gaggcttttg ctagtagggc taagattgtt catattgata 3240
ttgactcggc tgagattggg aagaataaga ctcctcatgt gtctgtgtgt ggtgatgtta 3300
agctggcttt gcaagggatg aataaggttc ttgagaaccg agcggaggag cttaaacttg 3360
attttggagt ttggaggaat gagttgaacg tacagaaaca gaagtttccg ttgagcttta 3420
agacgtttgg ggaagctatt cctccacagt atgcgattaa ggtccttgat gagttgactg 3480
atggaaaagc cataataagt actggtgtcg ggcaacatca aatgtgggcg gcgcagttct 3540
acaattacaa gaaaccaagg cagtggctat catcaggagg ccttggagct atgggatttg 3600
gacttcctgc tgcgattgga gcgtctgttg ctaaccctga tgcgatagtt gtggatattg 3660
acggagatgg aagttttata atgaatgtgc aagagctagc cactattcgt gtagagaatc 3720
ttccagtgaa ggtactttta ttaaacaacc agcatcttgg catggttatg caatgggaag 3780
atcggttcta caaagctaac cgagcacaca catttctcgg agatccggct caggaggacg 3840
agatattccc gaacatgttg ctgtttgcag cagcttgcgg gattccagcg gcgagggtga 3900
caaagaaagc agatctccga gaagctattc agacaatgct ggatacacca ggaccttacc 3960
tgttggatgt gatttgtccg caccaagaac atgtgttgcc gatgatcccg aatggtggca 4020
ctttcaacga tgtcataacg gaaggagatg gccggattaa atactgagag ctcgaatttc 4080
cccgatcgtt caaacatttg gcaataaagt ttcttaagat tgaatcctgt tgccggtctt 4140
gcgatgatta tcatataatt tctgttgaat tacgttaagc atgtaataat taacatgtaa 4200
tgcatgacgt tatttatgag atgggttttt atgattagag tcccgcaatt atacatttaa 4260
tacgcgatag aaaacaaaat atagcgcgca aactaggata aattatcgcg cgcggtgtca 4320
tctatgttac tagatcggga attcactggc cgtcgtttta caacgactca gctgcttggt 4380
aataattgtc attagattgt ttttatgcat agatgcactc gaaatcagcc aattttagac 4440
aagtatcaaa cggatgttaa ttcagtacat taaagacgtc cgcaatgtgt tattaagttg 4500
tctaagttgt ccttggtttt attagttgtt attgttgttt tgtcttttca atttctagta 4560

gtcaattggg aactggaagt acaaagttga atttttagac ttatgtatac aaattgaaaa 4620

tgtatcaaaa agtagtgaca cttataaaaa gaacaattct ttcttctgag aacatcttca 4680

acccacctct attttactct ccattctcta tatttagtga gtaaaataga gaatgggcac 4740

tccaacccac ctccatatca ctctccattc ttcatattta gagagtcata ttatttttat 4800

tattattttt attactttct aattaatata ttattttaca tataatgtca ttaattaaat 4860

atctaatatt cataattctt tttaaatgtc atattatatt ttaaaaaata tattatttaa 4920

tatatactac tttcatcccg aattaatagt attttaattt tttctaattt tcgacaataa 4980

tataatttga ttttattatt aattttcact ataaataata cacaaaatta aaatgataag 5040

atgaaaaaat taatttaaaa atacaataca taatatgata atttaaatac aggataacaa 5100

tacaatacat gacataataa tttaaataca agataaaata caatacataa cataataatc 5160

aattcgtgat gaaacaagaa tcatgccaaa aagatattga acgtgcattc gaagttttgc 5220

aatcacgttt tgcaattatt gcaagaccgt cacgtttttg gagaaaggaa gtgtgacatg 5280

atataatgac tacatgtatt atactgcaca ccatgataat tgaggatgaa catgatctta 5340

atgcaccaaa tcaagatgcc gtagaggctc caactccaac gacaaaaatg atggtagatg 5400

aaaatcttcg gtttgaacaa tttttagcta gacataaaaa agttaaggac aaaaatgctc 5460

attttgaact ccgtaatgca ttaatagagc atttatgaca gcaacgtgat aatttcgaaa 5520

cttgagtgtt tatgtaatta tatttcactt ttatttgaat ttgtccatta atttgtatat 5580

tatataatat ttatttacaa tttatgttat ttaaaaattt agaataaaat aaaattttga 5640

aaaaataaaa ttttatatca catgaaaatt atataaagta attattgggg aaaaaaataa 5700

aggatttata ttatgaaata agaaaataag aatgaataga aataataata atataatatt 5760

gaggagaaat aattattttt tttagagagt gaaatagaga attgggttga aattgattgt 5820

ctgaaaaaat aaaaaactct atatttggag agtaaaatag agtgtagggt tggagacgtc 5880

a 5881

<210> 45
<211> 14246
<212> DNA
<213> artificial

<220>
<223> synthetic construct sequence

<400> 45
gaaactcagt tggagattac gtttaaggga tggcctacat ggataaggtt atctacaatg 60

atgcaatgga gataataata gtactcctat gtgatttctt cgttttaatt tgtttatttt 120

attttcattt aatttgattt gatacgtagt ttaaaaaaat aaaaaagata tttaaatttt 180

gtgatgttag atgaaacata cgttaaatgc atcaaaatgt catttaaatt tgtcttgaac 240

atatagtatt agaaagttga aattaaagag ctatcaaaaa agaagaagat atgactaaaa 300

aaaatagcac aagcaaattg aaactaaaag agtaattaac aataacaatt tttttttaaa 360
atgatacata atactcactc tttctcaata tgtggcacaa aaatatcaag aatcaaacta 420
tttaattttg actataaatt tagaaataaa ttttttaaat ttcttaaaaa acaaaattta 480
caaagcaaat actatataaa aaatattata aattataata attaataatt caaaatagtt 540
aaccatacaa actgaatttt gggattgagt ttctatttaa attttcgtga gtaaagatta 600
aagttcaaat taacttttta taaaatgagt tttcaatttt aaaaagtcaa acaaaatata 660
tatagaatat aaagtgagaa accaaataaa acaagaatag caaggattat ccatttagga 720
aataagatga aagaatcaaa tcaactaata aaaactttaa tcctataaga taagcttgcc 780
aataatttaa aatagcatga tgcgtctaag agggtatcaa tatattgtta tcctttaaag 840
accaagatag tgtttcagga aagttatttc atttccaaat ttaaataaaa gaaaataatt 900
attcaaaatt cttatgtctt ttttattgat ttatagtcga agatctttca aaaataattt 960
ttctataggt aaaaaaggat aatttcaatt tcacacagga aacagctatg accatgatta 1020
cgccaagctg gcgcgccaag cttgcatgcc tgcaggtcga ctctagagga tctagaatca 1080
ccgaacctcc cctcggtaca gctcctccag ttctaccatg aatttcatcc actgattcct 1140
cttcaatcgc cattgcagat tctctcgatc tatgctcaaa aaatcccgag ataaaaccct 1200
agatctgctt caaatgctct gataccatgt aatttcagtg aattctaact aaacaatgga 1260
gagaattaac tattttagaa agactgattg aaggagaaga agagagaaaa attctatatt 1320
gaactcatga accaaaatga atgaaaaaaa taatgagaag aactatacta ttacaatcta 1380
tatatctcta tttatattct aatctgaagc agttaattta actgactcta acaactagac 1440
tgataggtgt acattttctg ttagtgcact gcagtgcatt taactaactg cttaacataa 1500
agaatgttgt tcgaacttca ttcgaatagc ttcaatgaga agcaaacatg tgtacctgta 1560
aagacacaca gtaaaagtgt taataatgaa taaatatgaa taaatcaaat aataaattaa 1620
aaataaaaac acatccaatt aacattggag gtcttgaaaa tcgatggtaa ttaacaaaga 1680
cccttgtgaa atttaagtct gtaattgaaa atttgagtat aggttagggg acatttgact 1740
attttctcat tttctttatc tttttcctaa tttgtggcag acaagtgagg aggccccact 1800
gtaattgatt catgcttttg ctttcttgac tttttggaac aatactatgc atcatatttg 1860
gtcttaatta ttcctctgtt tatttccaga attttgagct ctatacatct aataacaaag 1920
caagcagagg atatatagtt tcatcaacta aaaaggttag tcaactcatc taatatttgc 1980
tactctcatc tctattgaag tacagttatg gaaaagtaga agtgatgtaa gaaaaatgaa 2040
agaactttag taggttagtt ggatctaaca aagagaaagg gaaataaatt gcaggagaaa 2100
gagagaggtt aaatacttac tcacaccacc gatttacaac aaatcactta attgtggtta 2160
gttaatgtat actttcacct cattaaatta ttacttaccc atgataagtt gtattaattt 2220

ggtattaata tccggtgcgg gtgaattctt accgggtgag agggatgggg ttggagagtg 2280

tggagtgaac agaagcagat gttttagatt ttttctaaga tgacgaaaga ttcccctcac 2340

taatgaaaat atattactat acgctattag agatagaaag gttcggtacc agttggtctc 2400

gtttctggat gaaccccatt tttacaagtc attttcttca attcaaatcg caagtgtacc 2460

tttatcatct tccactaatt aagtcctctt aagttcgcgt gaaaatagtg aaattattga 2520

ttattcttat catttcatct tctttctcct gataaagttt tatgtacttt ttatgcatca 2580

ggtcttgaga acttggaaag gaaaagtaga atcatggaaa aacgaaaaga taatgaagaa 2640

gcaaacaact cattggtatg ttatttgata gagtgaactg taaagtattg aattgtagat 2700

atcatgtggc tttaaaaatt tgatatgtgt tattttggca ggagtcattt tctgctcttc 2760

gcaaggatgc tgccaatgtt ctggatttcc tagagagatt aaagaatgaa gaagatcaaa 2820

aggctgttga tgtggatctg attgaaagcc tgaaattgaa gctgacattt atttgtacat 2880

atgtccagct ttcttattcc gatttggaga agtttgaaga tataatgact agaaaaagac 2940

aagaggttga gaatctgctt caaccaattt tggatgatga tggcaaagac gtcgggtgta 3000

aatatgtcct tactagcctc gccggtaata tggatgactg tataagcttg tatcatcgtt 3060

ctaaatcaga tgccaccatg atggatgagc aattgggctt cctcctcttg aatctctctc 3120

atctatccaa gcatcgtgct gaaaagatgt ttcctggagt gactcaatat gaggttcttc 3180

agaatgtatg tggcaacata agagatttcc atggattgat agtgaattgt tgcattaagc 3240

atgagatggt tgagaatgtc ttatctctgt ttcaactgat ggctgagaga gtaggacgct 3300

tcctttggga ggatcaggct gatgaagact ctcaactctc cgagctagat gaggatgatc 3360

agaatgataa agaccctcaa ctcttcaagc tagcacatct actcttgaag attgttccaa 3420

ctgaattgga ggttatgcac atatgttata aaactttgaa agcttcaact tcaacagaaa 3480

ttggacgctt cattaagaag ctcctggaaa cctctccgga cattctcaga gaatatctga 3540

ttcatctaca agagcatatg ataactgtta ttacccctaa cacttcaggg gctcgaaaca 3600

ttcatgtcat gatggaattc ctattgatta ttctttctga tatgccgccc aaggacttta 3660

ttcatcatga caaacttttt gatctcttgg ctcgtgttgt agcacttacc agggaggtat 3720

caactcttgt acgcgacttg gaagagaaat taaggattaa agagagtact gacgaaacaa 3780

attgtgcaac cctaaagttt ctggaaaata ttgaactcct taaggaagat ctcaaacatg 3840

tttatctgaa agtcccggat tcatctcaat attgcttccc catgagtgat ggacctctct 3900

tcatgcatct gctacagaga cacttagatg atttgctgga ttccaatgct tattcaattg 3960

ctttgataaa ggaacaaatt gggctggtga aagaagactt ggaattcata agatcttttt 4020

tcgcgaatat tgagcaagga ttgtataaag atctctggga acgtgttcta gatgtggcat 4080

atgaggcaaa agatgtcata gattcaatta ttgttcgaga taatggtctc ttacatctta 4140

ttttctcact tcccattacc agaaagaaga tgatgcttat caaagaagag gtctctgatt 4200

tacatgagaa catttccaag aacagaggtc tcatcgttgt gaactctccc aagaaaccag 4260
ttgagagcaa gtcattgaca actgataaaa taattgtagg ttttggtgag gagacaaact 4320
tgatacttag aaagctcacc agtggaccgg cagatctaga tgtcatttcg atcattggta 4380
tgccgggttt aggtaaaact actttggcgt acaaagtata caatgataaa tcagtttcta 4440
gccatttcga ccttcgtgca tggtgcacgg tcgaccaagt atatgacgag aagaagttgt 4500
tggataaaat tttcaatcaa gttagtgact caaattcaaa attgagtgag aatattgatg 4560
ttgctgataa actacggaaa caattgtttg gaaagaggta tcttattgtc ttagatgacg 4620
tgtgggatac taatacatgg gatgagctaa caagaccttt tcctgatggt atgaaaggaa 4680
gtagaattat tttgacaact cgagaaaaga aagttgcttt gcatggaaag ctctacactg 4740
atcctcttaa ccttcgattg ctaagatcag aagaaagttg ggagttatta gagaaaaggg 4800
catttggaaa cgagagttgc cctgatgaac tattggatgt tggtaaagaa atagccgaaa 4860
attgtaaagg gcttcctttg gtggtggatc tgattgctgg aatcattgct gggagggaaa 4920
agaaaaagag tgtgtggctt gaagttgtaa ataatttgca ttcctttatt ttgaagaatg 4980
aagtggaagt gatgaaagtt atagaaataa gttatgacca cttacctgat cacctgaagc 5040
catgcttgct gtactttgca agtgcgccga aggactgggt aacgacaatc catgagttga 5100
aacttatttg ggttttgaa ggatttgtgg aaaagacaga tatgaagagt ctggaagaag 5160
tggtgaaaat ttatttggat gatttaattt ccagtagctt ggtaatttgt ttcaatgaga 5220
taggtgatta ccctacttgc caacttcatg atcttgtgca tgacttttgt ttgataaaag 5280
caagaaagga aaagttgtgt gatcggataa gttcaagtgc tccatcagat ttgttgccac 5340
gtcaaattag cattgattat gatgatgatg aagagcactt tgggcttaat tttgtcctgt 5400
tcggttcaaa taagaaaagg cattccggta aacacctcta ttctttgacc ataaatggag 5460
atgagctgga cgaccatctt tctgatacat ttcatctaag acacttgagg cttcttagaa 5520
ccttgcacct ggaatcctct tttatcatgg ttaaagattc tttgctgaat gaaatatgca 5580
tgttgaatca tttgaggtac ttaagcattg ggacagaagt taaatctctg cctttgtctt 5640
tctcaaacct ctggaatcta gaaatcttgt ttgtggataa caaagaatca accttgatac 5700
tattaccgag aatttgggat cttgtaaagt tgcaagtgct gttcacgact gcttgttctt 5760
tctttgatat ggatgcagat gaatcaatac tgatagcaga ggacacaaag ttagagaact 5820
tgacagcatt aggggaactc gtgctttcct attggaaaga tacagaggat attttcaaaa 5880
ggcttcccaa tcttcaagtg cttcatttca aactcaagga gtcatgggat tattcaacag 5940
agcaatattg gttcccgaaa ttggatttcc taactgaact agaaaaactc actgtagatt 6000
ttgaaagatc aaacacaaat gacagtgggt cctctgcagc cataaatcgg ccatgggatt 6060
ttcactttcc ttcgagtttg aaaagattgc aattgcatga atttcctctg acatccgatt 6120

cactatcaac aatagcgaga ctgctgaacc ttgaagagtt gtacctttat cgtacaatca 6180
tccatgggga agaatggaac atgggagaag aagacacctt tgagaatctc aaatgtttga 6240
tgttgagtca agtgattctt tccaagtggg aggttggaga ggaatctttt cccacgcttg 6300
agaaattaga actgtcggac tgtcataatc ttgaggagat tccgtctagt tttggggata 6360
tttattcctt gaaaattatc gaacttgtaa ggagccctca acttgaaaat tccgctctca 6420
agattaagga atatgctgaa gatatgaggg gaggggacga gcttcagatc cttggccaga 6480
aggatatccc gttatttaag tagtttttga gcattatggt tgaaaagtag attgcacttt 6540
gctgggtaga ttgtatatgg ttaagaaaat tctgttacag ttgttatgaa acatttttat 6600
ttgacttttc tgagtttctt ttagaaaact cagaagtttt taacaaaaat tatagttttt 6660
ataaatacaa tgtggatttg cctttggctg tccaacttgg tctgaagtct catatgctca 6720
gagcactatc gttcaacctc aatcaaggta ctgatttaaa atgacatcta tactacttta 6780
tcacaaaccc aacgaacttt catctcaaaa gctaggccag gaagtgaaga ggttgtagag 6840
agcttataag cactcatgac ttccttttct cgaacattca accaacgtag gctgaaatcc 6900
cactctgaac gaaaataagt gtttgtttat caaattaact ctcgtagtag aacactgaaa 6960
taccttcttc taaacgttca acaaatggga tttccagcac tcaaagtgaa tgaaaggttc 7020
acattaatct tcaaaaagaa ttacgacaat tcatgaccac aagtacattg acagcaccat 7080
ttcaacagaa gaacaagtca atgctgcatc ttcatcaata atccgagtgt cgaacctcct 7140
tcctgacact gtcctgtata tgtaaagttt ctcaacaggg caactttctg gtctcgtatc 7200
tggatgaccc ctctcgtcta taacttcaac attaagccct ggcaacttct ggaccaacag 7260
cttacatgct tcaaaactta ctgaacaatt agacatccaa agggatcgca ttgtctccag 7320
ctttgcagca ttagccaaca gagcctcatc gccaaagggg cagtctctaa tctcgaattt 7380
gaaaaaattg ttgttgtatg actttcctct gacatccgat gcactatcaa caatagcaag 7440
actggaggtt ggagaggaat cctttattat acaatcattc agggagaaga atggaacatg 7500
ggggaggaag acacttttga gaatctgaaa tgtgttagag ccacaagcta cagaagtatt 7560
gaatttgtca tgaatatcaa cattcttcat cctagttaat tctttttcaa tttttaatag 7620
actctcattt taatcactaa tattcttcta tttgtgactt cttttctgca ggtggcaact 7680
ttaaattcat aaagtatagg attgatgaca aactcgaaaa atatcttaat gaggtgaagt 7740
ttgagcagtc agcagatggt ggttccaact ctaagttgac aagcacatac tatcccggag 7800
ggcgatttca agcctgatgc atatggttag tgtggctaga gcagacagga tgtattacct 7860
ggatatctac caagacgaat ccacaatcag ttttatgtca agcaatacat gaagtaactc 7920
ccgatagaac agtaaaagca agatgtgtag gtgtatctcg actctaagag attgtacatt 7980
cctctttgag atttttactg ctaatacaaa tttacacctc agaagcgaat ctagaatttc 8040
tagagcatga atgcaccact aatgaaagga gaaaaaagga agtatgaagt gggaatttga 8100

tccttgtttc taggtatata aaatttatca ttcaactata cttcatttag caaacaactc 8160
tctttgccat tatttctcaa acaagggctt ctaatattgc taaactaaag actgtcaaaa 8220
ggtaagttca tcttcaaact ctcttgttta ctttatctaa aggggaacta tgaaaaacaa 8280
gaaacatcag gaatgtcccg taaacaaagc agcctcatgc acaaaacatc caacgttggt 8340
aggattaatg gagggatcgc atcccaggag gatactgtag aaaaattagt ggcttctttc 8400
accgctcaaa cccatgatct ataggttaca tggagacaac tttatggttg ctcgtaggct 8460
cccgtcaatt ctcataaacc acaacaccaa agttgcatca gacatcatct tcattcacaa 8520
gctgacaatc tccacaagtc ttagtcaact tgtaatatga atattagcca ggtagacgta 8580
catatttaca aaattgagtt tcctatataa tatggtttga aggaatgaaa catgatgggg 8640
agggtagata aaataatata tgaggcataa aaataggaaa gatatttgta gtgagaggtt 8700
ttgacttttt atgctgcttt tgatcttcag tttcttgtat tctttttcta ctgctttcct 8760
cttctttctc ctgagtaaag ttttatgtag gtacttttta tacgtccgat cgtgagaact 8820
tgaaagaaag ctctctatag ctatgttagg tgcccacata aaaaaatgaa atattacaaa 8880
aaccctgata ataaaataca ctaatctaag atattcactg caacatacat gcaaaatata 8940
tatatataaa ttttcatgaa aattataaca aataatagat gtgaacatat aactttaaaa 9000
ataatattac atccataaag cttaaattct agatccccgg tcgactctag aggatcccca 9060
ctccatccgt tcactttgat ttgtcatgtt gcacttttcg aaagtcaatt tgactaattt 9120
ttaaagctaa attagattac actaattcaa tattttaaac agaaaaatta gatattcaaa 9180
aactatacaa aaaatattat acattgcaat tttttgcata tcaatatgat aaaaaaatat 9240
atcgtaaaat attagtcaaa atttttataa tttgactcaa atcatgaaaa gtataataat 9300
taatagtgga cggaggaagt attgtctttc cagatttgtg gccatttttg gtccaagggc 9360
cattagcagt tctcttcatt ttctacttct gtctcatatt agatgggcat cttactaaaa 9420
atatttgtct catattactt gattatttat taaatcaaaa agaattaatt aatttttctt 9480
cattttaccc ctacaattaa tatagtttta aaagttttaa acaaattttg aagaatcaaa 9540
atttcttttt gcaagagact tattaatata aacaaaggat aaaataataa aatttgtcaa 9600
tttattgacg atcacttaat aatcatataa aatagaaaat gtttatctaa tatgagacgg 9660
agaaaatata tcctaaaata tttttggaca gatatgtgat attctaacca ttcactagac 9720
tatattatgc attttagccg ccaatgactt atttcagctt taattaatta ggaaagagga 9780
aactgccaat gaggaagagt aggggcgtag ttgctgtcga cgaaaaaaag ataatactca 9840
ctcttttcga tttttatttt tatttatcac ttttaaccta tcatgtaaaa agataattat 9900
ttttttcatg ctttatcctt agtattaaac aatttaatag ggattatttt gtaaaatatt 9960
tatatgaata attgttttcg taatgaattt gtccggtcaa acaatgataa ataaaaatga 10020

atgaagagag tagaaaacaa aacaaaagaa caagttgaca acttgagaga ttaaaagggt 10080
ccaaaacgcc ttggattttg agattccata tgtgaaattt ccatgaaata attgaatttg 10140
tattattaca agtcaaactt tccatttcat tccaactagc catcttggtt tcaaaattac 10200
acattcattc attcacagat ctaatattct taatagtgat ttccacatat ggctgaagct 10260
ttcattcaag ttctgctaga caatctcact tctttcctca aaggggaact tgtattgctt 10320
ttcggttttc aagatgagtt ccaaaggctt tcaagcatgt tttctacaat tcaagccgtc 10380
cttgaagatg ctcaggagaa gcaactcaac aacaagcctc tagaaaattg gttgcaaaaa 10440
ctcaatgctg ctacatatga agtcgatgac atcttggatg aatataaaac caaggccaca 10500
agattctccc agtctgaata tggccgttat catccaaagg ttatcccttt ccgtcacaag 10560
gtcgggaaaa ggatggacca agtgatgaaa aaactaaagg caattgctga ggaaagaaag 10620
aattttcatt tgcacgaaaa aattgtagag agacaagctg ttagacggga aacaggtact 10680
catcttaaat tagtattaca acaactaagt ttatattcat ttttttggca attatcaaat 10740
tcagaaaagg gttaaatata ctcatgtcct atcgtaaata gtgtatatat acctctcgtt 10800
gtactttcga tctgaatata cttgtcaaat ctggcaagct cagaatcaaa ttatccaccc 10860
caacttttaa atactcgata tctttagaaa tccacctgtc taactcatcc actacccatt 10920
ccctttgctt tgaattcttt tctttaccta taaacttgga acactcgatc cgttttgctt 10980
ttcttaacaa agcagctcag agaaaagagg ttttcttcta ttctgtttct ctgtgtgctg 11040
cacttgggtc cttaatccca ttaaaaacag ggcatgttaa tcccaacgac ggtagccttt 11100
cctgacagct gactgtaaat tttgtctaac aaagaaaaaa aaagattaga catgtttttc 11160
cttgtcattg attaggctgg atttctttca gagtggaaca taggggatat attggaccaa 11220
aagtagaatg ggtatatatt taaagtattt ctgatagaac aggagtatat tgtgcgaaaa 11280
tatcctctat tttctgttgt ctcctaatga gtttgaatgt aataatattc tcatgtggac 11340
attgcttgca ccaggttctg tattaaccga accgcaggtt tatggaagag acaaagagaa 11400
agatgagata gtgaaaatcc taataaacaa tgttagtgat gcccaacacc tttcagtcct 11460
cccaatactt ggtatggggg gattaggaaa aacgactctt gcccaaatgg tcttcaatga 11520
ccagagagtt actgagcatt tccattccaa aatatggatt tgtgtctcgg aagattttga 11580
tgagaagagg ttaataaagg caattgtaga atctattgaa ggaaggccac tacttggtga 11640
gatggacttg gctccacttc aaaagaagct tcaggagttg ctgaatggaa aaagatactt 11700
gcttgtctta gatgatgttt ggaatgaaga tcaacagaag tgggctaatt taagagcagt 11760
cttgaaggtt ggagcaagtg gtgcttctgt tctaaccact actcgtcttg aaaaggttgg 11820
atcaattatg ggaacattgc aaccatatga actgtcaaat ctgtctcaag aagattgttg 11880
gttgttgttc atgcaacgtg catttggaca ccaagaagaa ataaatccaa accttgtggc 11940
aatcggaaag gagattgtga aaaaaagtgg tggtgtgcct ctagcagcca aaactcttgg 12000

aggtattttg tgcttcaaga gagaagaaag agcatgggaa catgtgagag acagtccgat 12060

ttggaatttg cctcaagatg aaagttctat tctgcctgcc ctgaggctta gttaccatca 12120

acttccactt gatttgaaac aatgctttgc gtattgtgcg gtgttcccaa aggatgccaa 12180

aatggaaaaa gaaaagctaa tctctctctg gatggcgcat ggttttcttt tatcaaaagg 12240

aaacatggag ctagaggatg tgggcgatga agtatggaaa gaattatact tgaggtcttt 12300

tttccaagag attgaagtta aagatggtaa aacttatttc aagatgcatg atctcatcca 12360

tgatttggca acatctctgt tttcagcaaa cacatcaagc agcaatatcc gtgaaataaa 12420

taaacacagt tacacacata tgatgtccat tggtttcgcc gaagtggtgt ttttttacac 12480

tcttcccccc ttggaaaagt ttatctcgtt aagagtgctt aatctaggtg attcgacatt 12540

taataagtta ccatcttcca ttggagatct agtacattta agatacttga acctgtatgg 12600

cagtggcatg cgtagtcttc caaagcagtt atgcaagctt caaaatctgc aaactcttga 12660

tctacaatat tgcaccaagc tttgttgttt gccaaaagaa acaagtaaac ttggtagtct 12720

ccgaaatctt ttacttgatg gtagccagtc attgacttgt atgccaccaa ggataggatc 12780

attgacatgc cttaagactc taggtcaatt tgttgttgga aggaagaaag gttatcaact 12840

tggtgaacta ggaaacctaa atctctatgg ctcaattaaa atctcgcatc ttgagagagt 12900

gaagaatgat aaggacgcaa aagaagccaa tttatctgca aaagggaatc tgcattcttt 12960

aagcatgagt tggaataact ttggaccaca tatatatgaa tcagaagaag ttaaagtgct 13020

tgaagccctc aaaccacact ccaatctgac ttctttaaaa atctatggct tcagaggaat 13080

ccatctccca gagtggatga atcactcagt attgaaaaat attgtctcta ttctaattag 13140

caacttcaga aactgctcat gcttaccacc ctttggtgat ctgccttgtc tagaaagtct 13200

agagttacac tggggggtctg cggatgtgga gtatgttgaa gaagtggata ttgatgttca 13260

ttctggattc cccacaagaa taaggtttcc atccttgagg aaacttgata tatgggactt 13320

tggtagtctg aaaggattgc tgaaaaagga aggagaagag caattccctg tgcttgaaga 13380

gatgataatt cacgagtgcc cttttctgac cctttcttct aatcttaggg ctcttacttc 13440

cctcagaatt tgctataata aagtagctac ttcattccca gaagagatgt tcaaaaacct 13500

tgcaaatctc aaatacttga caatctctcg gtgcaataat ctcaaagagc tgcctaccag 13560

cttggctagt ctgaatgctt tgaaaagtct aaaaattcaa ttgtgttgcg cactagagag 13620

tctccctgag gaagggctgg aaggtttatc ttcactcaca gagttatttg ttgaacactg 13680

taacatgcta aaatgtttac cagagggatt gcagcaccta acaaccctca caagtttaaa 13740

aattcgggga tgtccacaac tgatcaagcg gtgtgagaag ggaataggag aagactggca 13800

caaaatttct cacattccta atgtgaatat atatatttaa gttatttgct attgtttctt 13860

tgtttgtgag tctttttggt tcctgccatt gtgattgcat gtaatttttt tctagggttg 13920

tttctttatg agtctctctc tcattggatg taattttctt ttggaaacaa atctgtcaat 13980

tgatttgtat tatacgcttt cagaatctat tacttatttg taattgtttc tttgtttgta 14040

aattgtgagt atcttatttt atggaatttt ctgattttat tttgaaaaca aatcaatgat 14100

ttgtaagatc catctgtatt atactccctt cgtctcattt tatgtgtcac ctgtcggatt 14160

tcgagattca aacaaatcta tctttgatcg taaattttta atagatcttt taaacatttt 14220

gaattatcaa ttattgtgac tttagt 14246

<210> 46
<211> 5171
<212> DNA
<213> Solanum bulbocastanum

<400> 46
ttgatttgtc atgttgcact tttcgaaagt caatttgact aatttttaaa gctaaattag 60

attacactaa ttcaatattt taaacagaaa aattagatat tcaaaaacta tacaaaaaat 120

attatacatt gcaatttttt gcatatcaat atgataaaaa aatatatcgt aaaatattag 180

tcaaaatttt tataatttga ctcaaatcat gaaaagtata ataattaata gtggacggag 240

gaagtattgt ctttccagat ttgtggccat ttttggtcca agggccatta gcagttctct 300

tcattttcta cttctgtctc atattagatg ggcatcttac taaaaatatt tgtctcatat 360

tacttgatta tttattaaat caaaaagaat taattaattt tttctcattt taccccctaca 420

attaatatag ttttaaaagt tttaaacaaa ttttgaagaa tcaaaatttc tttttgcaag 480

agacttatta atataaacaa aggataaaat aataaaattt gtcaatttat tgacgatcac 540

ttaataatca tataaaatag aaaatgttta tctaatatga gacggagaaa atatatccta 600

aaatattttt ggacagatat gtgatattct aaccattcac tagactatat tatgcatttt 660

agccgccaat gacttatttc agctttaatt aattaggaaa gaggaaactg ccaatgagga 720

agagtagggg cgtagttgct gtcgacgaaa aaaagataat actcactctt ttcgattttt 780

atttttattt atcactttta acctatcatg taaaaagata attattttt tcatgcttta 840

tccttagtat taaacaattt aatagggatt attttgtaaa atatttatat gaataattgt 900

tttcgtaatg aatttgtccg gtcaaacaat gataaataaa aatgaatgaa gagagtagaa 960

aacaaaacaa aagaacaagt tgacaacttg agagattaaa agggtccaaa acgccttgga 1020

ttttgagatt ccatatgtga aatttccatg aaataattga atttgtatta ttacaagtca 1080

aactttccat ttcattccaa ctagccatct tggtttcaaa attacacatt cattcattca 1140

cagatctaat attcttaata gtgatttcca catatggctg aagctttcat tcaagttctg 1200

ctagacaatc tcacttcttt cctcaaaggg gaacttgtat tgcttttcgg ttttcaagat 1260

gagttccaaa ggctttcaag catgttttct acaattcaag ccgtccttga agatgctcag 1320

gagaagcaac tcaacaacaa gcctctagaa aattggttgc aaaaactcaa tgctgctaca 1380

tatgaagtcg atgacatctt ggatgaatat aaaaccaagg ccacaagatt ctcccagtct 1440
gaatatggcc gttatcatcc aaaggttatc cctttccgtc acaaggtcgg gaaaaggatg 1500
gaccaagtga tgaaaaaact aaaggcaatt gctgaggaaa gaaagaattt tcatttgcac 1560
gaaaaaattg tagagagaca agctgttaga cgggaaacag gtactcatct taaattagta 1620
ttacaacaac taagtttata ttcatttttt tggcaattat caaattcaga aaagggttaa 1680
atatactcat gtcctatcgt aaatagtgta tatatacctc tcgttgtact ttcgatctga 1740
atatacttgt caaatctggc aagctcagaa tcaaattatc caccccaact tttaaatact 1800
cgatatcttt agaaatccac ctgtctaact catccactac ccattccctt tgctttgaat 1860
tcttttcttt acctataaac ttggaacact cgatccgttt tgcttttctt aacaaagcag 1920
ctcagagaaa agaggttttc ttctattctg tttctctgtg tgctgcactt gggtccttaa 1980
tcccattaaa aacagggcat gttaatccca acgacggtag cctttcctga cagctgactg 2040
taaattttgt ctaacaaaga aaaaaaaaga ttagacatgt ttttccttgt cattgattag 2100
gctggatttc tttcagagtg gaacataggg gatatattgg accaaaagta gaatgggtat 2160
atatttaaag tatttctgat agaacaggag tatattgtgc gaaaatatcc tctattttct 2220
gttgtctcct aatgagtttg aatgtaataa tattctcatg tggacattgc ttgcaccagg 2280
ttctgtatta accgaaccgc aggtttatgg aagagacaaa gagaaagatg agatagtgaa 2340
aatcctaata aacaatgtta gtgatgccca acacctttca gtcctcccaa tacttggtat 2400
ggggggatta ggaaaaacga ctcttgccca aatggtcttc aatgaccaga gagttactga 2460
gcatttccat tccaaaatat ggatttgtgt ctcggaagat tttgatgaga agaggttaat 2520
aaaggcaatt gtagaatcta ttgaaggaag gccactactt ggtgagatgg acttggctcc 2580
acttcaaaag aagcttcagg agttgctgaa tggaaaaaga tacttgcttg tcttagatga 2640
tgtttggaat gaagatcaac agaagtgggc taatttaaga gcagtcttga aggttggagc 2700
aagtggtgct tctgttctaa ccactactcg tcttgaaaag gttggatcaa ttatgggaac 2760
attgcaacca tatgaactgt caaatctgtc tcaagaagat tgttggttgt tgttcatgca 2820
acgtgcattt ggacaccaag aagaaataaa tccaaacctt gtggcaatcg gaaaggagat 2880
tgtgaaaaaa agtggtggtg tgcctctagc agccaaaact cttggaggta ttttgtgctt 2940
caagagagaa gaaagagcat gggaacatgt gagagacagt ccgatttgga atttgcctca 3000
agatgaaagt tctattctgc ctgccctgag gcttagttac catcaacttc cacttgattt 3060
gaaacaatgc tttgcgtatt gtgcggtgtt cccaaaggat gccaaaatgg aaaaagaaaa 3120
gctaatctct ctctggatgg cgcatggttt tcttttatca aaaggaaaca tggagctaga 3180
ggatgtgggc gatgaagtat ggaaagaatt atacttgagg tctttttccc aagagattga 3240
agttaaagat ggtaaaactt atttcaagat gcatgatctc atccatgatt tggcaacatc 3300
tctgttttca gcaaacacat caagcagcaa tatccgtgaa ataaataaac acagttacac 3360

acatatgatg tccattggtt tcgccgaagt ggtgtttttt tacactcttc ccccttgga 3420
aaagtttatc tcgttaagag tgcttaatct aggtgattcg acatttaata agttaccatc 3480
ttccattgga gatctagtac atttaagata cttgaacctg tatggcagtg gcatgcgtag 3540
tcttccaaag cagttatgca agcttcaaaa tctgcaaact cttgatctac aatattgcac 3600
caagctttgt tgtttgccaa aagaaacaag taaacttggt agtctccgaa atcttttact 3660
tgatggtagc cagtcattga cttgtatgcc accaaggata ggatcattga catgccttaa 3720
gactctaggt caatttgttg ttggaaggaa gaaaggttat caacttggtg aactaggaaa 3780
cctaaatctc tatggctcaa ttaaaatctc gcatcttgag agagtgaaga atgataagga 3840
cgcaaaagaa gccaatttat ctgcaaaagg gaatctgcat tctttaagca tgagttggaa 3900
taactttgga ccacatatat atgaatcaga agaagttaaa gtgcttgaag ccctcaaacc 3960
acactccaat ctgacttctt taaaaatcta tggcttcaga ggaatccatc tcccagagtg 4020
gatgaatcac tcagtattga aaaatattgt ctctattcta attagcaact tcagaaactg 4080
ctcatgctta ccaccctttg gtgatctgcc ttgtctagaa agtctagagt tacactgggg 4140
gtctgcggat gtggagtatg ttgaagaagt ggatattgat gttcattctg gattccccac 4200
aagaataagg tttccatcct tgaggaaact tgatatatgg gactttggta gtctgaaagg 4260
attgctgaaa aaggaaggag aagagcaatt ccctgtgctt gaagagatga taattcacga 4320
gtgccctttt ctgacccttt cttctaatct tagggctctt acttccctca gaatttgcta 4380
taataaagta gctacttcat tcccagaaga gatgttcaaa aaccttgcaa atctcaaata 4440
cttgacaatc tctcggtgca ataatctcaa agagctgcct accagcttgg ctagtctgaa 4500
tgctttgaaa agtctaaaaa ttcaattgtg ttgcgcacta gagagtctcc ctgaggaagg 4560
gctggaaggt ttatcttcac tcacagagtt atttgttgaa cactgtaaca tgctaaaatg 4620
tttaccagag ggattgcagc acctaacaac cctcacaagt ttaaaaattc ggggatgtcc 4680
acaactgatc aagcggtgtg agaagggaat aggagaagac tggcacaaaa tttctcacat 4740
tcctaatgtg aatatatata tttaagttat ttgctattgt ttctttgttt gtgagtcttt 4800
ttggttcctg ccattgtgat tgcatgtaat ttttttctag ggttgtttct ttatgagtct 4860
ctctctcatt ggatgtaatt ttcttttgga aacaaatctg tcaattgatt tgtattatac 4920
gctttcagaa tctattactt atttgtaatt gtttctttgt ttgtaaattg tgagtatctt 4980
attttatgga attttctgat tttattttga aaacaaatca atgatttgta agatccatct 5040
gtattatact cccttcgtct cattttatgt gtcacctgtc ggatttcgag attcaaacaa 5100
atctatcttt gatcgtaaat ttttaataga tcttttaaac attttgaatt atcaattatt 5160
gtgactttag t 5171

<210> 47

<211> 7950
<212> DNA
<213> Solanum bulbocastanum

<400> 47
aacctcccct cggtacagct cctccagttc taccatgaat ttcatccact gattcctctt 60

caatcgccat tgcagattct ctcgatctat gctcaaaaaa tcccgagata aaaccctaga 120

tctgcttcaa atgctctgat accatgtaat ttcagtgaat tctaactaaa caatggagag 180

aattaactat tttagaaaga ctgattgaag gagaagaaga gagaaaaatt ctatattgaa 240

ctcatgaacc aaaatgaatg aaaaaaataa tgagaagaac tatactatta caatctatat 300

atctctattt atattctaat ctgaagcagt taatttaact gactctaaca actagactga 360

taggtgtaca ttttctgtta gtgcactgca gtgcatttaa ctaactgctt aacataaaga 420

atgttgttcg aacttcattc gaatagcttc aatgagaagc aaacatgtgt acctgtaaag 480

acacacagta aaagtgttaa taatgaataa atatgaataa atcaaataat aaattaaaaa 540

taaaaacaca tccaattaac attggaggtc ttgaaaatcg atggtaatta acaaagaccc 600

ttgtgaaatt taagtctgta attgaaaatt tgagtatagg ttaggggaca tttgactatt 660

ttctcatttt ctttatcttt ttcctaattt gtggcagaca agtgaggagg ccccactgta 720

attgattcat gcttttgctt tcttgacttt ttggaacaat actatgcatc atatttggtc 780

ttaattattc ctctgtttat ttccagaatt ttgagctcta tacatctaat aacaaagcaa 840

gcagaggata tatagtttca tcaactaaaa aggttagtca actcatctaa tatttgctac 900

tctcatctct attgaagtac agttatggaa aagtagaagt gatgtaagaa aaatgaaaga 960

actttagtag gttagttgga tctaacaaag agaaagggaa ataaattgca ggagaaagag 1020

agaggttaaa tacttactca caccaccgat ttacaacaaa tcacttaatt gtggttagtt 1080

aatgtatact ttcacctcat taaattatta cttacccatg ataagttgta ttaatttggt 1140

attaatatcc ggtgcgggtg aattcttacc gggtgagagg gatggggttg gagagtgtgg 1200

agtgaacaga agcagatgtt ttagattttt tctaagatga cgaaagattc ccctcactaa 1260

tgaaaatata ttactatacg ctattagaga tagaaaggtt cggtaccagt tggtctcgtt 1320

tctggatgaa ccccattttt acaagtcatt ttcttcaatt caaatcgcaa gtgtaccttt 1380

atcatcttcc actaattaag tcctcttaag ttcgcgtgaa aatagtgaaa ttattgatta 1440

ttcttatcat ttcatcttct ttctcctgat aaagttttat gtacttttta tgcatcaggt 1500

cttgagaact tggaaaggaa aagtagaatc atggaaaaac gaaaagataa tgaagaagca 1560

aacaactcat tggtatgtta tttgatagag tgaactgtaa agtattgaat tgtagatatc 1620

atgtggcttt aaaaatttga tatgtgttat tttggcagga gtcattttct gctcttcgca 1680

aggatgctgc caatgttctg gatttcctag agagattaaa gaatgaagaa gatcaaaagg 1740

ctgttgatgt ggatctgatt gaaagcctga aattgaagct gacatttatt tgtacatatg 1800

tccagctttc ttattccgat ttggagaagt ttgaagatat aatgactaga aaaagacaag 1860

aggttgagaa tctgcttcaa ccaattttgg atgatgatgg caaagacgtc gggtgtaaat 1920

atgtccttac tagcctcgcc ggtaatatgg atgactgtat aagcttgtat catcgttcta 1980

aatcagatgc caccatgatg gatgagcaat tgggcttcct cctcttgaat ctctctcatc 2040

tatccaagca tcgtgctgaa aagatgtttc ctggagtgac tcaatatgag gttcttcaga 2100

atgtatgtgg caacataaga gatttccatg gattgatagt gaattgttgc attaagcatg 2160

agatggttga gaatgtctta tctctgtttc aactgatggc tgagagagta ggacgcttcc 2220

tttgggagga tcaggctgat gaagactctc aactctccga gctagatgag gatgatcaga 2280

atgataaaga ccctcaactc ttcaagctag cacatctact cttgaagatt gttccaactg 2340

aattggaggt tatgcacata tgttataaaa ctttgaaagc ttcaacttca acagaaattg 2400

gacgcttcat taagaagctc ctggaaacct ctccggacat tctcagagaa tatctgattc 2460

atctacaaga gcatatgata actgttatta cccctaacac ttcagggggct cgaaacattc 2520

atgtcatgat ggaattccta ttgattattc tttctgatat gccgcccaag gactttattc 2580

atcatgacaa actttttgat ctcttggctc gtgttgtagc acttaccagg gaggtatcaa 2640

ctcttgtacg cgacttggaa gagaaattaa ggattaaaga gagtactgac gaaacaaatt 2700

gtgcaaccct aaagtttctg gaaaatattg aactccttaa ggaagatctc aaacatgttt 2760

atctgaaagt cccggattca tctcaatatt gcttccccat gagtgatgga cctctcttca 2820

tgcatctgct acagagacac ttagatgatt tgctggattc caatgcttat tcaattgctt 2880

tgataaagga acaaattggg ctggtgaaag aagacttgga attcataaga tctttttttcg 2940

cgaatattga gcaaggattg tataaagatc tctgggaacg tgttctagat gtggcatatg 3000

aggcaaaaga tgtcatagat tcaattattg ttcgagataa tggtctctta catcttattt 3060

tctcacttcc cattaccaga aagaagatga tgcttatcaa agaagaggtc tctgatttac 3120

atgagaacat ttccaagaac agaggtctca tcgttgtgaa ctctcccaag aaaccagttg 3180

agagcaagtc attgacaact gataaaataa ttgtaggttt tggtgaggag acaaacttga 3240

tacttagaaa gctcaccagt ggaccggcag atctagatgt catttcgatc attggtatgc 3300

cgggtttagg taaaactact ttggcgtaca aagtatacaa tgataaatca gtttctagcc 3360

atttcgacct tcgtgcatgg tgcacggtcg accaagtata tgacgagaag aagttgttgg 3420

ataaaatttt caatcaagtt agtgactcaa attcaaaatt gagtgagaat attgatgttg 3480

ctgataaact acggaaacaa ttgtttggaa agaggtatct tattgtctta gatgacgtgt 3540

gggatactaa tacatgggat gagctaacaa gaccttttcc tgatggtatg aaaggaagta 3600

gaattatttt gacaactcga gaaaagaaag ttgctttgca tggaaagctc tacactgatc 3660

ctcttaacct tcgattgcta agatcagaag aaagttggga gttattagag aaaagggcat 3720

ttggaaacga gagttgccct gatgaactat tggatgttgg taaagaaata gccgaaaatt 3780

gtaaagggct tcctttggtg gtggatctga ttgctggaat cattgctggg agggaaaaga 3840
aaaagagtgt gtggcttgaa gttgtaaata atttgcattc ctttattttg aagaatgaag 3900
tggaagtgat gaaagttata gaaataagtt atgaccactt acctgatcac ctgaagccat 3960
gcttgctgta ctttgcaagt gcgccgaagg actgggtaac gacaatccat gagttgaaac 4020
ttatttgggg ttttgaagga tttgtggaaa agacagatat gaagagtctg gaagaagtgg 4080
tgaaaattta tttggatgat ttaatttcca gtagcttggt aatttgtttc aatgagatag 4140
gtgattaccc tacttgccaa cttcatgatc ttgtgcatga cttttgtttg ataaaagcaa 4200
gaaaggaaaa gttgtgtgat cggataagtt caagtgctcc atcagatttg ttgccacgtc 4260
aaattagcat tgattatgat gatgatgaag agcactttgg gcttaatttt gtcctgttcg 4320
gttcaaataa gaaaaggcat tccggtaaac acctctattc tttgaccata aatggagatg 4380
agctggacga ccatctttct gatacatttc atctaagaca cttgaggctt cttagaacct 4440
tgcacctgga atcctctttt atcatggtta aagattcttt gctgaatgaa atatgcatgt 4500
tgaatcattt gaggtactta agcattggga cagaagttaa atctctgcct ttgtctttct 4560
caaacctctg gaatctagaa atcttgtttg tggataacaa agaatcaacc ttgatactat 4620
taccgagaat ttgggatctt gtaaagttgc aagtgctgtt cacgactgct tgttctttct 4680
ttgatatgga tgcagatgaa tcaatactga tagcagagga cacaaagtta gagaacttga 4740
cagcattagg ggaactcgtg ctttcctatt ggaaagatac agaggatatt ttcaaaaggc 4800
ttcccaatct tcaagtgctt catttcaaac tcaaggagtc atgggattat tcaacagagc 4860
aatattggtt cccgaaattg gatttcctaa ctgaactaga aaaactcact gtagattttg 4920
aaagatcaaa cacaaatgac agtgggtcct ctgcagccat aaatcggcca tgggattttc 4980
actttccttc gagtttgaaa agattgcaat tgcatgaatt tcctctgaca tccgattcac 5040
tatcaacaat agcgagactg ctgaaccttg aagagttgta cctttatcgt acaatcatcc 5100
atggggaaga atggaacatg ggagaagaag acacctttga gaatctcaaa tgtttgatgt 5160
tgagtcaagt gattctttcc aagtgggagg ttggagagga atcttttccc acgcttgaga 5220
aattagaact gtcggactgt cataatcttg aggagattcc gtctagtttt ggggatattt 5280
attccttgaa aattatcgaa cttgtaagga gccctcaact tgaaaattcc gctctcaaga 5340
ttaaggaata tgctgaagat atgaggggag gggacgagct tcagatcctt ggccagaagg 5400
atatcccgtt atttaagtag tttttgagca ttatggttga aaagtagatt gcactttgct 5460
gggtagattg tatatggtta agaaaattct gttacagttg ttatgaaaca tttttatttg 5520
acttttctga gtttctttta gaaaactcag aagtttttaa caaaaattat agtttttata 5580
aatacaatgt ggatttgcct ttggctgtcc aacttggtct gaagtctcat atgctcagag 5640
cactatcgtt caacctcaat caaggtactg atttaaaatg acatctatac tactttatca 5700

caaacccaac gaactttcat ctcaaaagct aggccaggaa gtgaagaggt tgtagagagc 5760
ttataagcac tcatgacttc cttttctcga acattcaacc aacgtaggct gaaatcccac 5820
tctgaacgaa aataagtgtt tgtttatcaa attaactctc gtagtagaac actgaaatac 5880
cttcttctaa acgttcaaca aatgggattt ccagcactca aagtgaatga aaggttcaca 5940
ttaatcttca aaaagaatta cgacaattca tgaccacaag tacattgaca gcaccatttc 6000
aacagaagaa caagtcaatg ctgcatcttc atcaataatc cgagtgtcga acctccttcc 6060
tgacactgtc ctgtatatgt aaagtttctc aacagggcaa ctttctggtc tcgtatctgg 6120
atgacccctc tcgtctataa cttcaacatt aagccctggc aacttctgga ccaacagctt 6180
acatgcttca aaacttactg aacaattaga catccaaagg gatcgcattg tctccagctt 6240
tgcagcatta gccaacagag cctcatcgcc aaaggggcag tctctaatct cgaatttgaa 6300
aaaattgttg ttgtatgact ttcctctgac atccgatgca ctatcaacaa tagcaagact 6360
ggaggttgga gaggaatcct ttattataca atcattcagg gagaagaatg gaacatgggg 6420
gaggaagaca cttttgagaa tctgaaatgt gttagagcca caagctacag aagtattgaa 6480
tttgtcatga atatcaacat tcttcatcct agttaattct ttttcaattt ttaatagact 6540
ctcattttaa tcactaatat tcttctattt gtgacttctt ttctgcaggt ggcaacttta 6600
aattcataaa gtataggatt gatgacaaac tcgaaaaata tcttaatgag gtgaagtttg 6660
agcagtcagc agatggtggt tccaactcta agttgacaag cacatactat cccggagggc 6720
gatttcaagc ctgatgcata tggttagtgt ggctagagca gacaggatgt attacctgga 6780
tatctaccaa gacgaatcca caatcagttt tatgtcaagc aatacatgaa gtaactcccg 6840
atagaacagt aaaagcaaga tgtgtaggtg tatctcgact ctaagagatt gtacattcct 6900
ctttgagatt tttactgcta atacaaattt acacctcaga agcgaatcta gaatttctag 6960
agcatgaatg caccactaat gaaaggagaa aaaaggaagt atgaagtggg aatttgatcc 7020
ttgtttctag gtatataaaa tttatcattc aactatactt catttagcaa acaactctct 7080
ttgccattat ttctcaaaca agggcttcta atattgctaa actaaagact gtcaaaaggt 7140
aagttcatct tcaaactctc ttgtttactt tatctaaagg ggaactatga aaaacaagaa 7200
acatcaggaa tgtcccgtaa acaaagcagc ctcatgcaca aaacatccaa cgttggtagg 7260
attaatggag ggatcgcatc ccaggaggat actgtagaaa aattagtggc ttctttcacc 7320
gctcaaaccc atgatctata ggttacatgg agacaacttt atggttgctc gtaggctccc 7380
gtcaattctc ataaaccaca acaccaaagt tgcatcagac atcatcttca ttcacaagct 7440
gacaatctcc acaagtctta gtcaacttgt aatatgaata ttagccaggt agacgtacat 7500
atttacaaaa ttgagtttcc tatataatat ggtttgaagg aatgaaacat gatggggagg 7560
gtagataaaa taatatatga ggcataaaaa taggaaagat atttgtagtg agaggttttg 7620
actttttatg ctgcttttga tcttcagttt cttgtattct tttctactg ctttcctctt 7680

ctttctcctg agtaaagttt tatgtaggta ctttttatac gtccgatcgt gagaacttga 7740

aagaaagctc tctatagcta tgttaggtgc ccacataaaa aaatgaaata ttacaaaaac 7800

cctgataata aaatacacta atctaagata ttcactgcaa catacatgca aaatatatat 7860

atataaattt tcatgaaaat tataacaaat aatagatgtg aacatataac tttaaaaata 7920

atattacatc cataaagctt aaattctaga 7950

<210> 48
<211> 2584
<212> DNA
<213> artificial

<220>
<223> synthetic construct sequence

<400> 48
gatcatgagc ggagaattaa gggagtcacg ttatgacccc cgccgatgac gcgggacaag 60

ccgttttacg tttggaactg acagaaccgc aacgttgaag gagccactca gccgcgggtt 120

tctggagttt aatgagctaa gcacatacgt cagaaaccat tattgcgcgt tcaaaagtcg 180

cctaaggtca ctatcagcta gcaaatattt cttgtcaaaa atgctccact gacgttccat 240

aaattcccct cggtatccaa ttagagtctc atattcactc tcaatccaga tccccgggta 300

ccatggcggc ggcaacaaca acaacaacaa catcttcttc gatctccttc tccaccaaac 360

catctccttc ctcctccaaa tcaccattac caatctccag attctccctc ccattctccc 420

taaaccccaa caaatcatcc tcctcctccc gccgccgcgg tatcaaatcc agctctccct 480

cctccatctc cgccgtgctc aacacaacca ccaatgtcac aaccactccc tctccaacca 540

aacctaccaa acccgaaaca ttcatctccc gattcgctcc agatcaaccc cgcaaaggcg 600

ctgatattct cgtcgaggct ttagaacgtc aaggcgtaga aaccgtattc gcttaccctg 660

gaggtgcatc aatggagatt caccaagcct taacccgctc ttcctcaatc cgtaacgtcc 720

ttcctcgtca cgaacaagga ggtgtattcg cagcagaagg atacgctcga tcctcaggta 780

aaccaggtat ctgtatagcc acttcaggtc ccggagctac aaatctcgtt agcggattag 840

ccgatgcgtt gttagatagt gttcctcttg tagcaatcac aggacaagtc cctcgtcgta 900

tgattggtac agatgcgttt caagagactc cgattgttga ggtaacgcgt tcgattacga 960

agcataacta tcttgtgatg gatgttgaag atattcctag gattattgag gaggctttct 1020

ttttagctac ttctggtaga cctggacctg ttttggttga tgttcctaaa gatattcaac 1080

aacagcttgc gattcctaat tgggaacagg ctatgagatt acctggttat atgtctagga 1140

tgcctaaacc tccggaagat tctcatttgg agcagattgt taggttgatt tctgagtcta 1200

agaagcctgt gttgtatgtt ggtggtggtt gtttgaactc tagcgatgaa ttgggtaggt 1260

ttgttgagct tacgggaatc cctgttgcga gtacgttgat ggggctggga tcttatcctt 1320

gtgatgatga gttgtcgtta catatgcttg gaatgcatgg gactgtgtat gcaaattacg 1380

ctgtggagca tagtgatttg ttgttggcgt ttggggtaag gtttgatgat cgtgtcacgg 1440

gtaaacttga ggcttttgct agtagggcta agattgttca tattgatatt gactcggctg 1500

agattgggaa gaataagact cctcatgtgt ctgtgtgtgg tgatgttaag ctggctttgc 1560

aagggatgaa taaggttctt gagaaccgag cggaggagct taaacttgat tttggagttt 1620

ggaggaatga gttgaacgta cagaaacaga agtttccgtt gagctttaag acgtttgggg 1680

aagctattcc tccacagtat gcgattaagg tccttgatga gttgactgat ggaaaagcca 1740

taataagtac tggtgtcggg caacatcaaa tgtgggcggc gcagttctac aattacaaga 1800

aaccaaggca gtggctatca tcaggaggcc ttggagctat gggatttgga cttcctgctg 1860

cgattggagc gtctgttgct aaccctgatg cgatagttgt ggatattgac ggagatggaa 1920

gttttataat gaatgtgcaa gagctagcca ctattcgtgt agagaatctt ccagtgaagg 1980

tacttttatt aaacaaccag catcttggca tggttatgca atgggaagat cggttctaca 2040

aagctaaccg agcacacaca tttctcggag atccggctca ggaggacgag atattcccga 2100

acatgttgct gtttgcagca gcttgcggga ttccagcggc gagggtgaca aagaaagcag 2160

atctccgaga agctattcag acaatgctgg atacaccagg accttacctg ttggatgtga 2220

tttgtccgca ccaagaacat gtgttgccga tgatcccgaa tggtggcact ttcaacgatg 2280

tcataacgga aggagatggc cggattaaat actgagagct cgaatttccc cgatcgttca 2340

aacatttggc aataaagttt cttaagattg aatcctgttg ccggtcttgc gatgattatc 2400

atataatttc tgttgaatta cgttaagcat gtaataatta acatgtaatg catgacgtta 2460

tttatgagat gggtttttat gattagagtc ccgcaattat acatttaata cgcgatagaa 2520

aacaaaatat agcgcgcaaa ctaggataaa ttatcgcgcg cggtgtcatc tatgttacta 2580

gatc 2584

<210> 49
<211> 27
<212> DNA
<213> artificial

<220>
<223> Primer

<400> 49
tgttgaacac tgtaacatgc taaaatg 27

<210> 50
<211> 21
<212> DNA
<213> artificial

<220>
<223> Primer

<400> 50
agttgtggac atccccgaat t 21

<210> 51
<211> 28
<212> DNA
<213> artificial

<220>
<223> Primer

<400> 51
agagggattg cagcacctaa caaccctc 28

<210> 52
<211> 25
<212> DNA
<213> artificial

<220>
<223> Primer

<400> 52
ttcaaaaccc caaataagtt tcaac 25

<210> 53
<211> 21
<212> DNA
<213> artificial

<220>
<223> Primer

<400> 53
ccatgcttgc tgtactttgc a 21

<210> 54
<211> 20
<212> DNA
<213> artificial

<220>
<223> Primer

<400> 54
cgttacccag tccttcggcg 20

<210> 55
<211> 20
<212> DNA
<213> artificial

<220>
<223> Primer

<400> 55
aacgatgtca taacggaagg 20

<210> 56

<211> 25
<212> DNA
<213> artificial

<220>
<223> Primer

<400> 56
agagcatttg aagcagatct agggt 25

<210> 57
<211> 25
<212> DNA
<213> artificial

<220>
<223> Primer

<400> 57
cggattaaat actgagagct cgaat 25

<210> 58
<211> 22
<212> DNA
<213> artificial

<220>
<223> Primer

<400> 58
cagatctagg gttttatctc gg 22

<210> 59
<211> 20
<212> DNA
<213> artificial

<220>
<223> Primer

<400> 59
tgccggtctt gcgatgatta 20

<210> 60
<211> 25
<212> DNA
<213> artificial

<220>
<223> Primer

<400> 60
agatctaggg ttttatctcg ggatt 25

<210> 61
<211> 16
<212> DNA
<213> artificial

<220>
<223> artificial

<220>
<221> misc_feature
<222> (3)..(3)
<223> W may be A or T

<220>
<221> misc_feature
<222> (5)..(5)
<223> N may be A or T or G or C

<220>
<221> misc_feature
<222> (8)..(8)
<223> W may be A or T

<220>
<221> misc_feature
<222> (10)..(10)
<223> N may be A or T or G or C

<220>
<221> misc_feature
<222> (13)..(13)
<223> S may be G or C

<400> 61
tgwgnagwan casaga 16

<210> 62
<211> 16
<212> DNA
<213> artificial

<220>
<223> Primer

<220>
<221> misc_feature
<222> (3)..(3)
<223> W may by A or T

<220>
<221> misc_feature
<222> (5)..(5)
<223> N may be A or T ot G or C

<220>
<221> misc_feature
<222> (8)..(8)
<223> W may be A or T

<220>
<221> misc_feature
<222> (10)..(10)
<223> N may be A or T or G or C

<220>
<221> misc_feature
<222> (13)..(13)
<223> W may be A or T

<400> 62
agwgnagwan cawagg 16

<210> 63
<211> 16
<212> DNA
<213> artificial

<220>
<223> Primer

<220>
<221> misc_feature
<222> (3)..(3)
<223> W may be A or T

<220>
<221> misc_feature
<222> (6)..(6)
<223> N may be A or T ot G or C

<220>
<221> misc_feature
<222> (8)..(8)
<223> N may be A or T or G or C

<220>
<221> misc_feature
<222> (11)..(11)
<223> N may be A or T or G or C

<220>
<221> misc_feature
<222> (13)..(13)
<223> S may be G or C

<400> 63
cawcgncnga nasgaa 16

<210> 64
<211> 16
<212> DNA
<213> artificial

<220>
<223> Primer

<220>
<221> misc_feature
<222> (3)..(3)
<223> S may be G or C

<220>
<221> misc_feature
<222> (5)..(5)
<223> N may be A or T or G or C

<220>
<221> misc_feature
<222> (8)..(8)
<223> N may be A or T or G or C

<220>
<221> misc_feature
<222> (11)..(11)
<223> N may be A or T or G or C

<220>
<221> misc_feature
<222> (13)..(13)
<223> W may be A or T

<400> 64
tcstncgnac ntwgga 16

<210> 65
<211> 25
<212> DNA
<213> artificial

<220>
<223> Primer

<400> 65
taattcagta cattaaagac gtccg 25

<210> 66
<211> 23
<212> DNA
<213> artificial

<220>
<223> Primer

<400> 66
gtcccatagt catttcttga tca 23

<210> 67
<211> 26
<212> DNA
<213> artificial

<220>
<223> Primer

<400> 67
tgtctctgat aggctaataa actatg 26

<210> 68
<211> 21
<212> DNA
<213> artificial

<220>
<223> Primer

<400> 68
tagatctgat tgtcgtttcc c 21

<210> 69
<211> 23

<212> DNA
<213> artificial

<220>
<223> Primer

<400> 69
atgacgttat ttatgagatg ggt 23

<210> 70
<211> 20
<212> DNA
<213> artificial

<220>
<223> Primer

<400> 70
atttaaaagg caaaacgtgc 20

<210> 71
<211> 23
<212> DNA
<213> artificial

<220>
<223> Primer

<400> 71
ttcatgtcaa gttcaatttc agg 23

<210> 72
<211> 21
<212> DNA
<213> artificial

<220>
<223> Primer

<400> 72
actcacatta attgcgttgc g 21

<210> 73
<211> 26
<212> DNA
<213> artificial

<220>
<223> Primer

<400> 73
gcttggtaat aattgtcatt agattg 26

<210> 74
<211> 23
<212> DNA
<213> artificial

<220>
<223> Primer

<400> 74
gccttgacct ttgaattatt tac 23

<210> 75
<211> 25
<212> DNA
<213> artificial

<220>
<223> Primer

<400> 75
tctgatgcag aattttctaa ctcaa 25

<210> 76
<211> 26
<212> DNA
<213> artificial

<220>
<223> Primer

<400> 76
ttcctactag atctgattgt cgtttc 26